# EUROPEAN PATENT SPECIFICATION

(11) **EP 4 389 225 B1**
(45) Date of publication and mention of the grant of the patent: **07.10.2026**
(21) Application number: 24164571.2
(22) Date of filing: 19.12.2014
(51) Int. Cl.: A61K 31/4439, A61K 31/506, A61K 31/519, A61K 31/437, A61K 31/337, A61P 35/00, A61K 31/4184

(54) **CANCER TREATMENTS USING COMBINATIONS OF TYPE 2 MEK AND ERK INHIBITORS**
KREBSBEHANDLUNGEN UNTER VERWENDUNG VON KOMBINATIONEN AUS TYP-2-MEK- UND ERK-HEMMERN
TRAITEMENTS DE CANCER UTILISANT DES COMBINAISONS D'INHIBITEURS ERK ET MEK DE TYPE 2

(30) Priority: 20.12.2013 US 201361919625 P
(43) Date of publication of application: 26.06.2024
(62) Document number(s) of the earlier application(s) in accordance with Art. 76 EPC: 21193832.9 / 3 984 537; 14871217.7 / 3 082 779
(73) Proprietor: Biomed Valley Discoveries, Inc., Kansas City, Missouri 64111 (US)
(72) Inventor: SAHA, Saurabh, Massachusetts, 02481 (US); DECRESCENZO, Gary, Missouri, 64152 (US); ROIX, Jeffrey James, London, W1G 7BB (GB); WELSCH, Dean, Missouri, 64152 (US)
(74) Representative: Vossius & Partner Patentanwälte Rechtsanwälte mbB

(56) References cited:
- WO-A1-2005/113541
- HATZIVASSILIOU G ET AL: "ERK inhibition overcomes acquired resistance to MEK Inhibitors", MOLECULAR CANCER THERAPEUTICS, AMERICAN ASSOCIATION OF CANCER RESEARCH, US, vol. 11, no. 5, 11 May 2012 (2012-05-11), pages 1143 - 1154, XP002730627, ISSN: 1535-7163, [retrieved on 20120308], DOI: 10.1158/1535-7163.MCT-11-1010
- E. J. MORRIS ET AL: "Discovery of a Novel ERK Inhibitor with Activity in Models of Acquired Resistance to BRAF and MEK Inhibitors", CANCER DISCOVERY, vol. 3, no. 7, 1 July 2013 (2013-07-01), US, pages 742 - 750, XP055219076, ISSN: 2159-8274, DOI: 10.1158/2159-8290.CD-13-0070
- NEUZILLET CINDY ET AL: "MEK in cancer and cancer therapy", PHARMACOLOGY AND THERAPEUTICS, ELSEVIER, GB, vol. 141, no. 2, 9 October 2013 (2013-10-09), pages 160 - 171, XP028814295, ISSN: 0163-7258, DOI: 10.1016/J.PHARMTHERA.2013.10.001

## Description

### CROSS REFERENCE TO RELATED APPLICATIONS

This application claims benefit of U.S. Patent Application Serial No. 61/919,625, filed on December 20, 2013.

### FIELD OF INVENTION

The present invention provides, *inter alia,* methods, pharmaceutical compositions, and kits for use in treating or ameliorating the effects of a cancer in a subject using a first anti-cancer agent, which is BVD-523 or a pharmaceutically acceptable salt thereof and a second anti-cancer agent, which is a type 2 MEK inhibitor or a pharmaceutically acceptable salt thereof.

### INCORPORATION BY REFERENCE OF SEQUENCE LISTING

This application contains references to amino acids and/or nucleic acid sequences that have been filed concurrently herewith as sequence listing text file "0375603.txt", file size of 474 KB, created on December 19, 2014.

### BACKGROUND OF THE INVENTION

Within cellular signaling networks, Ras and Raf play significant roles in the regulation of various biological processes, including cell growth, proliferation, differentiation, inflammatory responses, and programmed cell death. Notably, mutations in ras genes were the first genetic alterations identified in human cancer. Activating mutations of HRAS, NRAS, and KRAS ('RAS'), as well as BRAF are found frequently in several types of cancer.

A MEK inhibitor is an agent that inhibits the mitogen - activated protein kinase enzymes, MEK1 and/or MEK2. Depending on their target and effect, i.e. MEK1, MEK2 or both, MEK inhibitors may be classified as type 1 MEK inhibitors, type 2 MEK inhibitors or pan MEK inhibitors. MEK inhibitors are known to modulate, e.g., the MAPK pathway, which is often over-active in many cancers, and, therefore, have been used in cancer therapy. Unfortunately, many cancers become resistant to MEK inhibitor treatment over time.

Extracellular-signal-regulated kinases (ERKs) are protein kinases that are involved in cell cycle regulation, including the regulation of meiosis, mitosis, and postmitotic functions in differentiated cells. Disruption of the ERK pathway is common in cancers. However, to date, little progress has been made developing effective ERK inhibitors for the treatment of cancer.

E. J. Morris et al. "Discovery of a Novel ERK Inhibitor with Activity in Models of Acquired Resistance to BRAF and MEK Inhibitors", CANCER DISCOVERY, vol. 3, no. 7, 1 July 2013, pages 742-750 discloses combination of some ERK and MEK1 inhibitors to reach a synergistic effect in the inhibition of cancer cell growth, including cells with BRAF mutations.

As the understanding of the molecular basis of cancer grows, there is an increased emphasis on developing drugs that specifically target particular nodes in pathways that lead to cancer. In view of the deficiencies noted above, there is, *inter alia,* a need for effective molecularly targeted cancer treatments, including combination therapies. The present application is directed to meeting these and other needs.

### SUMMARY OF THE INVENTION

The subject-matter of the present application is defined in the appended claims. Any references in the description to methods of treatment refer to the compounds, pharmaceutical compositions and medicaments of the present invention for use in a method for treatment of the human (or animal) body by therapy (or for diagnosis).

One embodiment of the present invention is a method of treating or ameliorating the effects of a cancer in a subject in need thereof. The method comprises administering to the subject an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, to treat or ameliorate the effects of the cancer.

Another embodiment of the present invention is a method of treating or ameliorating the effects of a cancer in a subject in need thereof. The method comprises administering to the subject an effective amount of (i) a first anti-cancer agent, which is BVD-523 or a pharmaceutically acceptable salt thereof and (ii) a second anti-cancer agent, which is trametinib or a pharmaceutically acceptable salt thereof, to treat or ameliorate the effects of the cancer.

A further embodiment of the present invention is a method of effecting cancer cell death. The method comprises contacting the cancer cell with an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof.

An additional embodiment of the present invention is a kit for use in treating or ameliorating the effects of a cancer in a subject in need thereof. The kit comprises an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, packaged together with instructions for their use.

Another embodiment of the present invention is a pharmaceutical composition for use in treating or ameliorating the effects of cancer in a subject in need thereof. The pharmaceutical composition comprises a pharmaceutically acceptable diluent or carrier and an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, wherein administration of the first and second anti-cancer agents provides a synergistic effect compared to administration of either anti-cancer agent alone.

A further embodiment of the present invention is a method of treating or ameliorating the effects of a cancer in a subject in need thereof. The method comprises administering to the subject an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973 (Hoffmann-La Roche), RG422 (Chugai Pharmaceutical Co.), RO4987655 (Hoffmann-La Roche), RO5126766 (Hoffmann-La Roche), SL327 (Sigma), WX-554 (Wilex), YopJ polypeptide (Mittal *et al.,* 2010), pharmaceutically acceptable salts thereof, and combinations thereof, to treat or ameliorate the effects of the cancer.

An additional embodiment of the present invention is a method of effecting cancer cell death. The method comprises contacting the cancer cell with an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof.

Another embodiment of the present invention is a kit for use in treating or ameliorating the effects of a cancer in a subject in need thereof. The kit comprises an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof, packaged together with instructions for their use.

A further embodiment of the present invention is a pharmaceutical composition for use in treating or ameliorating the effects of a cancer in a subject in need thereof. The pharmaceutical composition comprises a pharmaceutically acceptable diluent or carrier and an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof, wherein administration of the first and second anti-cancer agents provides a synergistic effect compared to administration of either anti-cancer agent alone.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

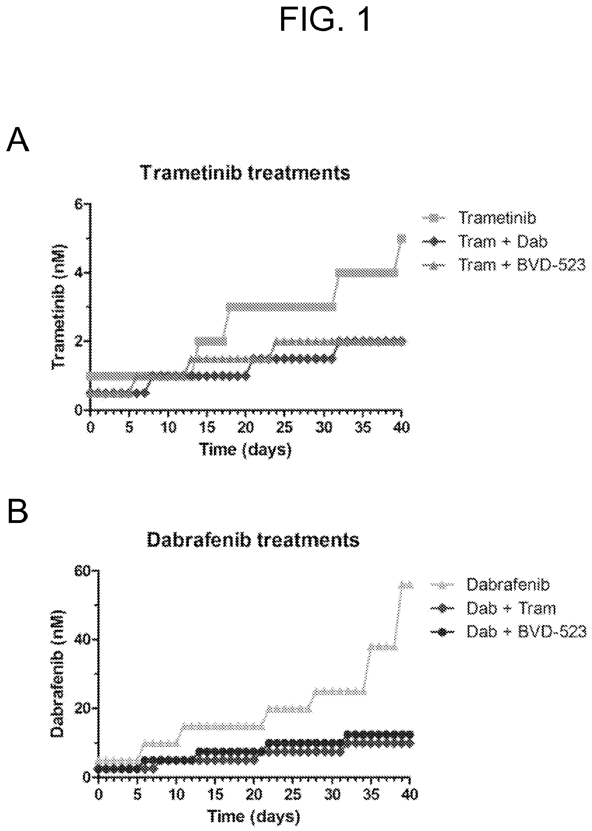

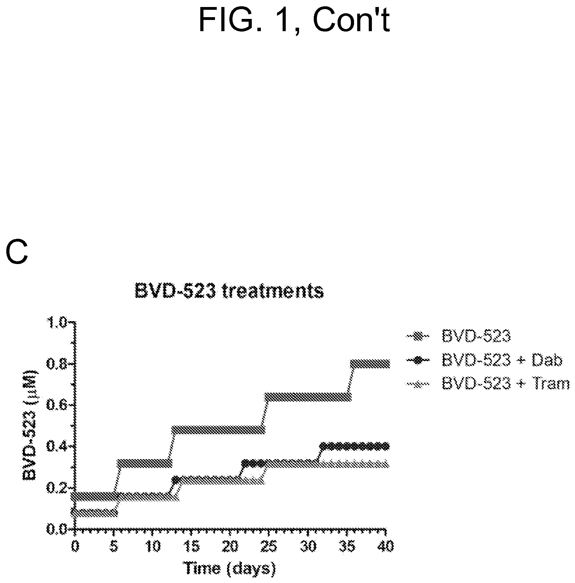

FIGS. 1A-C show the progress of a dose escalation study in a human malignant melanoma cell line (A375 cells) for month 1. Various treatments (trametinib (a type 2 MEK inhibitor), dabrafenib (a BRAF inhibitor), and BVD-523 (an ERK1/2 inhibior)) are as labeled.

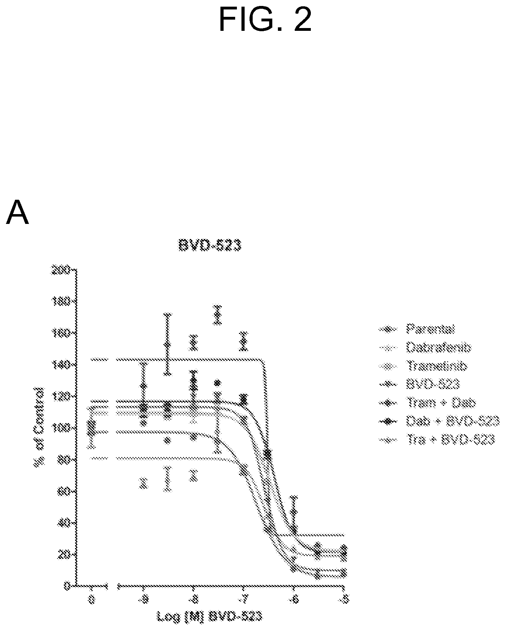

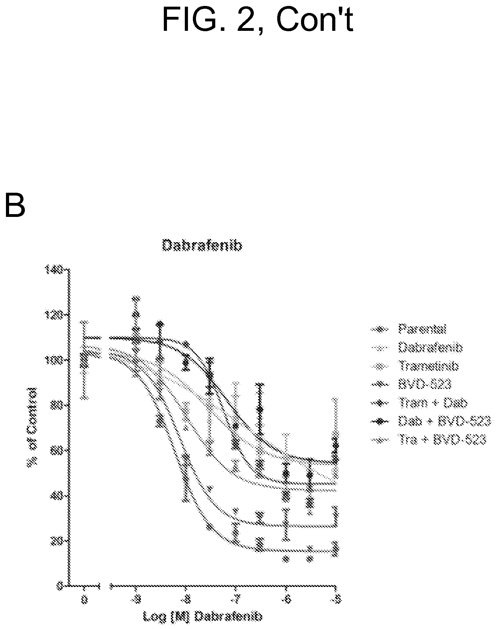

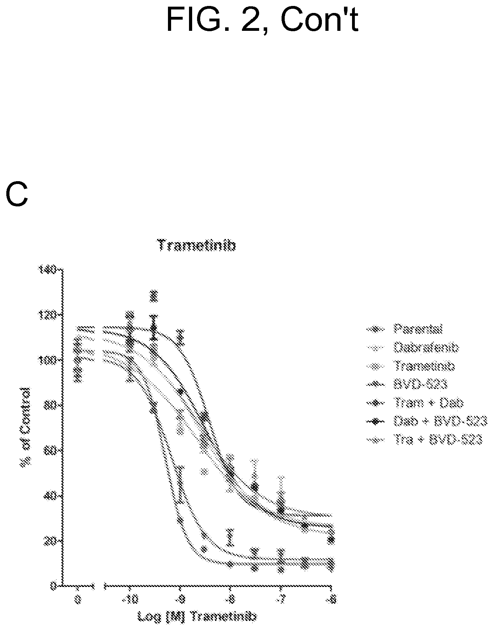

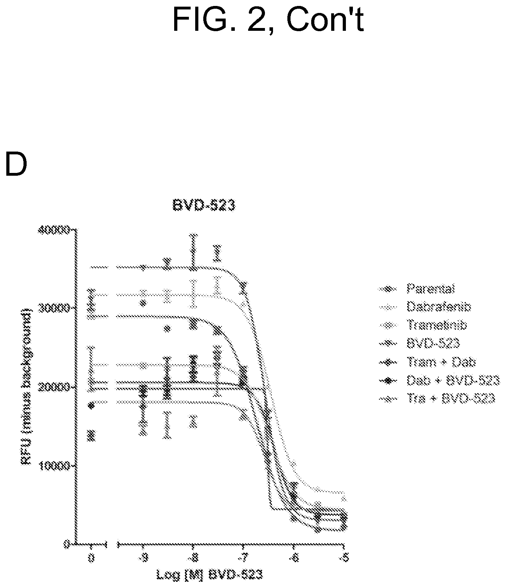

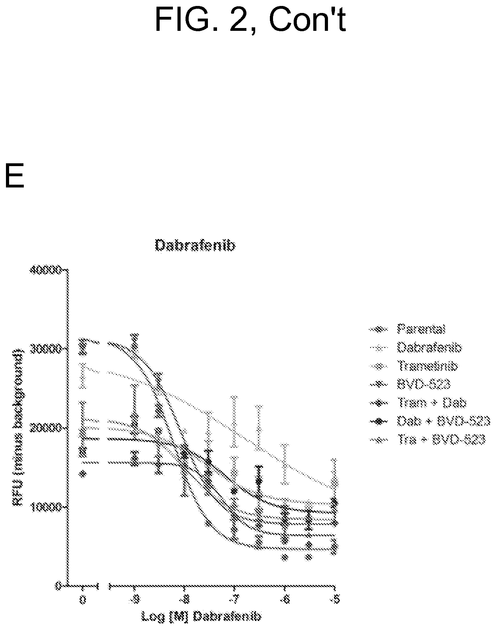

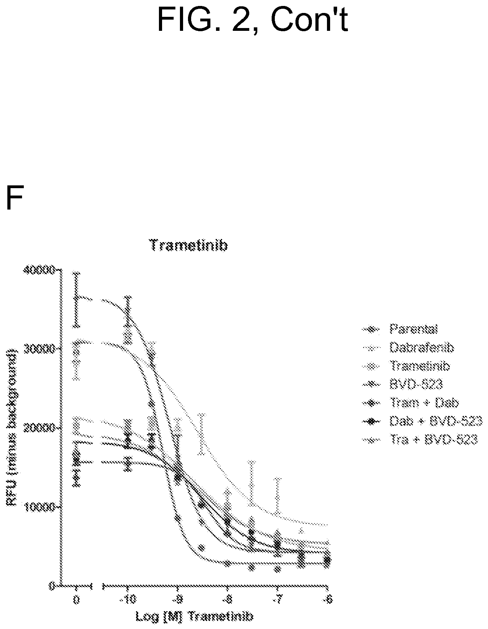

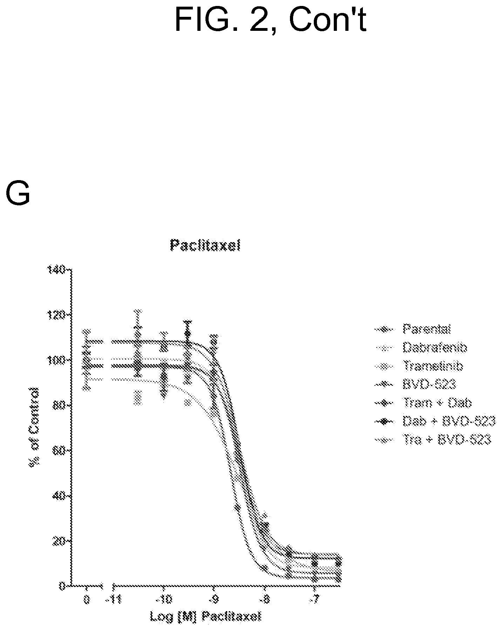

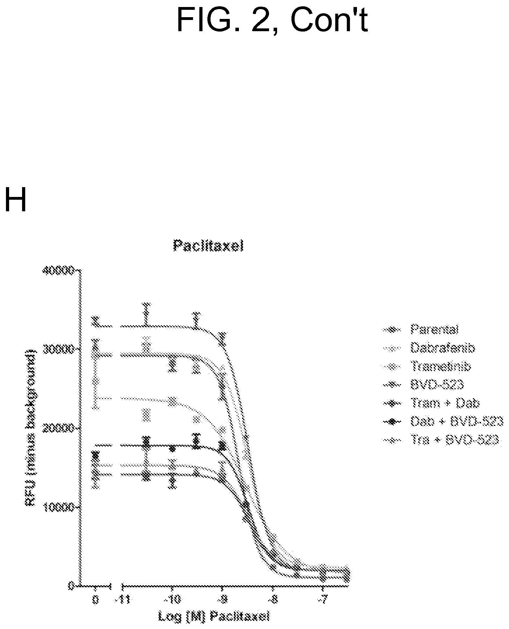

FIGS. 2A-H show the results of a proliferation assay that tracks changes in sensitivity to the escalated agent(s) at month 1. Various treatments (trametinib, dabrafenib, BVD-523, and pacitaxel) are as labeled on the top of the graph. The caption to the right of the graph shows the various types of cells generated from the dose escalation study. For example, "dabrafenib" refers to the cells that have been treated with the highest dose of dabrafenib from month 1 of the dose escalation study. Parental refers to the control cells that have not been treated with drugs. FIGS. 2A-2C and 2G are normalized to control, whereas FIGS. 2D-2F and 2H show the raw data.

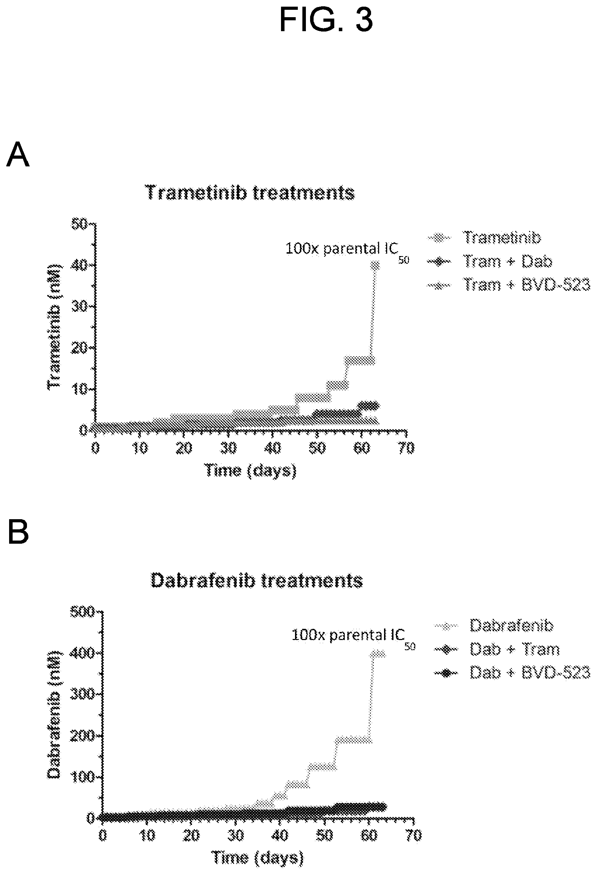

FIGS. 3A-3D show the progress of a dose escalation study in A375 cells for month 2. Various treatments (trametinib, dabrafenib, and BVD-523) are as labeled.

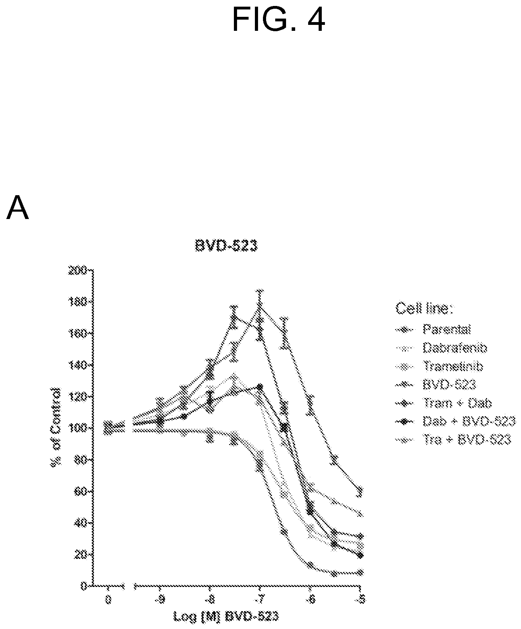

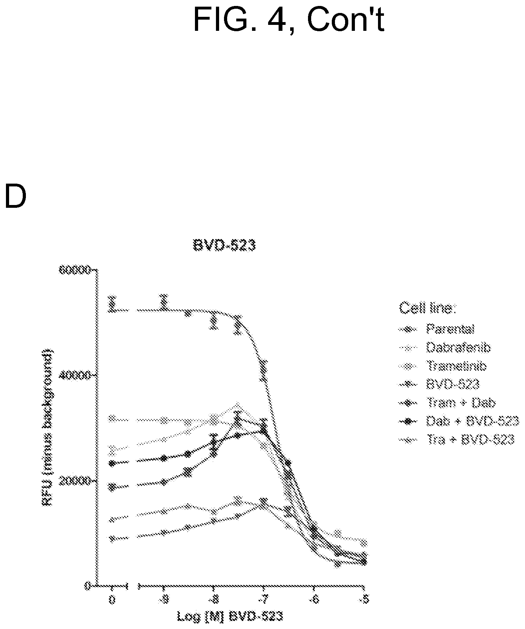

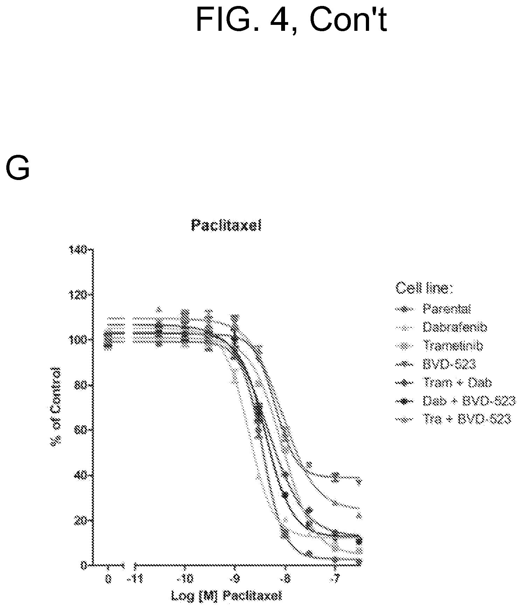

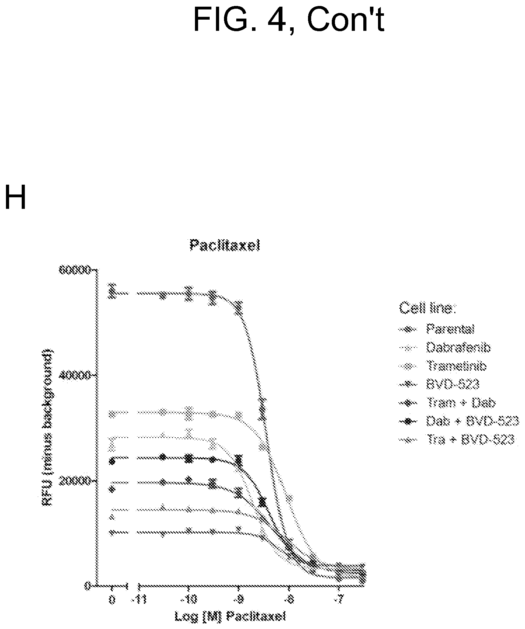

FIGS. 4A-H show the results of a proliferation assay that tracks changes in sensitivity to the escalated agent(s) at month 2. Various treatments (trametinib, dabrafenib, BVD-523, and pacitaxel) are as labeled on the top of the graph. The caption to the right of the graph shows the various types of cells generated from the dose escalation study. For example, "dabrafenib" refers to the cells that have been treated with the highest dose of dabrafenib from month 2 of the dose escalation study. Parental refers to the control cells that have not been treated with drugs. FIGS. 4A-4C and 4G are normalized to control, whereas FIGS. 4D-4F and 4H show the raw data.

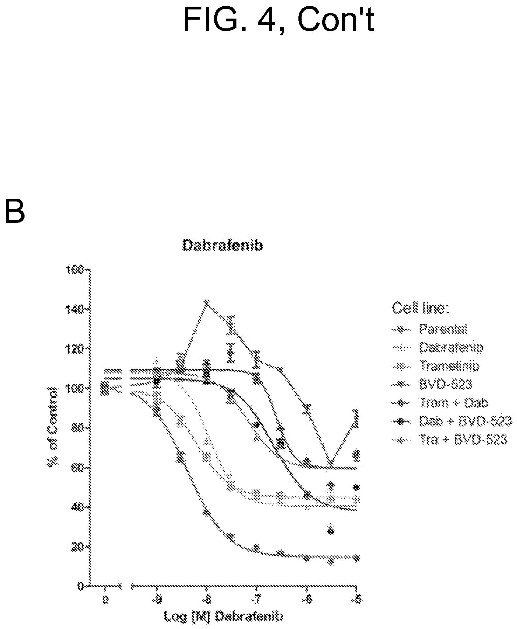

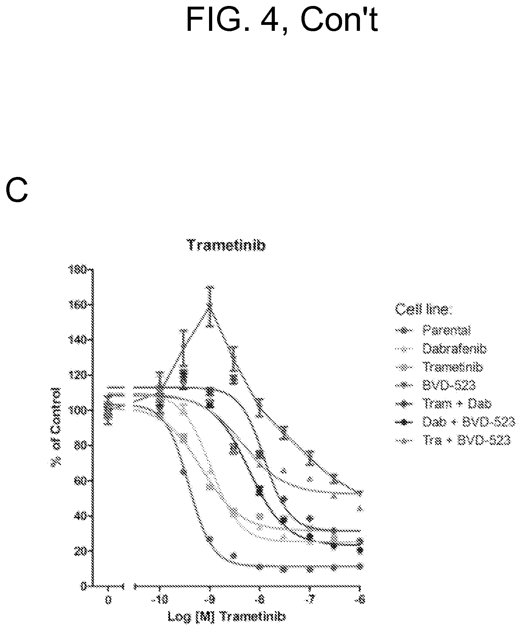

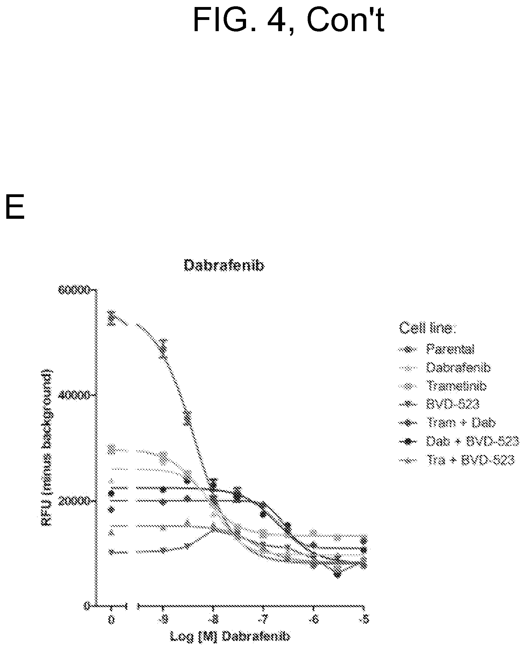

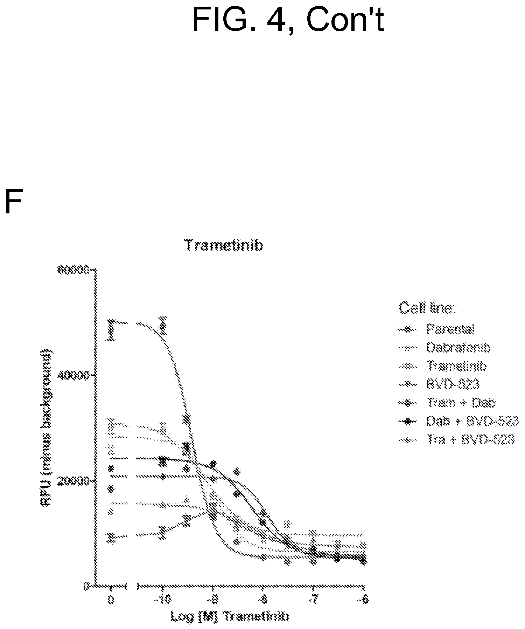

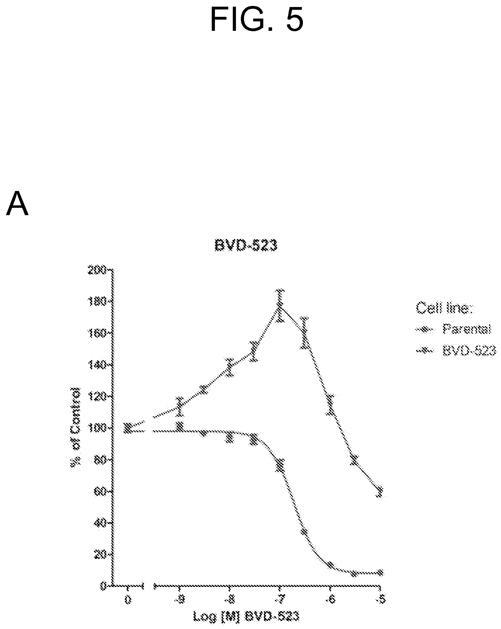

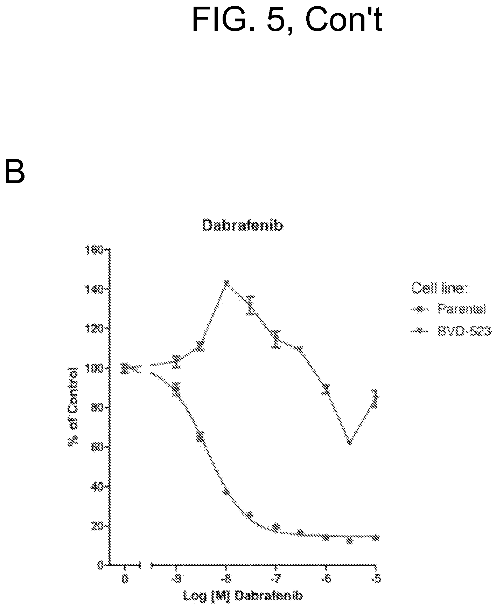

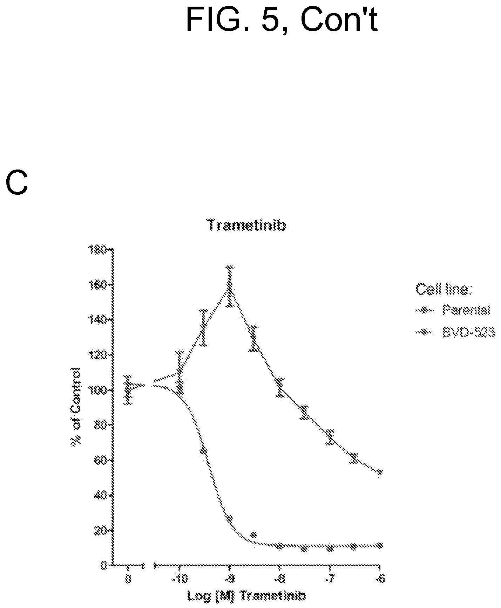

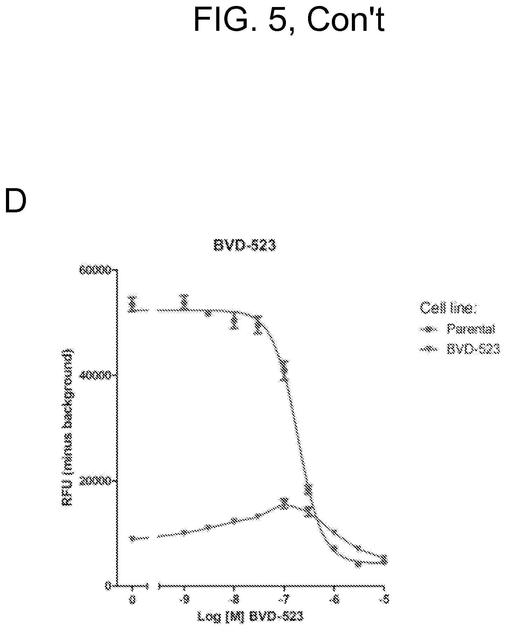

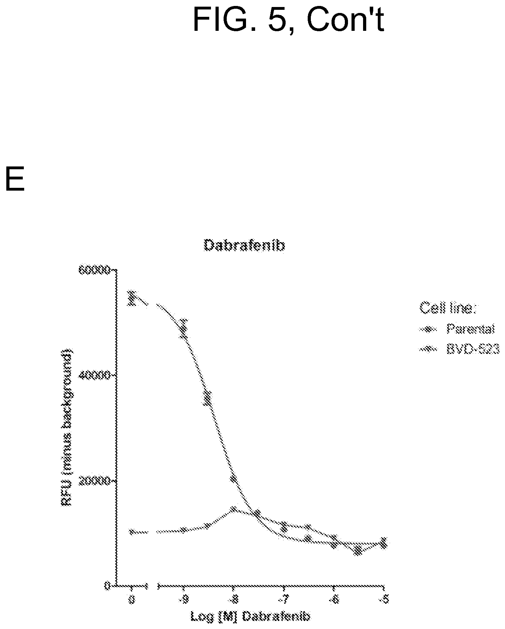

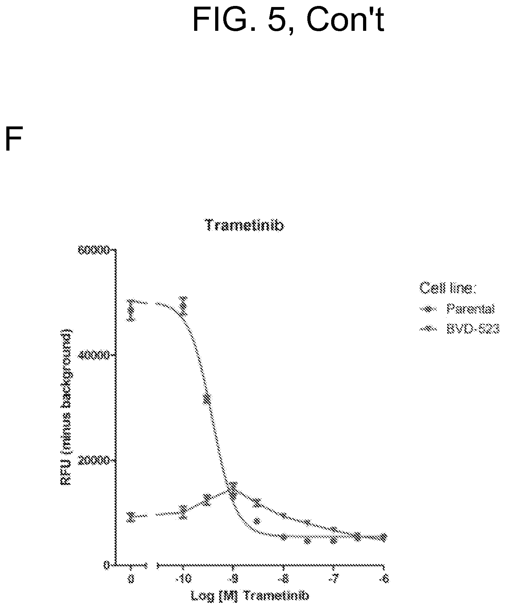

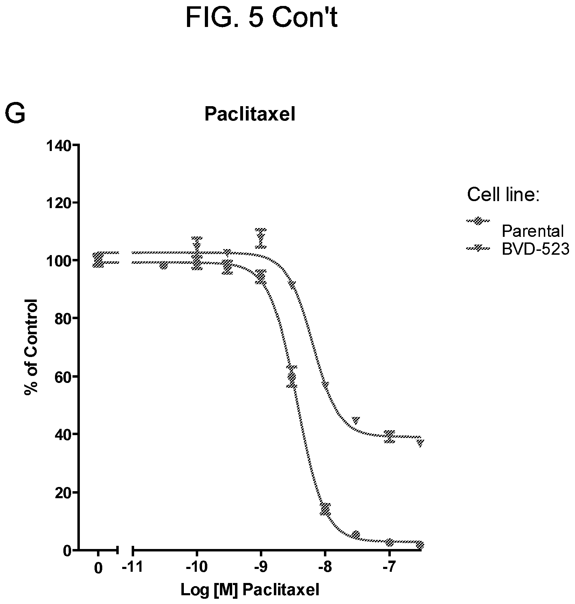

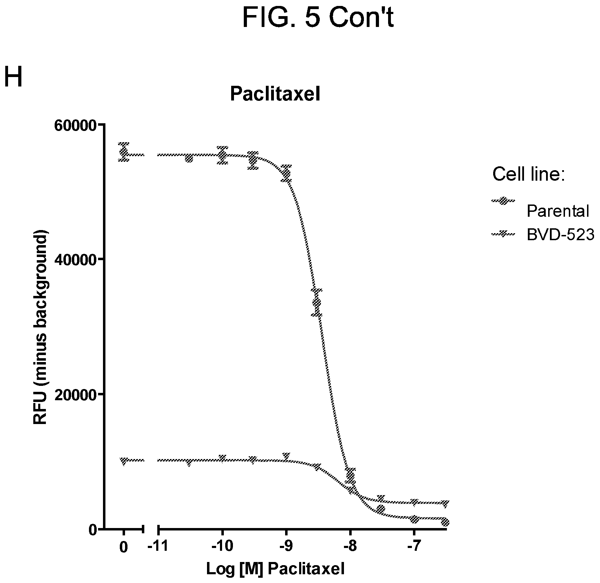

FIGS. 5A-H show only the parental and BVD-523 cell line data from FIG. 4. Various treatments (trametinib, dabrafenib, BVD-523, and pacitaxel) are as labeled. FIGS. 5A-5C and 5G are normalized to control, whereas FIGS. 5D-5F and 5H show the raw data.

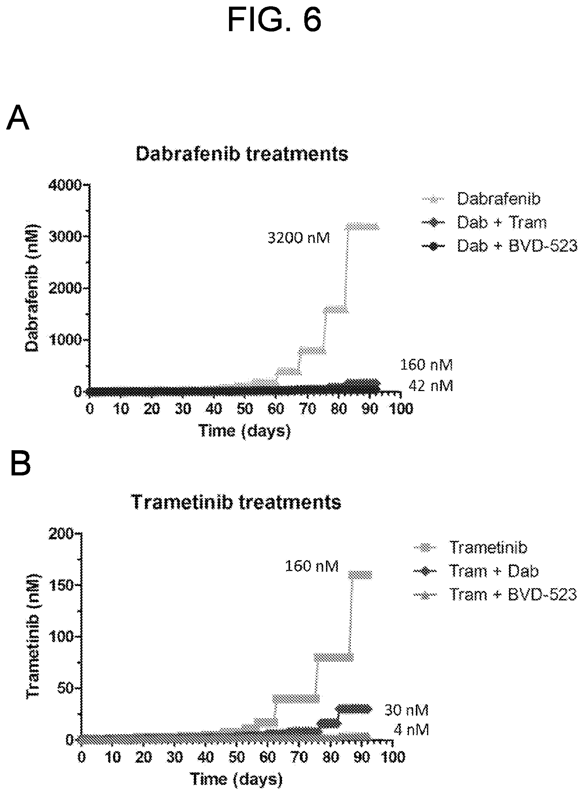

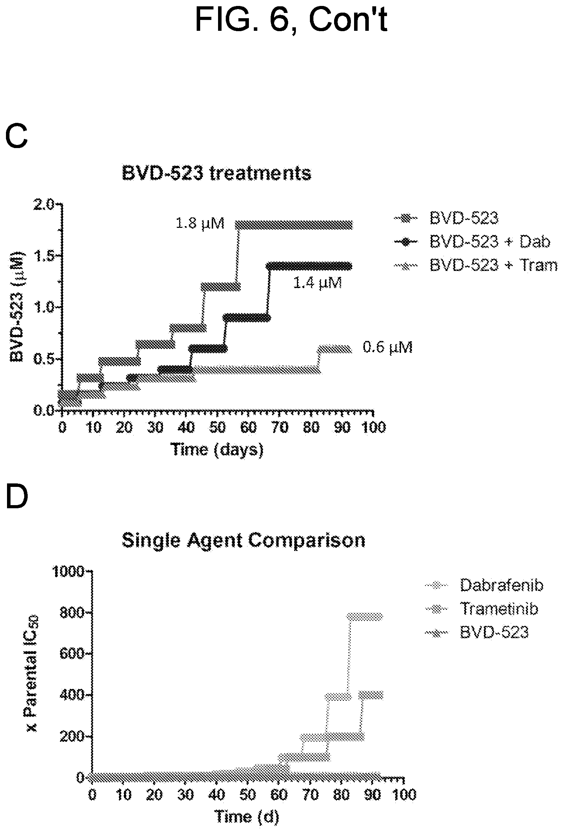

FIGS. 6A-D show the progress of the dose escalation study in a human malignant cell line (A375 cells) for month 3. Various treatments (trametinib, dabrafenib, and BVD-523) are as labeled.

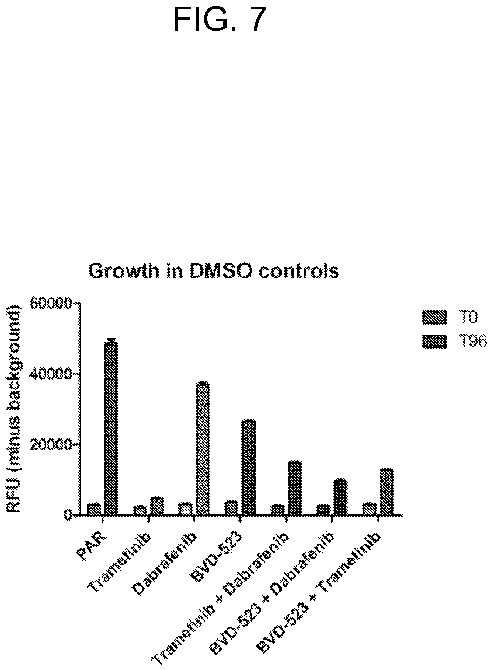

FIG. 7 is a histogram showing the results of a proliferation assay as applied to cells grown in the DMSO control wells from the dose escalation assay.

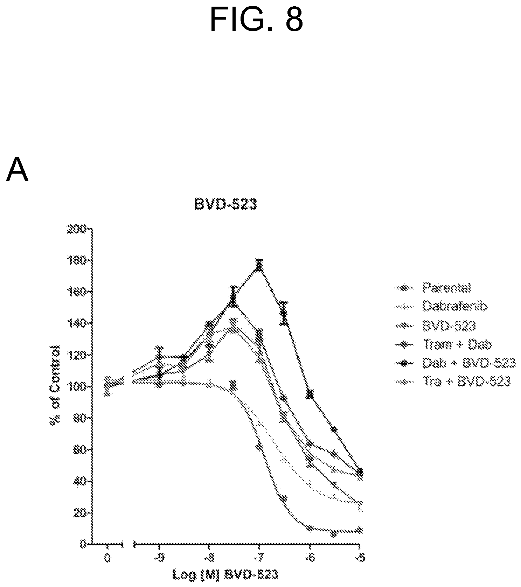

FIGS. 8A-D are a set of line graphs showing proliferation assays for month 3 of the study. Various treatments (trametinib, dabrafenib, BVD-523, and pacitaxel) are as labeled on the top of the graph. The caption to the right of the graph shows the various types of cells generated from the dose escalation study. For example, "dabrafenib" refers to the cells that have been treated with the highest dose of dabrafenib from month 3 of the dose escalation study. Parental refers to the control cells that have not been treated with drugs.

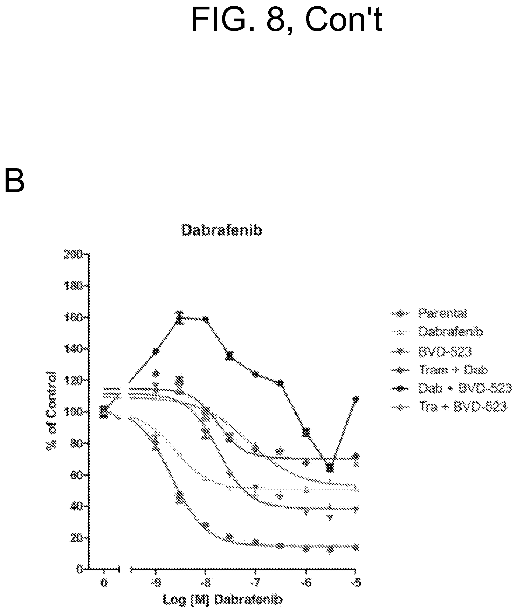

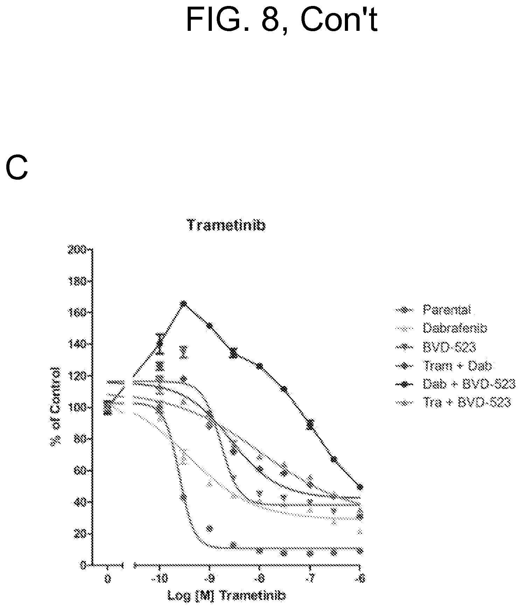

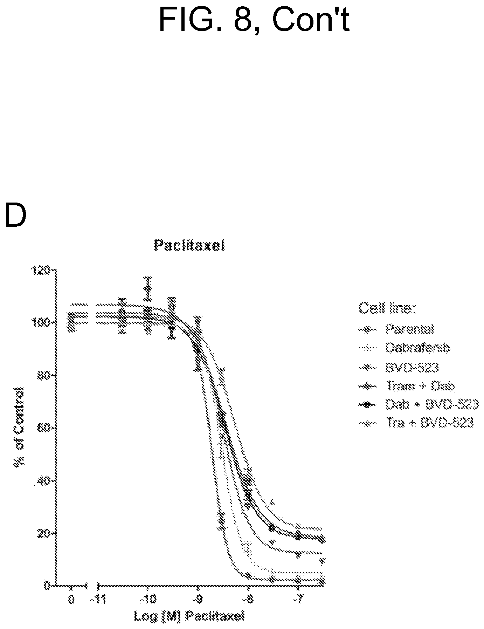

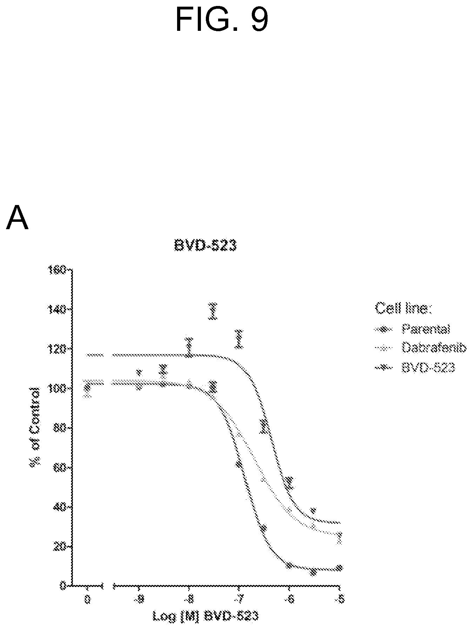

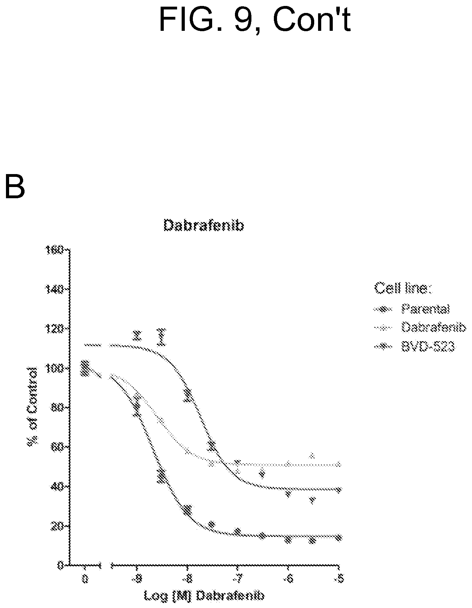

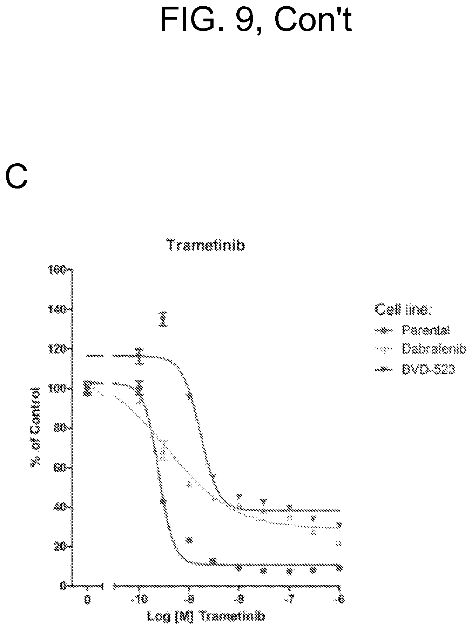

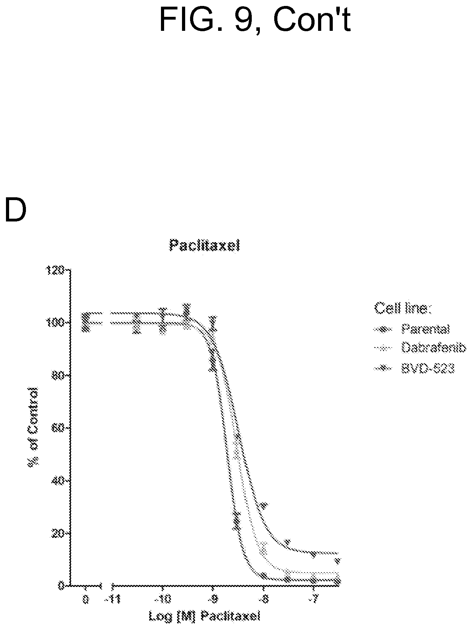

FIGS. 9A-D show only the parental, dabrafenib, and BVD-523 cell line data from FIG. 8.

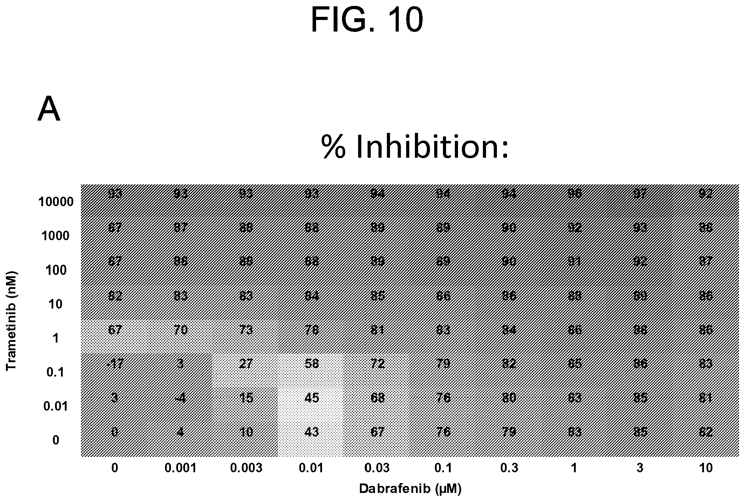

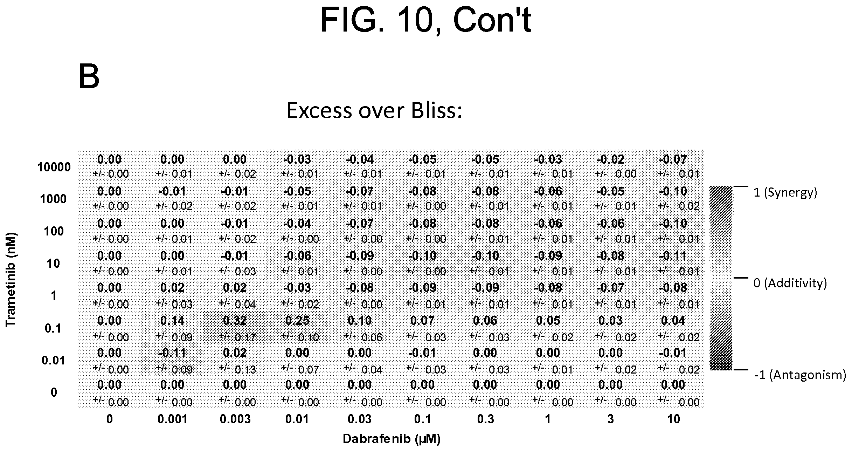

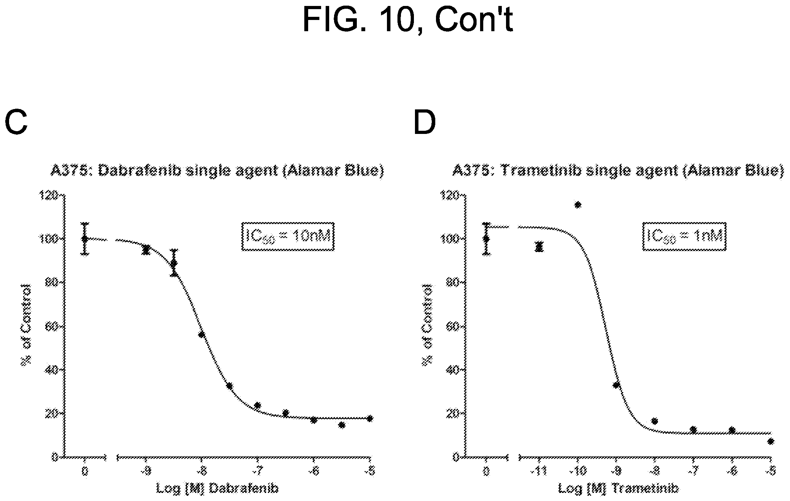

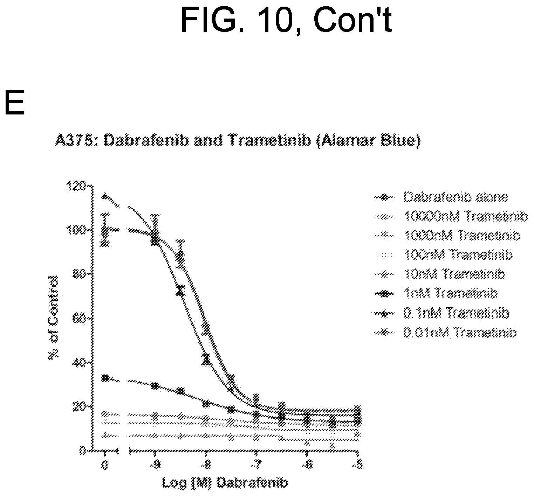

FIG. 10A is a dose matrix showing % inhibition of the trametinib/dabrafenib combination in A375 cells using the Alamar Blue cell viability assay. FIG. 10B is a dose matrix showing excess over Bliss for the trametinib/dabrafenib combination. FIGS. 10C and 10D show % viability relative to DMSO only treated controls for dabrafenib and trametinib single agent treatments in A375 cells using the Alamar Blue cell viability assay. FIG. 10E shows % viability relative to DMSO only treated controls for dabrafenib and trametinib combination treatments in A375 cells using the Alamar Blue cell viability assay.

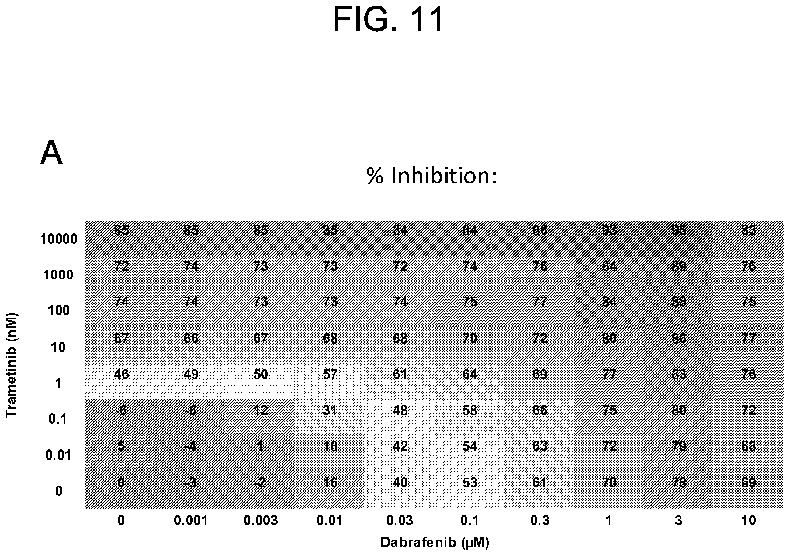

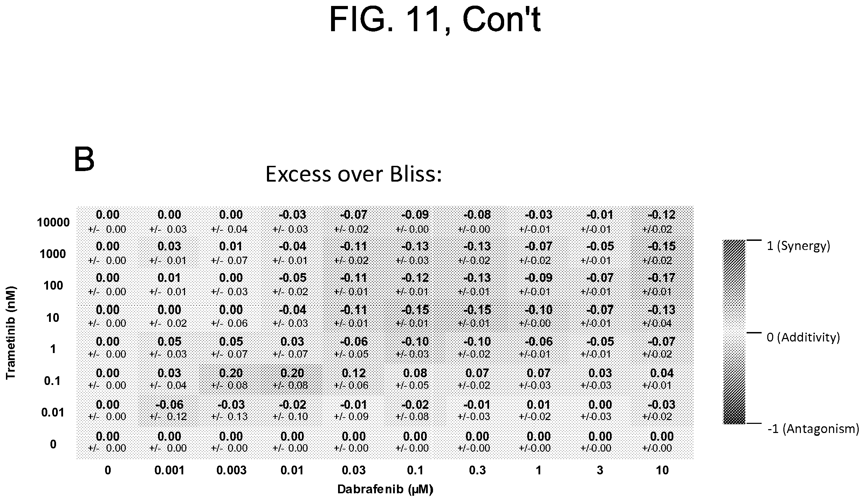

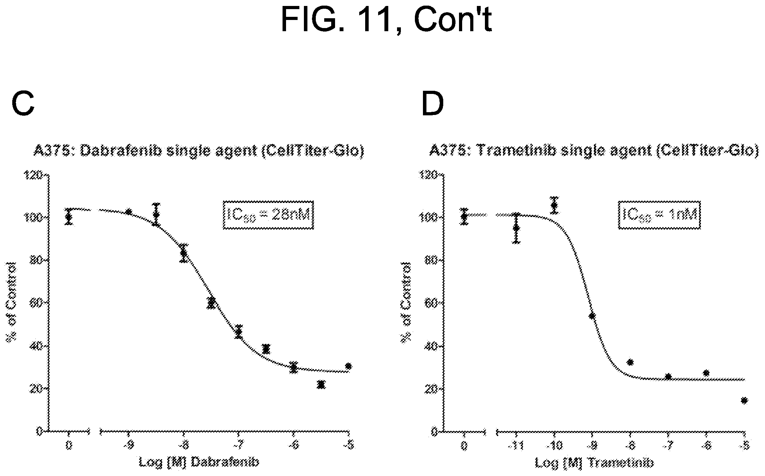

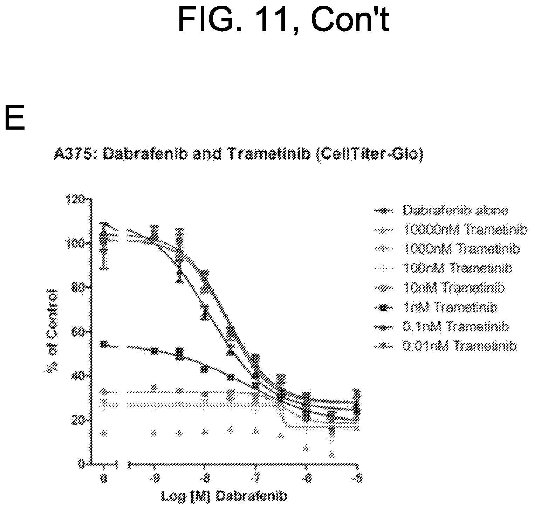

FIG. 11A is a dose matrix showing % inhibition of the trametinib/dabrafenib combination in A375 cells using the CellTiter-Glo cell viability assay. FIG. 11B is a dose matrix showing excess over Bliss for the trametinib/dabrafenib combination. FIGS. 11C and 11D show % viability relative to DMSO only treated controls for dabrafenib and trametinib single agent treatments in A375 cells using the CellTiter-Glo cell viability assay. FIG. 11E shows % viability relative to DMSO only treated controls for dabrafenib and trametinib combination treatments in A375 cells using the CellTiter-Glo cell viability assay.

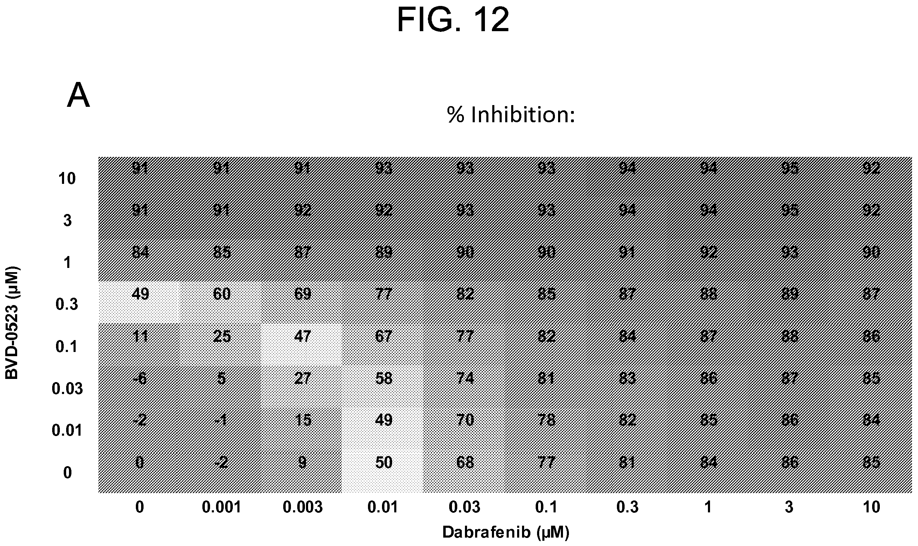

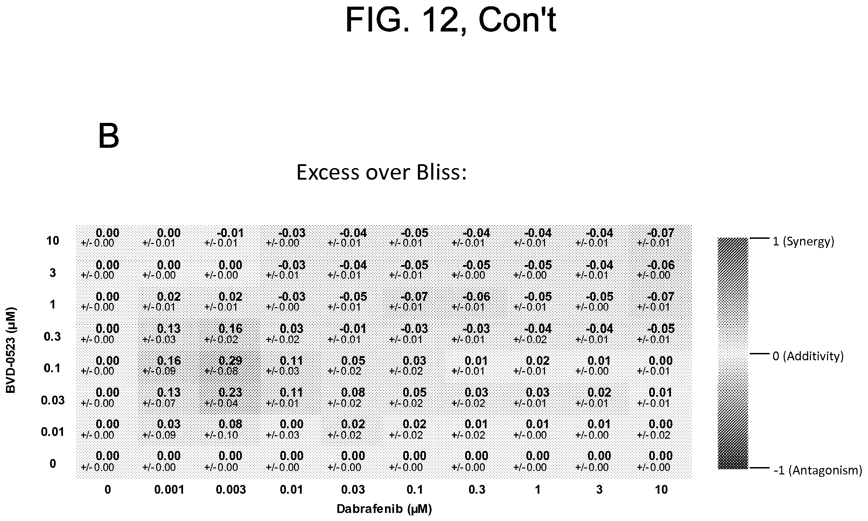

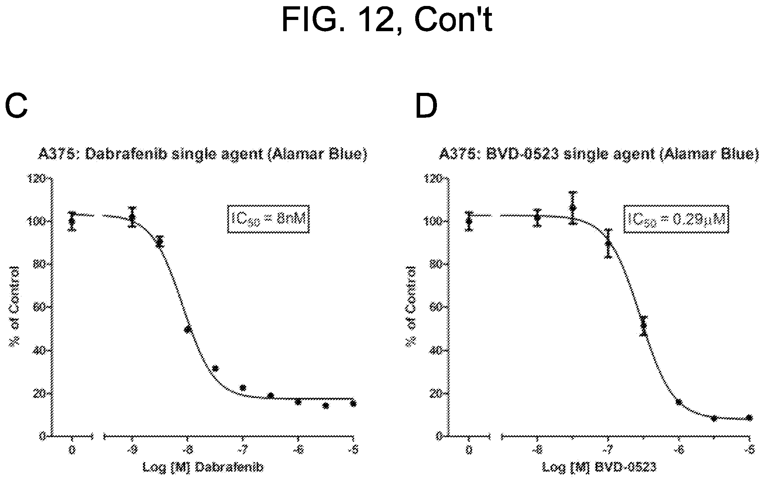

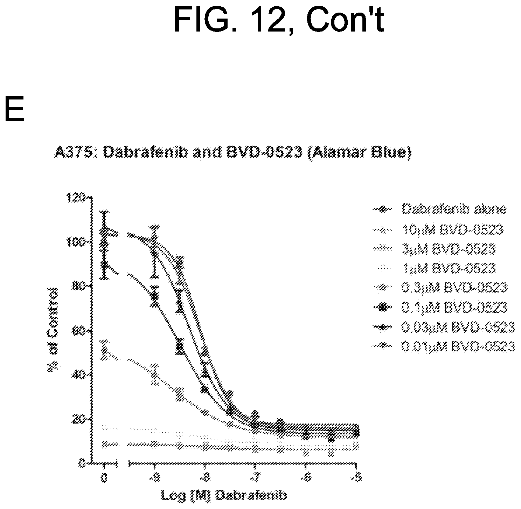

FIG. 12A is a dose matrix showing % inhibition of the BVD-523/dabrafenib combination in A375 cells using the Alamar Blue cell viability assay. FIG. 12B is a dose matrix showing excess over Bliss for the BVD-523/dabrafenib combination. FIGS. 12C and 12D show % viability relative to DMSO only treated controls for dabrafenib and BVD-523 single agent treatments in A375 cells using the Alamar Blue cell viability assay. FIG. 12E shows % viability relative to DMSO only treated controls for dabrafenib and BVD-523 combination treatments in A375 cells using the Alamar Blue cell viability assay.

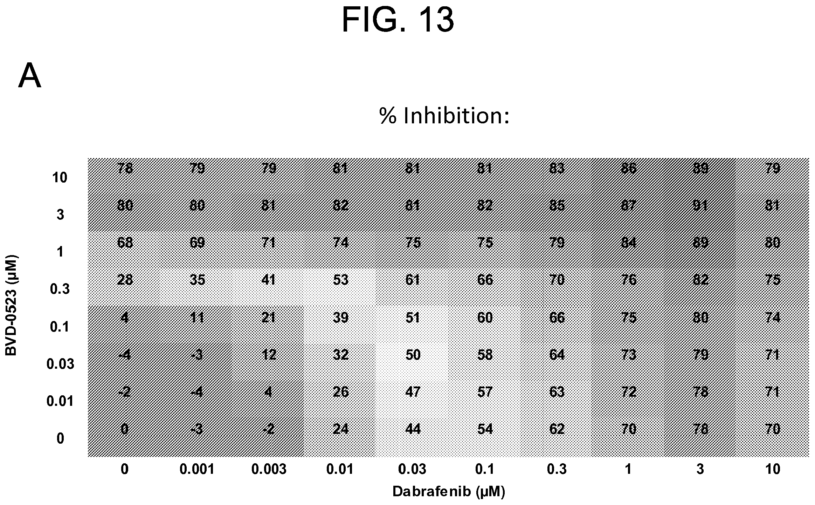

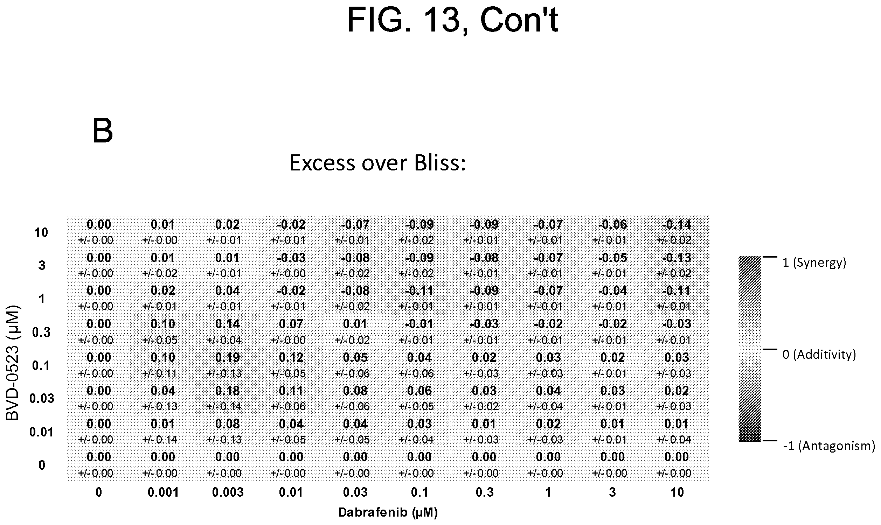

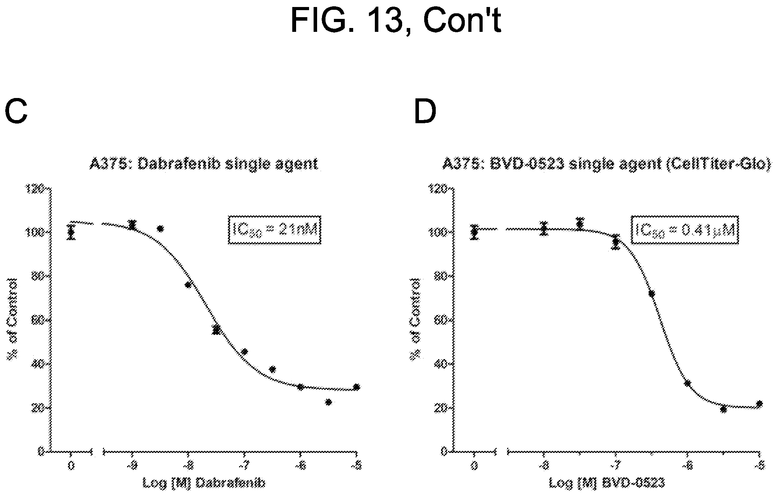

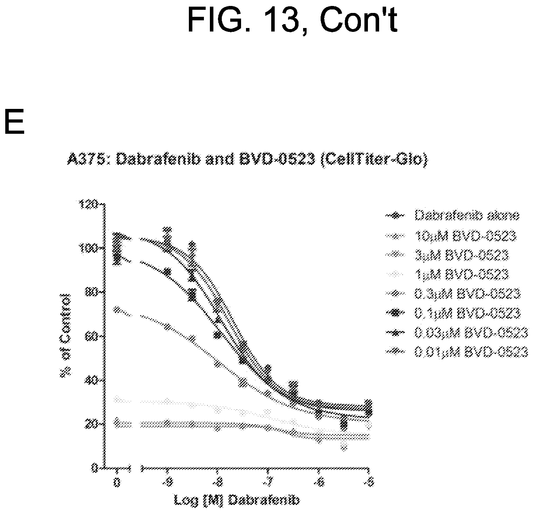

FIG. 13A is a dose matrix showing % inhibition of the BVD-523/dabrafenib combination in A375 cells using the CellTiter-Glo cell viability assay. FIG. 13B is a dose matrix showing excess over Bliss for the BVD-523/dabrafenib combination. FIGS. 13C and 13D show % viability relative to DMSO only treated controls for dabrafenib and BVD-523 single agent treatments in A375 cells using the CellTiter-Glo cell viability assay. FIG. 13E shows % viability relative to DMSO only treated controls for dabrafenib and BVD-523 combination treatments in A375 cells using the CellTiter-Glo cell viability assay.

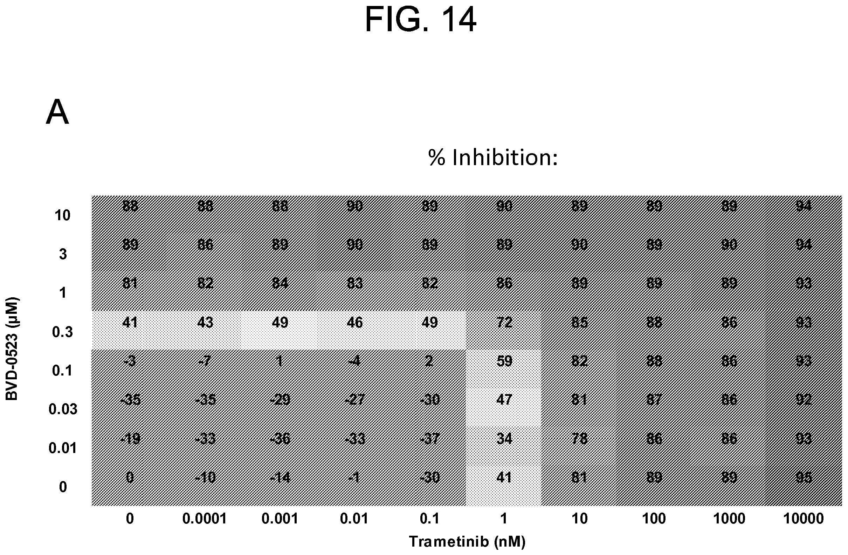

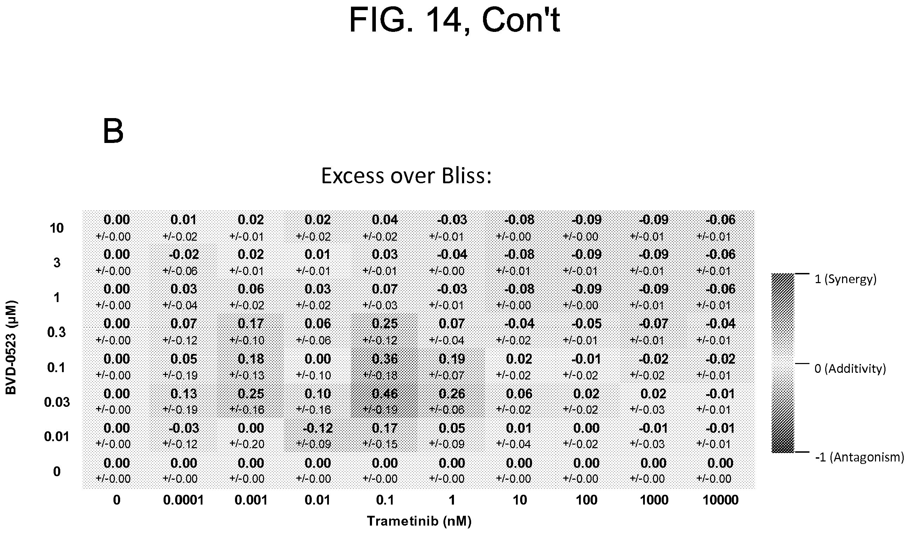

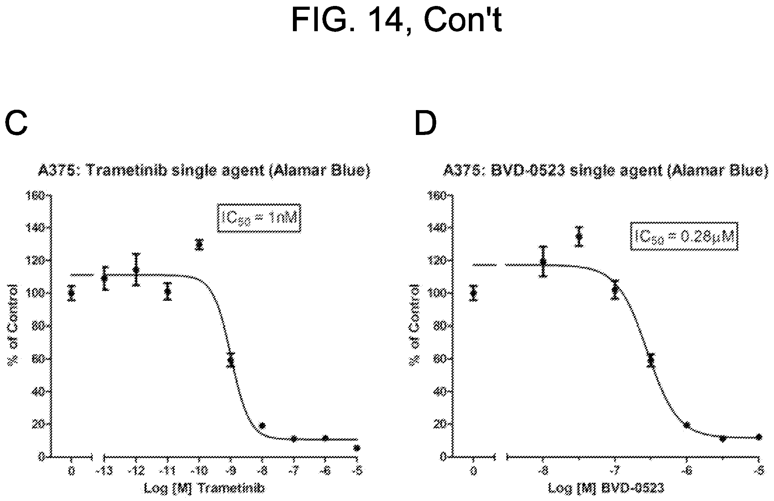

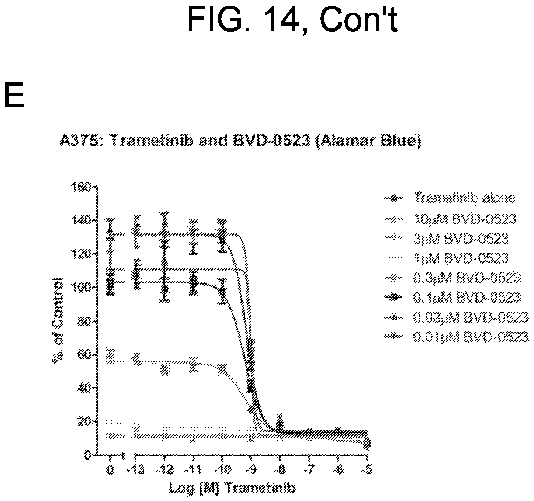

FIG. 14A is a dose matrix showing % inhibition of the trametinib/BVD-523 combination in A375 cells using the Alamar Blue cell viability assay. FIG. 14B is a dose matrix showing excess over Bliss for the trametinib/BVD-523 combination. FIGS. 14C and 14D show % viability relative to DMSO only treated controls for BVD-523 and trametinib single agent treatments in A375 cells using the Alamar Blue cell viability assay. FIG. 14E shows % viability relative to DMSO only treated controls for BVD-523 and trametinib combination treatments in A375 cells using the Alamar Blue cell viability assay.

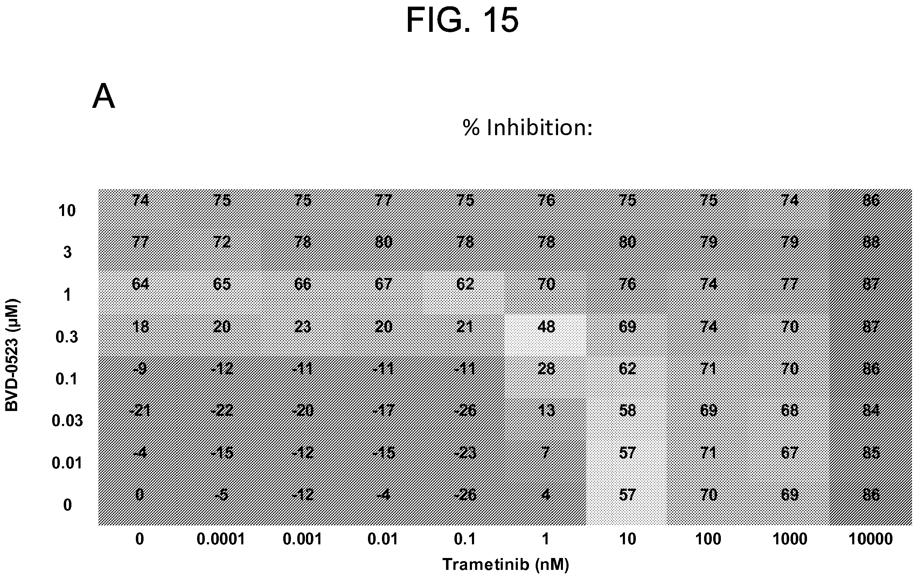

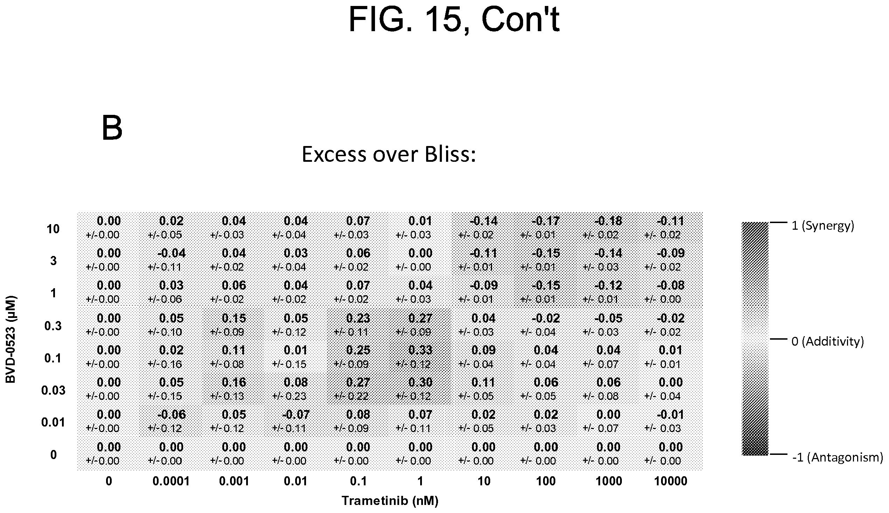

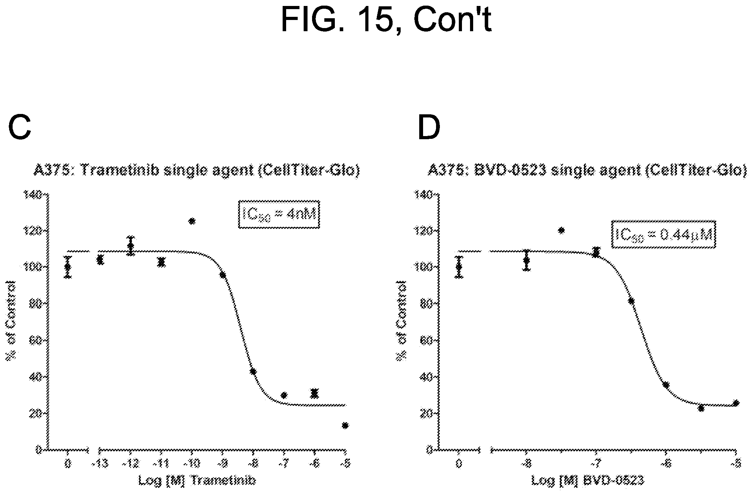

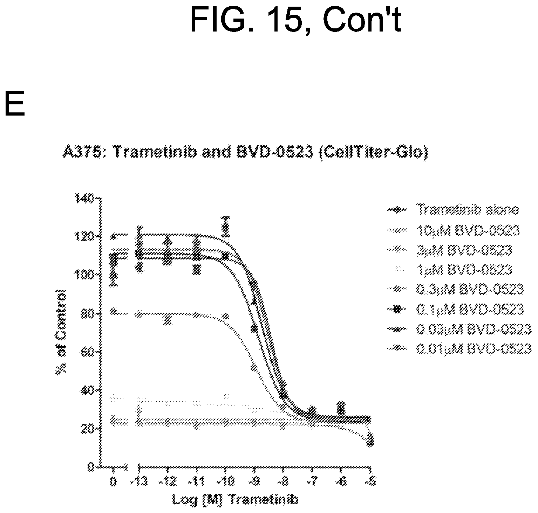

FIG. 15A is a dose matrix showing % inhibition of the trametinib/BVD-523 combination in A375 cells using the CellTiter-Glo cell viability assay. FIG. 15B is a dose matrix showing excess over Bliss for the trametinib/BVD-523 combination. FIGS. 15C and 15D show % viability relative to DMSO only treated controls for BVD-523 and trametinib single agent treatments in A375 cells using the CellTiter-Glo cell viability assay. FIG. 15E shows % viability relative to DMSO only treated controls for BVD-523 and trametinib combination treatments in A375 cells using the CellTiter-Glo cell viability assay.

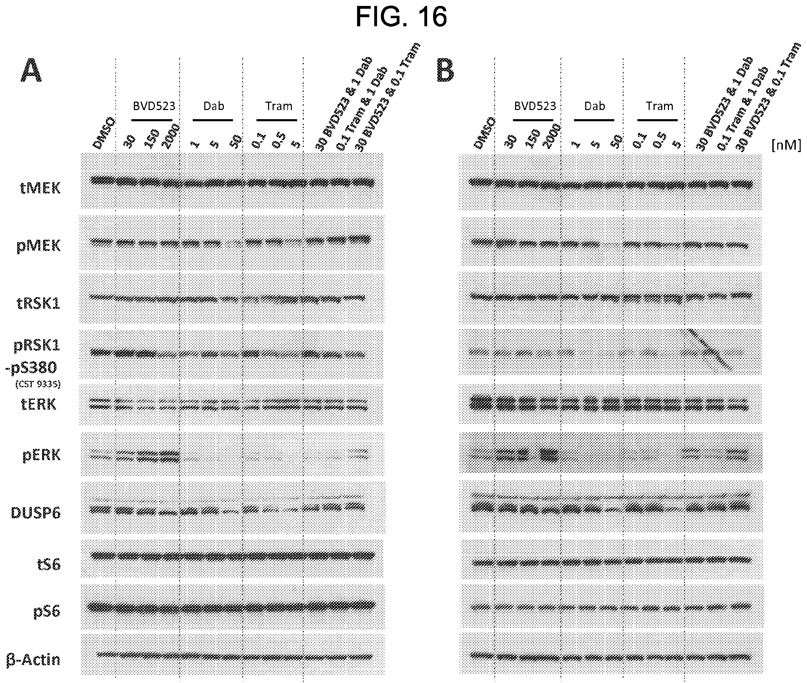

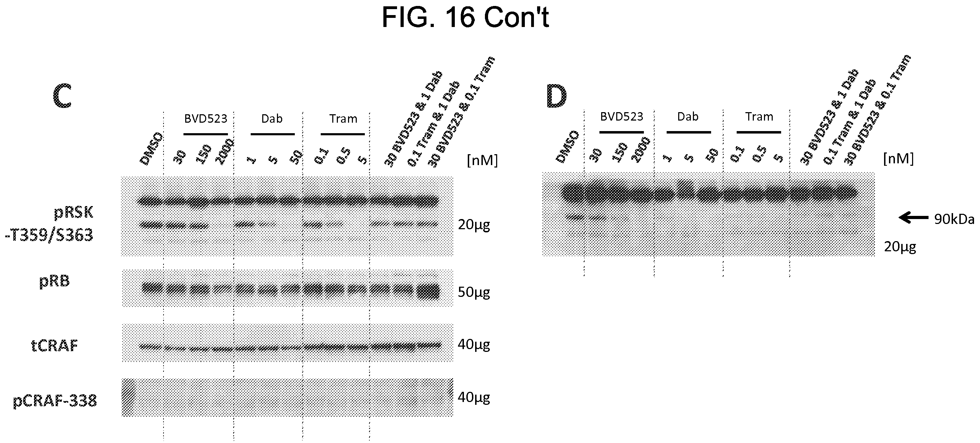

FIGS. 16A-D are a set of images showing Western blot analysis of MAPK signaling in A375 cells after a 4 hour treatment with various concentrations (in nM) of BVD-523, dabrafenib (Dab), and Trametinib (Tram). 40 µg of total protein was loaded in each lane except where indicated otherwise. In this experiment, duplicate samples were collected. FIGS. 16A and 16B show results from duplicate samples. Similarly, FIGS. 16C and 16D also show results from duplicate samples. In FIGS. 16A and 16B, pRSK1 had a relatively weak signal in A375 cells compared to other markers. A different pRSK1-S380 antibody from Cell Signaling (cat. #11989) was tested but did not give a detectable signal (data not shown). In FIGS. 16C and 16D, pCRAF-338 gave a minimal signal.

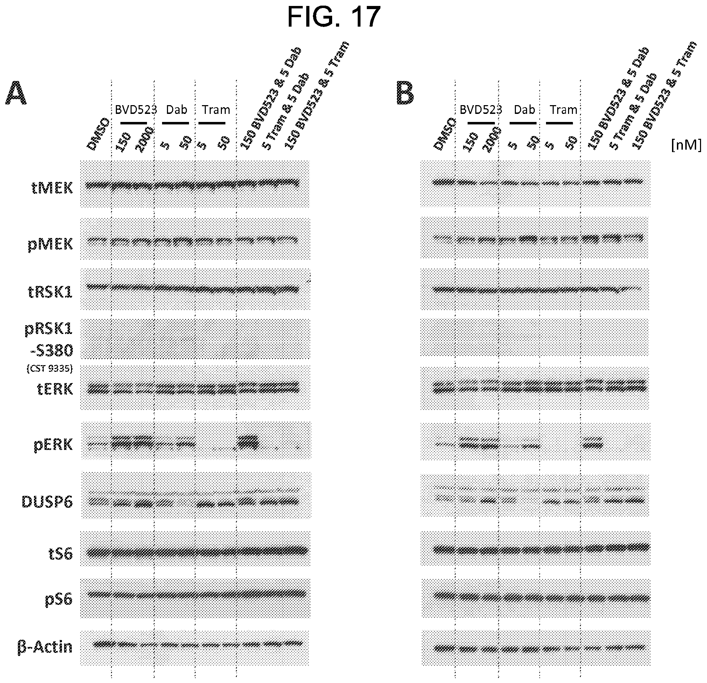

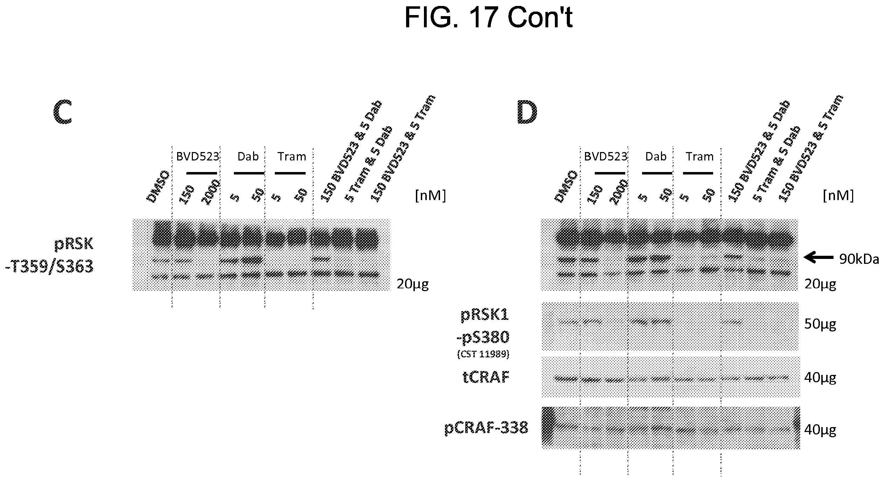

FIGS. 17A-D are a set of images showing Western blot analysis of MAPK signaling in a human colorectal carcinoma cell line (HCT116 cells) after a 4 hour treatment with various concentrations (in nM) of BVD-523, dabrafenib (Dab), and Trametinib (Tram). 40 µg of total protein was loaded in each lane except where indicated otherwise. In this experiment, duplicate samples were collected. FIGS. 17A and 17B show results from duplicate samples. Similarly, FIGS. 17C and 17D also show results from duplicate samples. In FIGS. 17A-17B, pRSK1 levels appear to be very low in HCT116 cells, and in FIGS. 17C and 17D, pCRAF-338 signal was also very weak.

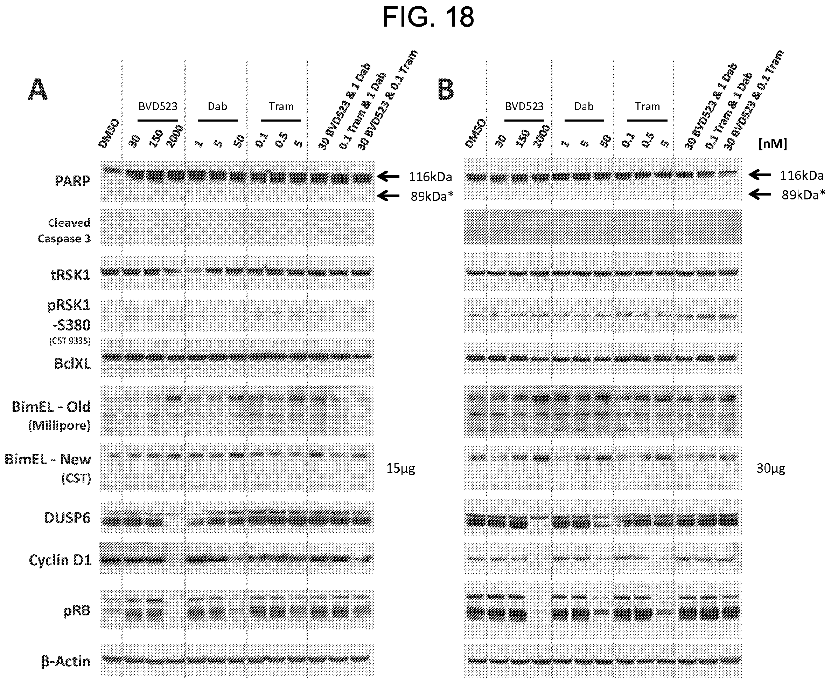

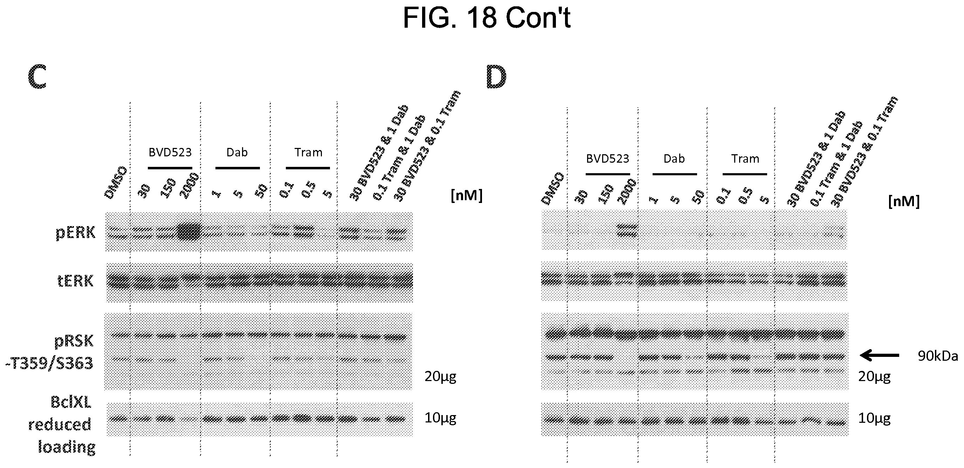

FIGS. 18A-D are a set of images showing Western blot analysis of cell cycle and apoptosis signalling in A375 melanoma cells after a 24 hour treatment with various concentrations (in nM) of BVD-523 ("BVD523"), trametinib ("Tram") and/or dabrafenib ("Dab") as labelled. 50 µg of total protein was loaded in each lane except where indicated otherwise. In this experiment, duplicate samples were collected. FIGS. 18A and 18B show results from duplicate samples. Similarly, FIGS. 18C and 18D also show results from duplicate samples. In FIGS. 18A and 18B, no band of a size corresponding to cleaved PARP (89 kDa) was apparent.

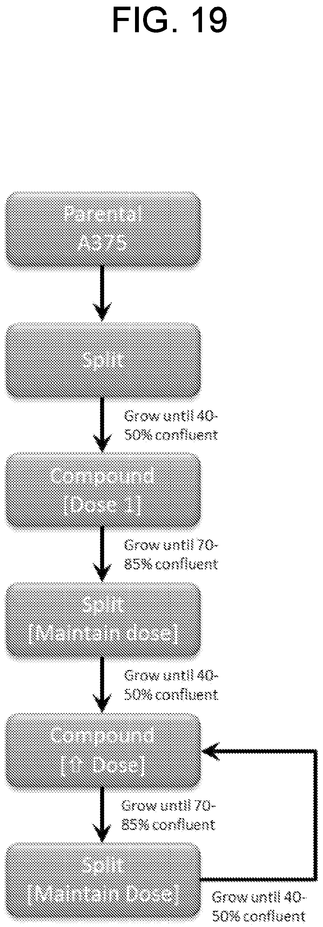

FIG. 19 is a flowchart showing the dose escalation protocol used herein.

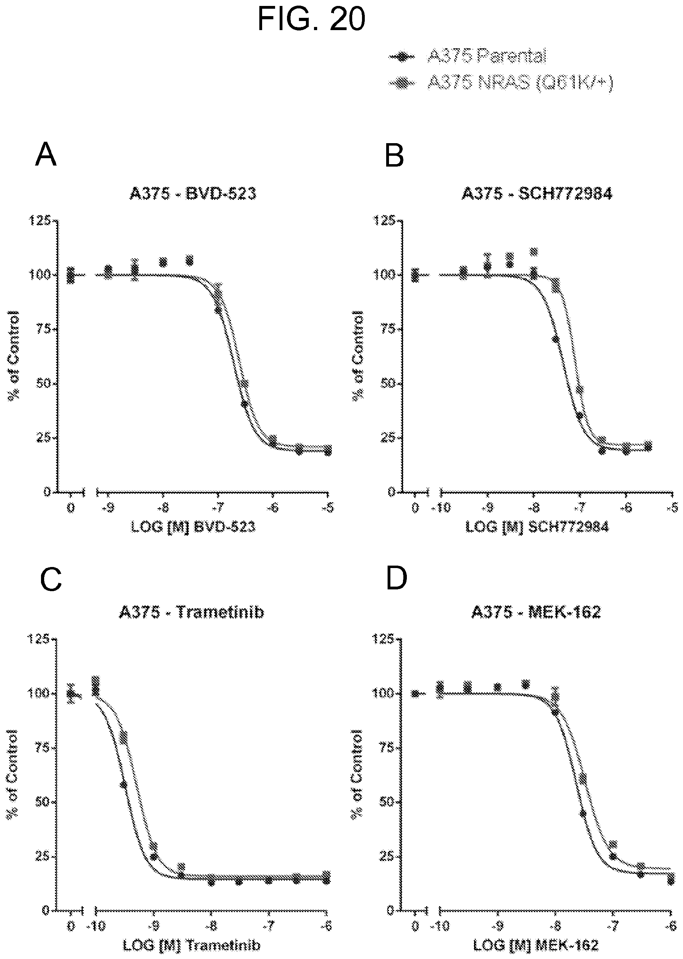

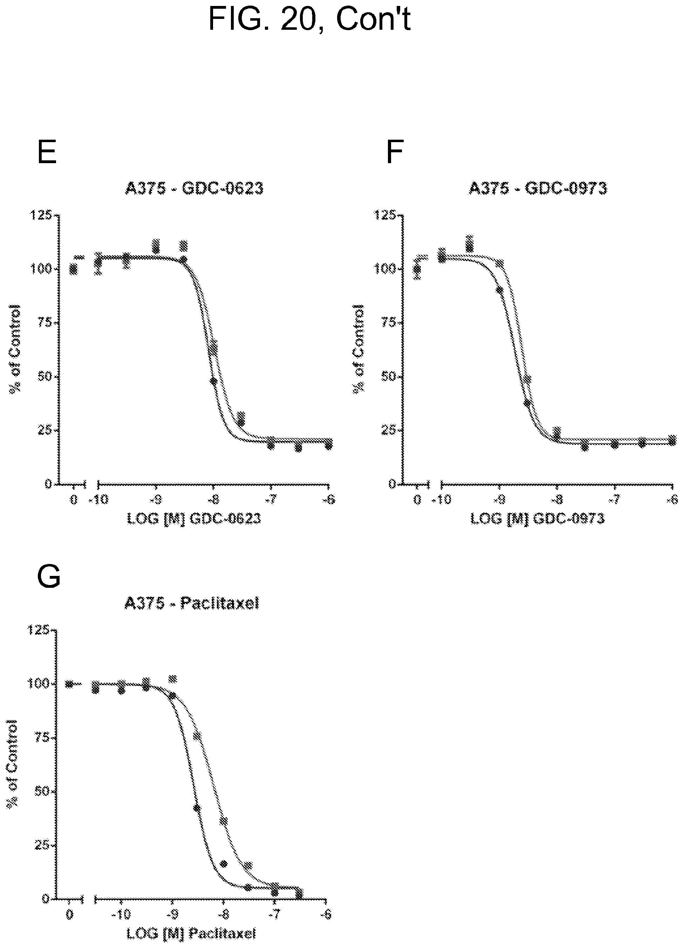

FIG. 20 shows the results of single agent proliferation assays in parental A375 and A375 NRAS (Q61K/+) cells. Proliferation results are shown for treatment with BVD-523 (FIG. 20A), SCH772984 (FIG. 20B), Trametinib (FIG. 20C), MEK-162 (FIG. 20D), GDC-0623 (FIG. 20E), GDC-0973 (FIG. 20F), and Paclitaxel (FIG. 20G).

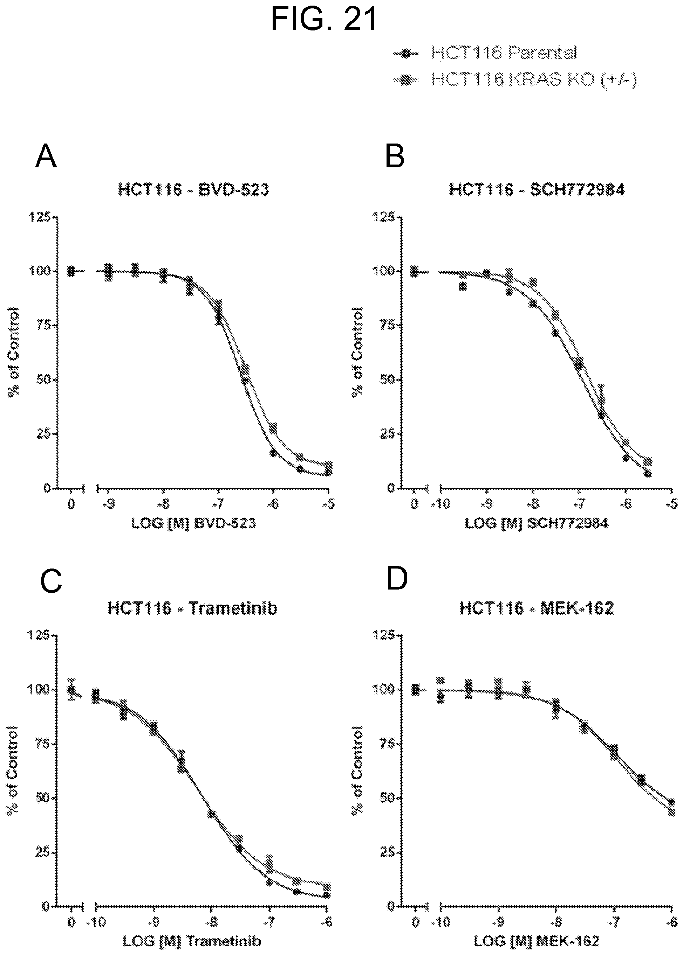

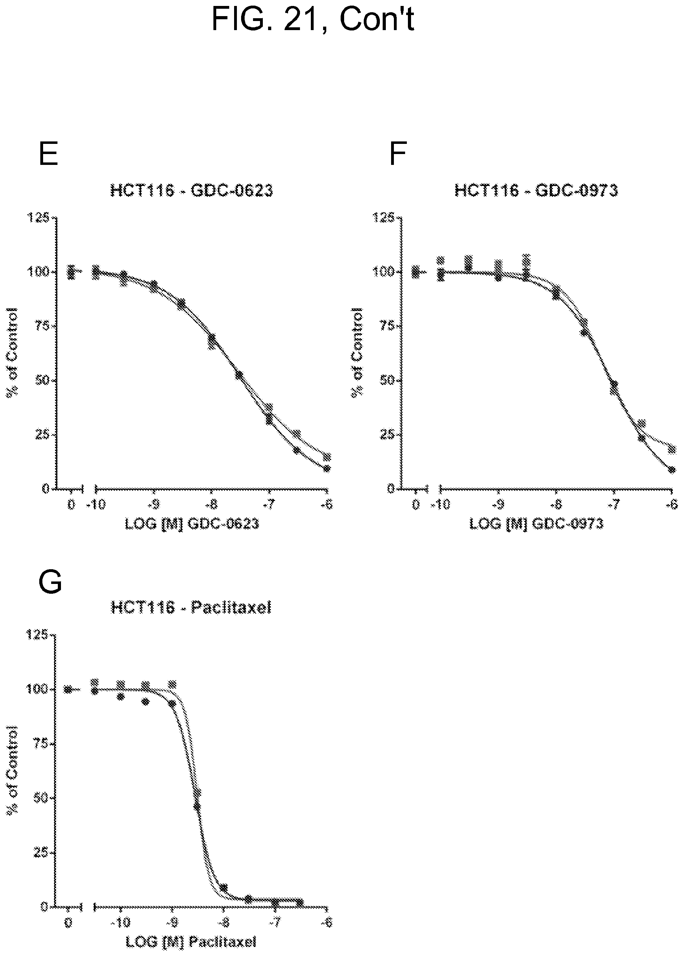

FIG. 21 shows the results of single agent proliferation assays in parental HCT116 and A375 KRAS KO (-/+) cells. Proliferation results are shown for treatment with BVD-523 (FIG. 21A), SCH772984 (FIG. 21B), Trametinib (FIG. 21C), MEK-162 (FIG. 21D), GDC-0623 (FIG. 21E), GDC-0973 (FIG. 21F), and Paclitaxel (FIG. 21G).

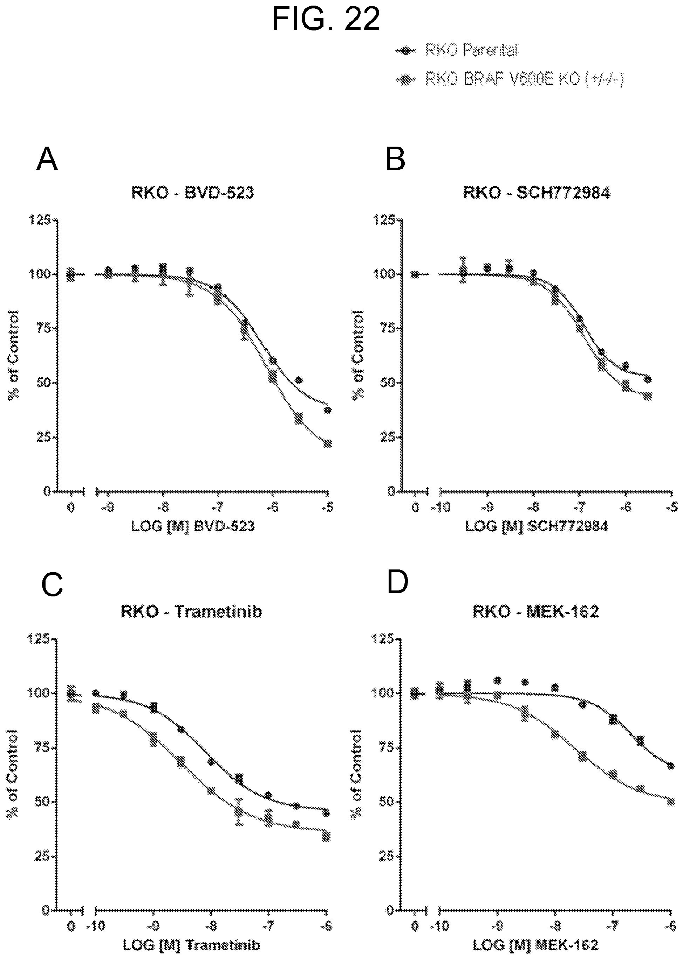

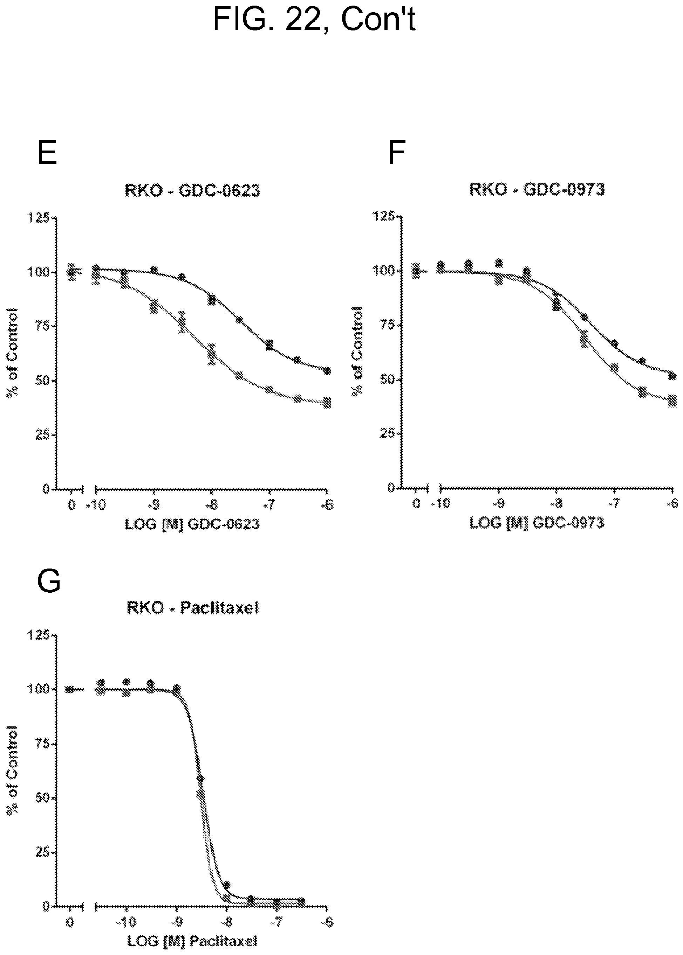

FIG. 22 shows the results of single agent proliferation assays in parental RKO and RKO BRAF V600E KO (+/-/-) cells. Proliferation results are shown for treatment with BVD-523 (FIG. 22A), SCH772984 (FIG. 22B), Trametinib (FIG. 22C), MEK-162 (FIG. 22D), GDC-0623 (FIG. 22E), GDC-0973 (FIG. 22F), and Paclitaxel (FIG. 22G).

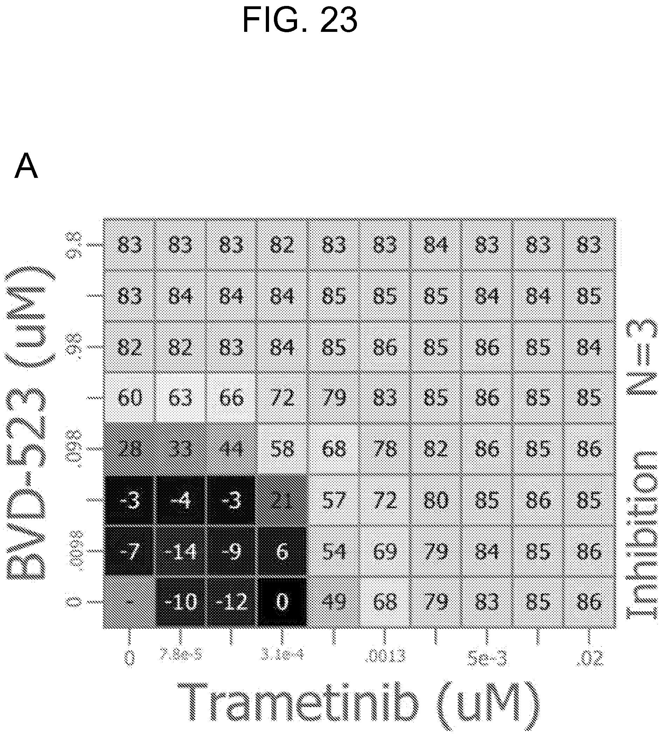

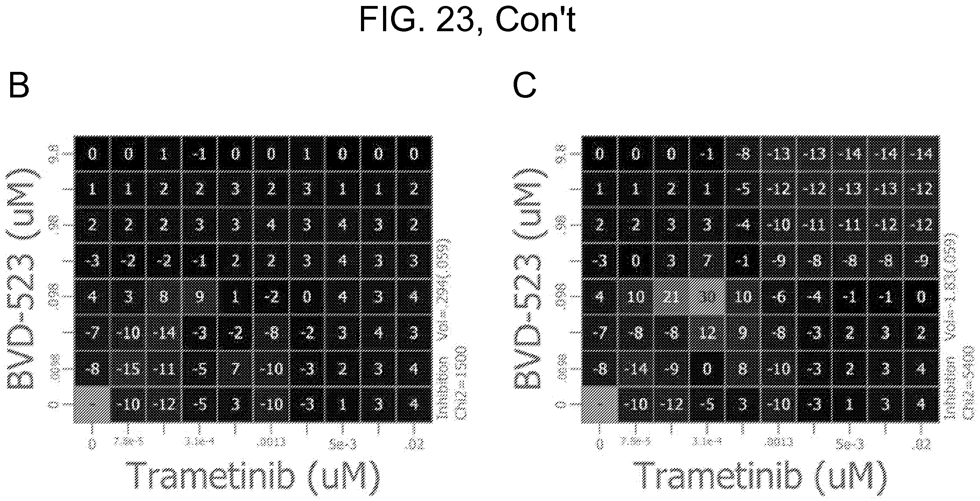

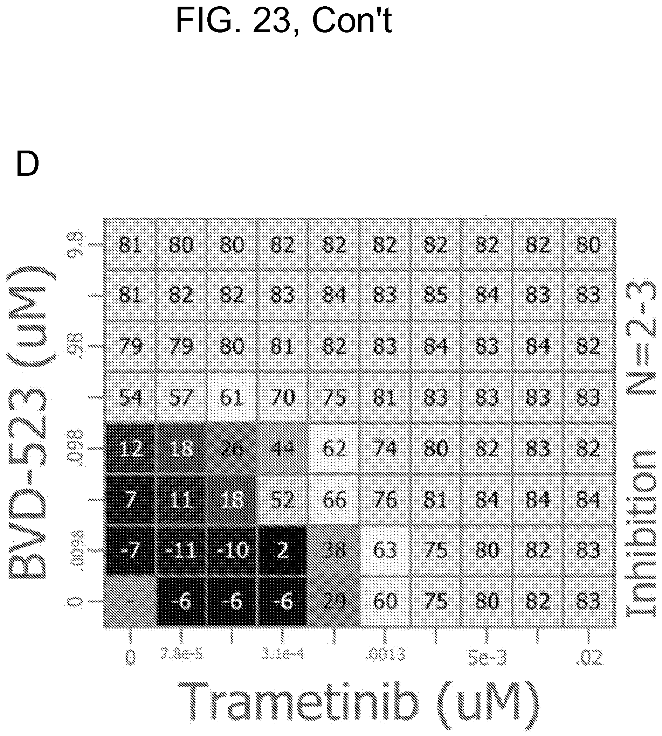

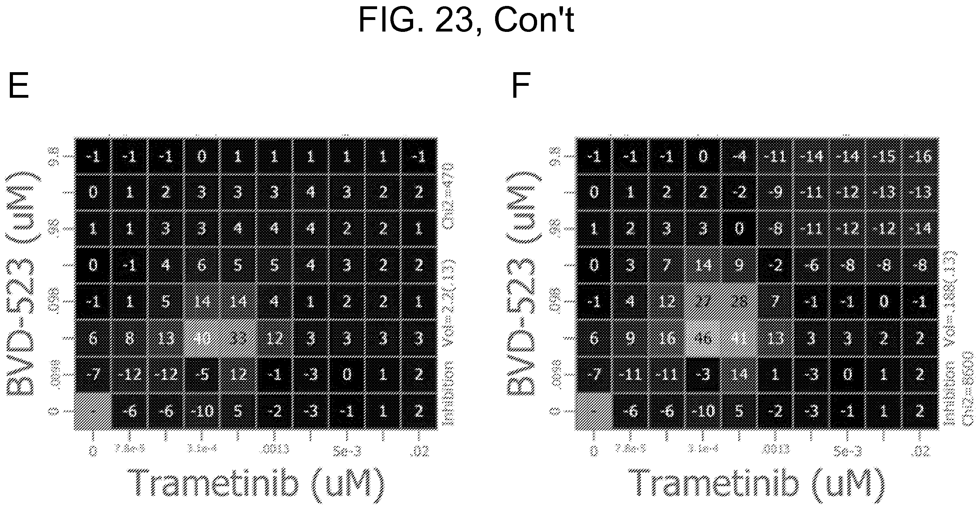

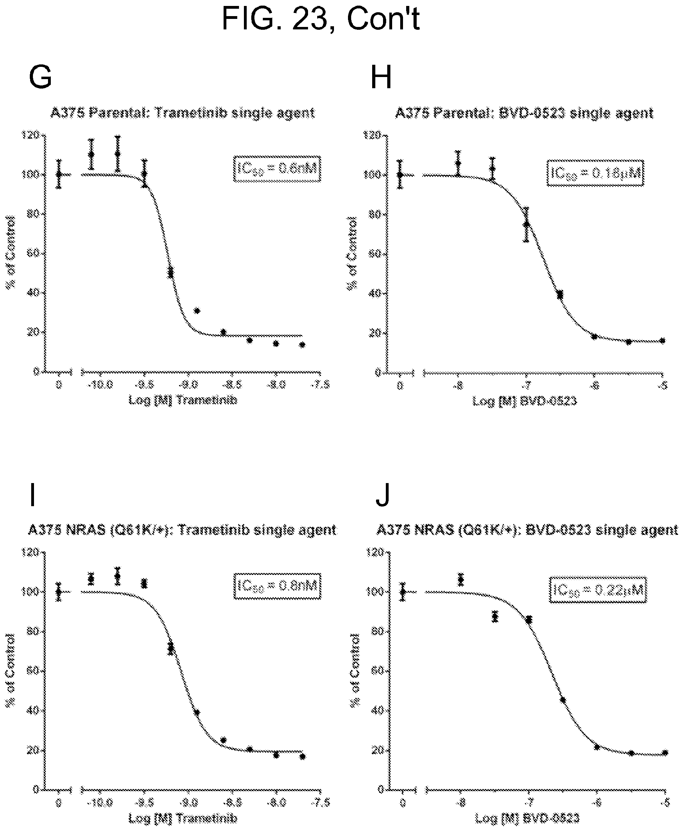

FIG. 23 shows the results of the combination of BVD-523 and Trametinib in parental A375 and A375 NRAS (Q61K/+) cells. FIG. 23A shows a dose matrix showing inhibition (%) for the combination in parental A375 cells. FIG. 23B shows Loewe excess for the combination in 23A and FIG. 23C shows Bliss excess for the combination in 23A. FIG. 23D shows a dose matrix showing inhibition (%) for the combination in A375 NRAS (Q61K/+) cells. FIG. 23E shows Loewe excess for the combination in 23D and FIG. 23F shows Bliss excess for the combination in 23D. FIG. 23G - FIG. 23H show the results of single agent proliferation assays for the combination in 23A. FIG. 23I - FIG. 23J show the results of single agent proliferation assays for the combination in 23D.

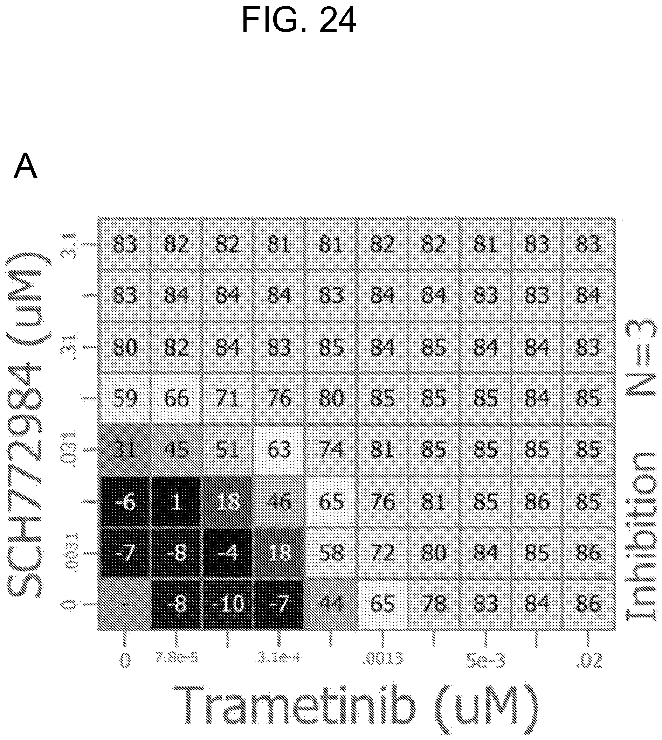

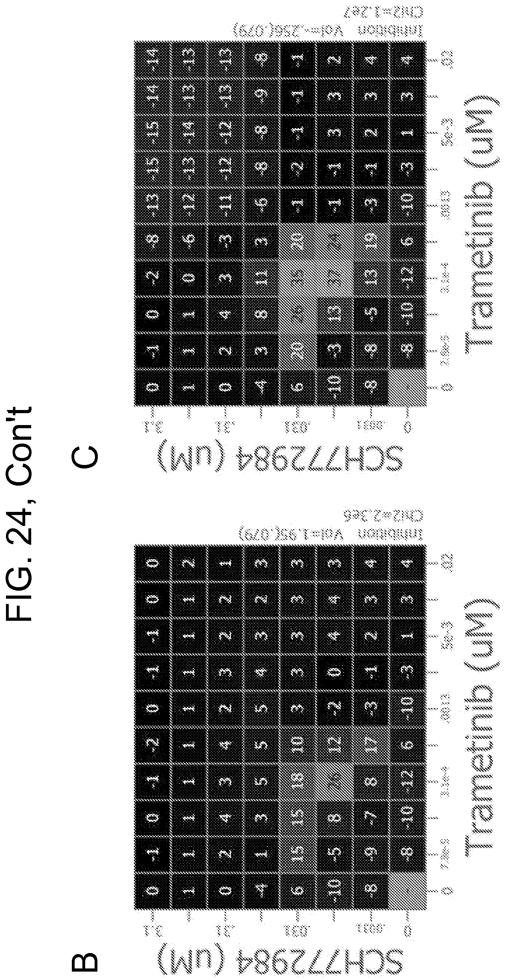

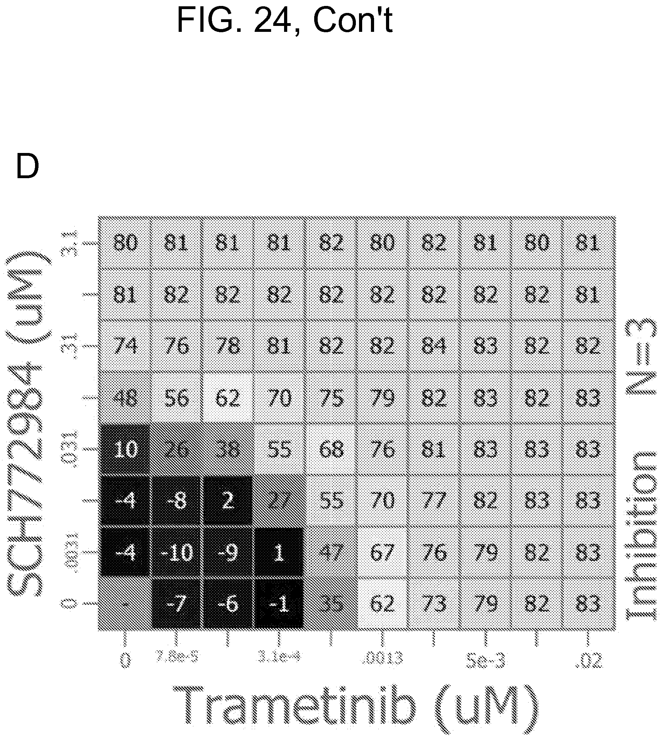

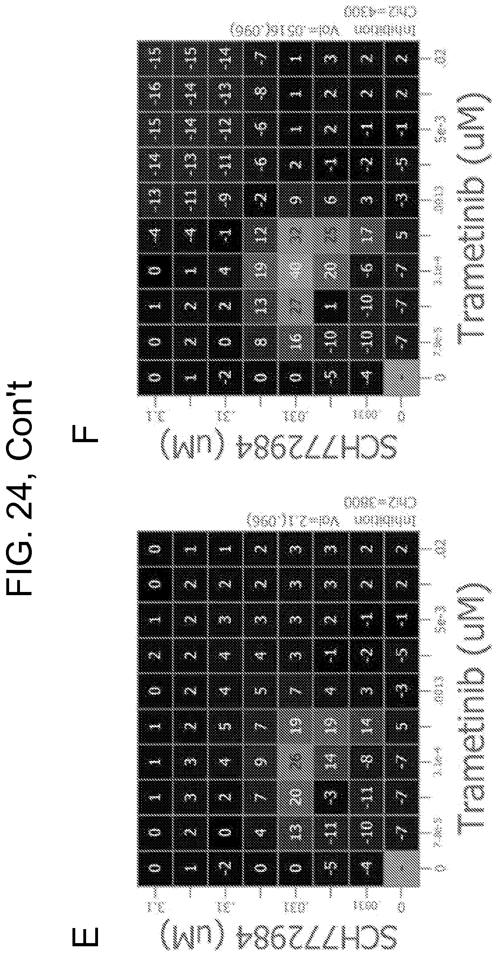

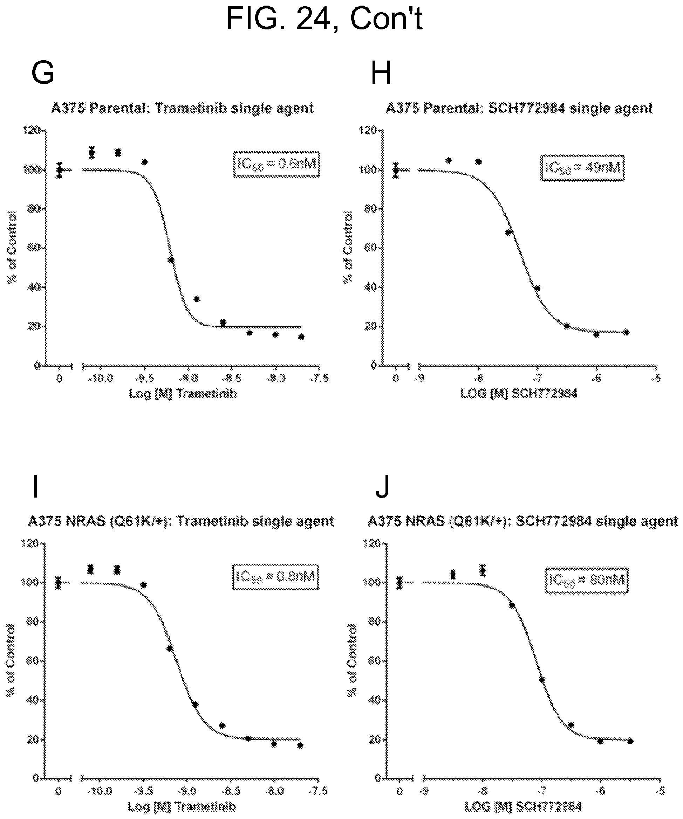

FIG. 24 shows the results of the combination of SCH772984 and Trametinib in parental A375 and A375 NRAS (Q61K/+) cells. FIG. 24A shows a dose matrix showing inhibition (%) for the combination in parental A375 cells. FIG. 24B shows Loewe excess for the combination in 24A and FIG. 24C shows Bliss excess for the combination in 24A. FIG. 24D shows a dose matrix showing inhibition (%) for the combination in A375 NRAS (Q61K/+) cells. FIG. 24E shows Loewe excess for the combination in 24D and FIG. 24F shows Bliss excess for the combination in 24D. FIG. 24G - FIG. 24H show the results of single agent proliferation assays for the combination in 24A. FIG. 24I - FIG. 24J show the results of single agent proliferation assays for the combination in 24D.

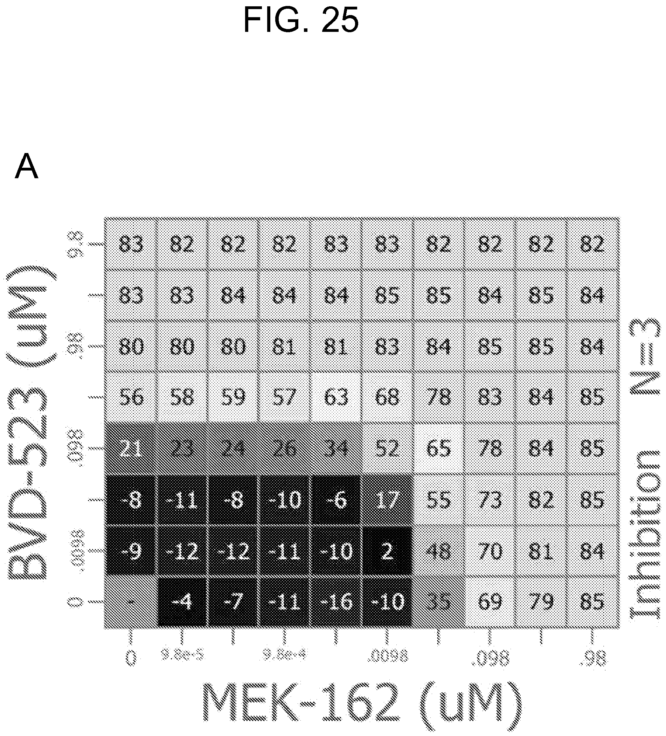

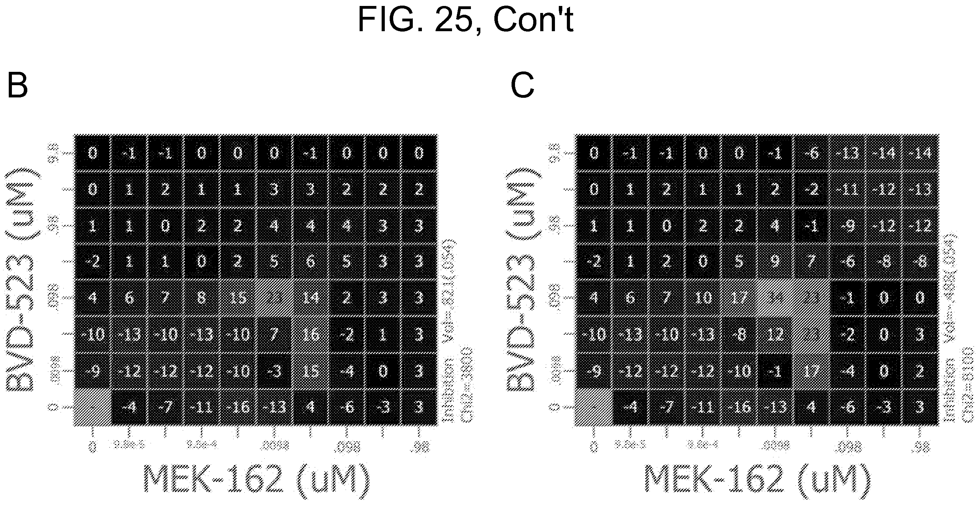

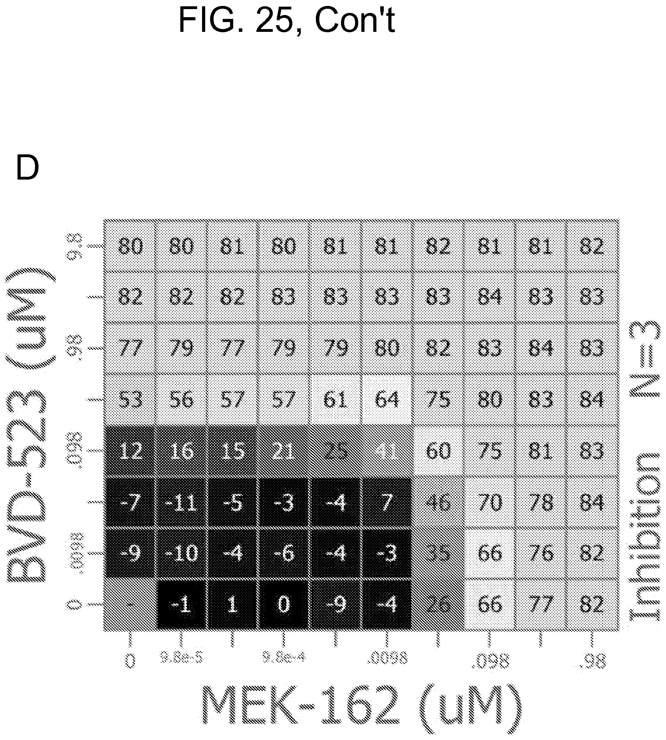

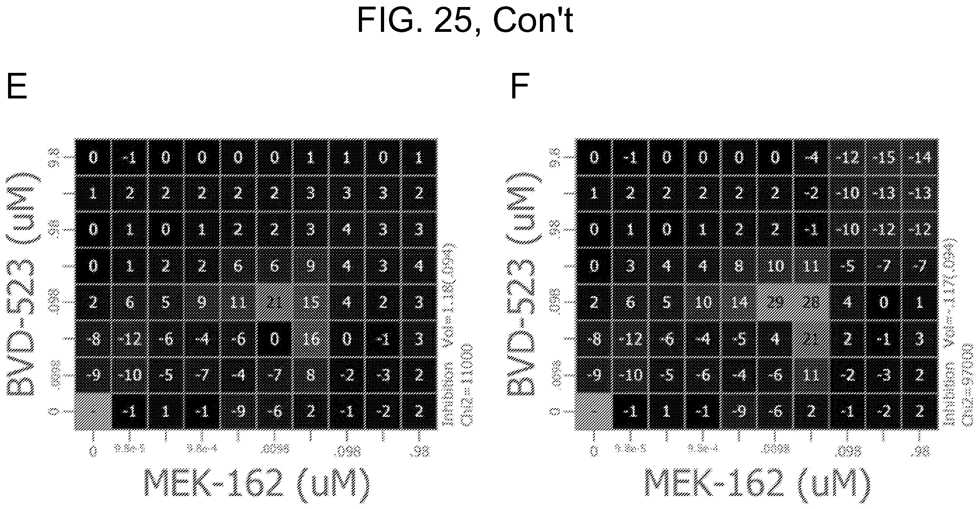

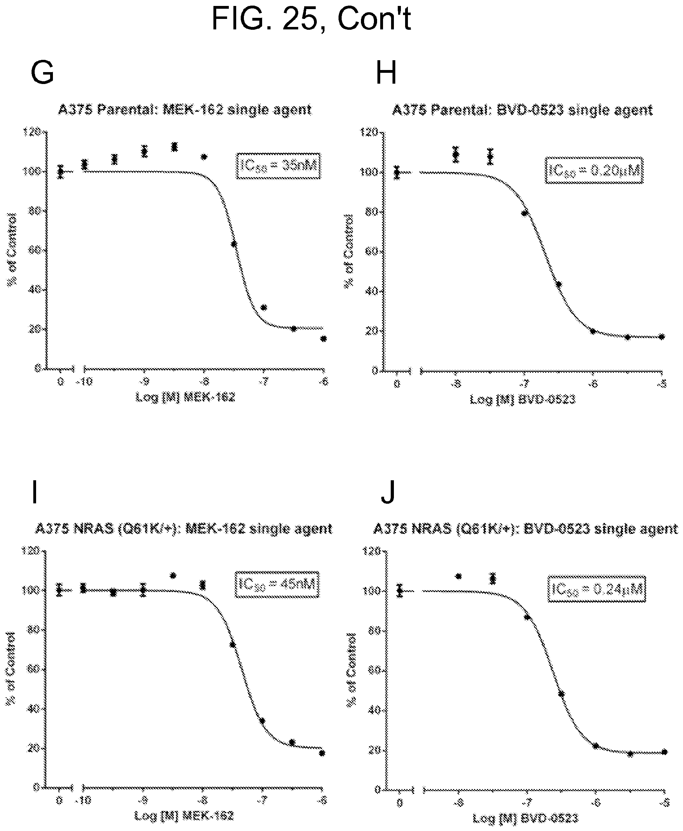

FIG. 25 shows the results of the combination of BVD-523 and MEK-162 in parental A375 and A375 NRAS (Q61K/+) cells. FIG. 25A shows a dose matrix showing inhibition (%) for the combination in parental A375 cells. FIG. 25B shows Loewe excess for the combination in 25A and FIG. 25C shows Bliss excess for the combination in 25A. FIG. 25D shows a dose matrix showing inhibition (%) for the combination in A375 NRAS (Q61K/+) cells. FIG. 25E shows Loewe excess for the combination in 25D and FIG. 25F shows Bliss excess for the combination in 25D. FIG. 25G - FIG. 25H show the results of single agent proliferation assays for the combination in 25A. FIG. 25I - FIG. 25J show the results of single agent proliferation assays for the combination in 25D.

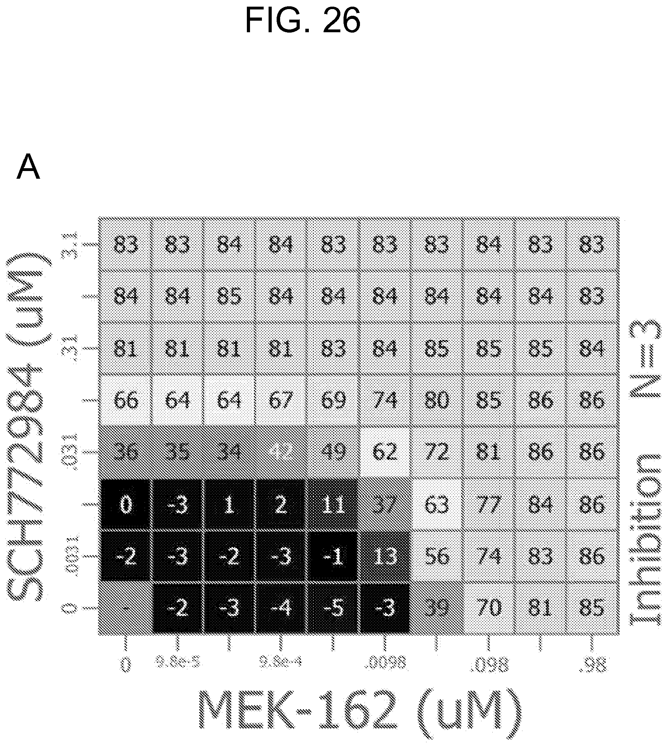

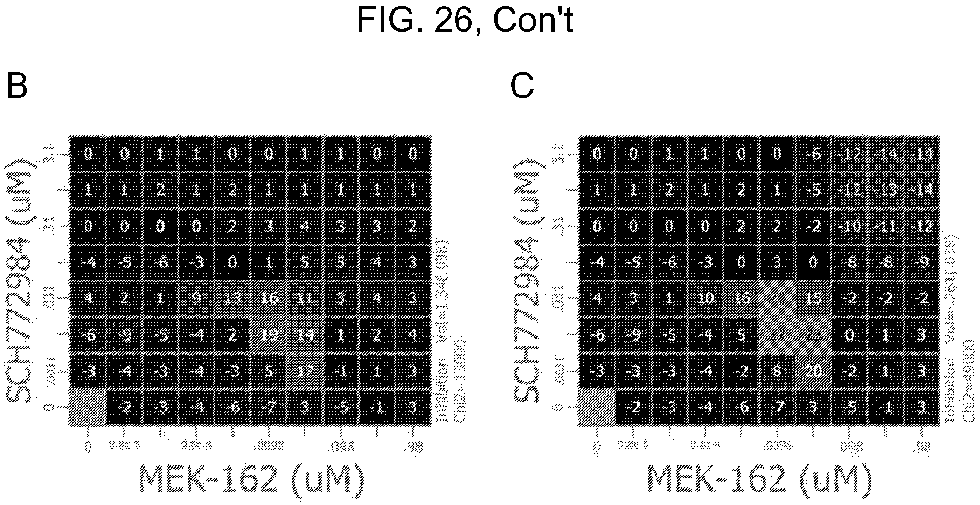

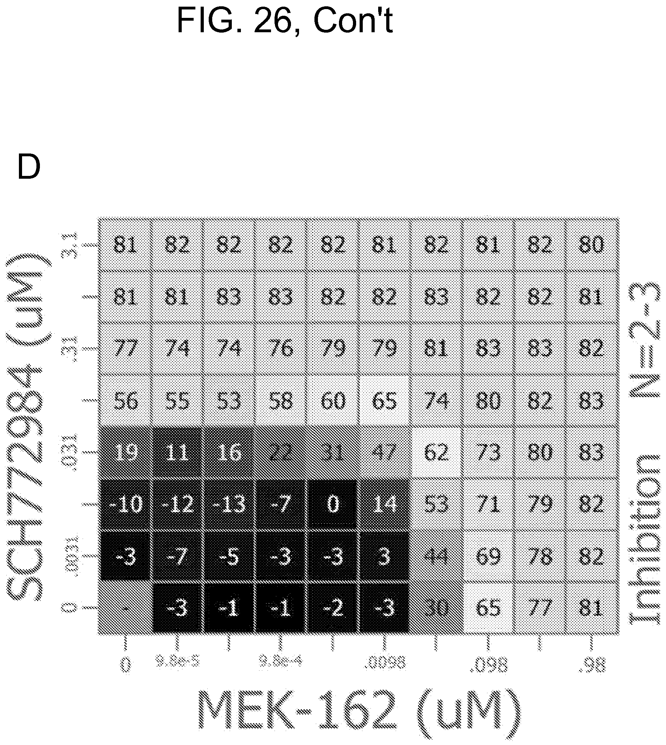

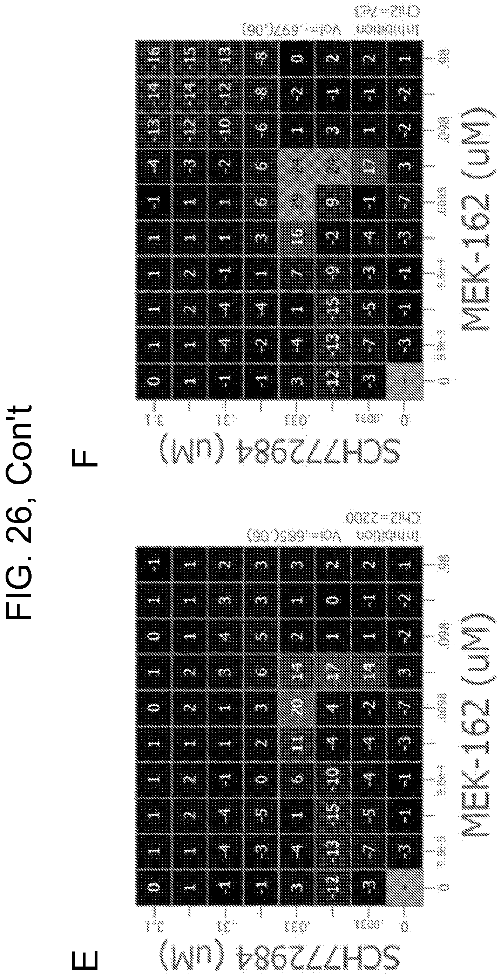

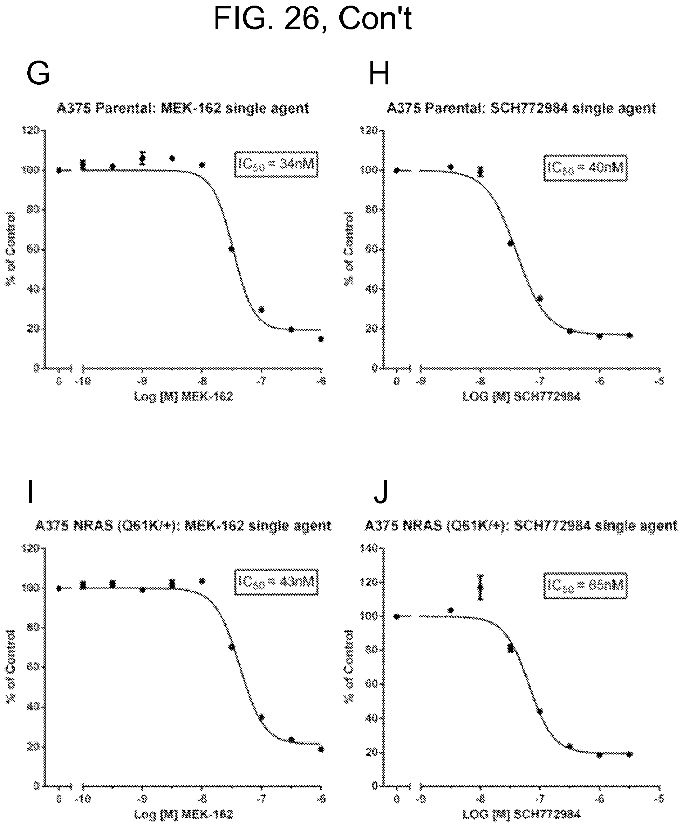

FIG. 26 shows the results of the combination of SCH772984 and MEK-162 in parental A375 and A375 NRAS (Q61K/+) cells. FIG. 26A shows a dose matrix showing inhibition (%) for the combination in parental A375 cells. FIG. 26B shows Loewe excess for the combination in 26A and FIG. 26C shows Bliss excess for the combination in 26A. FIG. 26D shows a dose matrix showing inhibition (%) for the combination in A375 NRAS (Q61K/+) cells. FIG. 26E shows Loewe excess for the combination in 26D and FIG. 26F shows Bliss excess for the combination in 26D. FIG. 26G - FIG. 26H show the results of single agent proliferation assays for the combination in 26A. FIG. 26I - FIG. 26J show the results of single agent proliferation assays for the combination in 26D.

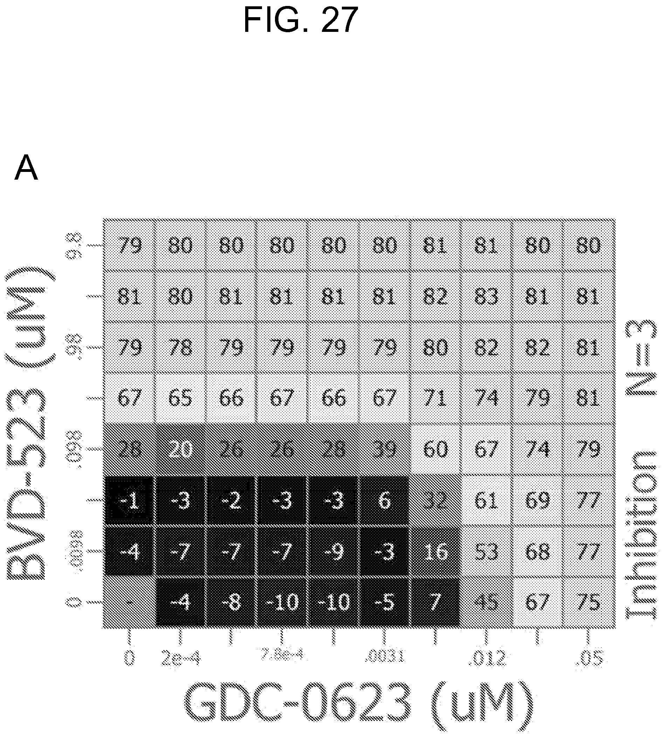

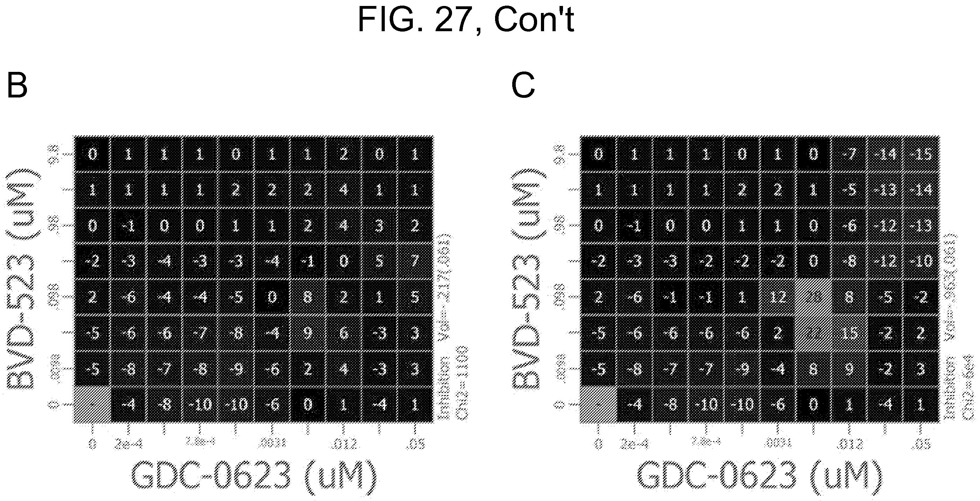

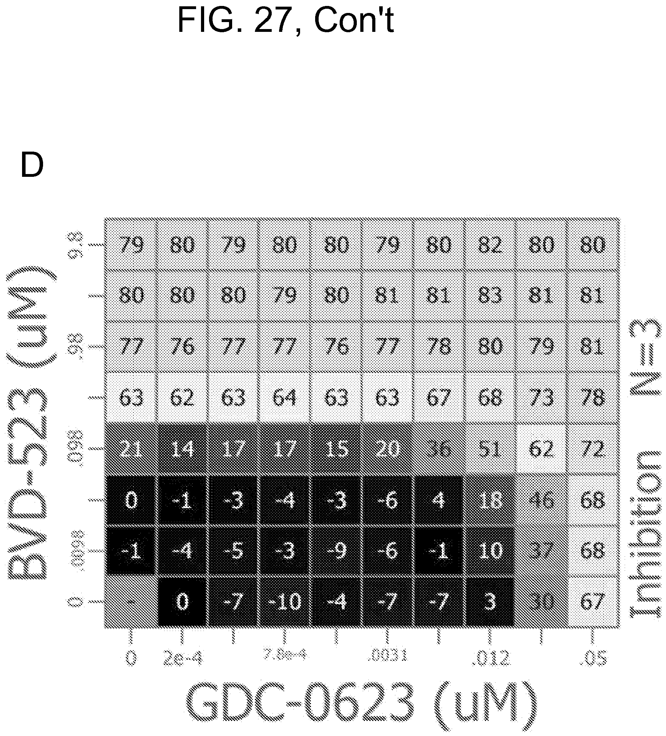

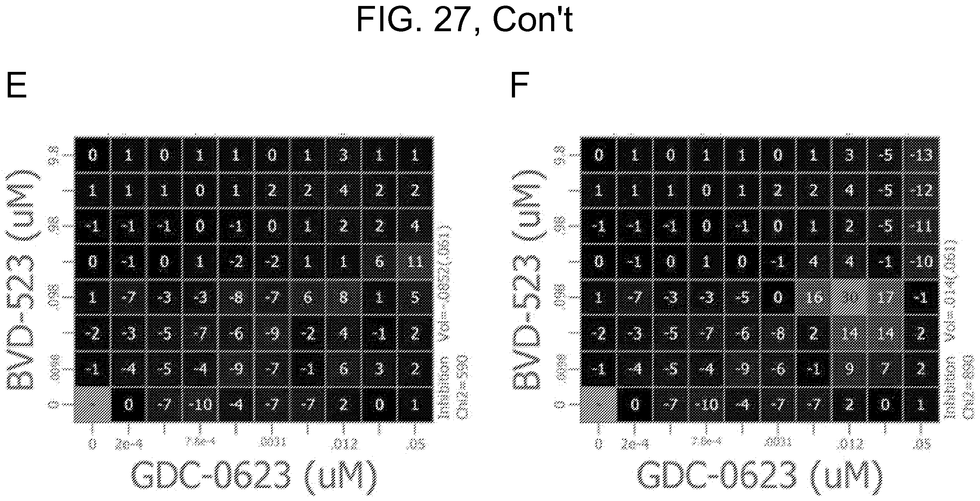

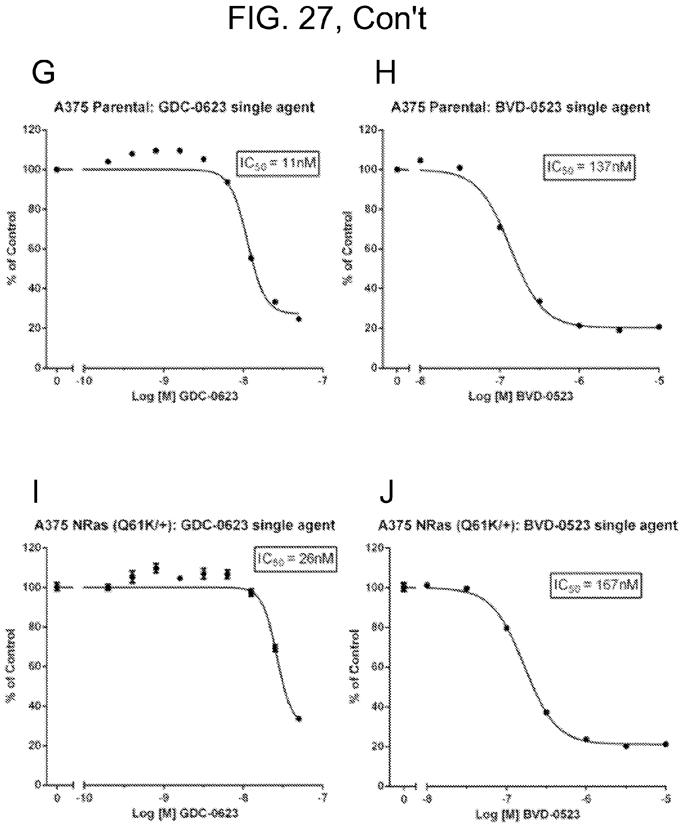

FIG. 27 shows the results of the combination of BVD-523 and GDC-0623 in parental A375 and A375 NRAS (Q61K/+) cells. FIG. 27A shows a dose matrix showing inhibition (%) for the combination in parental A375 cells. FIG. 27B shows Loewe excess for the combination in 27A and FIG. 27C shows Bliss excess for the combination in 27A. FIG. 27D shows a dose matrix showing inhibition (%) for the combination in A375 NRAS (Q61K/+) cells. FIG. 27E shows Loewe excess for the combination in 27D and FIG. 27F shows Bliss excess for the combination in 27D. FIG. 27G - FIG. 27H show the results of single agent proliferation assays for the combination in 27A. FIG. 27I - FIG. 27J show the results of single agent proliferation assays for the combination in 27D.

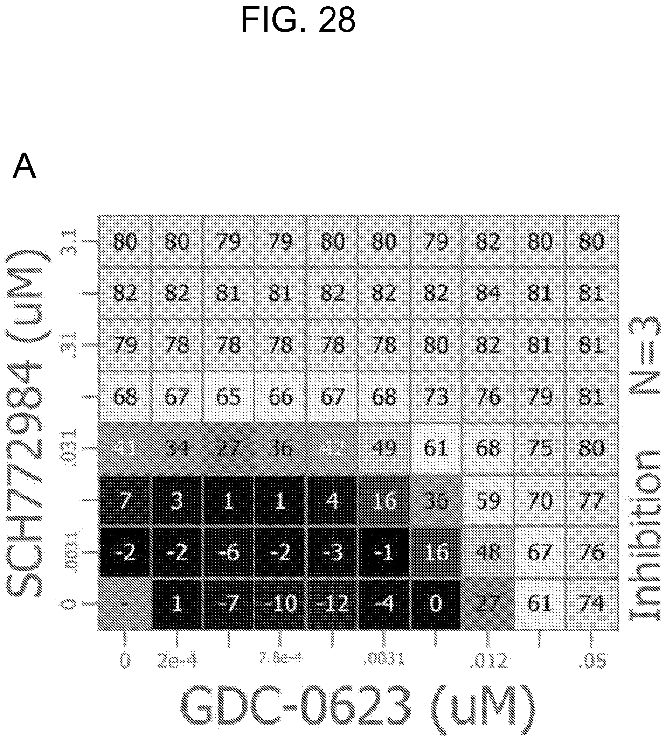

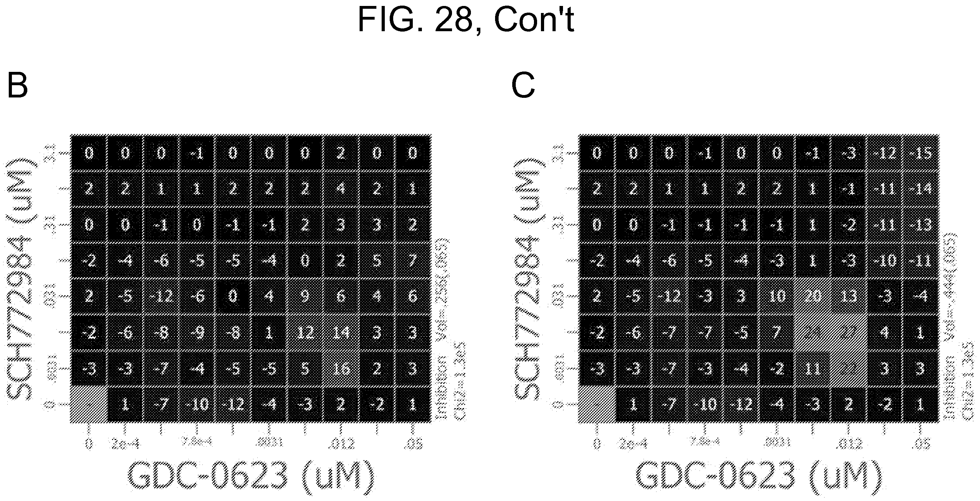

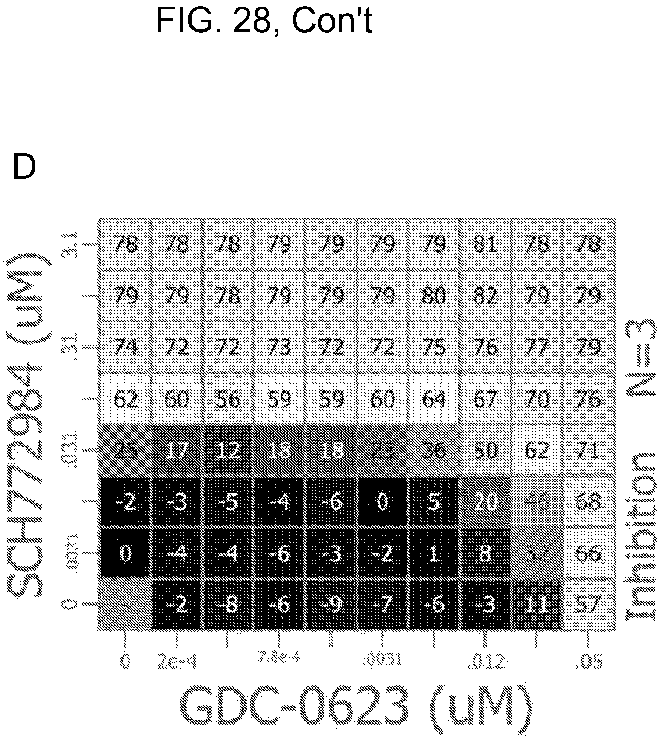

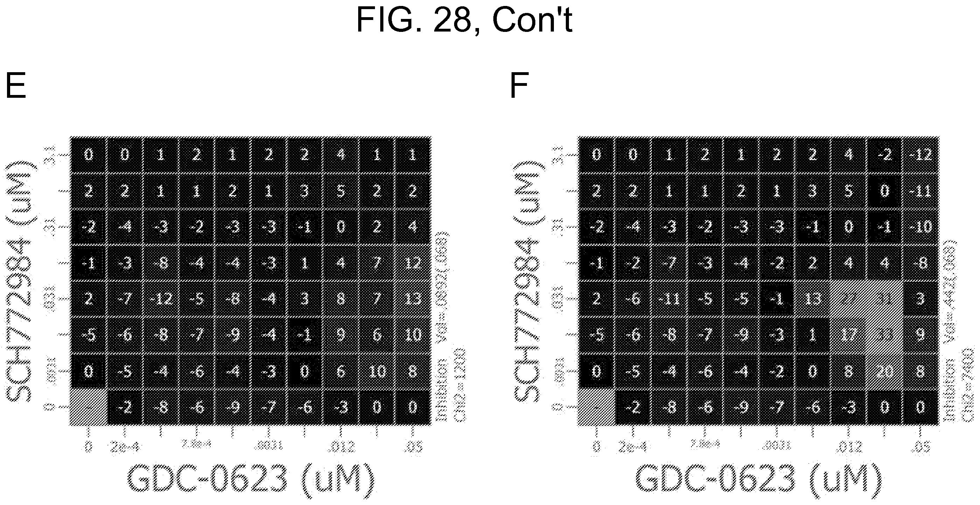

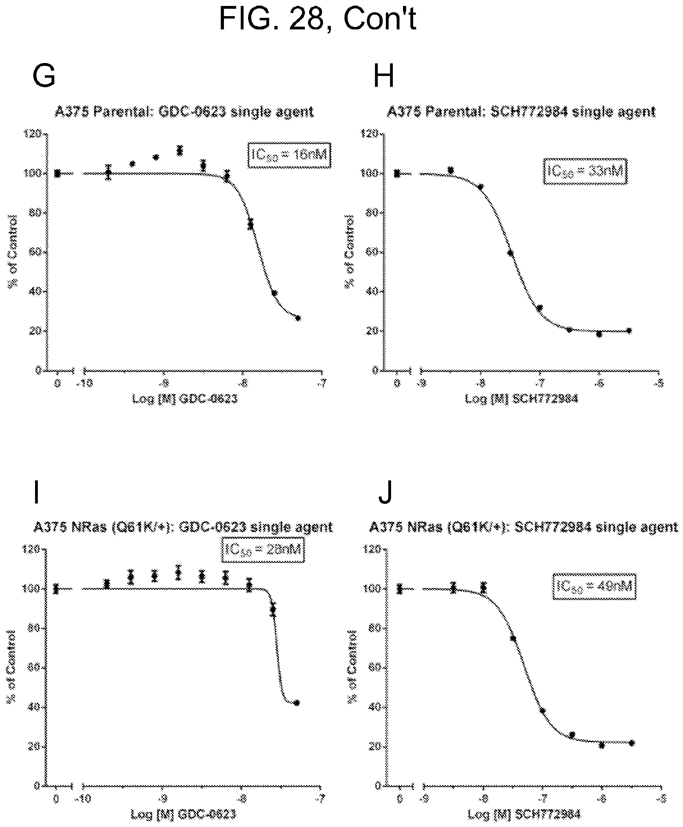

FIG. 28 shows the results of the combination of SCH772984 and GDC-0623 in parental A375 and A375 NRAS (Q61K/+) cells. FIG. 28A shows a dose matrix showing inhibition (%) for the combination in parental A375 cells. FIG. 28B shows Loewe excess for the combination in 28A and FIG. 28C shows Bliss excess for the combination in 28A. FIG. 28D shows a dose matrix showing inhibition (%) for the combination in A375 NRAS (Q61K/+) cells. FIG. 28E shows Loewe excess for the combination in 28D and FIG. 28F shows Bliss excess for the combination in 28D. FIG. 28G - FIG. 28H show the results of single agent proliferation assays for the combination in 28A. FIG. 28I - FIG. 28J show the results of single agent proliferation assays for the combination in 28D.

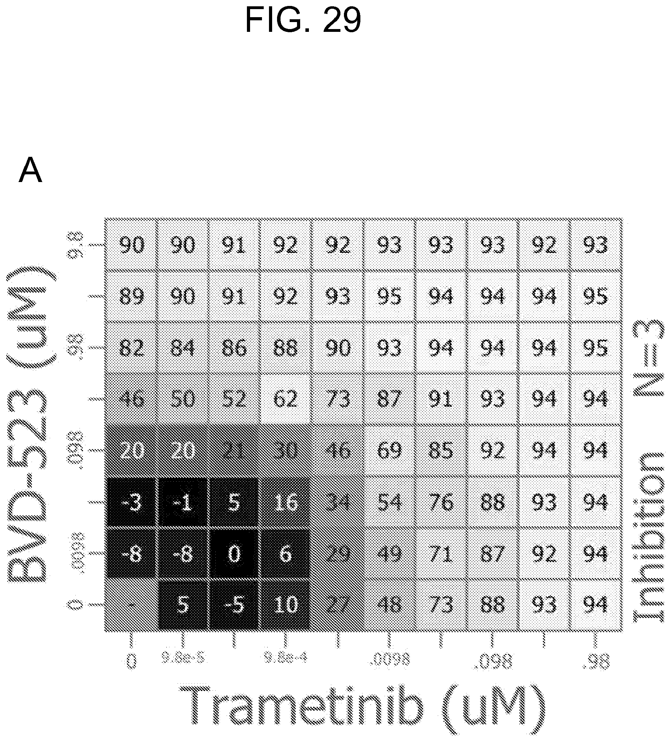

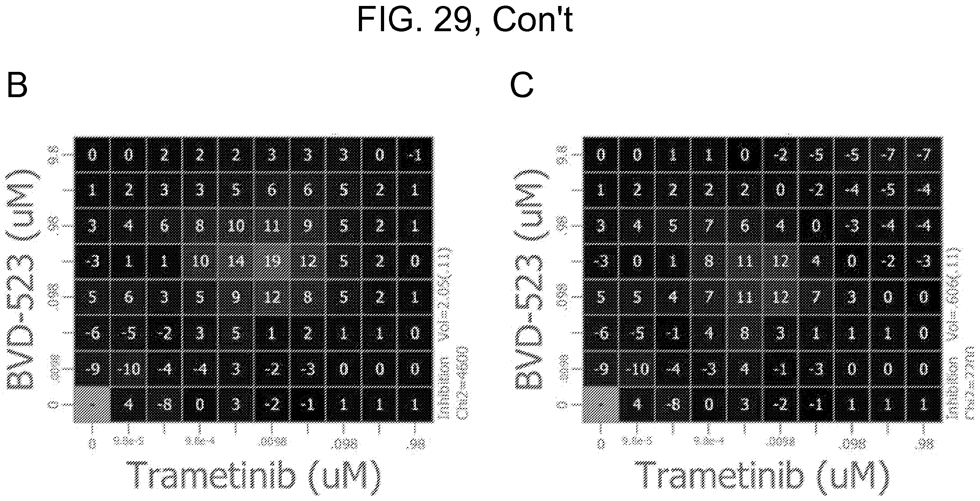

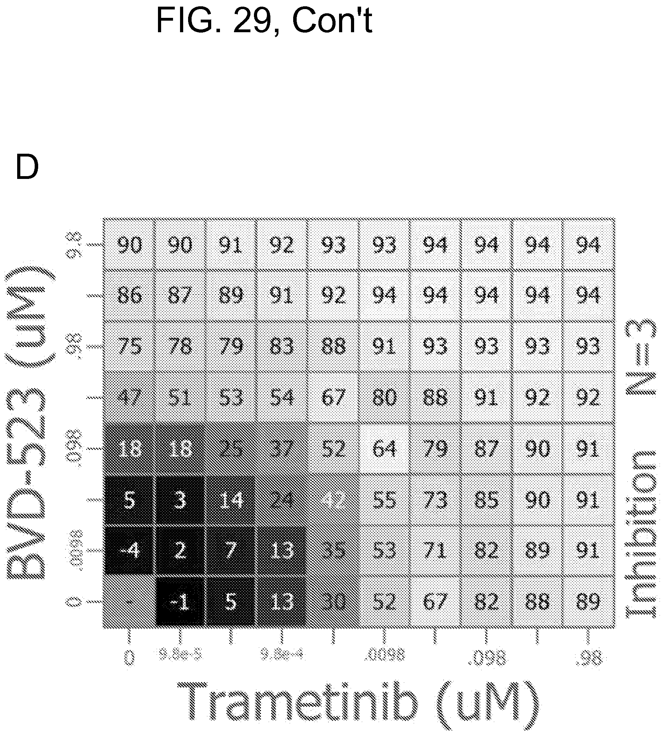

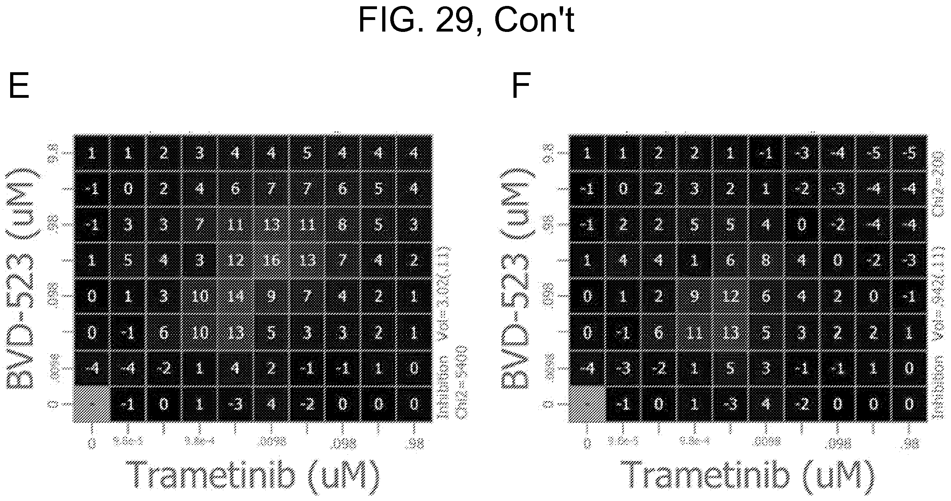

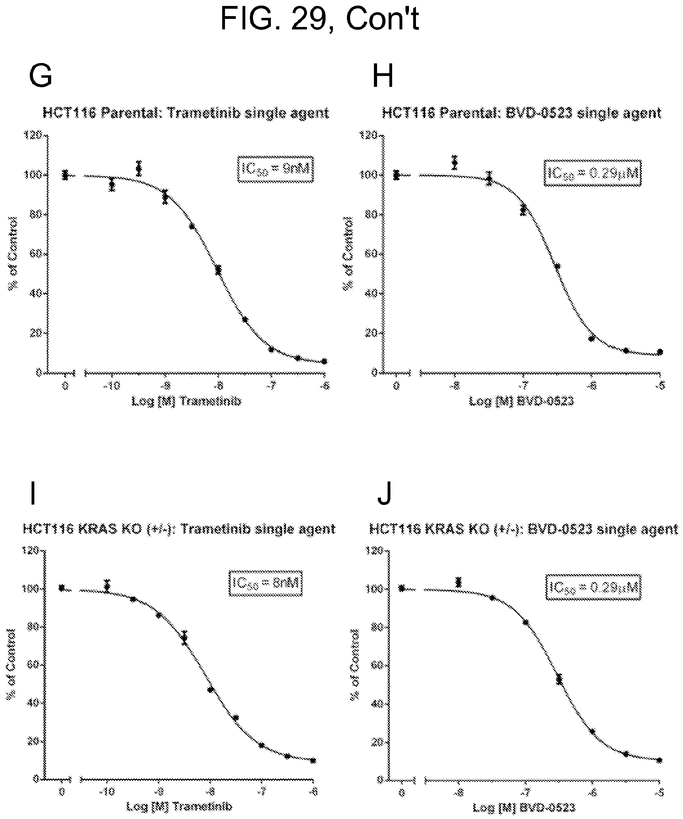

FIG. 29 shows the results of the combination of BVD-523 and Trametinib in parental HCT116 and HCT116 KRAS KO (+/-) cells. FIG. 29A shows a dose matrix showing inhibition (%) for the combination in parental HCT116 cells. FIG. 29B shows Loewe excess for the combination in 29A and FIG. 29C shows Bliss excess for the combination in 29A. FIG. 29D shows a dose matrix showing inhibition (%) for the combination in HCT116 KRAS KO (+/-) cells. FIG. 29E shows Loewe excess for the combination in 29D and FIG. 29F shows Bliss excess for the combination in 29D. FIG. 29G - FIG.29H show the results of single agent proliferation assays for the combination in 29A. FIG. 29I - FIG. 29J show the results of single agent proliferation assays for the combination in 29D.

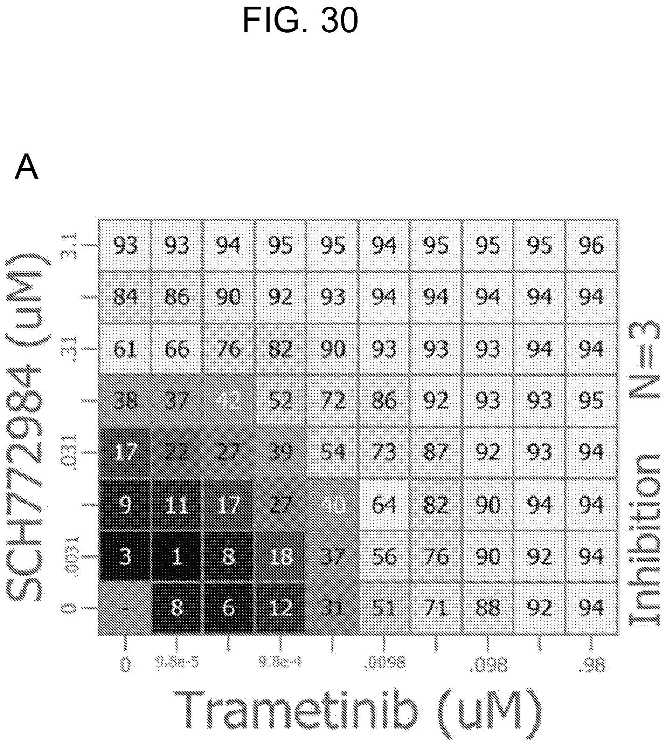

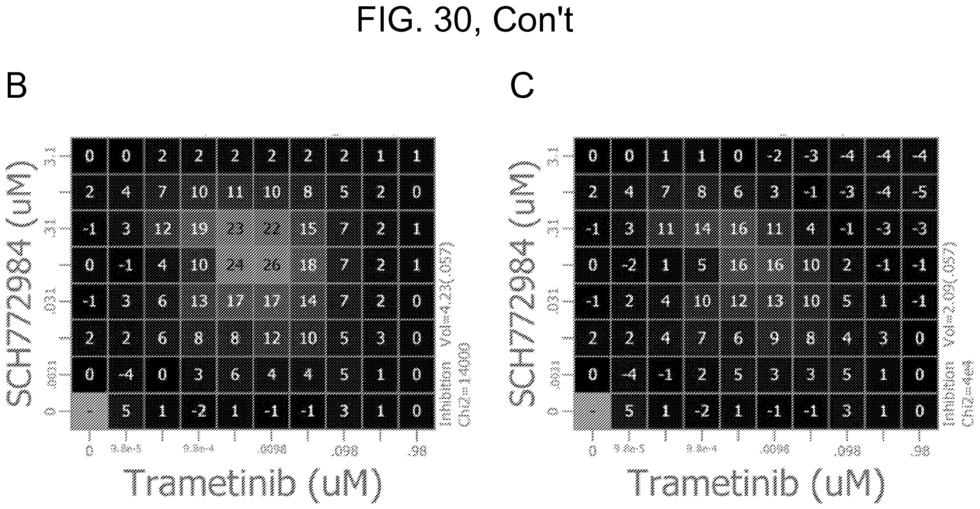

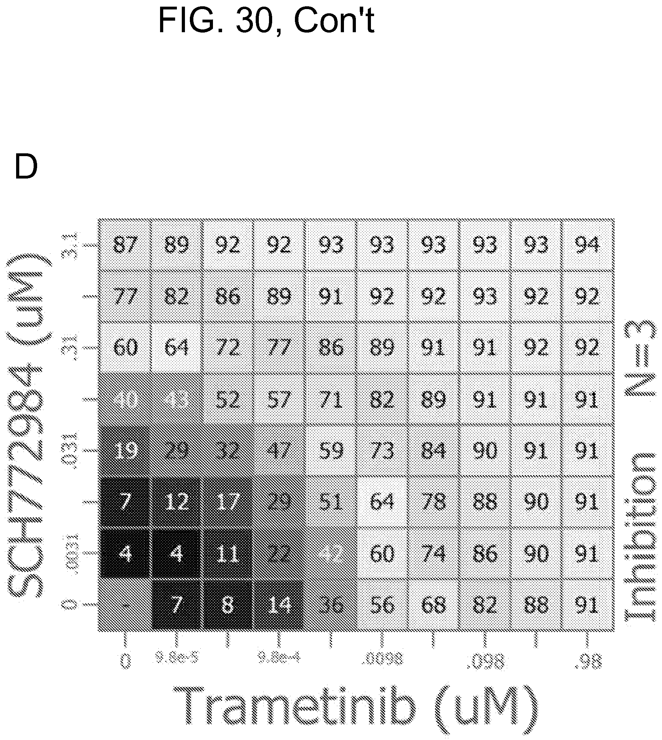

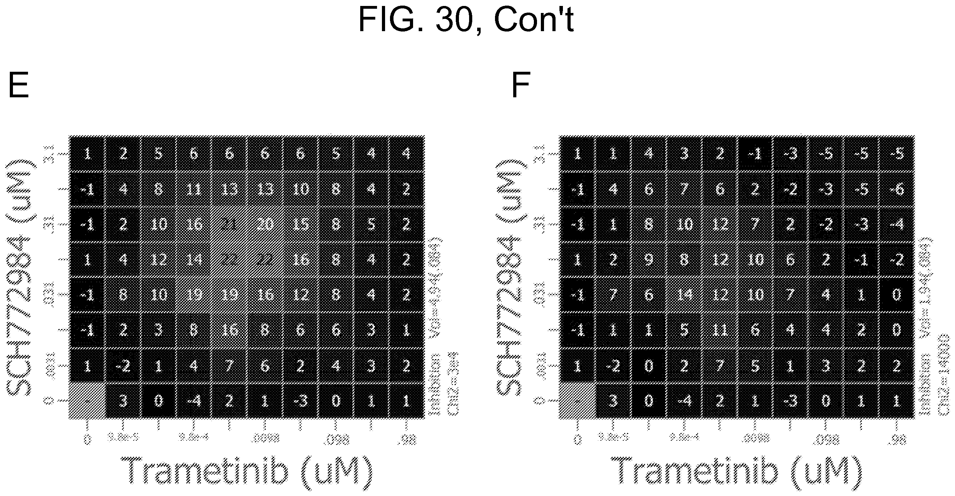

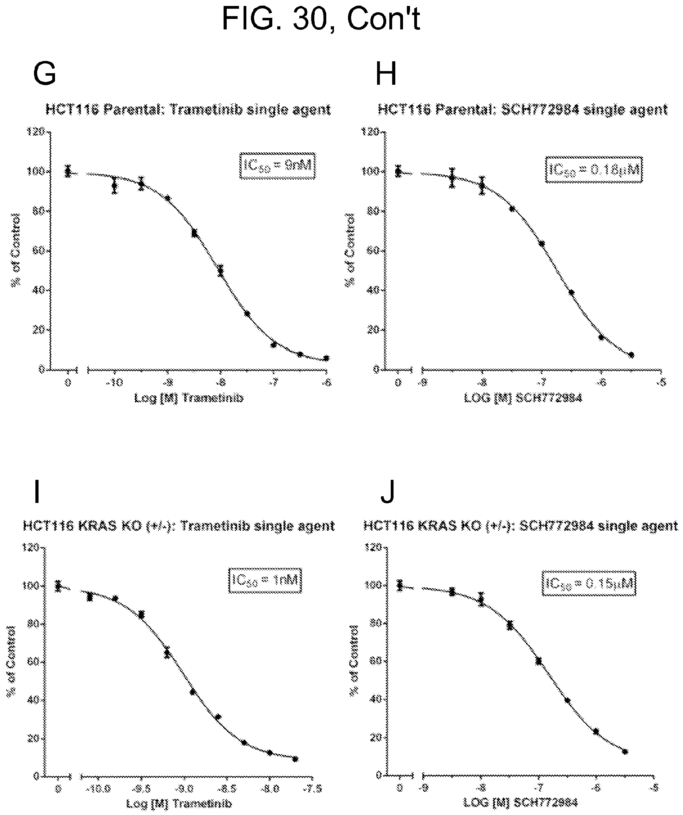

FIG. 30 shows the results of the combination of SCH772984 and Trametinib in parental HCT116 and HCT116 KRAS KO (+/-) cells. FIG. 30A shows a dose matrix showing inhibition (%) for the combination in parental HCT116 cells. FIG. 30B shows Loewe excess for the combination in 30A and FIG. 30C shows Bliss excess for the combination in 30A. FIG. 30D shows a dose matrix showing inhibition (%) for the combination in HCT116 KRAS KO (+/-) cells. FIG. 30E shows Loewe excess for the combination in 30D and FIG. 30F shows Bliss excess for the combination in 30D. FIG. 30G - FIG.30H show the results of single agent proliferation assays for the combination in 30A. FIG. 30I - FIG. 30J show the results of single agent proliferation assays for the combination in 30D.

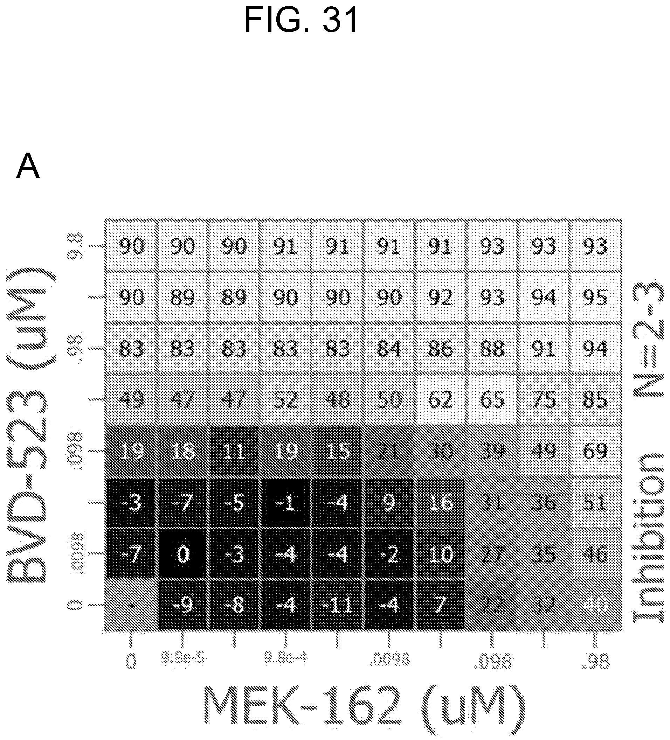

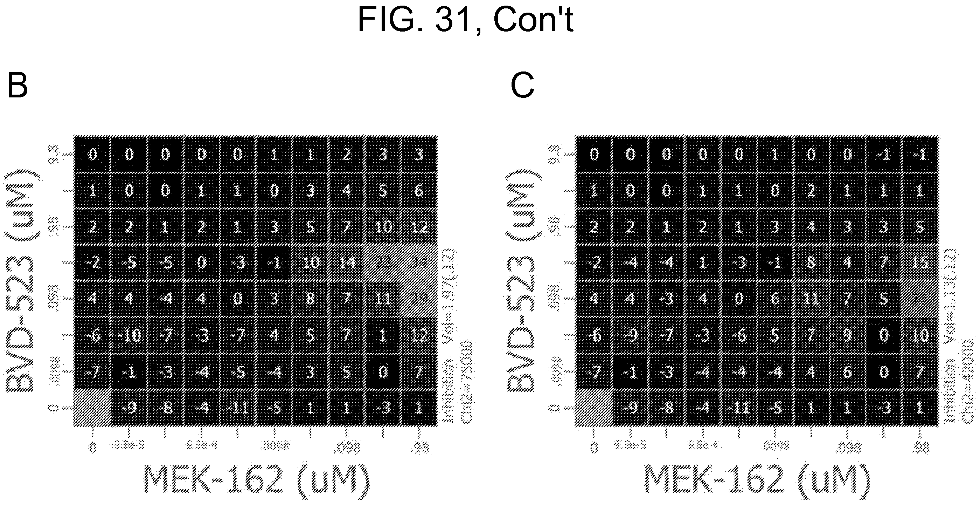

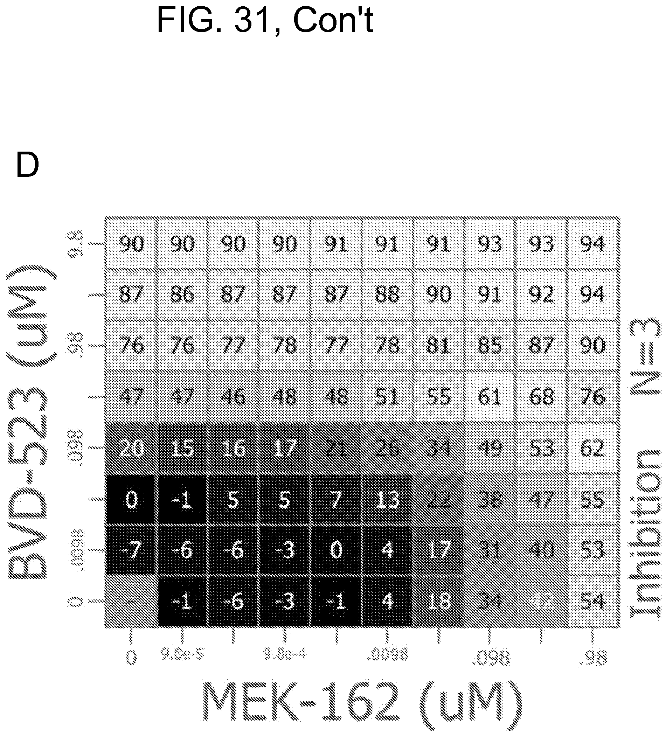

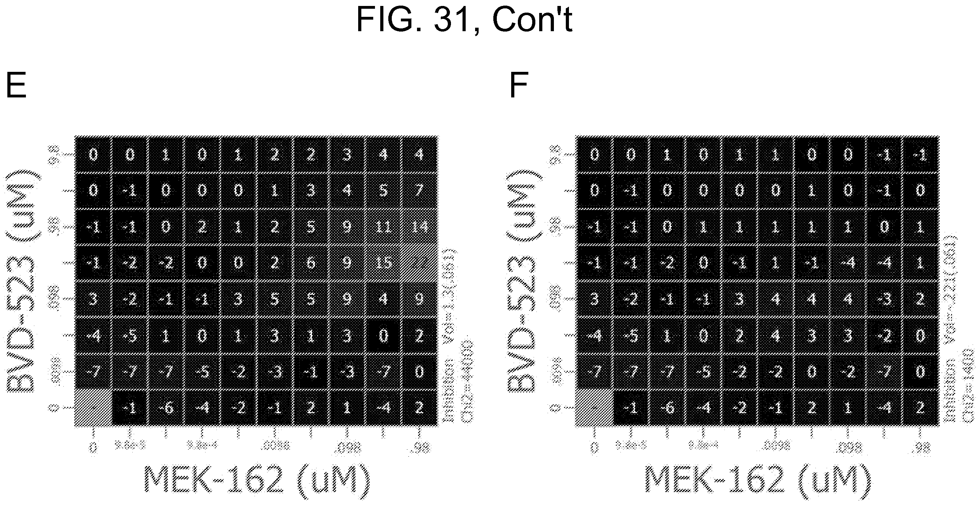

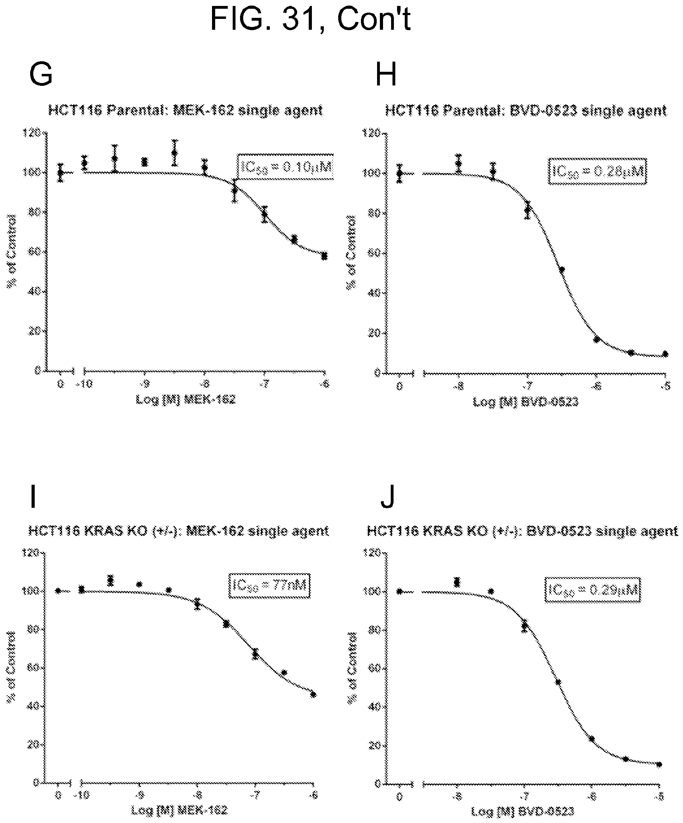

FIG. 31 shows the results of the combination of BVD-523 and MEK-162 in parental HCT116 and HCT116 KRAS KO (+/-) cells. FIG. 31A shows a dose matrix showing inhibition (%) for the combination in parental HCT116 cells. FIG. 31B shows Loewe excess for the combination in 31A and FIG. 31C shows Bliss excess for the combination in 31A. FIG. 31D shows a dose matrix showing inhibition (%) for the combination in HCT116 KRAS KO (+/-) cells. FIG. 31E shows Loewe excess for the combination in 31D and FIG. 31F shows Bliss excess for the combination in 31D. FIG. 31G - FIG.31H show the results of single agent proliferation assays for the combination in 31A. FIG. 31I - FIG. 31J show the results of single agent proliferation assays for the combination in 31D.

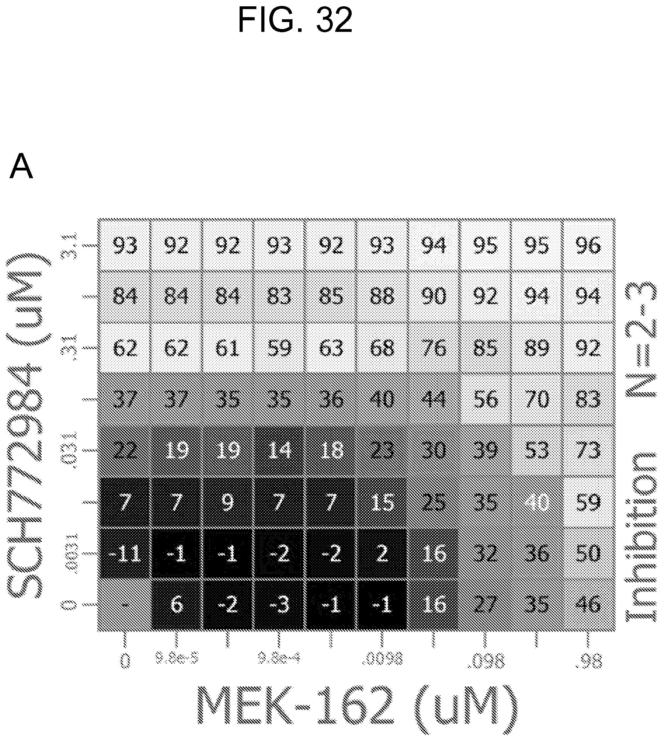

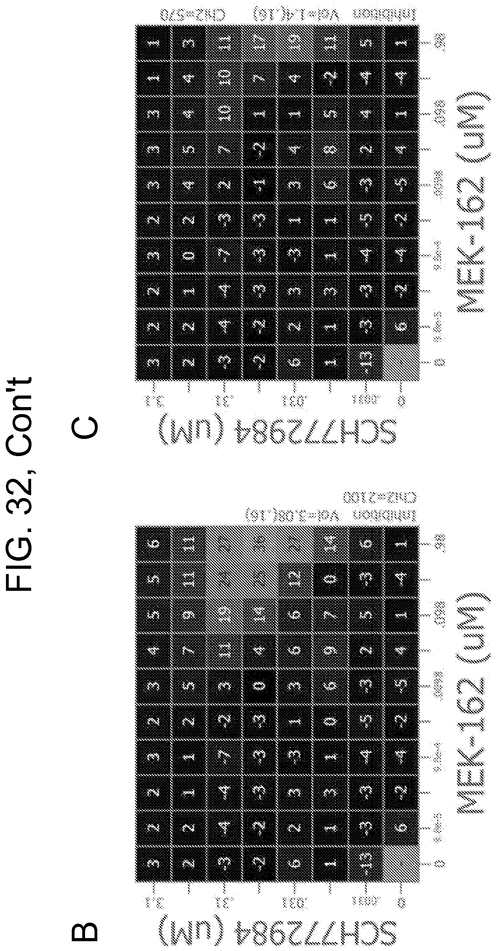

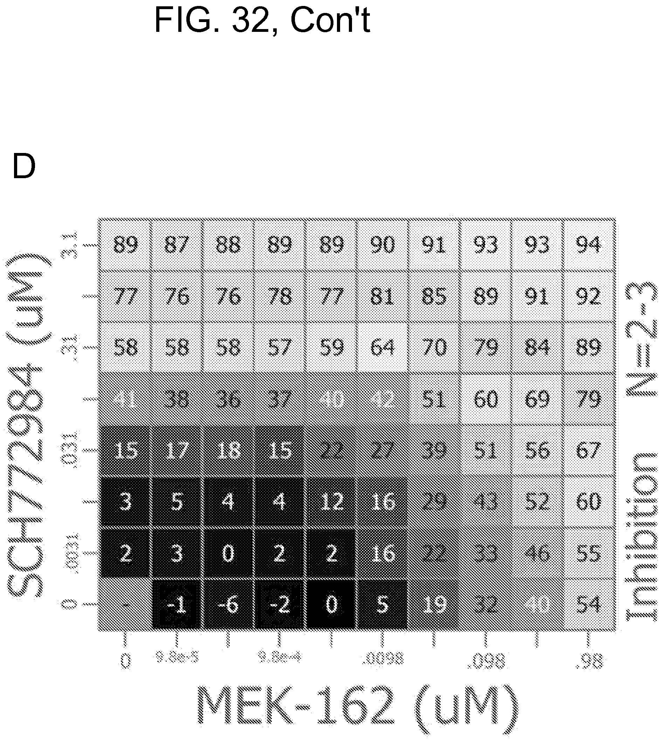

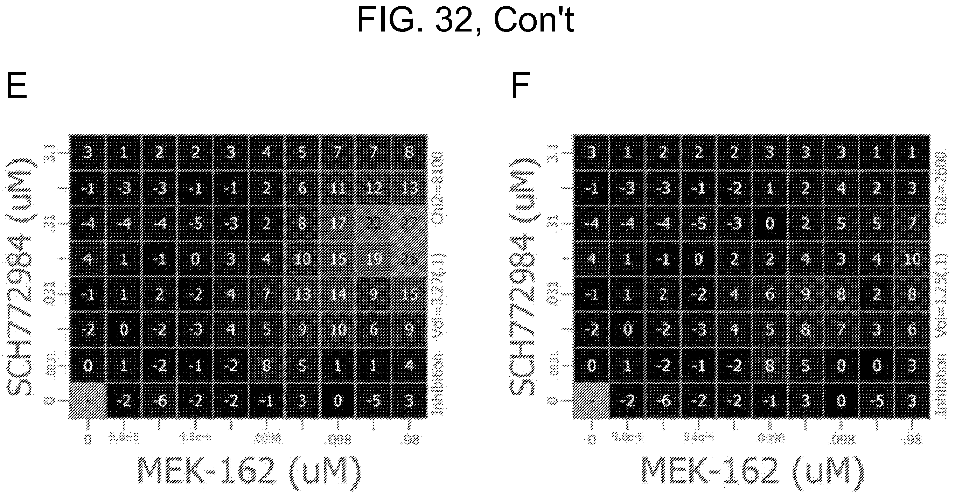

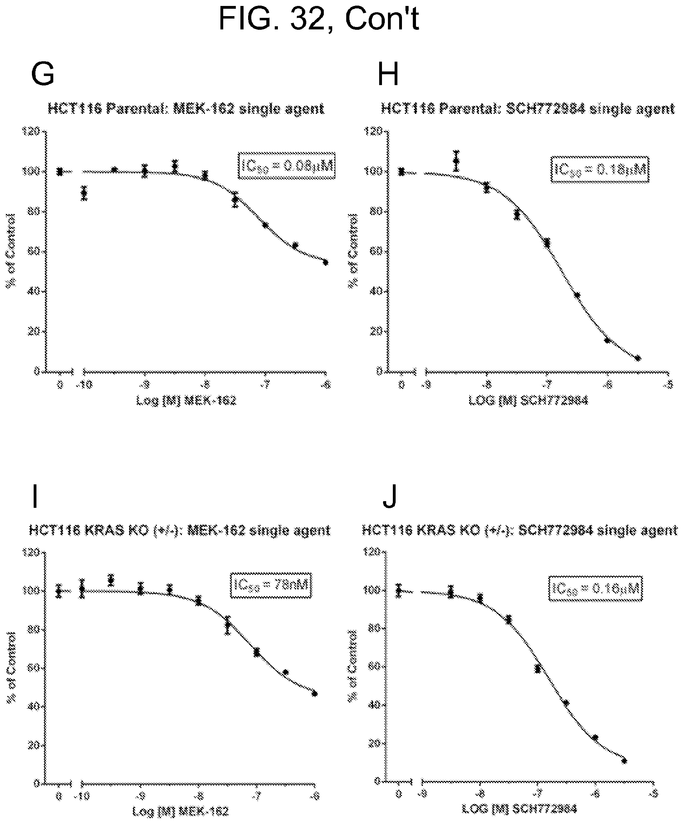

FIG. 32 shows the results of the combination of SCH772984 and MEK-162 in parental HCT116 and HCT116 KRAS KO (+/-) cells. FIG. 32A shows a dose matrix showing inhibition (%) for the combination in parental HCT116 cells. FIG. 32B shows Loewe excess for the combination in 32A and FIG. 32C shows Bliss excess for the combination in 32A. FIG. 32D shows a dose matrix showing inhibition (%) for the combination in HCT116 KRAS KO (+/-) cells. FIG. 32E shows Loewe excess for the combination in 32D and FIG. 32F shows Bliss excess for the combination in 32D. FIG. 32G - FIG. 32H show the results of single agent proliferation assays for the combination in 32A. FIG. 32I - FIG. 32J show the results of single agent proliferation assays for the combination in 32D.

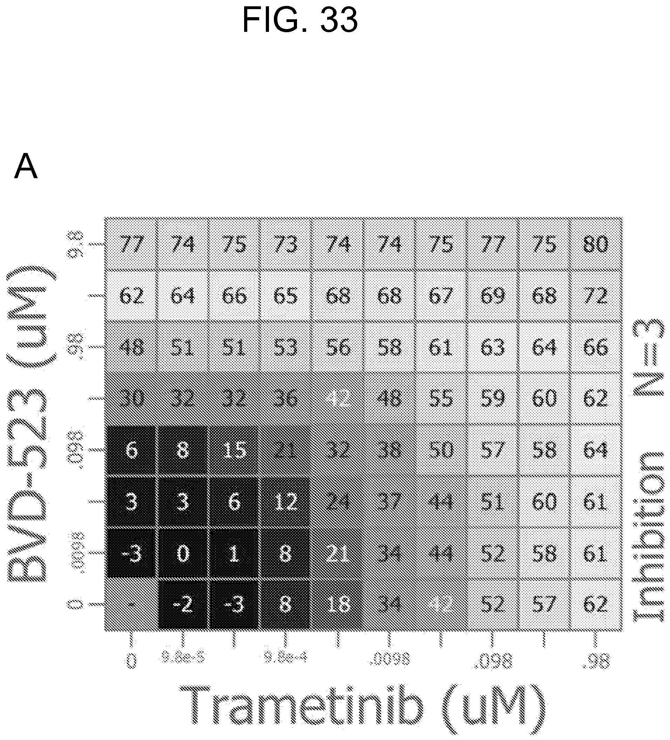

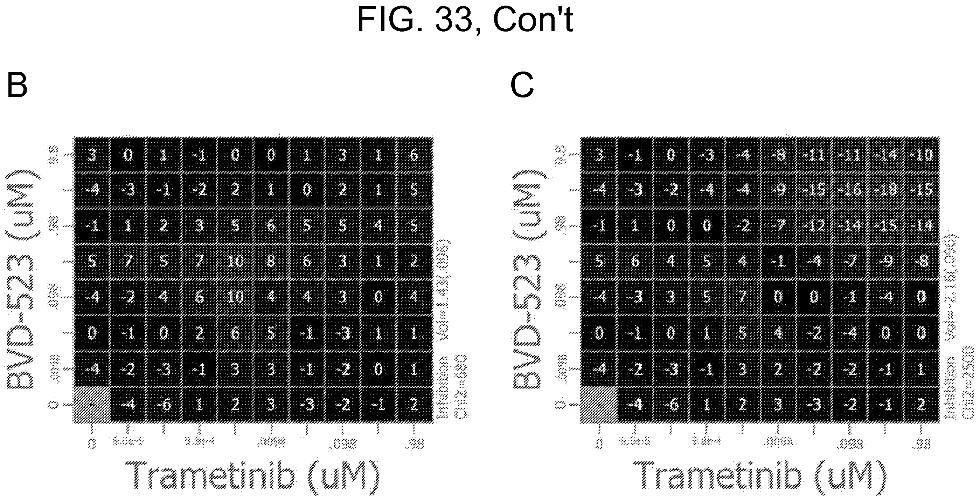

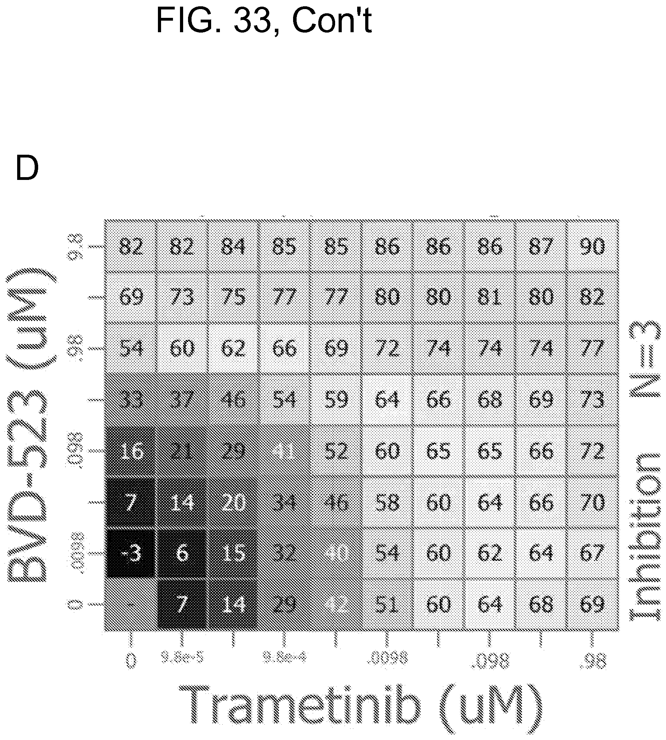

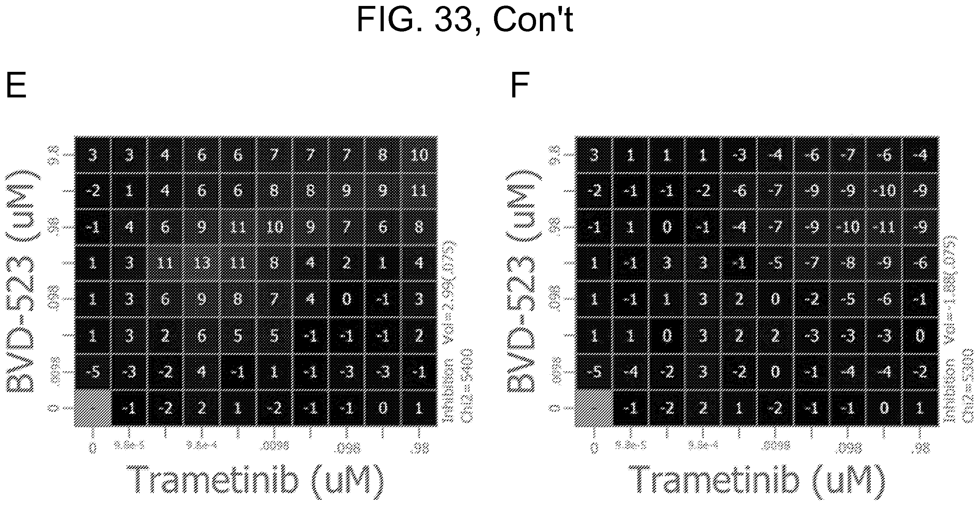

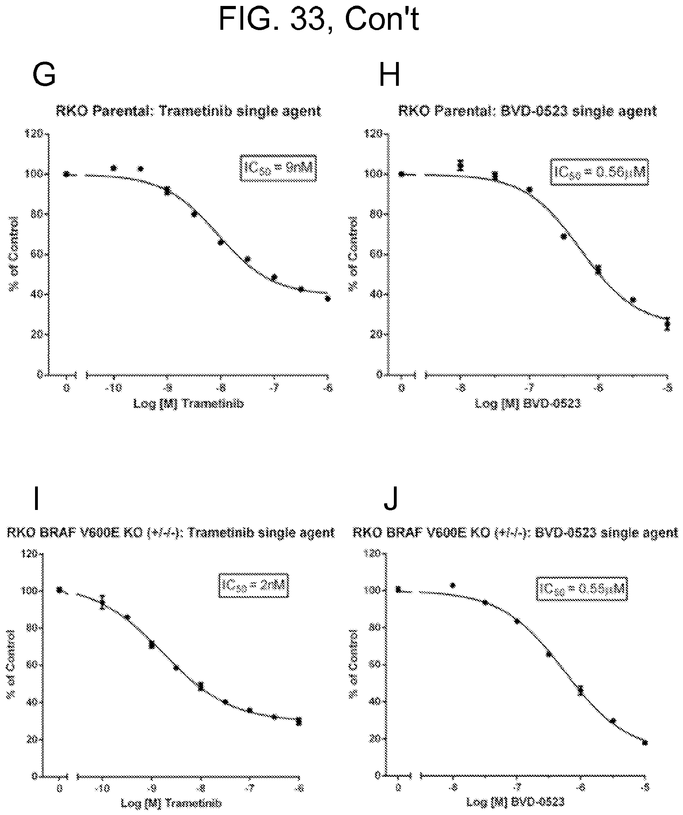

FIG. 33 shows the results of the combination of BVD-523 and Trametinib in parental RKO and RKO BRAF V600E KO (+/-/-) cells. FIG. 33A shows a dose matrix showing inhibition (%) for the combination in parental RKO cells. FIG. 33B shows Loewe excess for the combination in 33A and FIG. 33C shows Bliss excess for the combination in 33A. FIG. 33D shows a dose matrix showing inhibition (%) for the combination in RKO BRAF V600E KO (+/-/-) cells. FIG. 33E shows Loewe excess for the combination in 33D and FIG. 33F shows Bliss excess for the combination in 33D. FIG. 33G - FIG. 33H show the results of single agent proliferation assays for the combination in 33A. FIG. 33I - FIG. 33J show the results of single agent proliferation assays for the combination in 33D.

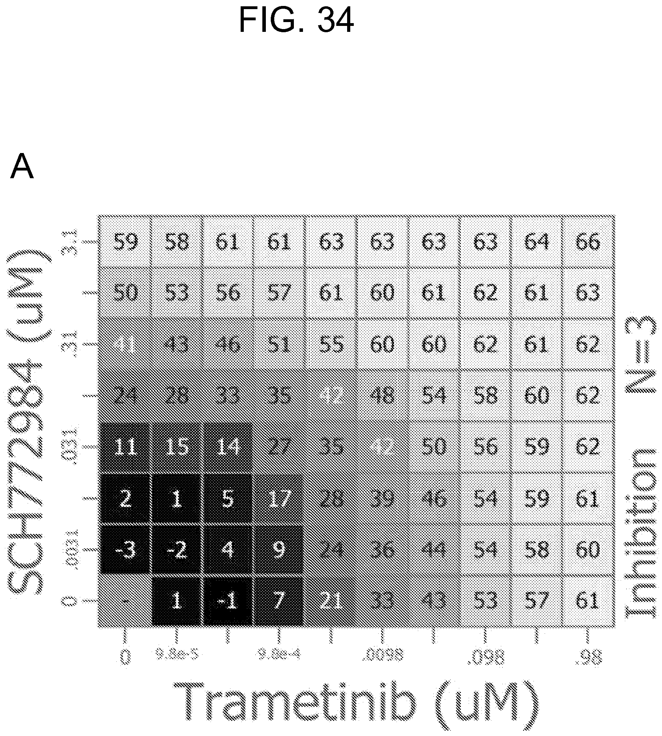

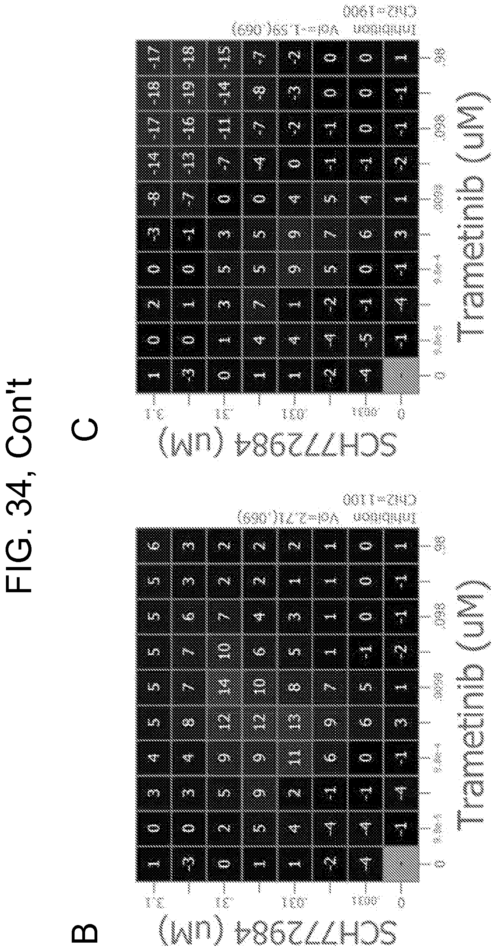

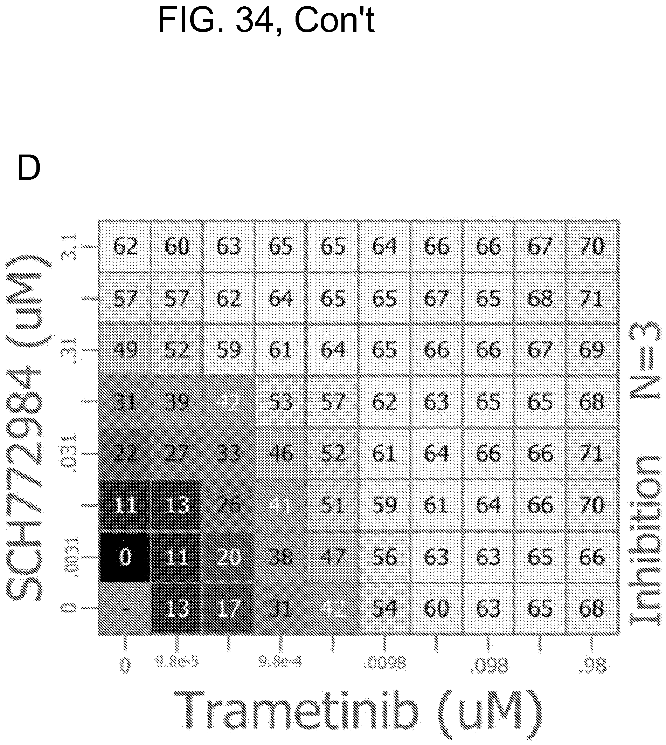

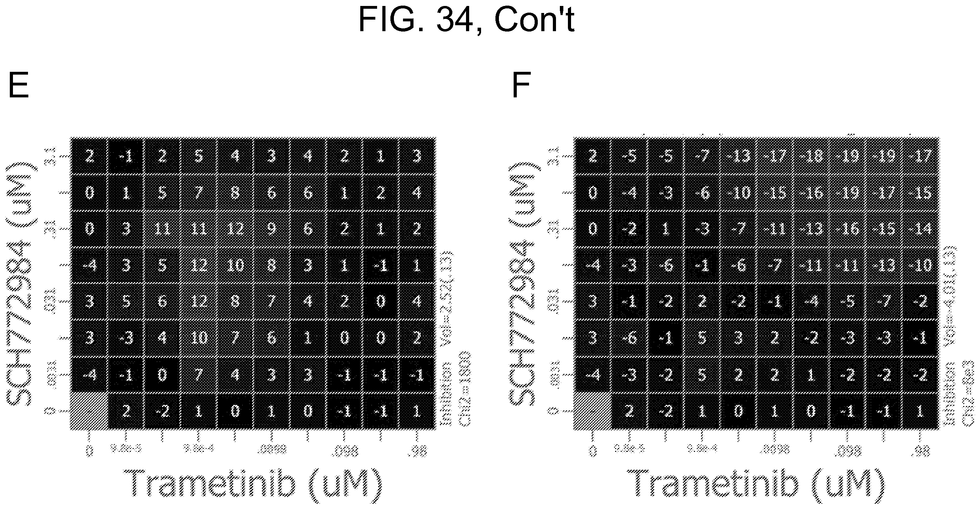

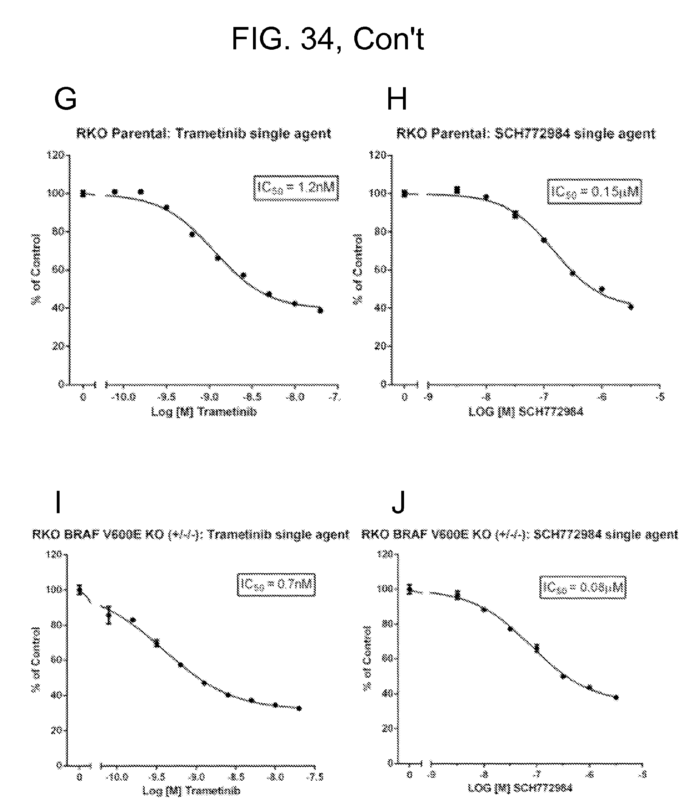

FIG. 34 shows the results of the combination of SCH772984 and Trametinib in parental RKO and RKO BRAF V600E KO (+/-/-) cells. FIG. 34A shows a dose matrix showing inhibition (%) for the combination in parental RKO cells. FIG. 34B shows Loewe excess for the combination in 34A and FIG. 34C shows Bliss excess for the combination in 34A. FIG. 34D shows a dose matrix showing inhibition (%) for the combination in RKO BRAF V600E KO (+/-/-) cells. FIG. 34E shows Loewe excess for the combination in 34D and FIG. 34F shows Bliss excess for the combination in 34D. FIG. 34G - FIG. 34H show the results of single agent proliferation assays for the combination in 34A. FIG. 34I - FIG. 34J show the results of single agent proliferation assays for the combination in 34D.

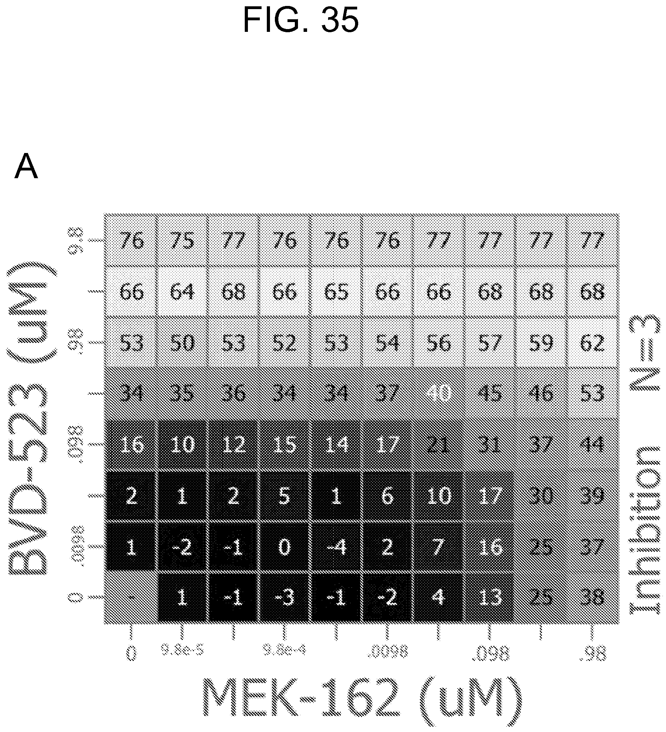

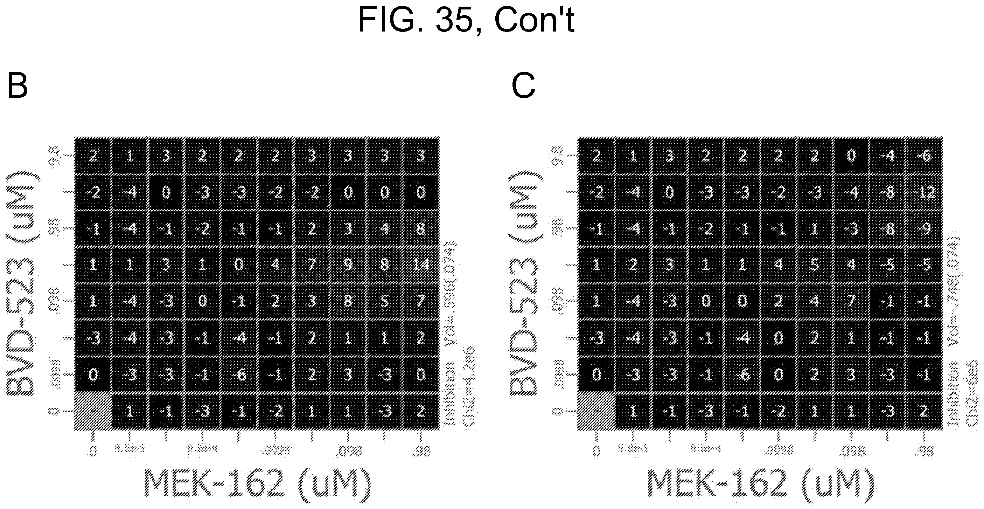

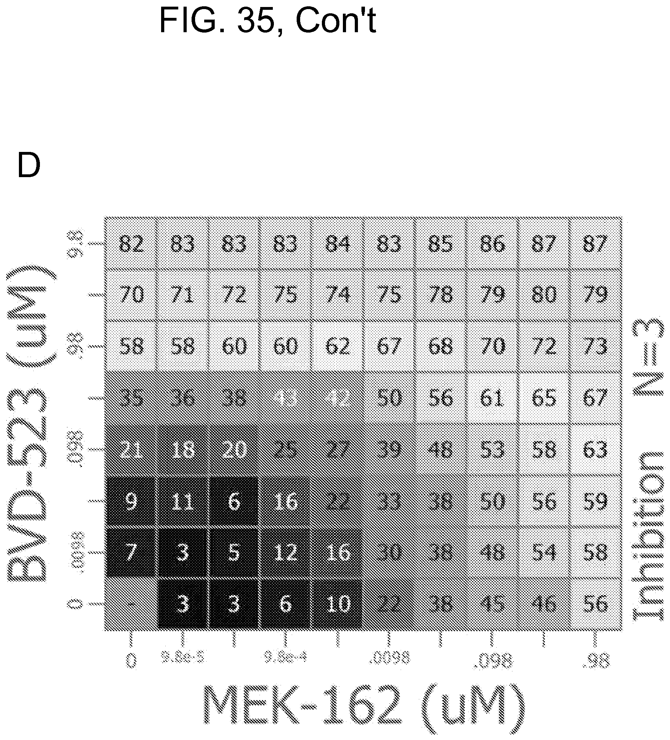

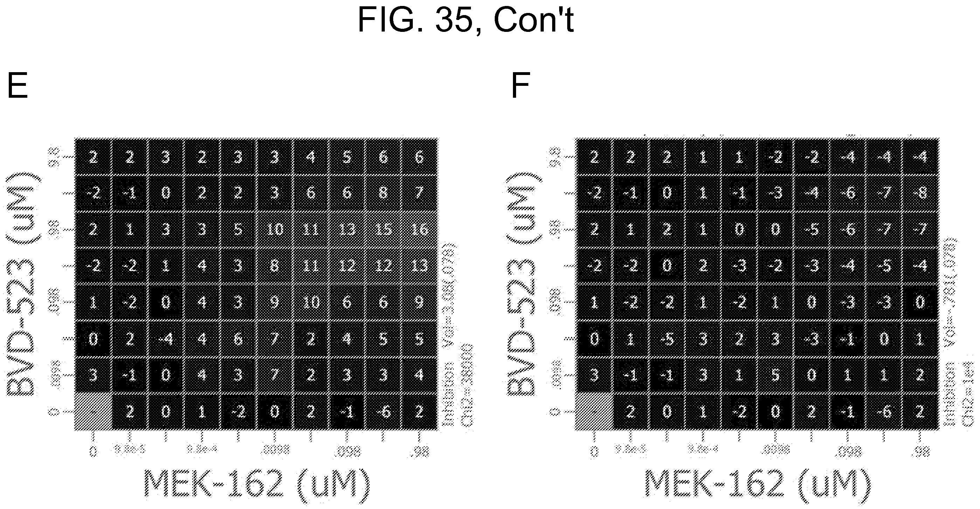

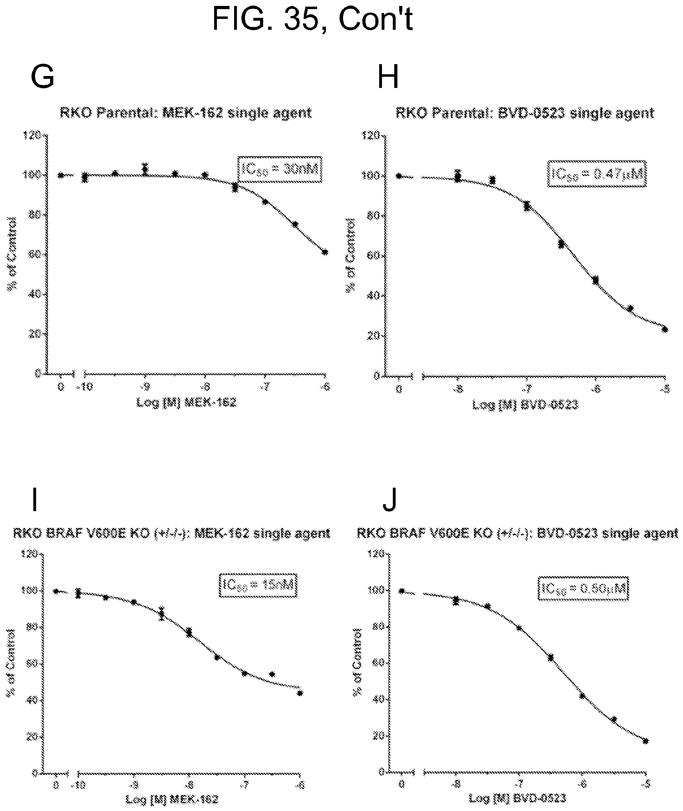

FIG. 35 shows the results of the combination of BVD-523 and MEK-162 in parental RKO and RKO BRAF V600E KO (+/-/-) cells. FIG. 35A shows a dose matrix showing inhibition (%) for the combination in parental RKO cells. FIG. 35B shows Loewe excess for the combination in 35A and FIG. 35C shows Bliss excess for the combination in 35A. FIG. 35D shows a dose matrix showing inhibition (%) for the combination in RKO BRAF V600E KO (+/-/-) cells. FIG. 35E shows Loewe excess for the combination in 35D and FIG. 35F shows Bliss excess for the combination in 35D. FIG. 35G - FIG. 35H show the results of single agent proliferation assays for the combination in 35A. FIG. 35I - FIG. 35J show the results of single agent proliferation assays for the combination in 35D.

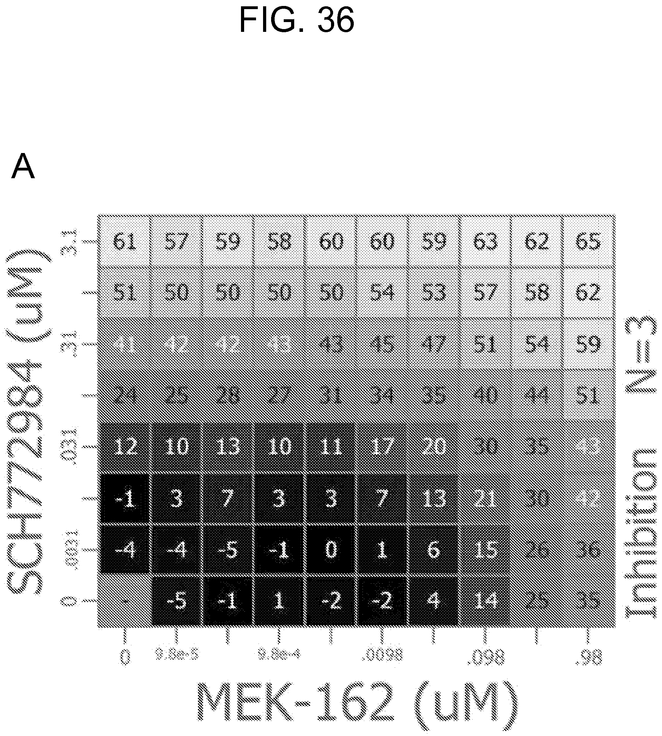

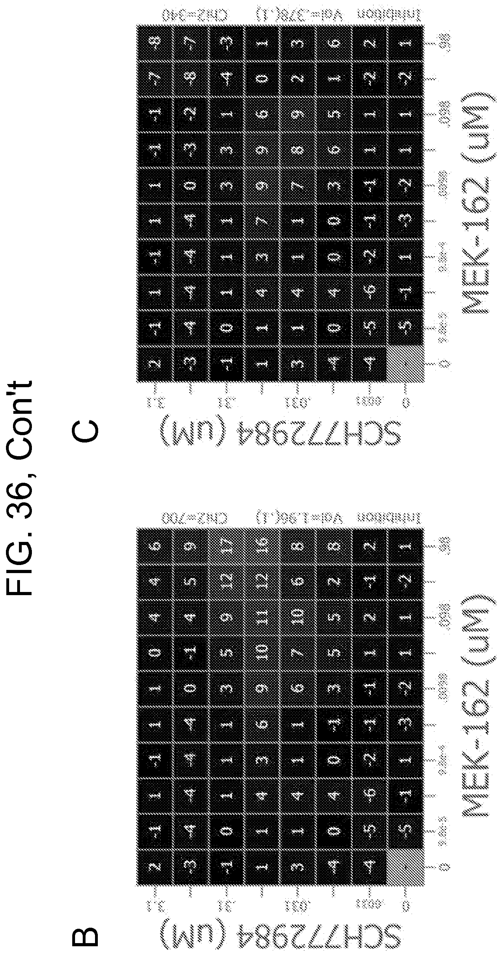

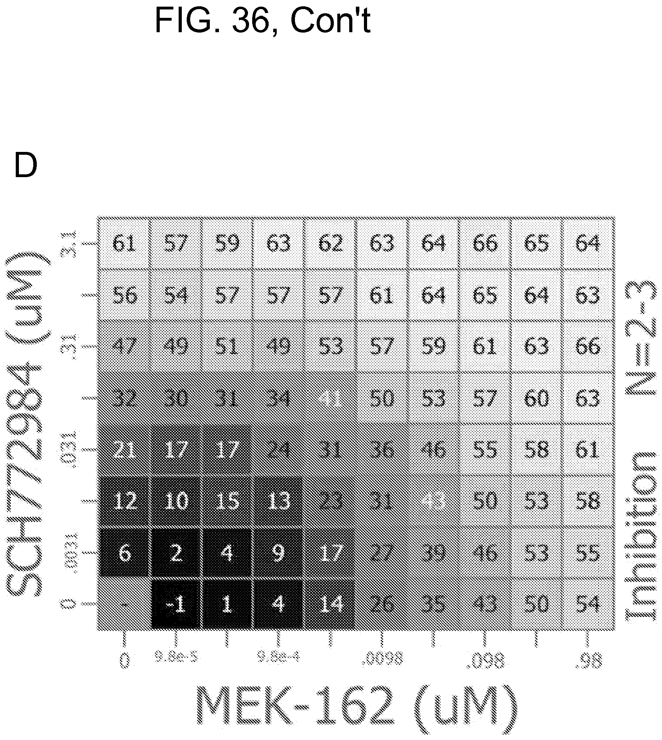

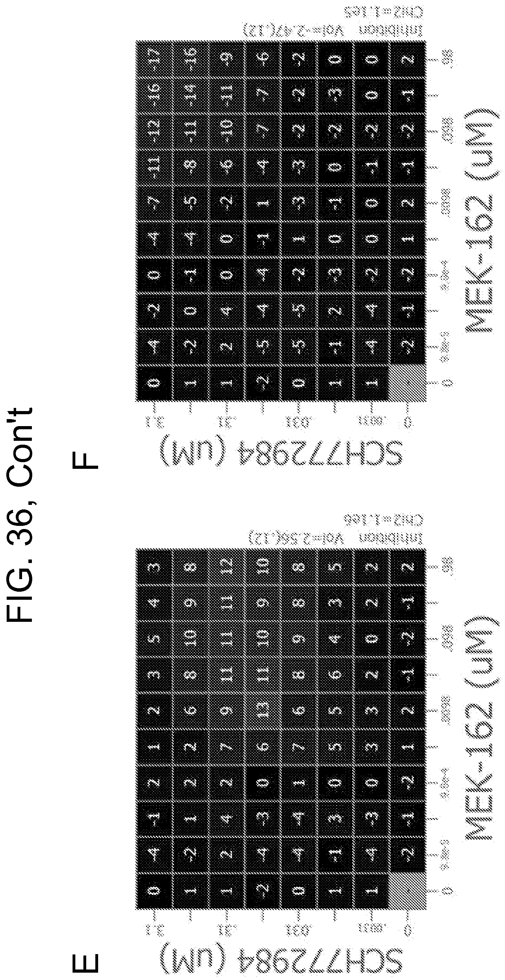

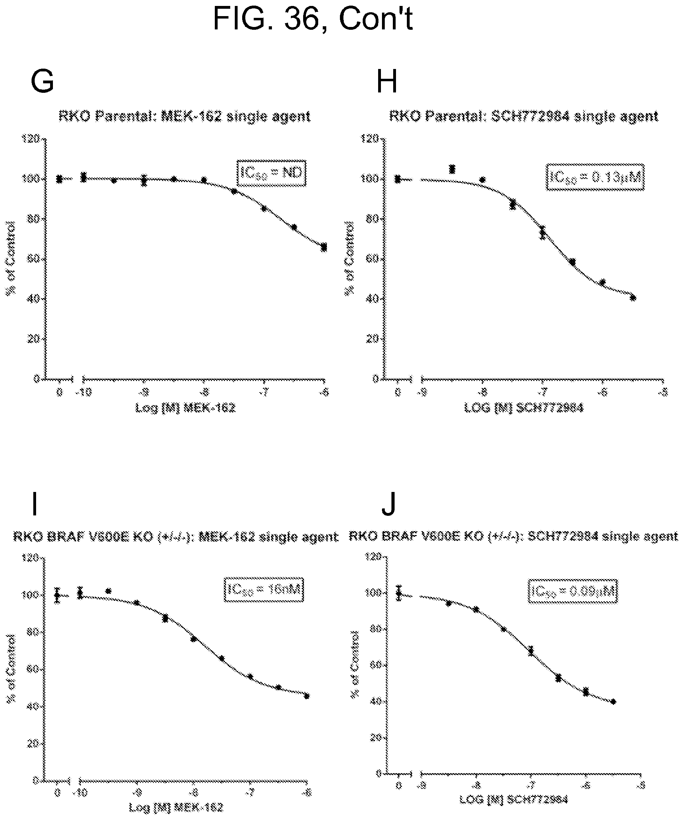

FIG. 36 shows the results of the combination of SCH772984 and MEK-162 in parental RKO and RKO BRAF V600E KO (+/-/-) cells. FIG. 36A shows a dose matrix showing inhibition (%) for the combination in parental RKO cells. FIG. 36B shows Loewe excess for the combination in 36A and FIG. 36C shows Bliss excess for the combination in 36A. FIG. 36D shows a dose matrix showing inhibition (%) for the combination in RKO BRAF V600E KO (+/-/-) cells. FIG. 36E shows Loewe excess for the combination in 36D and FIG. 36F shows Bliss excess for the combination in 36D. FIG. 36G - FIG. 36H show the results of single agent proliferation assays for the combination in 36A. FIG. 36I - FIG. 36J show the results of single agent proliferation assays for the combination in 36D.

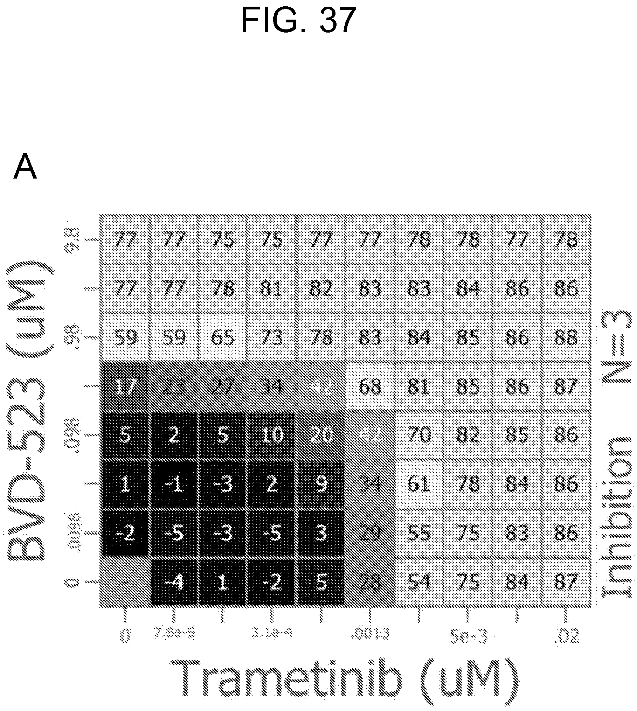

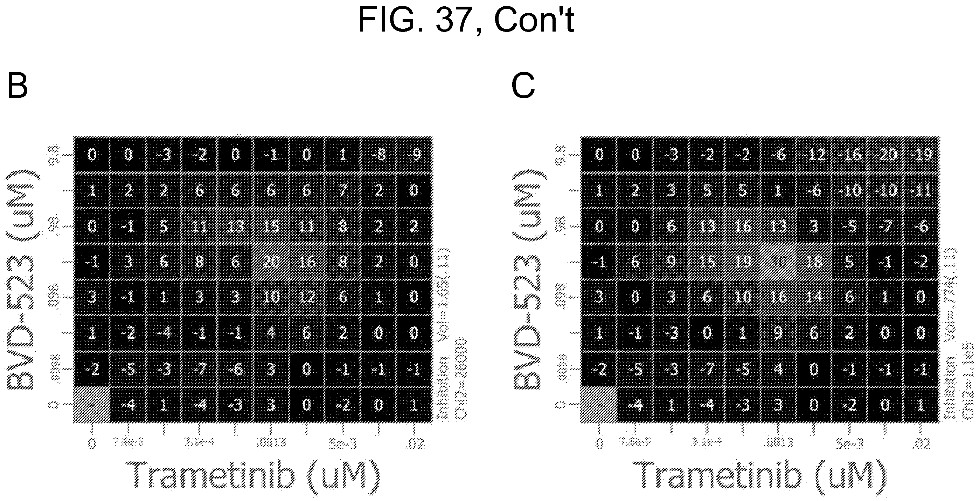

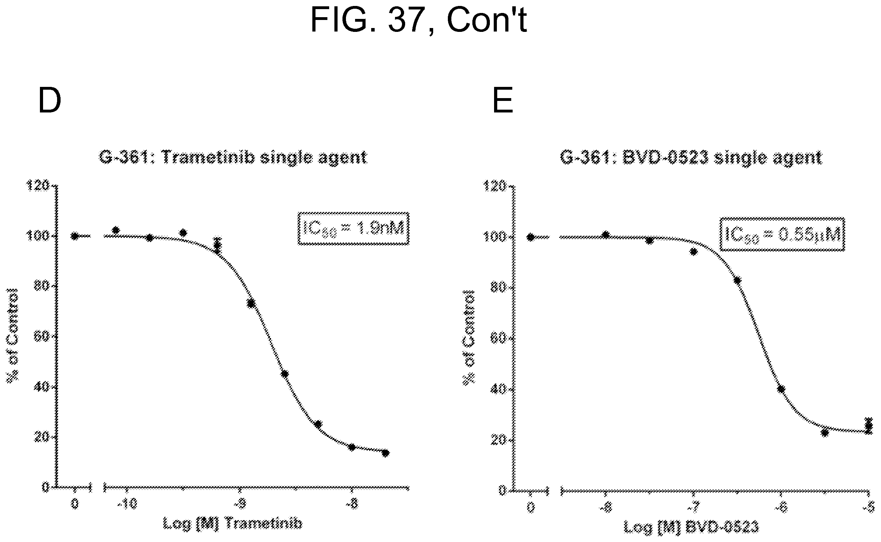

FIG. 37 shows the results of the combination of BVD-523 and Trametinib in G-361 cells. FIG. 37A shows a dose matrix showing inhibition (%) for the combination. FIG. 37B shows Loewe excess for the combination in 37A and FIG. 37C shows Bliss excess for the combination in 37A. FIG. 37D - FIG. 37E show the results of single agent proliferation assays for the combination in 37A.

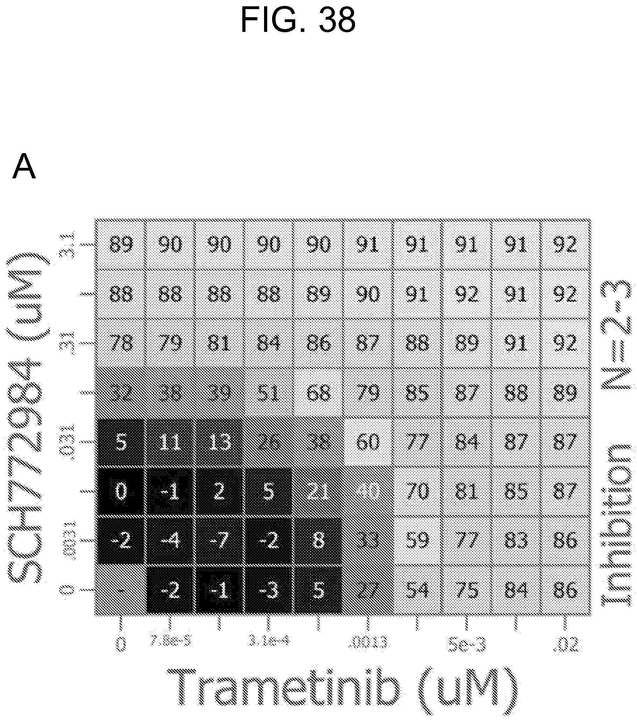

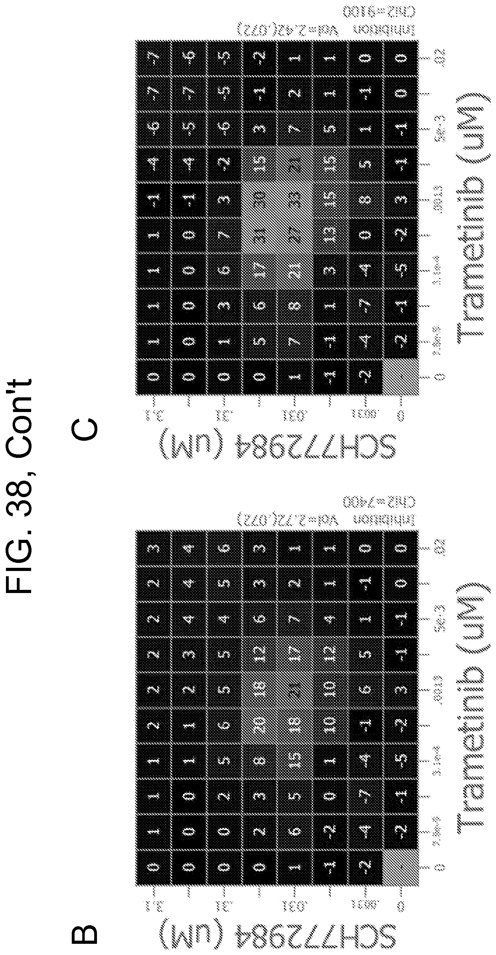

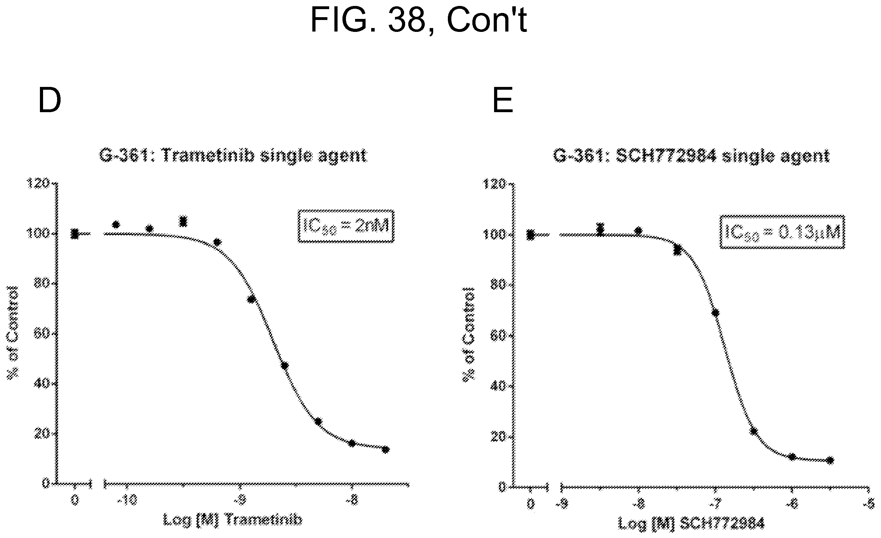

FIG. 38 shows the results of the combination of SCH772984 and Trametinib in G-361 cells. FIG. 38A shows a dose matrix showing inhibition (%) for the combination. FIG. 38B shows Loewe excess for the combination in 38A and FIG. 38C shows Bliss excess for the combination in 38A. FIG. 38D - FIG. 38E show the results of single agent proliferation assays for the combination in 38A.

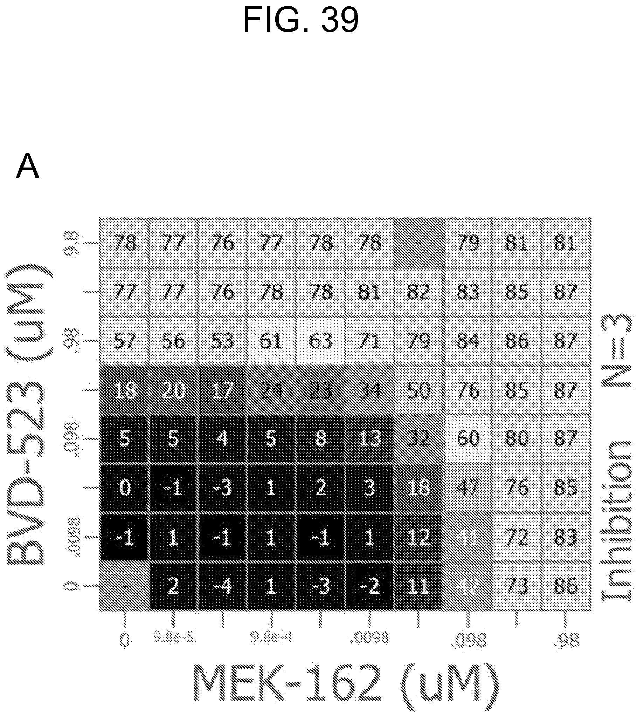

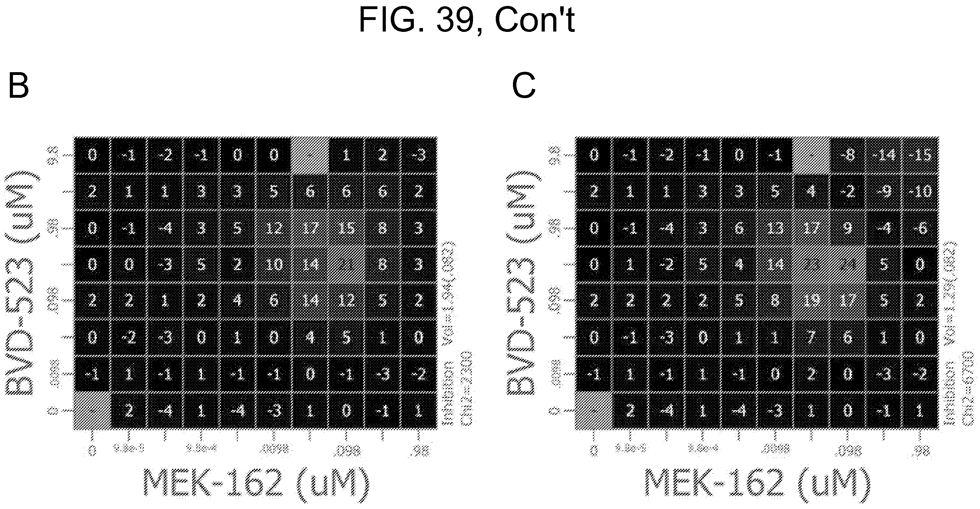

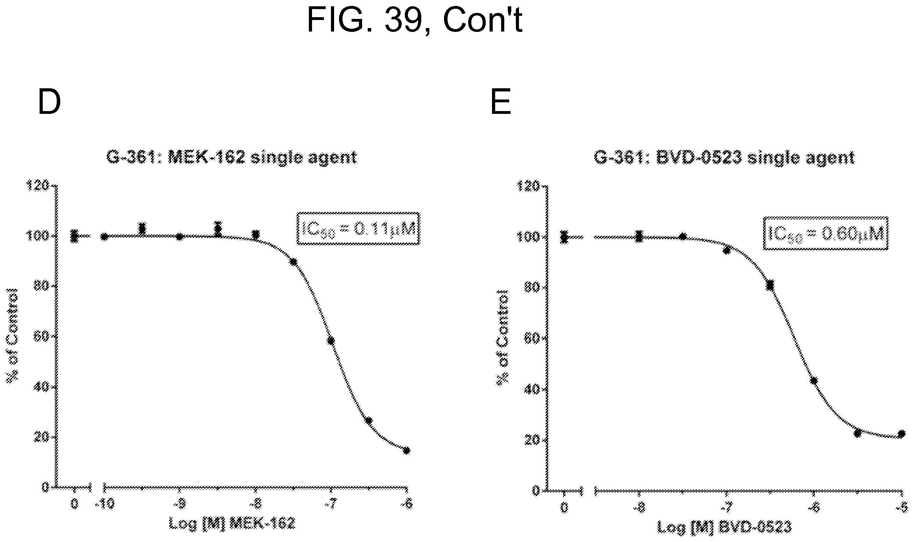

FIG. 39 shows the results of the combination of BVD-523 and MEK-162 in G-361 cells. FIG. 39A shows a dose matrix showing inhibition (%) for the combination. FIG. 39B shows Loewe excess for the combination in 39A and FIG. 39C shows Bliss excess for the combination in 39A. FIG. 39D - FIG. 39E show the results of single agent proliferation assays for the combination in 39A.

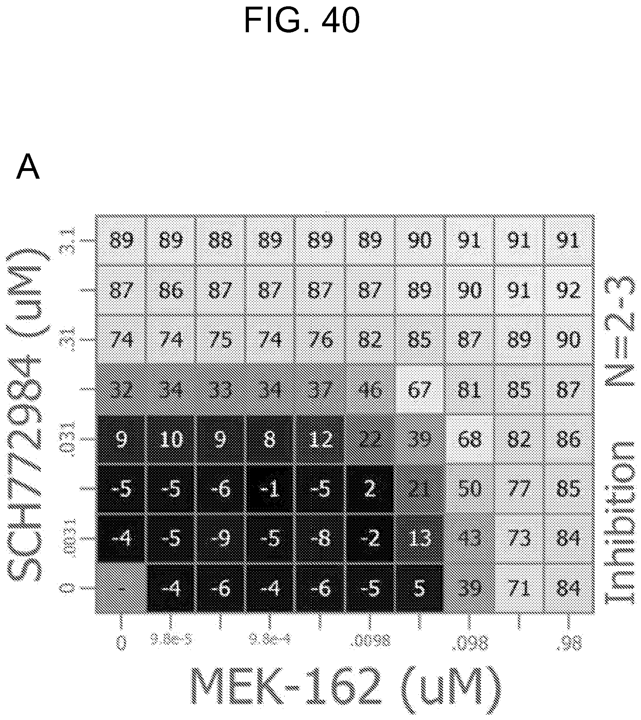

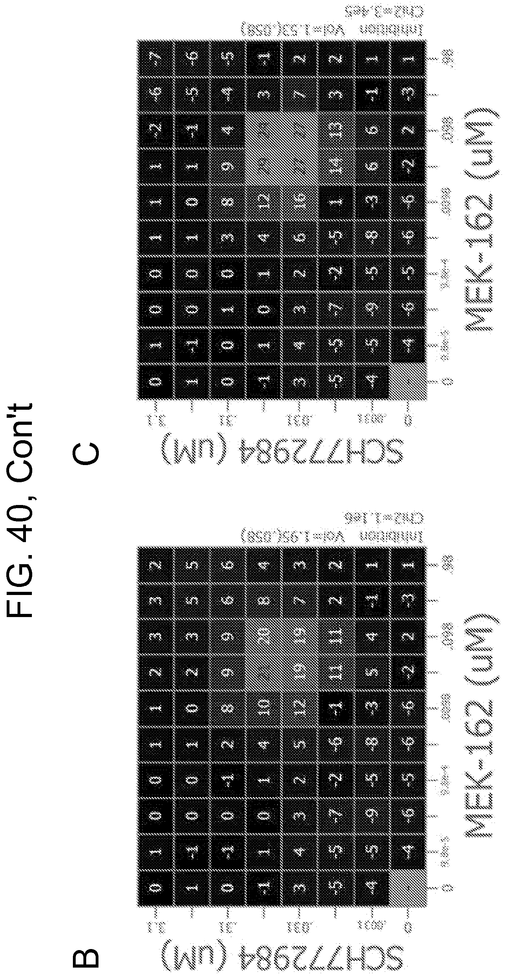

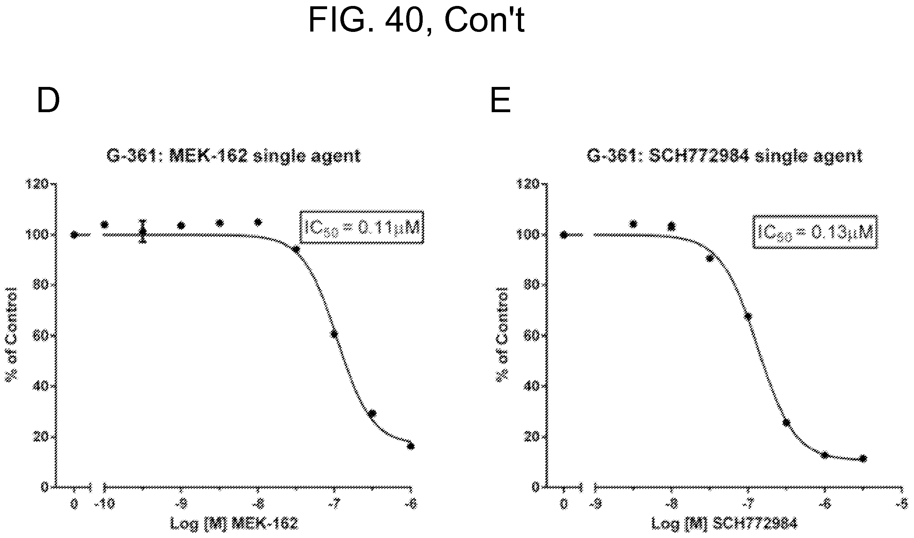

FIG. 40 shows the results of the combination of SCH772984 and MEK-162 in G-361 cells. FIG. 40A shows a dose matrix showing inhibition (%) for the combination. FIG. 40B shows Loewe excess for the combination in 40A and FIG. 40C shows Bliss excess for the combination in 40A. FIG. 40D - FIG. 40E show the results of single agent proliferation assays for the combination in 40A.

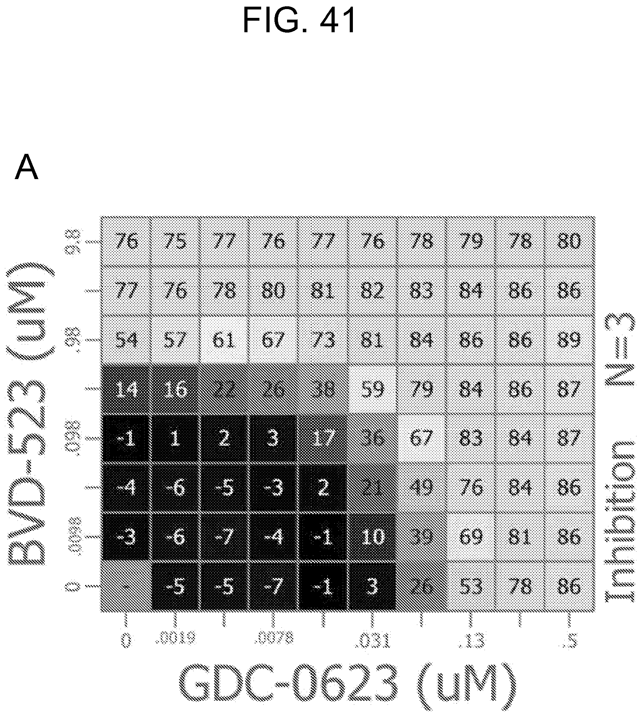

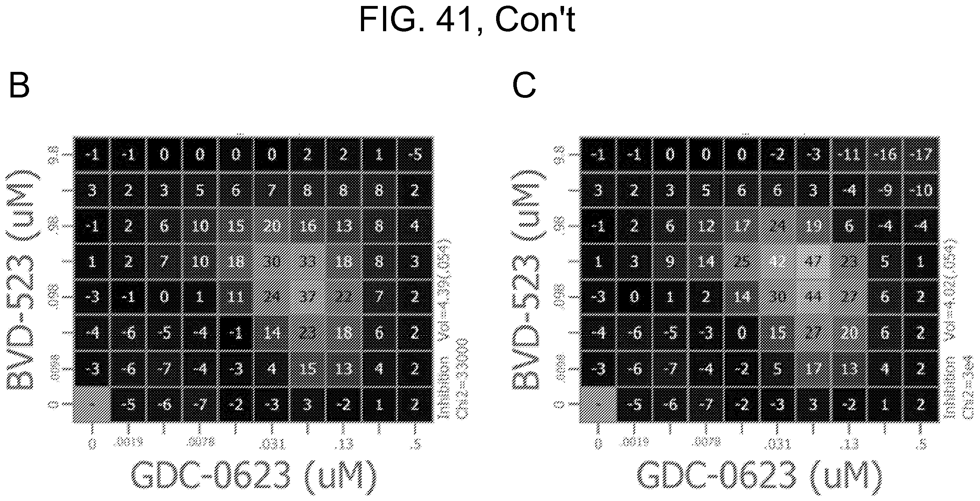

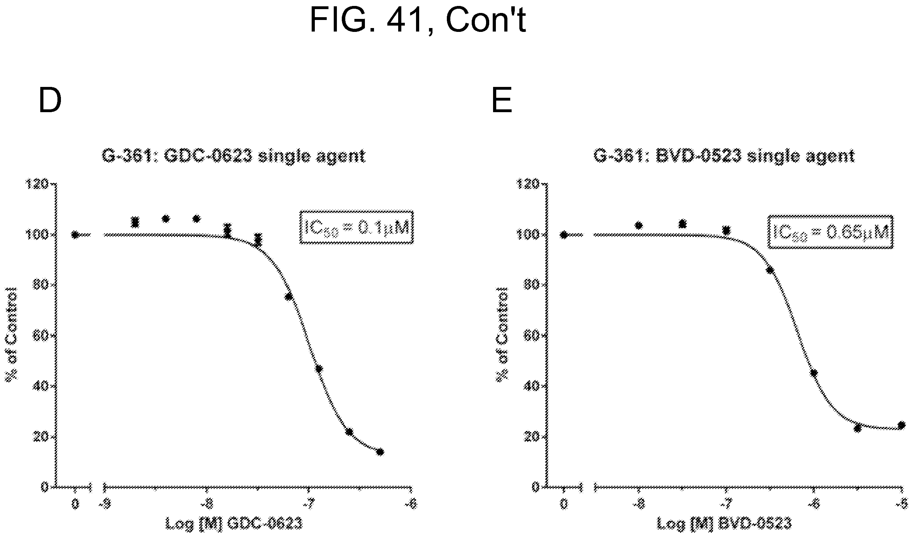

FIG. 41 shows the results of the combination of BVD-523 and GDC-0623 in G-361 cells. FIG. 41A shows a dose matrix showing inhibition (%) for the combination. FIG. 41B shows Loewe excess for the combination in 41A and FIG. 41C shows Bliss excess for the combination in 41A. FIG. 41D - FIG. 41E show the results of single agent proliferation assays for the combination in 41A.

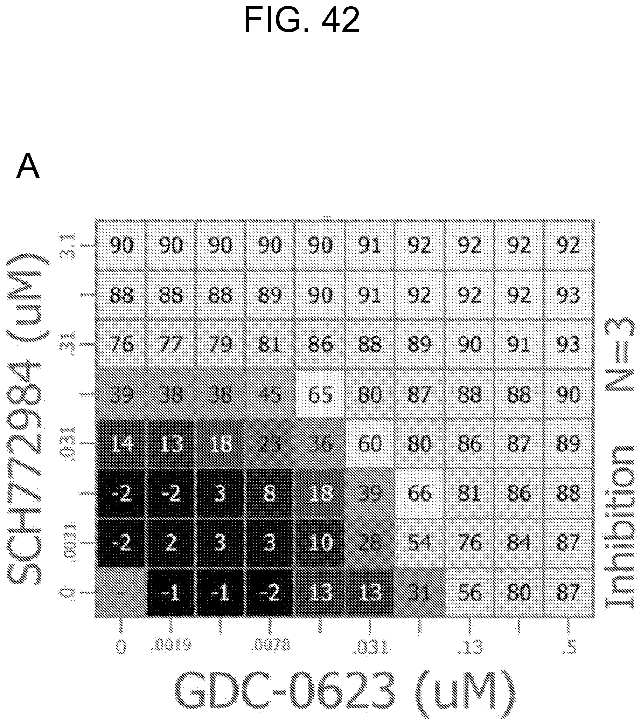

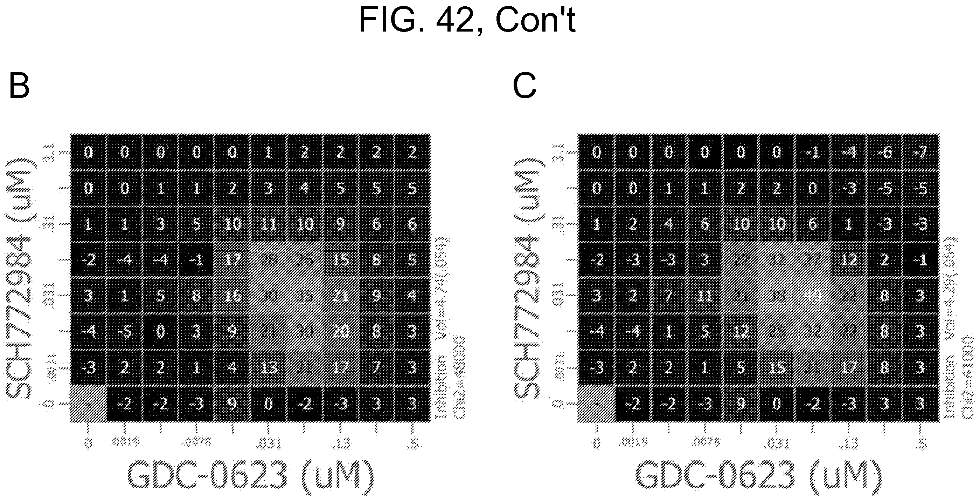

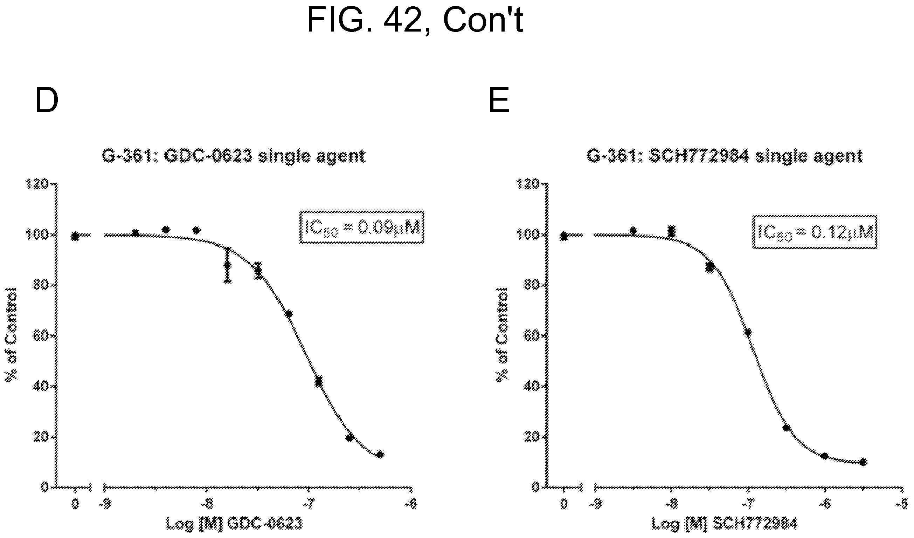

FIG. 42 shows the results of the combination of SCH772984 and GDC-0623 in G-361 cells. FIG. 42A shows a dose matrix showing inhibition (%) for the combination. FIG. 42B shows Loewe excess for the combination in 42A and FIG. 42C shows Bliss excess for the combination in 42A. FIG. 42D - FIG. 42E show the results of single agent proliferation assays for the combination in 42A.

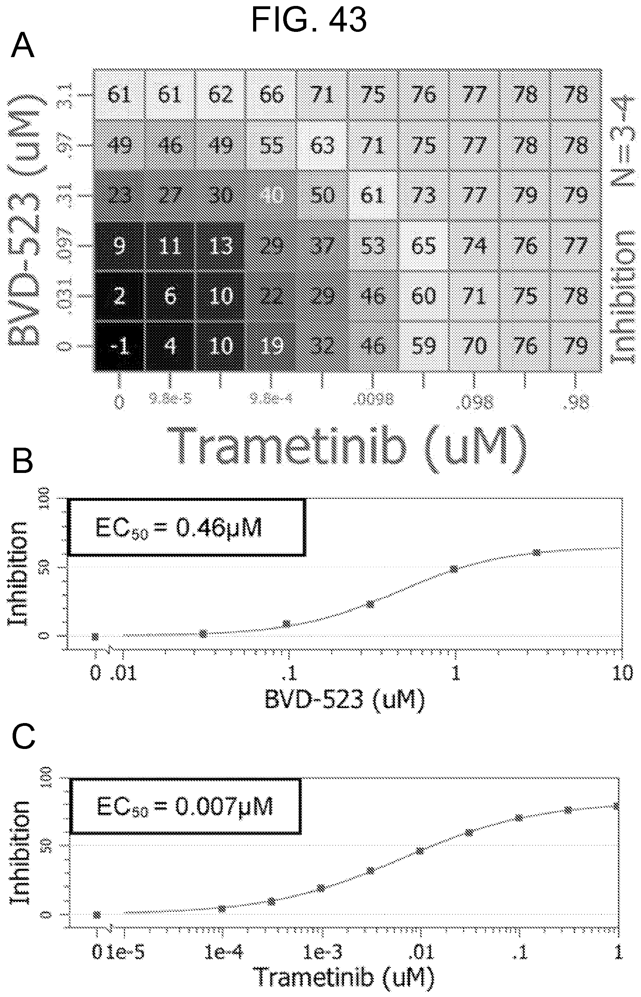

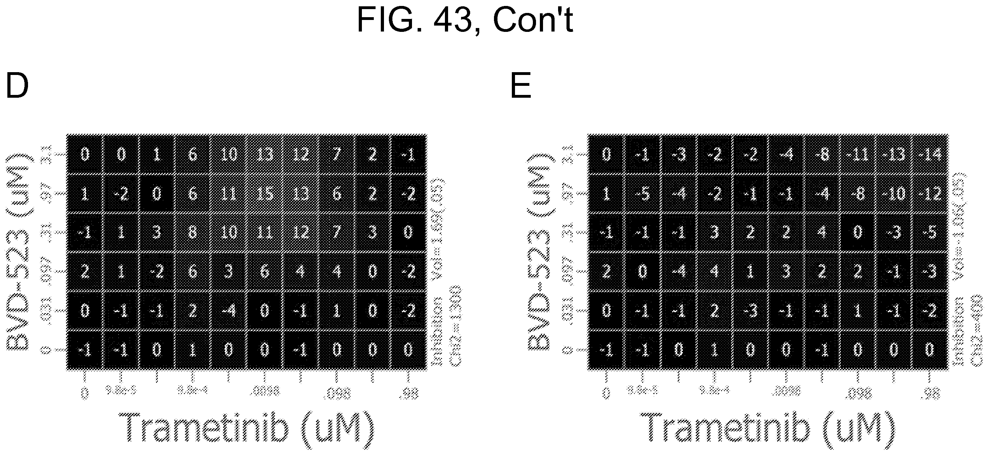

FIG. 43 shows the results of the combination of BVD-523 and Trametinib in A549 cells. FIG. 43A shows a dose matrix showing inhibition (%) for the combination. FIG. 43B - FIG. 43C show the results of single agent proliferation assays for the combination in 43A. FIG. 43D shows Loewe excess for the combination in 43A and FIG. 43E shows Bliss excess for the combination in 43A.

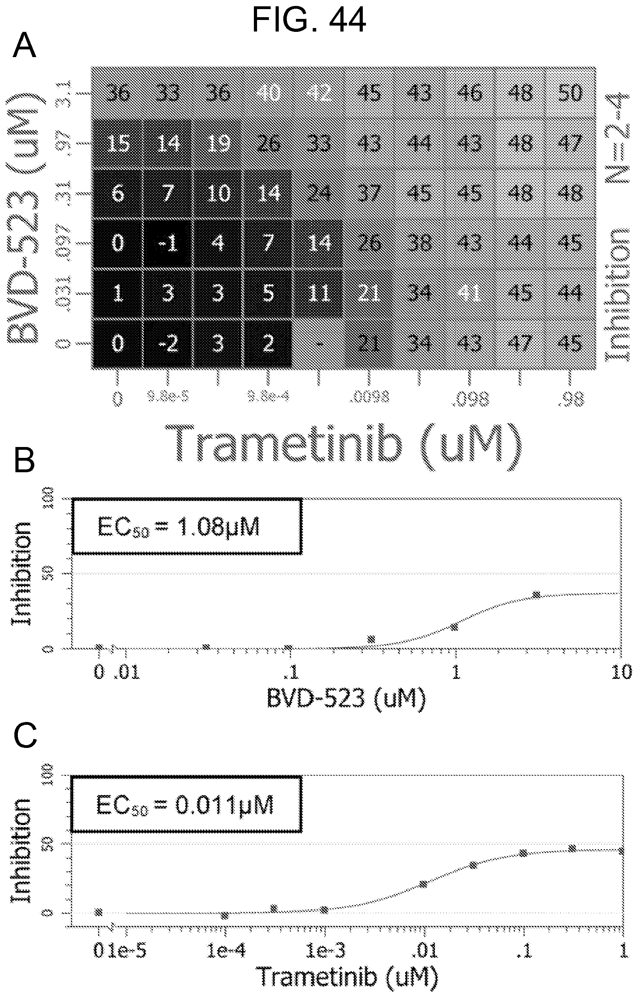

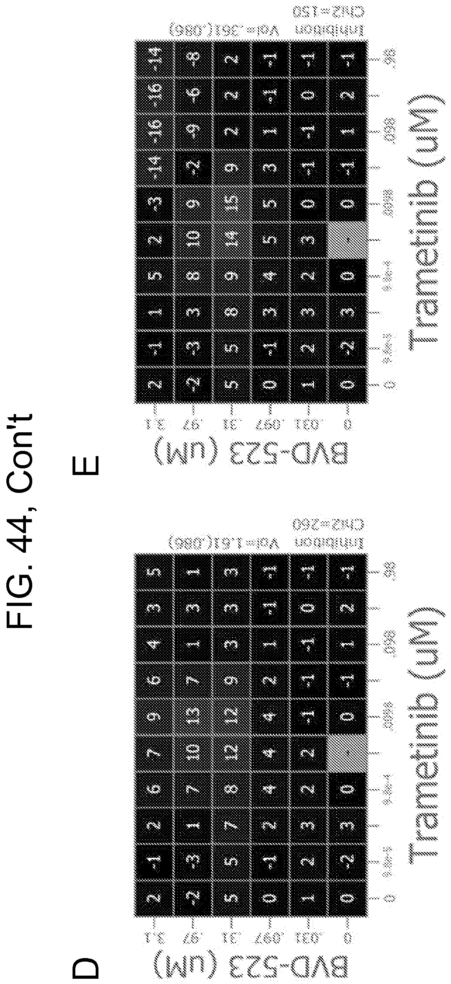

FIG. 44 shows the results of the combination of BVD-523 and Trametinib in H2122 cells. FIG. 44A shows a dose matrix showing inhibition (%) for the combination. FIG. 44B - FIG. 44C show the results of single agent proliferation assays for the combination in 44A. FIG. 44D shows Loewe excess for the combination in 44A and FIG. 44E shows Bliss excess for the combination in 44A.

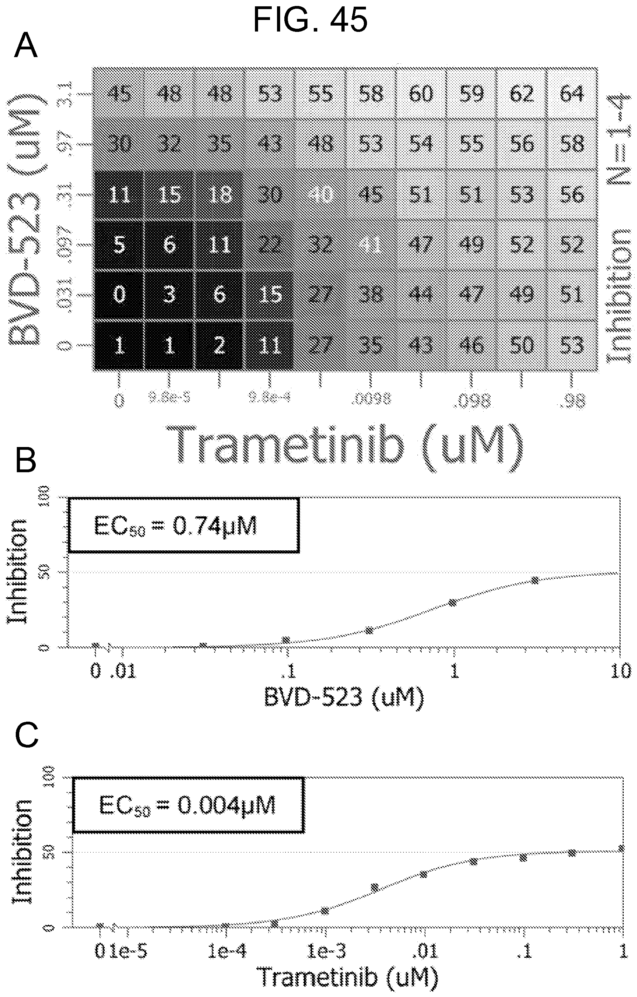

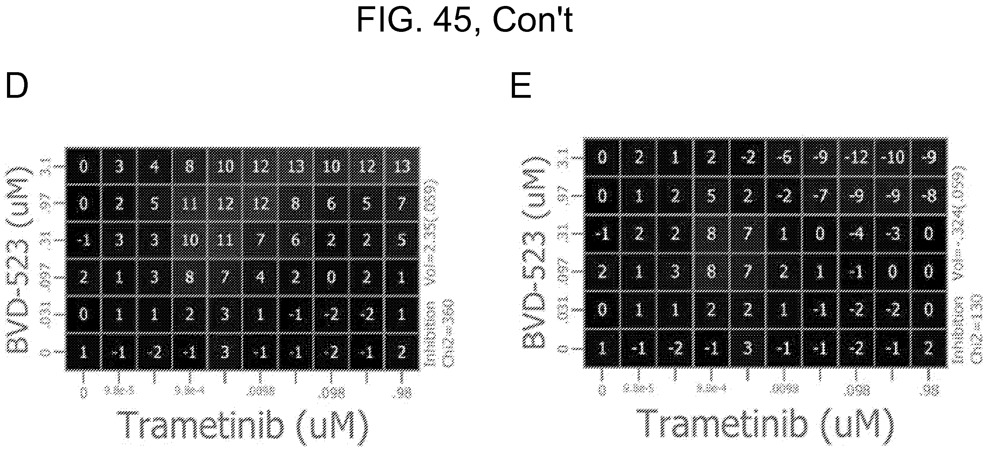

FIG. 45 shows the results of the combination of BVD-523 and Trametinib in H1437 cells. FIG. 45A shows a dose matrix showing inhibition (%) for the combination. FIG. 45B - FIG. 45C show the results of single agent proliferation assays for the combination in 45A. FIG. 45D shows Loewe excess for the combination in 45A and FIG. 45E shows Bliss excess for the combination in 45A.

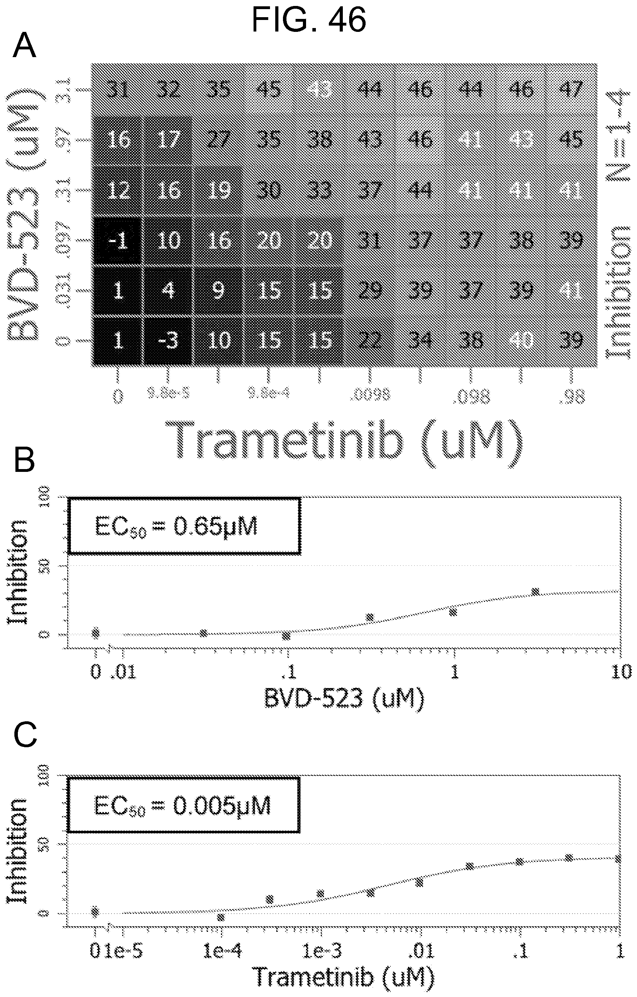

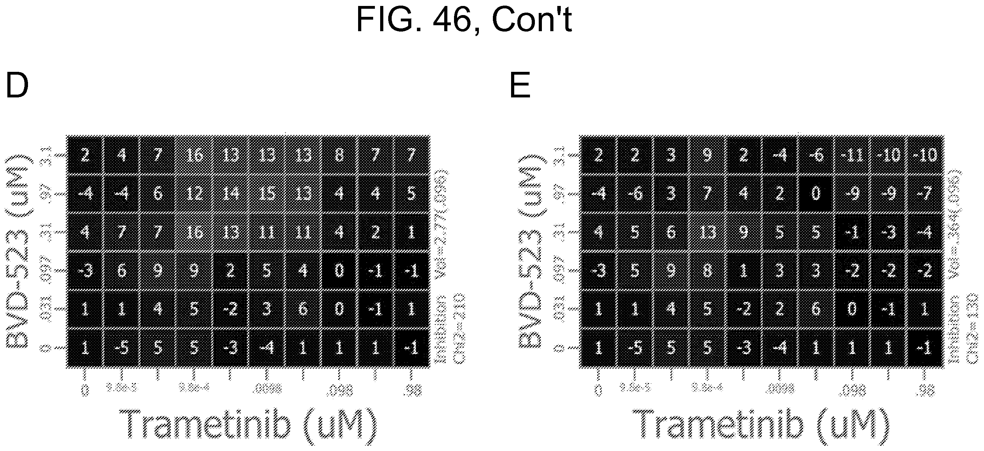

FIG. 46 shows the results of the combination of BVD-523 and Trametinib in H226 cells. FIG. 46A shows a dose matrix showing inhibition (%) for the combination. FIG. 46B - FIG. 46C show the results of single agent proliferation assays for the combination in 46A. FIG. 46D shows Loewe excess for the combination in 46A and FIG. 46E shows Bliss excess for the combination in 46A.

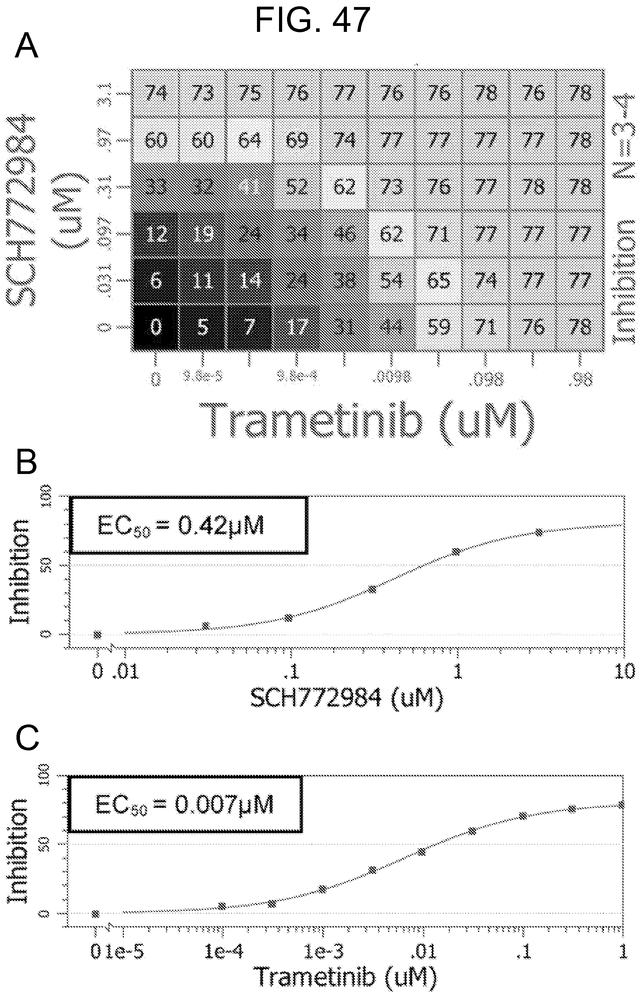

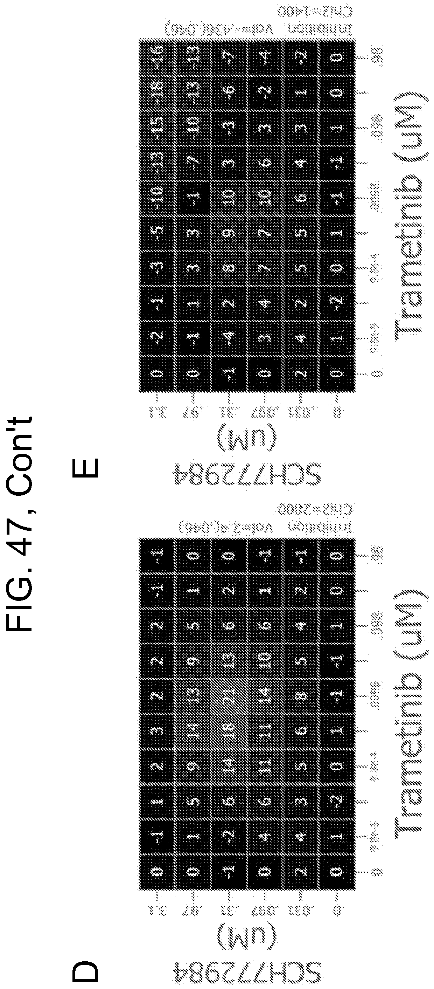

FIG. 47 shows the results of the combination of SCH772984 and Trametinib in A549 cells. FIG. 47A shows a dose matrix showing inhibition (%) for the combination. FIG. 47B - FIG. 47C show the results of single agent proliferation assays for the combination in 47A. FIG. 47D shows Loewe excess for the combination in 47A and FIG. 47E shows Bliss excess for the combination in 47A.

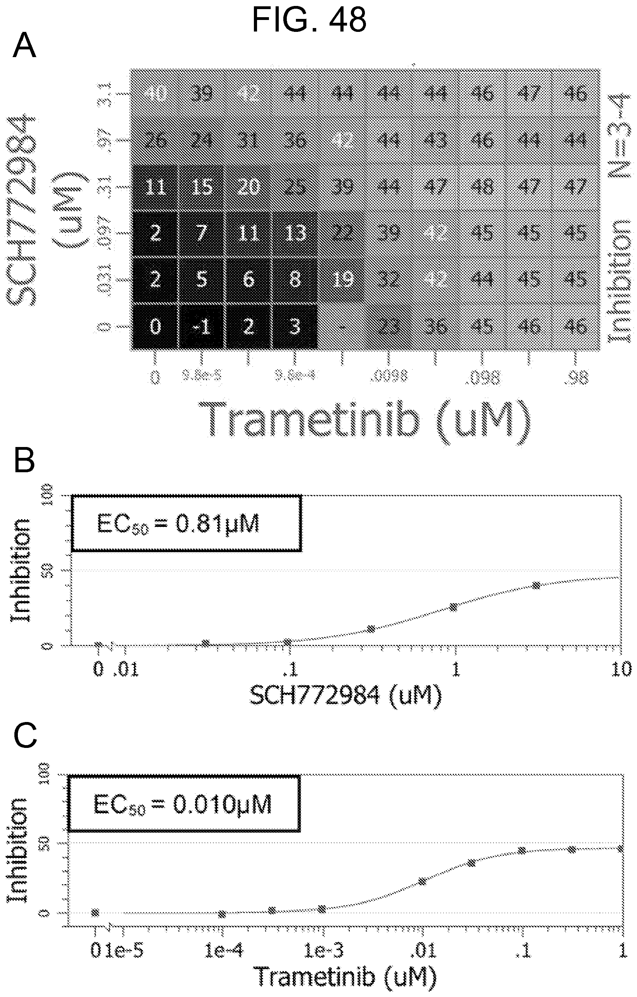

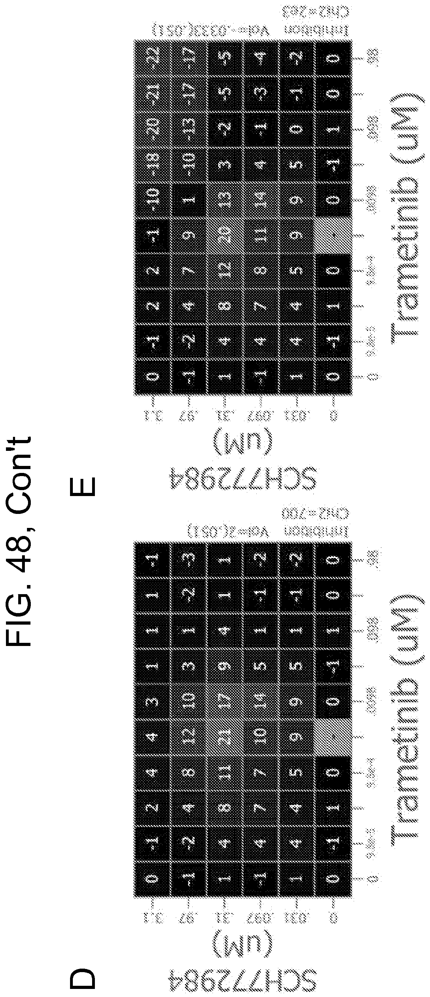

FIG. 48 shows the results of the combination of SCH772984 and Trametinib in H2122 cells. FIG. 48A shows a dose matrix showing inhibition (%) for the combination. FIG. 48B - FIG. 48C show the results of single agent proliferation assays for the combination in 48A. FIG. 48D shows Loewe excess for the combination in 48A and FIG. 48E shows Bliss excess for the combination in 48A.

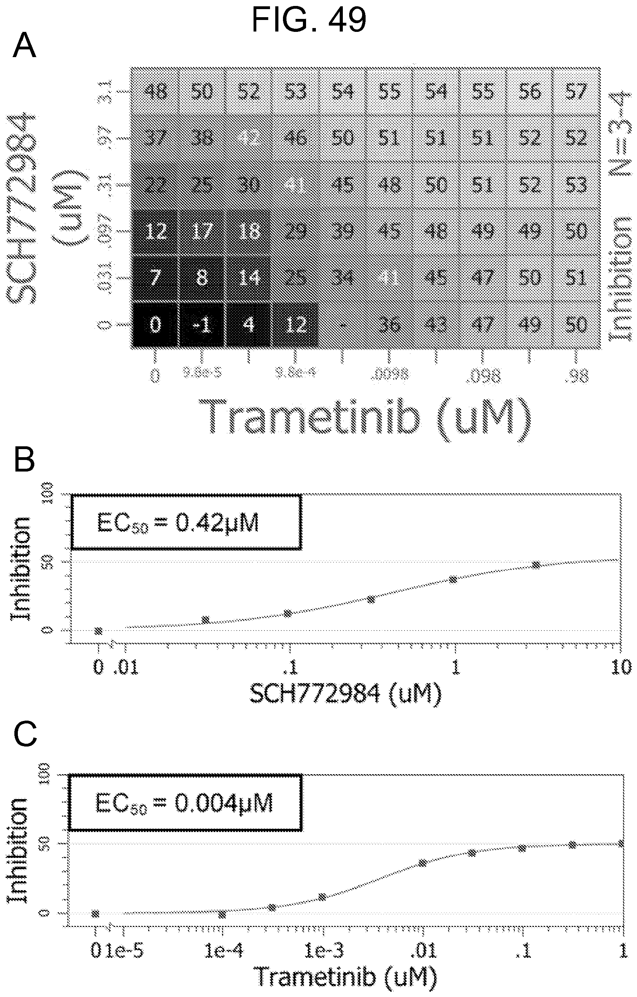

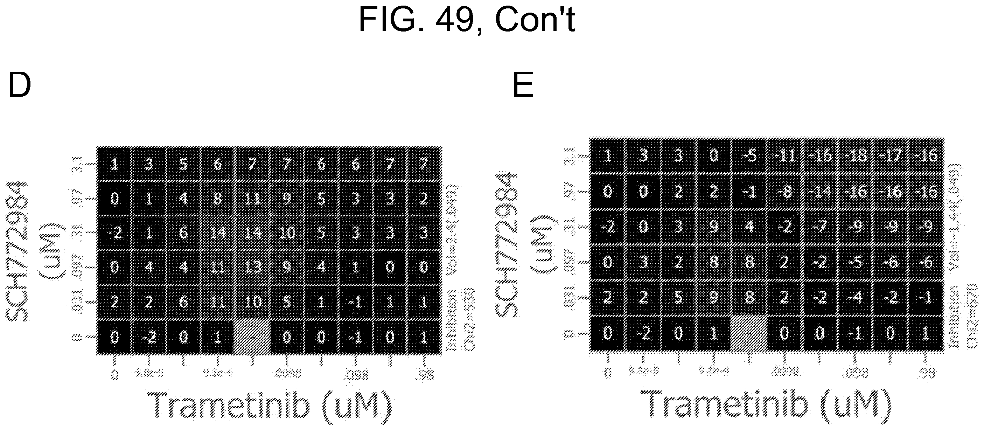

FIG. 49 shows the results of the combination of SCH772984 and Trametinib in H1437 cells. FIG. 49A shows a dose matrix showing inhibition (%) for the combination. FIG. 49B - FIG. 49C show the results of single agent proliferation assays for the combination in 49A. FIG. 49D shows Loewe excess for the combination in 49A and FIG. 49E shows Bliss excess for the combination in 49A.

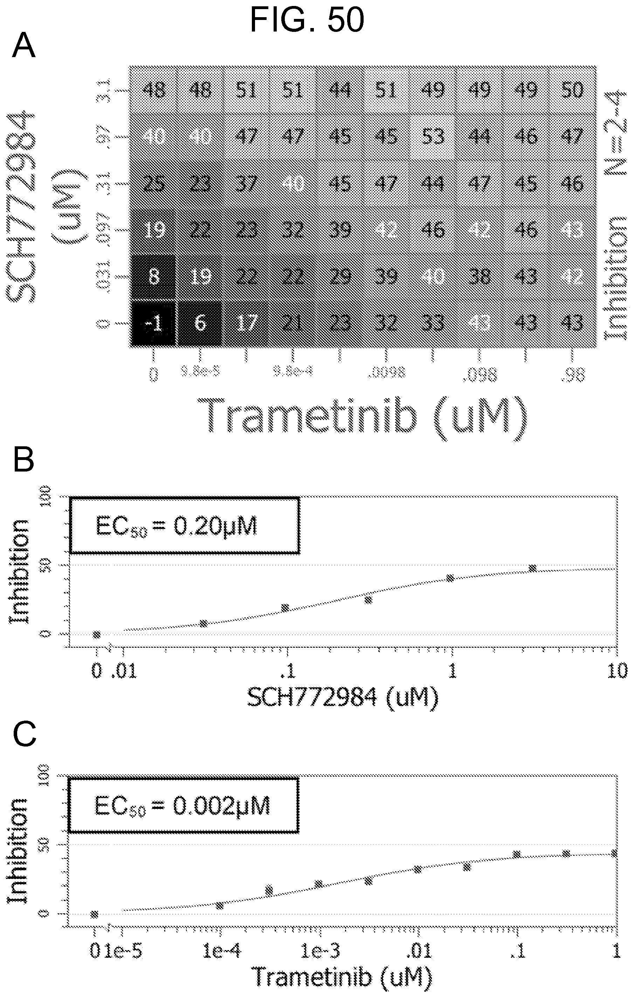

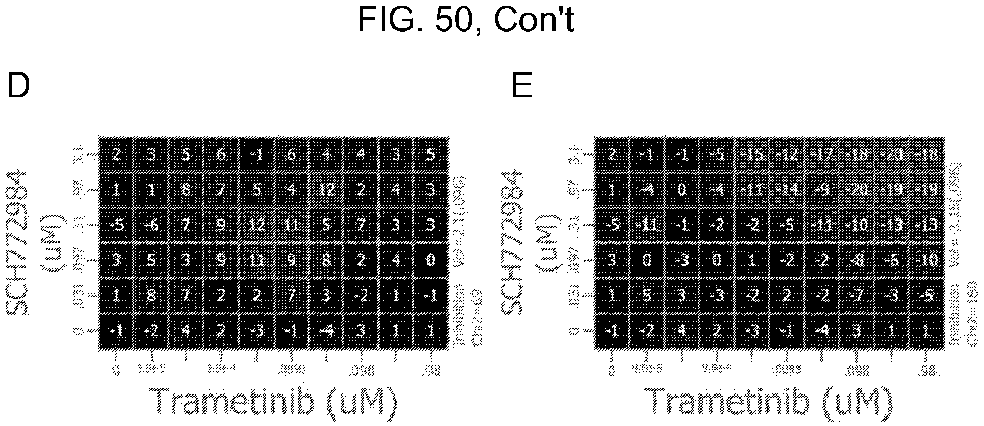

FIG. 50 shows the results of the combination of SCH772984 and Trametinib in H226 cells. FIG. 50A shows a dose matrix showing inhibition (%) for the combination. FIG. 50B - FIG. 50C show the results of single agent proliferation assays for the combination in 50A. FIG. 50D shows Loewe excess for the combination in 50A and FIG. 50E shows Bliss excess for the combination in 50A.

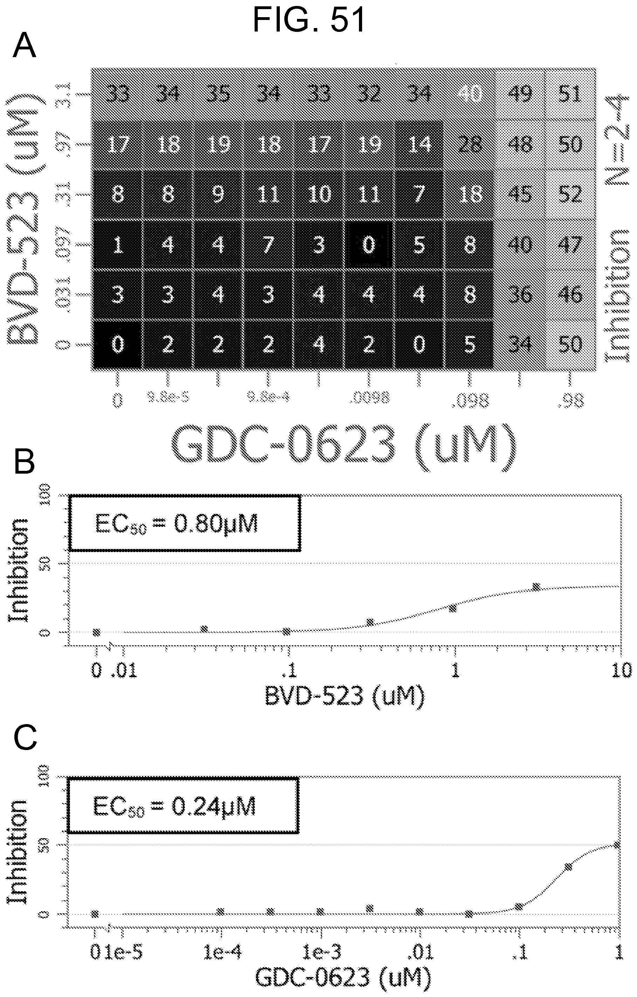

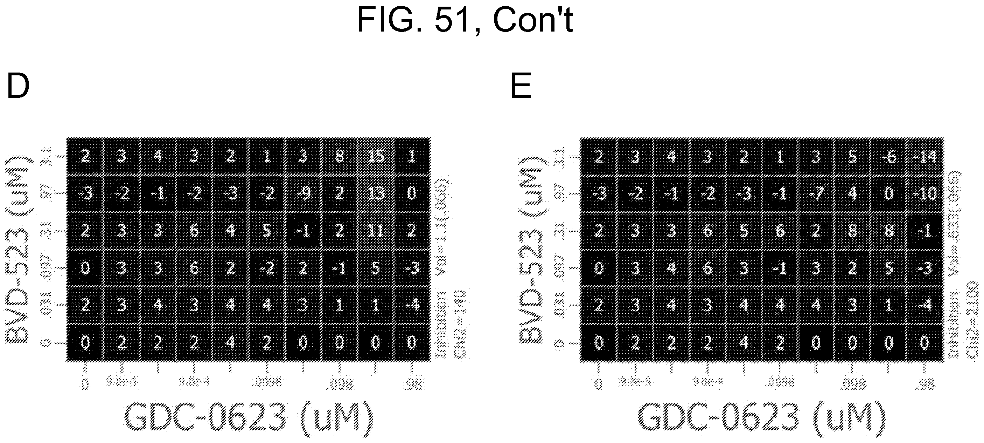

FIG. 51 shows the results of the combination of BVD-523 and GDC-0623 in H2122 cells. FIG. 51A shows a dose matrix showing inhibition (%) for the combination. FIG. 51B - FIG. 51C show the results of single agent proliferation assays for the combination in 51A. FIG. 51D shows Loewe excess for the combination in 51A and FIG. 51E shows Bliss excess for the combination in 51A.

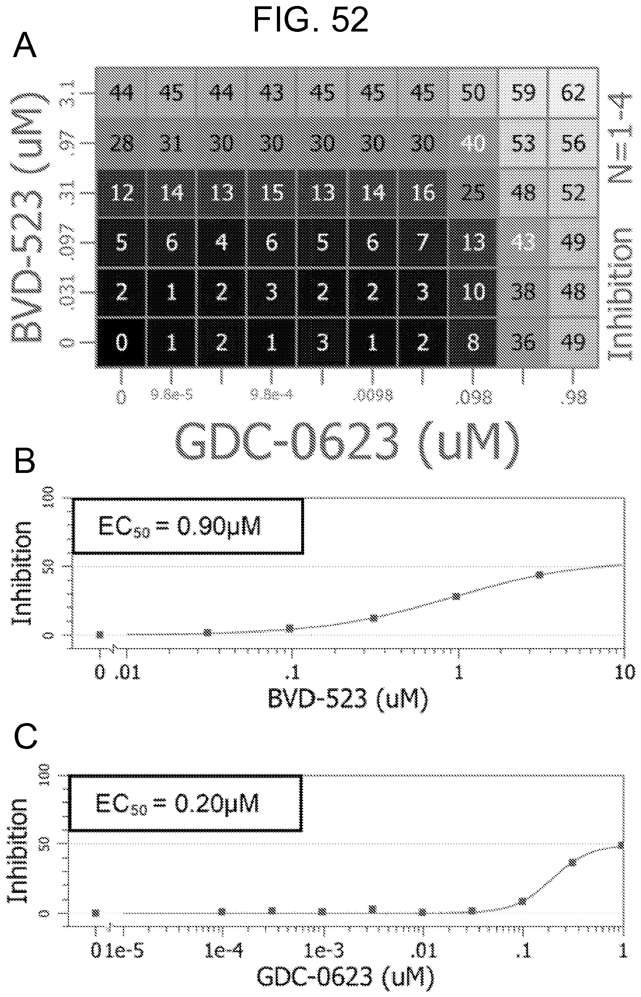

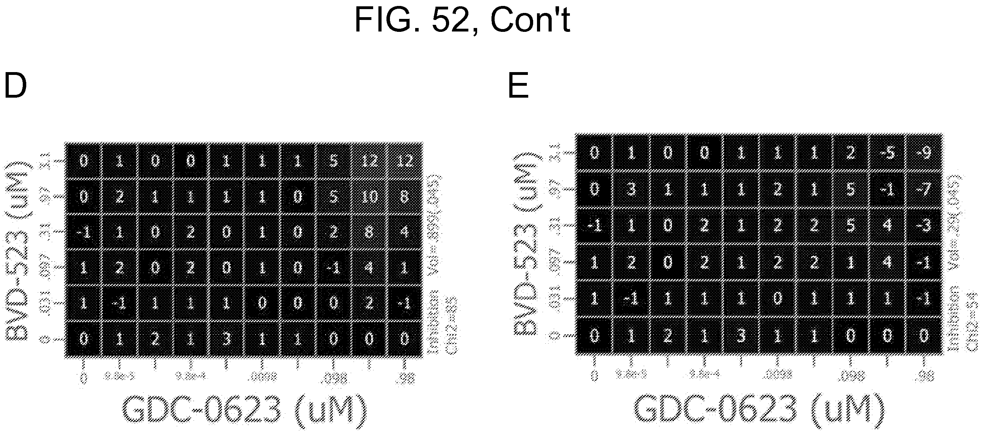

FIG. 52 shows the results of the combination of BVD-523 and GDC-0623 in H1437 cells. FIG. 52A shows a dose matrix showing inhibition (%) for the combination. FIG. 52B - FIG. 52C show the results of single agent proliferation assays for the combination in 52A. FIG. 52D shows Loewe excess for the combination in 52A and FIG. 52E shows Bliss excess for the combination in 52A.

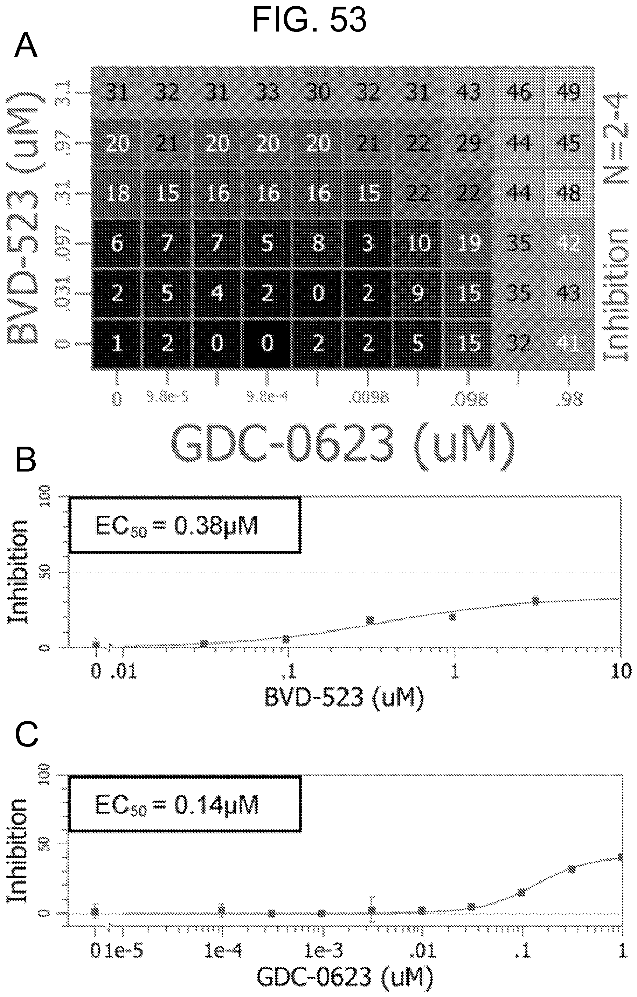

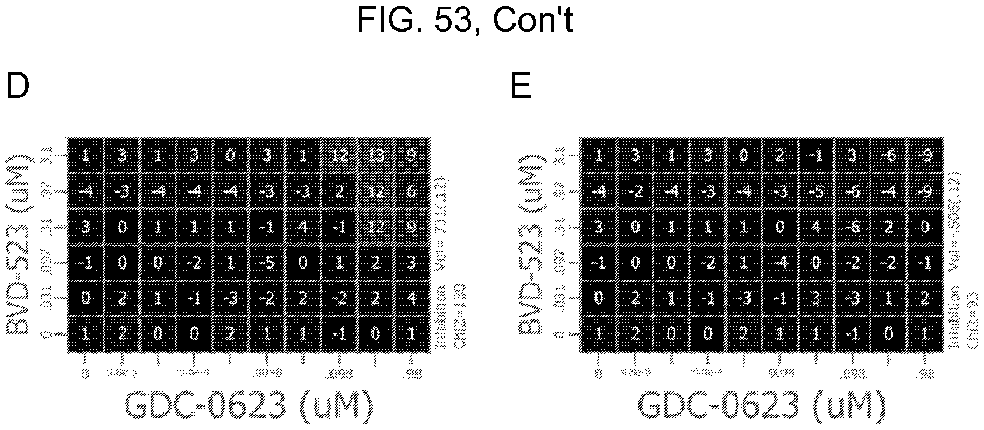

FIG. 53 shows the results of the combination of BVD-523 and GDC-0623 in H226 cells. FIG. 53A shows a dose matrix showing inhibition (%) for the combination. FIG. 53B - FIG. 53C show the results of single agent proliferation assays for the combination in 53A. FIG. 53D shows Loewe excess for the combination in 53A and FIG. 53E shows Bliss excess for the combination in 53A.

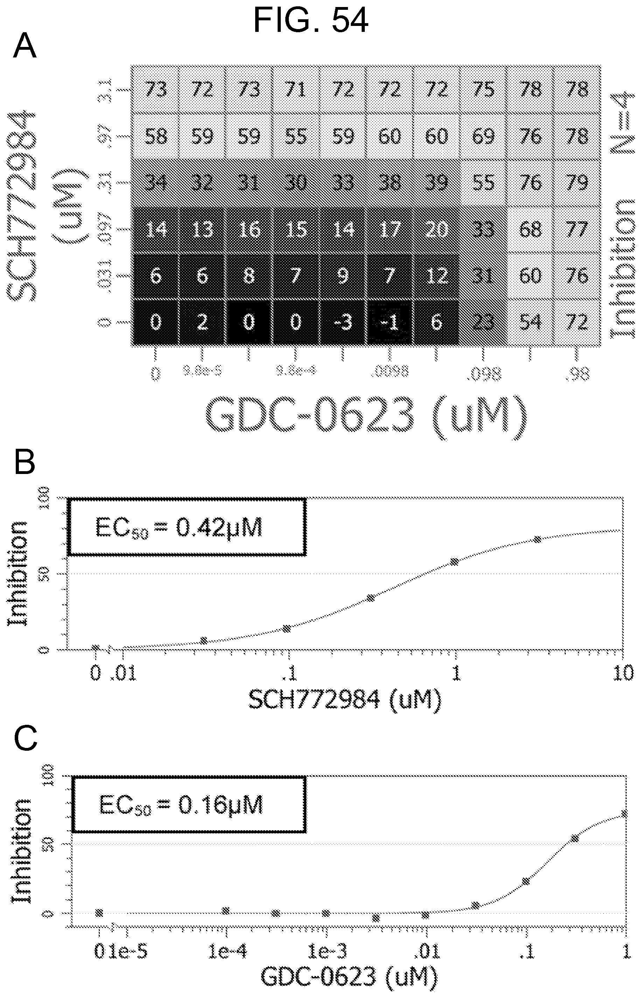

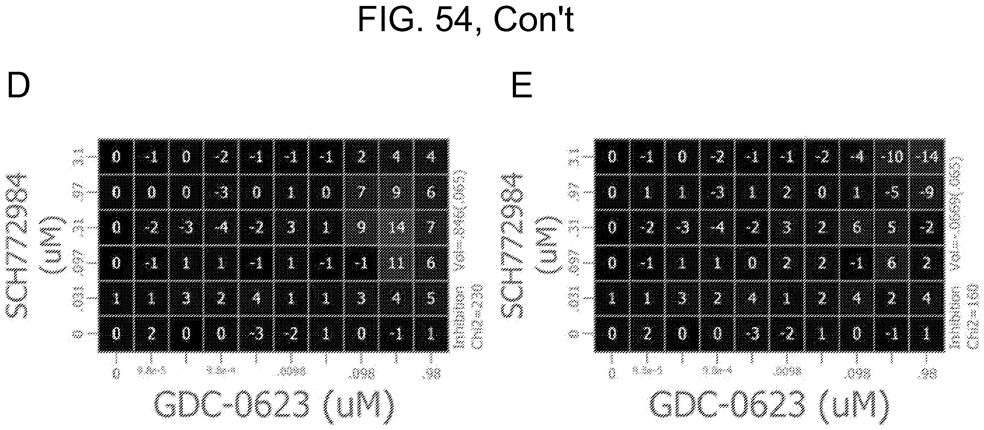

FIG. 54 shows the results of the combination of SCH772984 and GDC-0623 in A549 cells. FIG. 54A shows a dose matrix showing inhibition (%) for the combination. FIG. 54B - FIG. 54C show the results of single agent proliferation assays for the combination in 54A. FIG. 54D shows Loewe excess for the combination in 54A and FIG. 54E shows Bliss excess for the combination in 54A.

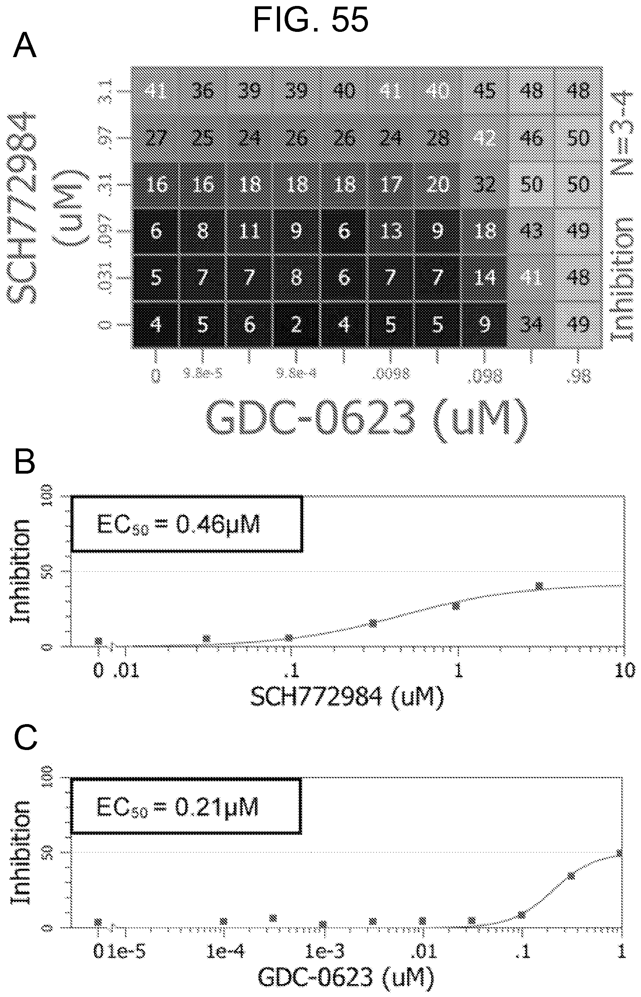

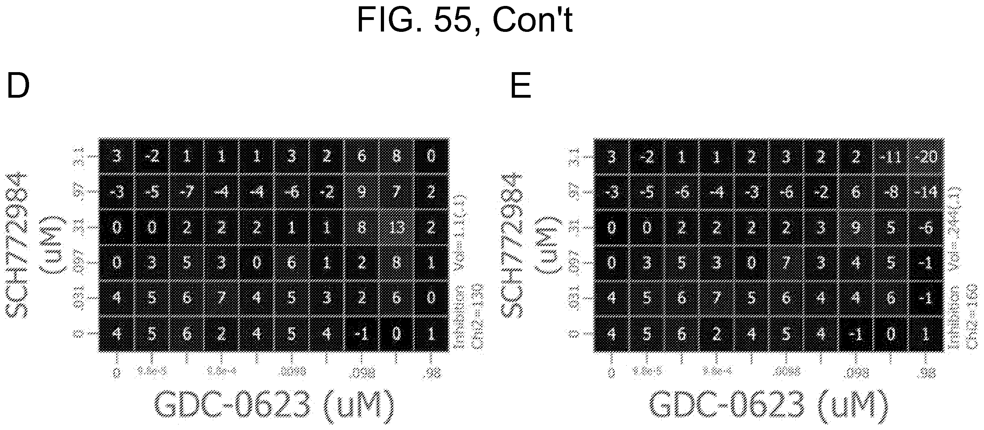

FIG. 55 shows the results of the combination of SCH772984 and GDC-0623 in H2122 cells. FIG. 55A shows a dose matrix showing inhibition (%) for the combination. FIG. 55B - FIG. 55C show the results of single agent proliferation assays for the combination in 55A. FIG. 55D shows Loewe excess for the combination in 55A and FIG. 55E shows Bliss excess for the combination in 55A.

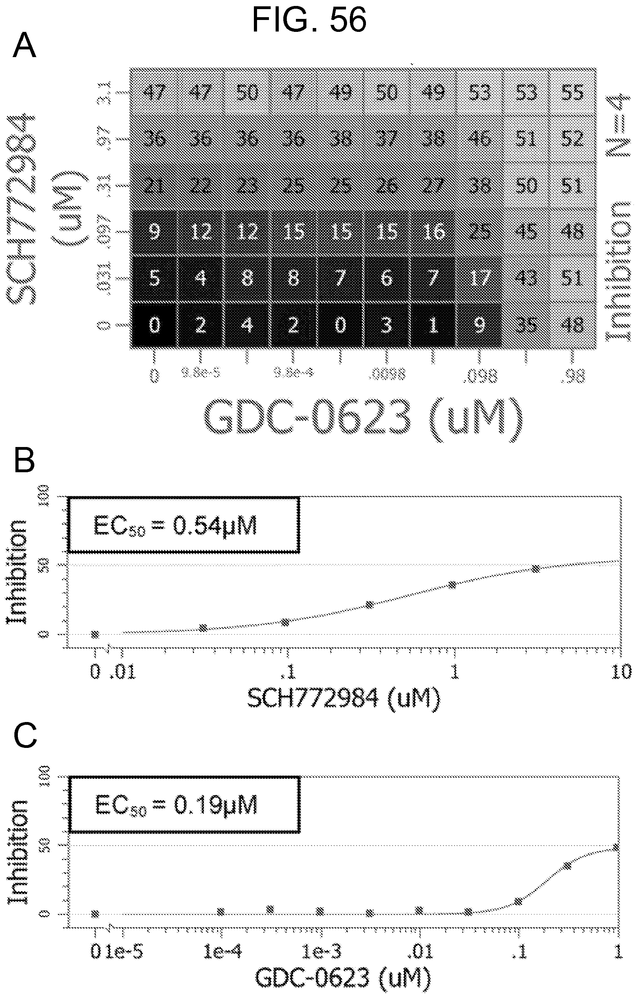

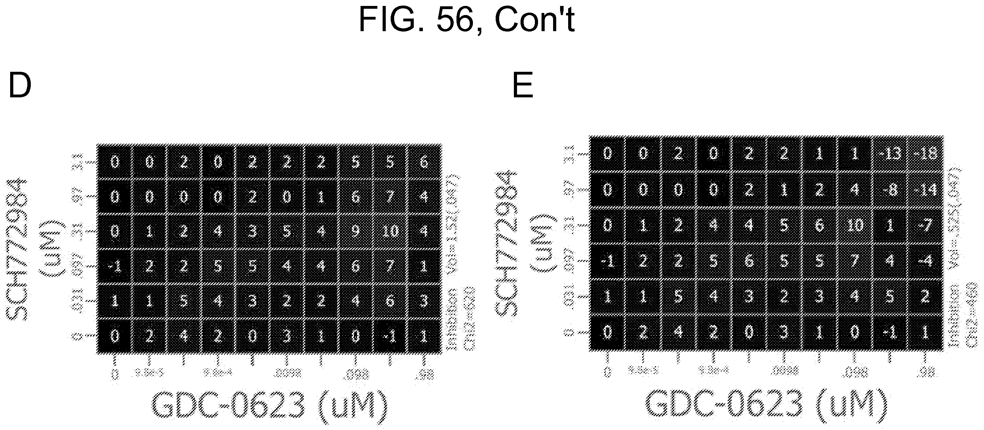

FIG. 56 shows the results of the combination of SCH772984 and GDC-0623 in H1437 cells. FIG. 56A shows a dose matrix showing inhibition (%) for the combination. FIG. 56B - FIG. 56C show the results of single agent proliferation assays for the combination in 56A. FIG. 56D shows Loewe excess for the combination in 56A and FIG. 56E shows Bliss excess for the combination in 56A.

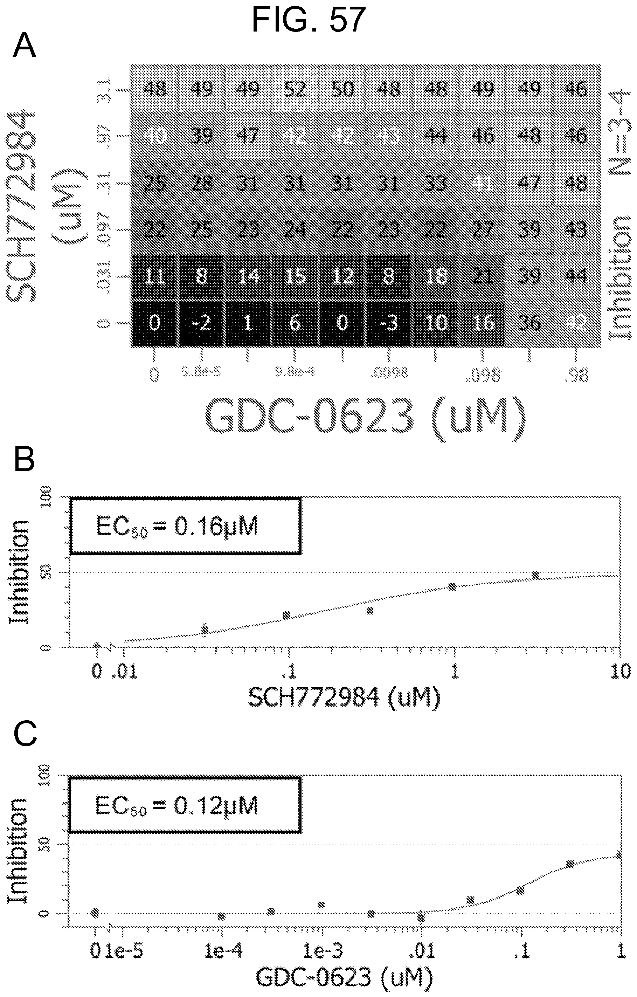

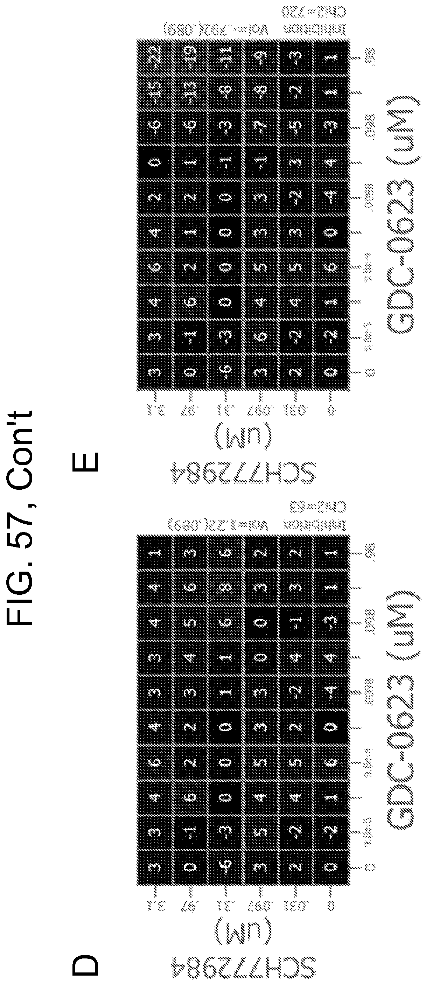

FIG. 57 shows the results of the combination of SCH772984 and GDC-0623 in H226 cells. FIG. 57A shows a dose matrix showing inhibition (%) for the combination. FIG. 57B - FIG. 57C show the results of single agent proliferation assays for the combination in 57A. FIG. 57D shows Loewe excess for the combination in 57A and FIG. 57E shows Bliss excess for the combination in 57A.

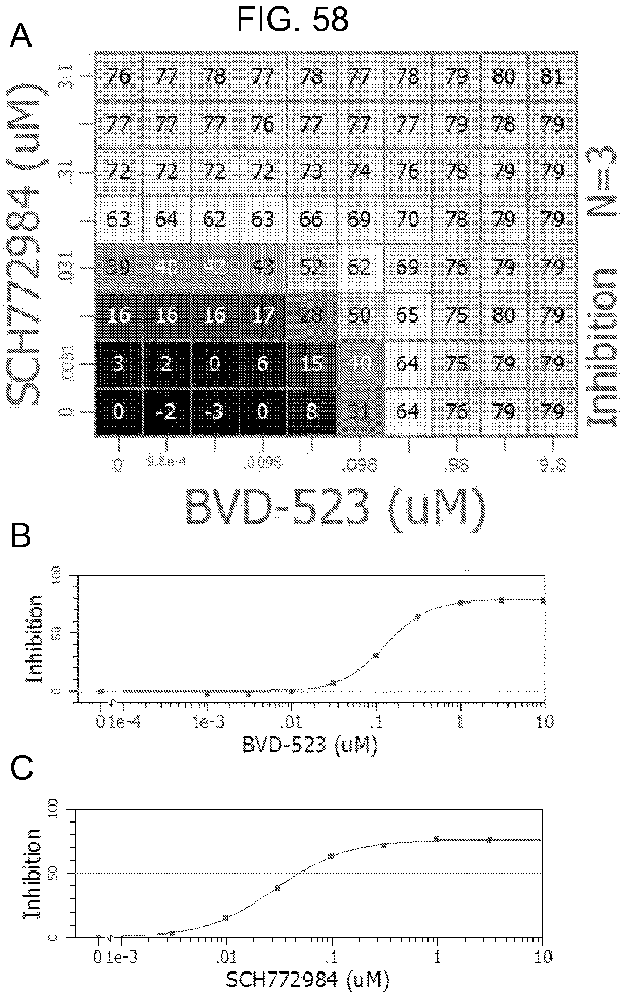

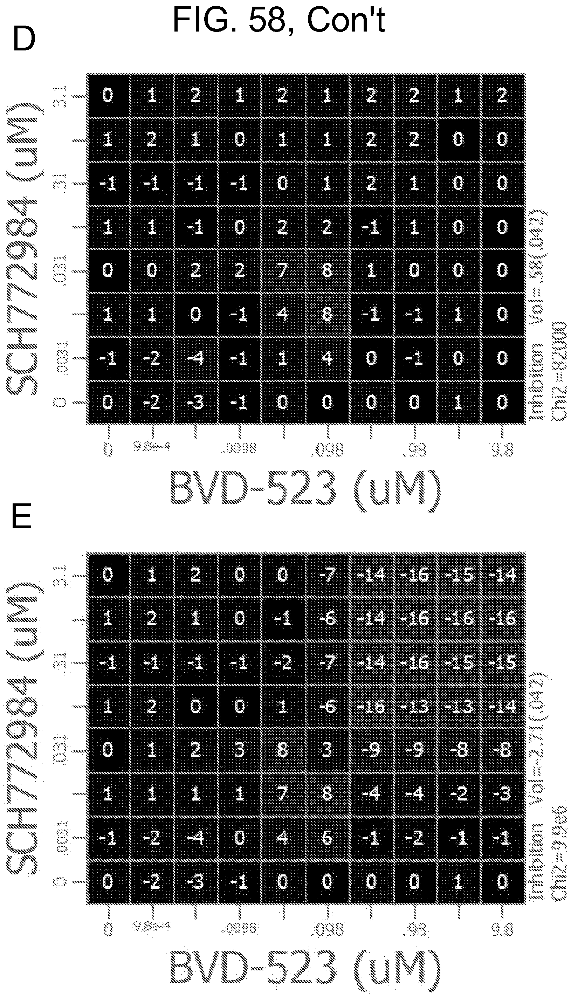

FIG. 58 shows the results of the combination of BVD-523 and SCH772984. FIG. 58A shows a dose matrix showing inhibition (%) for the combination in A375 cells. FIG. 58B - FIG. 58C show the results of single agent proliferation assays for the combination in 58A. FIG. 58D shows Loewe excess for the combination in 58A and FIG. 58E shows Bliss excess for the combination in 58A.

### DETAILED DESCRIPTION OF THE INVENTION

One embodiment of the present invention is a method of treating or ameliorating the effects of a cancer in a subject in need thereof. The method comprises administering to the subject an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, to treat or ameliorate the effects of the cancer.

As used herein, the terms "treat," "treating," "treatment" and grammatical variations thereof mean subjecting an individual subject to a protocol, regimen, process or remedy, in which it is desired to obtain a physiologic response or outcome in that subject, e.g., a patient. In particular, the methods and compositions of the present invention may be used to slow the development of disease symptoms or delay the onset of the disease or condition, or halt the progression of disease development. However, because every treated subject may not respond to a particular treatment protocol, regimen, process or remedy, treating does not require that the desired physiologic response or outcome be achieved in each and every subject or subject population, e.g., patient population. Accordingly, a given subject or subject population, e.g., patient population may fail to respond or respond inadequately to treatment.

As used herein, the terms "ameliorate", "ameliorating" and grammatical variations thereof mean to decrease the severity of the symptoms of a disease in a subject.

In the present invention, cancers include both solid and hemotologic cancers. Non-limiting examples of solid cancers include adrenocortical carcinoma, anal cancer, bladder cancer, bone cancer (such as osteosarcoma), brain cancer, breast cancer, carcinoid cancer, carcinoma, cervical cancer, colon cancer, endometrial cancer, esophageal cancer, extrahepatic bile duct cancer, Ewing family of cancers, extracranial germ cell cancer, eye cancer, gallbladder cancer, gastric cancer, germ cell tumor, gestational trophoblastic tumor, head and neck cancer, hypopharyngeal cancer, islet cell carcinoma, kidney cancer, large intestine cancer, laryngeal cancer, leukemia, lip and oral cavity cancer, liver tumor/cancer, lung tumor/cancer, lymphoma, malignant mesothelioma, Merkel cell carcinoma, mycosis fungoides, myelodysplastic syndrome, myeloproliferative disorders, nasopharyngeal cancer, neuroblastoma, oral cancer, oropharyngeal cancer, osteosarcoma, ovarian epithelial cancer, ovarian germ cell cancer, pancreatic cancer, paranasal sinus and nasal cavity cancer, parathyroid cancer, penile cancer, pituitary cancer, plasma cell neoplasm, prostate cancer, rhabdomyosarcoma, rectal cancer, renal cell cancer, transitional cell cancer of the renal pelvis and ureter, salivary gland cancer, Sezary syndrome, skin cancers (such as cutaneous t-cell lymphoma, Kaposi's sarcoma, mast cell tumor, and melanoma), small intestine cancer, soft tissue sarcoma, stomach cancer, testicular cancer, thymoma, thyroid cancer, urethral cancer, uterine cancer, vaginal cancer, vulvar cancer, and Wilms' tumor.

Examples of hematologic cancers include, but are not limited to, leukemias, such as adult/childhood acute lymphoblastic leukemia, adult/childhood acute myeloid leukemia, chronic lymphocytic leukemia, chronic myelogenous leukemia, and hairy cell leukemia, lymphomas, such as AIDS-related lymphoma, cutaneous T-cell lymphoma, adult/childhood Hodgkin lymphoma, mycosis fungoides, adult/childhood non-Hodgkin lymphoma, primary central nervous system lymphoma, Sézary syndrome, cutaneous T-cell lymphoma, and Waldenstrom macroglobulinemia, as well as other proliferative disorders such as chronic myeloproliferative disorders, Langerhans cell histiocytosis, multiple myeloma/plasma cell neoplasm, myelodysplastic syndromes, and myelodysplastic/myeloproliferative neoplasms.

A preferred set of cancers that may be treated according to the present invention include a cancer of the large intestine, breast cancer, pancreatic cancer, skin cancer, endometrial cancer, neuroblastoma, leukemia, lymphoma, liver cancer, lung cancer, testicular cancer, and thyroid cancer. Preferably, the cancer is melanoma.

As used herein, a "subject" is a mammal, preferably, a human. In addition to humans, categories of mammals within the scope of the present invention include, for example, farm animals, domestic animals, laboratory animals, etc. Some examples of farm animals include cows, pigs, horses, goats, etc. Some examples of domestic animals include dogs, cats, etc. Some examples of laboratory animals include primates, rats, mice, rabbits, guinea pigs, etc.

In the present invention, BVD-523 corresponds to a compound according to formula (I): 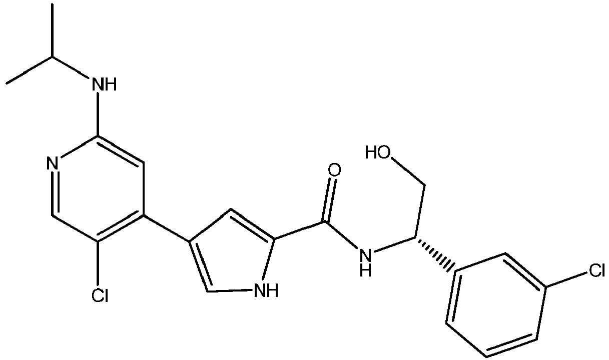 and pharmaceutically acceptable salts thereof. BVD-523 may be synthesized according to the methods disclosed, e.g., in U.S. Patent No. 7,354,939. Enantiomers and racemic mixtures of both enantiomers of BVD-523 are also contemplated within the scope of the present invention. BVD-523 is an ERK1/2 inhibitor with a mechanism of action that is believed to be, e.g., unique and distinct from certain other ERK1/2 inhibitors, such as SCH772984 and the pyrimidinal structure used by Hatzivassiliou *et al.* (2012). For example, other ERK1/2 inhibitors, such as SCH772984, inhibit autophosphorylation of ERK (Morris *et al.,* 2013), whereas BVD-523 allows for the autophosphorylation of ERK while still inhibiting ERK. (See, e.g., FIG. 18).

As used herein, a "MEK inhibitor", such as a type 2 MEK inhibitor means those substances that (i) directly interact with MEK *(i.e.* MEK2), e.g. by binding to MEK (i.e. MEK2) and (ii) decrease the expression or the activity of MEK (i.e. MEK2). Therefore, inhibitors that act upstream of MEK (i.e. MEK2), such as RAS inhibitors and RAF inhibitors, are not MEK *(i.e.* MEK2) inhibitors according to the present invention. As noted above, MEK inhibitors may be classified into two types depending on whether the inhibitor competes with ATP. As used herein, "Type 1" MEK inhibitors mean those inhibitors that compete with ATP for binding to MEK. "Type 2" MEK inhibitors means those that do not compete with ATP for binding to MEK.

Non-limiting examples of type 2 MEK inhibitors according to the present invention include anthrax toxin, lethal factor portion of anthrax toxin, ARRY-142886 (6-(4-bromo-2-chloro-phenylamino)-7-fluoro-3-methyl-3H-benzoimidazole-5-carboxylic acid (2-hydroxy-ethoxy)-amide) (Array BioPharma), ARRY-438162 (Array BioPharma), AS-1940477 (Astellas), MEK162 (Array BioPharma), PD 098059 (2-(2'-amino-3'-methoxyphenyl)-oxanaphthalen-4-one), PD 184352 (CI-1040), PD-0325901 (Pfizer), pimasertib (Santhera Pharmaceuticals), refametinib (AstraZeneca), selumetinib (AZD6244) (AstraZeneca), TAK-733 (Takeda), trametinib (Japan Tobacco), U0126 (1,4-diamino-2,3-dicyano-1,4-bis(2-aminophenylthio)butadiene) (Sigma), RDEA119 (Ardea Biosciences/Bayer), pharmaceutically acceptable salts thereof, and combinations thereof. Preferably, the type 2 MEK inhibitor is trametinib or a pharmaceutically acceptable salt thereof.

In an additional aspect of this embodiment, the subject with cancer has a somatic RAS or BRAF mutation. As used herein, "somatic mutation" means a change occurring in any cell that is not destined to become a germ cell. The mutation may be, e.g., a substitution, deletion, insertion, or a fusion. Preferably, the RAS mutation is a mutation in H-RAS, N-RAS, or K-RAS. The following Tables 1, 2 and 3 show the SEQ ID Nos. of representative nucleic acid and amino acid sequences of wild type H-RAS, K-RAS, and N-RAS from various animals, respectively. These sequences may be used in methods for identifying subjects with a mutant RAS genotype (such as in the methods set forth below).

**Table 1 H-RAS sequences**

| **SEQ ID No.** | **polypeptide or nucleic acid sequence** | **Organism** | **Other Information** |
|---|---|---|---|
| 1 | nucleic acid | human | isoform 1 |
| 2 | polypeptide | human | isoform 1 |
| 3 | nucleic acid | human | isoform 2 |
| 4 | polypeptide | human | isoform 2 |
| 5 | nucleic acid | human | isoform 3 |
| 6 | polypeptide | human | isoform 3 |
| 7 | nucleic acid | rat (Rattus norvegicus) | variant 1 |
| 8 | polypeptide | rat (Rattus norvegicus) | variant 1 |
| 9 | nucleic acid | rat (Rattus norvegicus) | variant 2 |
| 10 | polypeptide | rat (Rattus norvegicus) | variant 2 |
| 11 | nucleic acid | mouse, Mus musculus | |
| 12 | polypeptide | mouse, Mus musculus | |
| 13 | nucleic acid | guinea pig, Cavia porcellus | variant 1 |
| 14 | polypeptide | guinea pig, Cavia porcellus | variant 1 |
| 15 | nucleic acid | guinea pig, Cavia porcellus | variant 2 |
| 16 | polypeptide | guinea pig, Cavia porcellus | variant 2 |
| 17 | nucleic acid | guinea pig, Cavia porcellus | variant 3 |
| 18 | polypeptide | guinea pig, Cavia porcellus | variant 3 |
| 19 | nucleic acid | guinea pig, Cavia porcellus | variant 4 |
| 20 | polypeptide | guinea pig, Cavia porcellus | variant 4 |
| 21 | nucleic acid | dog, Canis lupus familiaris | variant 1 |
| 22 | polypeptide | dog, Canis lupus | variant 1 |
| | | familiaris | |
| 23 | nucleic acid | dog, Canis lupus familiaris | variant 2 |
| 24 | polypeptide | dog, Canis lupus familiaris | variant 2 |
| 25 | nucleic acid | cat, Felis catus | variant 1 |
| 26 | polypeptide | cat, Felis catus | variant 1 |
| 27 | nucleic acid | cat, Felis catus | variant 2 |
| 28 | polypeptide | cat, Felis catus | variant 2 |
| 29 | nucleic acid | cow, Bos taurus | variant 1 |
| 30 | polypeptide | cow, Bos taurus | variant 1 |
| 31 | nucleic acid | cow, Bos taurus | variant 2 |
| 32 | polypeptide | cow, Bos taurus | variant 2 |
| 33 | nucleic acid | cow, Bos taurus | variant X1 |
| 34 | polypeptide | cow, Bos taurus | variant X1 |
| 35 | nucleic acid | chicken, Gallus gallus | |
| 36 | polypeptide | chicken, Gallus gallus | |

**Table 2 K- RAS sequences**

| **SEQ ID No.** | **polypeptide or nucleic acid sequence** | **Organism** | **Other Information** |
|---|---|---|---|
| 37 | nucleic acid | human | isoform a |
| 38 | polypeptide | human | isoform a |
| 39 | nucleic acid | human | isoform b |
| 40 | polypeptide | human | isoform b |
| 41 | nucleic acid | rat (Rattus norvegicus) | |
| 42 | polypeptide | rat (Rattus norvegicus) | |
| 43 | nucleic acid | mouse, Mus musculus | |
| 44 | polypeptide | mouse, Mus musculus | |
| 45 | nucleic acid | rabbit, Oryctolagus cuniculus | |
| 46 | polypeptide | rabbit, Oryctolagus cuniculus | |
| 47 | nucleic acid | guinea pig, Cavia porcellus | variant 1 |
| 48 | polypeptide | guinea pig, Cavia porcellus | variant 1 |
| 49 | nucleic acid | guinea pig, Cavia porcellus | variant 2 |
| 50 | polypeptide | guinea pig, Cavia porcellus | variant 2 |
| 51 | nucleic acid | dog, Canis lupus familiaris | variant 1 |
| 52 | polypeptide | dog, Canis lupus familiaris | variant 1 |
| 53 | nucleic acid | dog, Canis lupus familiaris | variant 2 |
| 54 | polypeptide | dog, Canis lupus familiaris | variant 2 |
| 55 | nucleic acid | cat, Felis catus | variant 1 |
| 56 | polypeptide | cat, Felis catus | variant 1 |
| 57 | nucleic acid | cat, Felis catus | variant 2 |
| 58 | polypeptide | cat, Felis catus | variant 2 |
| 59 | nucleic acid | cow, Bos taurus | |
| 60 | polypeptide | cow, Bos taurus | |
| 61 | nucleic acid | cow, Bos taurus | variant X2 |
| 62 | polypeptide | cow, Bos taurus | variant X2 |
| 63 | nucleic acid | cow, Bos taurus | variant X3 |
| 64 | polypeptide | cow, Bos taurus | variant X3 |
| 65 | nucleic acid | chicken, Gallus gallus | |
| 66 | polypeptide | chicken, Gallus gallus | |

**Table 3 N-RAS sequences**

| **SEQ ID No.** | **polypeptide or nucleic acid sequence** | **Organism** | **Other Information** |
|---|---|---|---|
| 67 | nucleic acid | human | |
| 68 | polypeptide | human | |
| 69 | nucleic acid | rat (Rattus norvegicus) | |
| 70 | polypeptide | rat (Rattus norvegicus) | |
| 71 | nucleic acid | mouse, Mus musculus | |
| 72 | polypeptide | mouse, Mus musculus | |
| 73 | nucleic acid | guinea pig, Cavia porcellus | |
| 74 | polypeptide | guinea pig, Cavia porcellus | |
| 75 | nucleic acid | guinea pig, Cavia porcellus | variant X1 |
| 76 | polypeptide | guinea pig, Cavia porcellus | variant X1 |
| 77 | nucleic acid | dog, Canis lupus familiaris | |
| 78 | polypeptide | dog, Canis lupus familiaris | |
| 79 | nucleic acid | cat, Felis catus | |
| 80 | polypeptide | cat, Felis catus | |
| 81 | nucleic acid | cow, Bos taurus | |
| 82 | polypeptide | cow, Bos taurus | |
| 83 | nucleic acid | chicken, Gallus gallus | |
| 84 | polypeptide | chicken, Gallus gallus | |

The following Table 4 shows the SEQ ID Nos. of representative nucleic acid and amino acid sequences of wild type BRAF from various animals. These wild type sequences may be used in methods (such as the methods set forth below) for identifying subjects with a mutant BRAF genotype.

**Table 4 BRAF sequences**

| **SEQ ID NO** | **Nucleic acid or polypeptide** | **Organism** | **Other information** |
|---|---|---|---|
| 85 | nucleic acid | human | |
| 86 | polypeptide | human | |
| 87 | nucleic acid | rat (Rattus norvegicus) | |
| 88 | polypeptide | rat (Rattus norvegicus) | |
| 89 | nucleic acid | mouse, Mus musculus | |
| 90 | polypeptide | mouse, Mus musculus | |
| 91 | nucleic acid | rabbit, Oryctolagus cuniculus | |
| 92 | polypeptide | rabbit, Oryctolagus cuniculus | |
| 93 | nucleic acid | guinea pig, Cavia porcellus | |
| 94 | polypeptide | guinea pig, Cavia porcellus | |
| 95 | nucleic acid | dog, Canis lupus familiaris | variant x1 |
| 96 | polypeptide | dog, Canis lupus familiaris | variant x1 |
| 97 | nucleic acid | dog, Canis lupus familiaris | variant x2 |
| 98 | polypeptide | dog, Canis lupus familiaris | variant x2 |
| 99 | nucleic acid | cat, Felis catus | |
| 100 | polypeptide | cat, Felis catus | |
| 101 | nucleic acid | cow, Bos taurus | variant X1 |
| 102 | polypeptide | cow, Bos taurus | variant X1 |
| 103 | nucleic acid | cow, Bos taurus | variant X2 |
| 104 | polypeptide | cow, Bos taurus | variant X2 |
| 105 | nucleic acid | cow, Bos taurus | variant X3 |
| 106 | polypeptide | cow, Bos taurus | variant X3 |
| 107 | nucleic acid | cow, Bos taurus | variant X4 |
| 108 | polypeptide | cow, Bos taurus | variant X4 |
| 109 | nucleic acid | cow, Bos taurus | variant X5 |
| 110 | polypeptide | cow, Bos taurus | variant X5 |
| 111 | nucleic acid | cow, Bos taurus | variant X6 |
| 112 | polypeptide | cow, Bos taurus | variant X6 |
| 113 | nucleic acid | cow, Bos taurus | variant X7 |
| 114 | polypeptide | cow, Bos taurus | variant X7 |
| 115 | nucleic acid | cow, Bos taurus | variant X8 |
| 116 | polypeptide | cow, Bos taurus | variant X8 |
| 117 | nucleic acid | cow, Bos taurus | variant X9 |
| 118 | polypeptide | cow, Bos taurus | variant X9 |
| 119 | nucleic acid | cow, Bos taurus | variant X10 |
| 120 | polypeptide | cow, Bos taurus | variant X10 |
| 121 | nucleic acid | cow, Bos taurus | variant X11 |
| 122 | polypeptide | cow, Bos taurus | variant X11 |
| 123 | nucleic acid | cow, Bos taurus | variant 2 |
| 124 | polypeptide | cow, Bos taurus | variant 2 |
| 125 | nucleic acid | horse, Equus caballus | |
| 126 | polypeptide | horse, Equus caballus | |
| 127 | nucleic acid | chicken, Gallus gallus | |
| 128 | polypeptide | chicken, Gallus gallus | |

Methods for identifying mutations in nucleic acids, such as the above identified RAS and BRAF genes, are known in the art. Nucleic acids may be obtained from biological samples. In the present invention, biological samples include, but are not limited to, blood, plasma, urine, skin, saliva, and biopsies. Biological samples are obtained from a subject by routine procedures and methods which are known in the art.

Non-limiting examples of methods for identifying mutations include PCR, sequencing, hybrid capture, in-solution capture, molecular inversion probes, fluorescent *in situ* hybridization (FISH) assay, and combinations thereof.

Various sequencing methods are known in the art. These include, but are not limited to, Sanger sequencing (also referred to as dideoxy sequencing) and various sequencing-by-synthesis (SBS) methods as disclosed in, e.g., Metzker 2005, sequencing by hybridization, by ligation (for example, WO 2005021786), by degradation (for example, U.S. Patent Nos. 5,622,824 and 6,140,053) and nanopore sequencing (which is commercially available from Oxford Nanopore Technologies, UK). In deep sequencing techniques, a given nucleotide in the sequence is read more than once during the sequencing process. Deep sequencing techniques are disclosed in e.g., U.S. Patent Publication No. 20120264632 and International Patent Publication No. WO2012125848.

PCR-based methods for detecting mutations are known in the art and employ PCR amplification, where each target sequence in the sample has a corresponding pair of unique, sequence-specific primers. For example, the polymerase chain reaction-restriction fragment length polymorphism (PCR-RFLP) method allows for rapid detection of mutations after the genomic sequences are amplified by PCR. The mutation is discriminated by digestion with specific restriction endonucleases and is identified by electrophoresis. *See, e.g.,* Ota *et al.,* 2007. Mutations may also be detected using real time PCR. *See, e.g.,* International Application publication No. WO2012046981.

Hybrid capture methods are known in the art and are disclosed in e.g., U.S. Patent Publication No. 20130203632 and U.S. Patent Nos. 8,389,219 and 8,288,520. These methods are based on the selective hybridization of the target genomic regions to user-designed oligonucleotides. The hybridization can be to oligonucleotides immobilized on high or low density microarrays (on-array capture), or solution-phase hybridization to oligonucleotides modified with a ligand (e.g. biotin) which can subsequently be immobilized to a solid surface, such as a bead (in-solution capture).

Molecular Inversion Probe (MIP) techniques are known in the art and are disclosed in e.g., Absalan *et al.,* 2008. This method uses MIP molecules, which are special "padlock" probes (Nilsson et al, 1994) for genotyping. A MIP molecule is a linear oligonucleotide that contains specific regions, universal sequences, restriction sites and a Tag (index) sequence (16-22 bp). A MIP hybridizes directly around the genetic marker/SNP of interest. The MIP method may also use a number of "padlock" probe sets that hybridize to genomic DNA in parallel (Hardenbol *et al.,* 2003). In case of a perfect match, genomic homology regions are ligated by undergoing an inversion in configuration (as suggested by the name of the technique) and creating a circular molecule. After the first restriction, all molecules are amplified with universal primers. Amplicons are restricted again to ensure short fragments for hybridization on a microarray. Generated short fragments are labeled and, through a Tag sequence, hybridized to a cTag (complementary strand for index) on an array. After the formation of Tag-cTag duplex, a signal is detected.

In another aspect of this embodiment, the method further comprises administering to the subject at least one additional therapeutic agent effective for treating or ameliorating the effects of the cancer. The additional therapeutic agent may be selected from the group consisting of an antibody or fragment thereof, a cytotoxic agent, a toxin, a radionuclide, an immunomodulator, a photoactive therapeutic agent, a radiosensitizing agent, a hormone, an anti-angiogenesis agent, and combinations thereof.

As used herein, an "antibody" encompasses naturally occurring immunoglobulins as well as non-naturally occurring immunoglobulins, including, for example, single chain antibodies, chimeric antibodies (e.g., humanized murine antibodies), and heteroconjugate antibodies (e.g., bispecific antibodies). Fragments of antibodies include those that bind antigen, (e.g., Fab', F(ab')₂, Fab, Fv, and rlgG). See also, e.g., Pierce Catalog and Handbook, 1994-1995 (Pierce Chemical Co., Rockford, III.); Kuby, J., Immunology, 3rd Ed., W.H. Freeman & Co., New York (1998). The term antibody also includes bivalent or bispecific molecules, diabodies, triabodies, and tetrabodies. The term "antibody" further includes both polyclonal and monoclonal antibodies.

Examples of therapeutic antibodies that may be used in the present invention include rituximab (Rituxan), Cetuximab (Erbitux), bevacizumab (Avastin), and Ibritumomab (Zevalin).

Cytotoxic agents according to the present invention include DNA damaging agents, antimetabolites, anti-microtubule agents, antibiotic agents, etc. DNA damaging agents include alkylating agents, platinum-based agents, intercalating agents, and inhibitors of DNA replication. Non-limiting examples of DNA alkylating agents include cyclophosphamide, mechlorethamine, uramustine, melphalan, chlorambucil, ifosfamide, carmustine, lomustine, streptozocin, busulfan, temozolomide, pharmaceutically acceptable salts thereof, prodrugs, and combinations thereof. Non-limiting examples of platinum-based agents include cisplatin, carboplatin, oxaliplatin, nedaplatin, satraplatin, triplatin tetranitrate, pharmaceutically acceptable salts thereof, prodrugs, and combinations thereof. Non-limiting examples of intercalating agents include doxorubicin, daunorubicin, idarubicin, mitoxantrone, pharmaceutically acceptable salts thereof, prodrugs, and combinations thereof. Non-limiting examples of inhibitors of DNA replication include irinotecan, topotecan, amsacrine, etoposide, etoposide phosphate, teniposide, pharmaceutically acceptable salts thereof, prodrugs, and combinations thereof. Antimetabolites include folate antagonists such as methotrexate and premetrexed, purine antagonists such as 6-mercaptopurine, dacarbazine, and fludarabine, and pyrimidine antagonists such as 5-fluorouracil, arabinosylcytosine, capecitabine, gemcitabine, decitabine, pharmaceutically acceptable salts thereof, prodrugs, and combinations thereof. Anti-microtubule agents include without limitation vinca alkaloids, paclitaxel (Taxol^{®}), docetaxel (Taxotere^{®}), and ixabepilone (Ixempra^{®}). Antibiotic agents include without limitation actinomycin, anthracyclines, valrubicin, epirubicin, bleomycin, plicamycin, mitomycin, pharmaceutically acceptable salts thereof, prodrugs, and combinations thereof.

Cytotoxic agents according to the present invention also include an inhibitor of the PI3K/Akt pathway. Non-limiting examples of an inhibitor of the PI3K/Akt pathway according to the present invention include A-674563 (CAS # 552325-73-2), AGL 2263, AMG-319 (Amgen, Thousand Oaks, CA), AS-041164 (5-benzo[1,3]dioxol-5-ylmethylene-thiazolidine-2,4-dione), AS-604850 (5-(2,2-Difluoro-benzo[1,3]dioxol-5-ylmethylene)-thiazolidine-2,4-dione), AS-605240 (5-quinoxilin-6-methylene-1,3-thiazolidine-2,4-dione), AT7867 (CAS # 857531-00-1), benzimidazole series, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 (CAS # 1007207-67-1), CHR-4432 (Chroma Therapeutics, Ltd., Abingdon, UK), FPA 124 (CAS # 902779-59-3), GS-1101 (CAL-101) (Gilead Sciences), GSK 690693 (CAS # 937174-76-0), H-89 (CAS # 127243-85-0), Honokiol, IC87114 (Gilead Science), IPI-145 (Intellikine Inc.), KAR-4139 (Karus Therapeutics, Chilworth, UK), KAR-4141 (Karus Therapeutics), KIN-1 (Karus Therapeutics), KT 5720 (CAS # 108068-98-0), Miltefosine, MK-2206 dihydrochloride (CAS # 1032350-13-2), ML-9 (CAS # 105637-50-1), Naltrindole Hydrochloride, OXY-111A (NormOxys Inc., Brighton, MA), perifosine, PHT-427 (CAS # 1191951-57-1), PI3 kinase delta inhibitor, Merck KGaA (Merck & Co., Whitehouse Station, NJ), PI3 kinase delta inhibitors, Genentech (Roche Holdings Inc.), PI3 kinase delta inhibitors, Incozen (Incozen Therapeutics, Pvt. Ltd., Hyderabad, India), PI3 kinase delta inhibitors-2, Incozen (Incozen Therapeutics), PI3 kinase inhibitor, Roche-4 (Roche Holdings Inc.), PI3 kinase inhibitors, Roche (Roche Holdings Inc.), PI3 kinase inhibitors, Roche-5 (Roche Holdings Inc.), PI3-alpha/delta inhibitors, Pathway Therapeutics (Pathway Therapeutics Ltd., South San Francisco, CA), PI3-delta inhibitors, Cellzome (Cellzome AG, Heidelberg, Germany), PI3-delta inhibitors, Intellikine (Intellikine Inc., La Jolla, CA), Pl3-delta inhibitors, Pathway Therapeutics-1 (Pathway Therapeutics Ltd.), Pl3-delta inhibitors, Pathway Therapeutics-2 (Pathway Therapeutics Ltd.), PI3-delta/gamma inhibitors, Cellzome (Cellzome AG), PI3-delta/gamma inhibitors, Cellzome (Cellzome AG), PI3-delta/gamma inhibitors, Intellikine (Intellikine Inc.), PI3-delta/gamma inhibitors, Intellikine (Intellikine Inc.), PI3-delta/gamma inhibitors, Pathway Therapeutics (Pathway Therapeutics Ltd.), PI3-delta/gamma inhibitors, Pathway Therapeutics (Pathway Therapeutics Ltd.), PI3-gamma inhibitor Evotec (Evotec), PI3-gamma inhibitor, Cellzome (Cellzome AG), PI3-gamma inhibitors, Pathway Therapeutics (Pathway Therapeutics Ltd.), PI3K delta/gamma inhibitors, Intellikine-1 (Intellikine Inc.), PI3K delta/gamma inhibitors, Intellikine-1 (Intellikine Inc.), pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, Tetrahydro Curcumin, TG100-115 (Targegen Inc., San Diego, CA), Triciribine, X-339 (Xcovery, West Palm Beach, FL), XL-499 (Evotech, Hamburg, Germany), pharmaceutically acceptable salts thereof, and combinations thereof.

In the present invention, the term "toxin" means an antigenic poison or venom of plant or animal origin. An example is diphtheria toxin or portions thereof.

In the present invention, the term "radionuclide" means a radioactive substance administered to the patient, e.g., intravenously or orally, after which it penetrates via the patient's normal metabolism into the target organ or tissue, where it delivers local radiation for a short time. Examples of radionuclides include, but are not limited to, I-125, At-211, Lu-177, Cu-67, I-131, Sm-153, Re-186, P-32, Re-188, In-114m, and Y-90.

In the present invention, the term "immunomodulator" means a substance that alters the immune response by augmenting or reducing the ability of the immune system to produce antibodies or sensitized cells that recognize and react with the antigen that initiated their production. Immunomodulators may be recombinant, synthetic, or natural preparations and include cytokines, corticosteroids, cytotoxic agents, thymosin, and immunoglobulins. Some immunomodulators are naturally present in the body, and certain of these are available in pharmacologic preparations. Examples of immunomodulators include, but are not limited to, granulocyte colony-stimulating factor (G-CSF), interferons, imiquimod and cellular membrane fractions from bacteria, IL-2, IL-7, IL-12, CCL3, CCL26, CXCL7, and synthetic cytosine phosphate-guanosine (CpG).

In the present invention, the term "photoactive therapeutic agent" means compounds and compositions that become active upon exposure to light. Certain examples of photoactive therapeutic agents are disclosed, e.g., in U.S. Patent Application Serial No. 2011/0152230 A1, "Photoactive Metal Nitrosyls For Blood Pressure Regulation And Cancer Therapy."

In the present invention, the term "radiosensitizing agent" means a compound that makes tumor cells more sensitive to radiation therapy. Examples of radiosensitizing agents include misonidazole, metronidazole, tirapazamine, and trans sodium crocetinate.

In the present invention, the term "hormone" means a substance released by cells in one part of a body that affects cells in another part of the body. Examples of hormones include, but are not limited to, prostaglandins, leukotrienes, prostacyclin, thromboxane, amylin, antimullerian hormone, adiponectin, adrenocorticotropic hormone, angiotensinogen, angiotensin, vasopressin, atriopeptin, brain natriuretic peptide, calcitonin, cholecystokinin, corticotropin-releasing hormone, encephalin, endothelin, erythropoietin, follicle-stimulating hormone, galanin, gastrin, ghrelin, glucagon, gonadotropin-releasing hormone, growth hormone-releasing hormone, human chorionic gonadotropin, human placental lactogen, growth hormone, inhibin, insulin, somatomedin, leptin, liptropin, luteinizing hormone, melanocyte stimulating hormone, motilin, orexin, oxytocin, pancreatic polypeptide, parathyroid hormone, prolactin, prolactin releasing hormone, relaxin, renin, secretin, somatostain, thrombopoietin, thyroid-stimulating hormone, testosterone, dehydroepiandrosterone, androstenedione, dihydrotestosterone, aldosterone, estradiol, estrone, estriol, cortisol, progesterone, calcitriol, and calcidiol.

Some compounds interfere with the activity of certain hormones or stop the production of certain hormones. These hormone-interfering compounds include, but are not limited to, tamoxifen (Nolvadex^{®}), anastrozole (Arimidex^{®}), letrozole (Femara^{®}), and fulvestrant (Faslodex^{®}). Such compounds are also within the meaning of hormone in the present invention.

As used herein, an "anti-angiogenesis" agent means a substance that reduces or inhibits the growth of new blood vessels, such as, e.g., an inhibitor of vascular endothelial growth factor (VEGF) and an inhibitor of endothelial cell migration. Anti-angiogenesis agents include without limitation 2-methoxyestradiol, angiostatin, bevacizumab, cartilage-derived angiogenesis inhibitory factor, endostatin, IFN-α, IL-12, itraconazole, linomide, platelet factor-4, prolactin, SU5416, suramin, tasquinimod, tecogalan, tetrathiomolybdate, thalidomide, thrombospondin, thrombospondin, TNP-470, ziv-aflibercept, pharmaceutically acceptable salts thereof, prodrugs, and combinations thereof.

In another aspect of this embodiment, administration of the first and second anti-cancer agents provides a synergistic effect compared to administration of either anti-cancer agent alone. As used herein, "synergistic" means more than additive. Synergistic effects may be measured by various assays known in the art, including but not limited to those disclosed herein, such as the excess over bliss assay.

Another embodiment of the present invention is a method of treating or ameliorating the effects of a cancer in a subject in need thereof. The method comprises administering to the subject an effective amount of (i) a first anti-cancer agent, which is BVD-523 or a pharmaceutically acceptable salt thereof and (ii) a second anti-cancer agent, which is trametinib or a pharmaceutically acceptable salt thereof, to treat or ameliorate the effects of the cancer.

Suitable and preferred subjects and various types of cancer are as disclosed herein. In this embodiment, the methods may be used to treat the cancers disclosed above, including those cancers with the mutational backgrounds identified above. Methods of identifying such mutations are also as set forth above.

In one aspect of this embodiment, the BVD-523 or a pharmaceutically acceptable salt thereof is administered in the form of a pharmaceutical composition further comprising a pharmaceutically acceptable carrier or diluent.

In a further aspect of this embodiment, the trametinib or a pharmaceutically acceptable salt thereof is administered in the form of a pharmaceutical composition further comprising a pharmaceutically acceptable carrier or diluent.

In an additional aspect of this embodiment, the method further comprises administering to the subject at least one additional therapeutic agent, preferably an inhibitor of the PI3K/Akt pathway, as disclosed herein.

In another aspect of this embodiment, administration of the first and second anti-cancer agents provides a synergistic effect compared to administration of either anti-cancer agent alone.

A further embodiment of the present invention is a method of effecting cancer cell death. The method comprises contacting the cancer cell with an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof.

Suitable and preferred type 2 MEK inhibitors are as disclosed herein. In this embodiment, effecting cancer cell death may be accomplished in cancer cells having various mutational backgrounds and/or that are characterized as disclosed above. Methods of identifying such mutations are also as set forth above.

In one aspect of this embodiment, the cancer cell is a mammalian cancer cell. Preferably, the mammalian cancer cell is obtained from a mammal selected from the group consisting of humans, primates, farm animals, and domestic animals. More preferably, the mammalian cancer cell is a human cancer cell.

The methods of this embodiment, which may be carried out *in vitro* or *in vivo,* may be used to effect cancer cell death, by e.g., killing cancer cells, in cells of the types of cancer disclosed herein.

In an additional aspect of this embodiment, the method further comprises contacting the cancer cell with at least one additional therapeutic agent, preferably an inhibitor of the PI3K/Akt pathway, as disclosed herein.

In another aspect of this embodiment, contacting the cancer cell with the first and second anti-cancer agents provides a synergistic effect compared to contacting the cancer cell with either anti-cancer agent alone. In this embodiment, "contacting" means bringing BVD-523 and the Type 2 MEK inhibitors, and optionally one or more additional therapeutic agents into close proximity to the cancer cells. This may be accomplished using conventional techniques of drug delivery to mammals or in the *in vitro* situation by, e.g., providing BVD-523 and the Type 2 MEK inhibitors, and optionally other therapeutic agents to a culture media in which the cancer cells are located.

An additional embodiment of the present invention is a kit for treating or ameliorating the effects of a cancer in a subject in need thereof. The kit comprises an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, packaged together with instructions for their use.

The kits may also include suitable storage containers, e.g., ampules, vials, tubes, etc., for each anti-cancer agent of the present invention (which, e.g., may be in the form of pharmaceutical compositions) and other reagents, e.g., buffers, balanced salt solutions, etc., for use in administering the anti-cancer agents to subjects. The anti-cancer agents of the invention and other reagents may be present in the kits in any convenient form, such as, e.g., in a solution or in a powder form. The kits may further include a packaging container, optionally having one or more partitions for housing the pharmaceutical composition and other optional reagents.

In this embodiment, suitable and preferred type 2 MEK inhibitors and subjects are as set forth above. In this embodiment, the kit may be used to treat the cancers disclosed above, including those cancers with the mutational backgrounds identified herein. Methods of identifying such mutations are as set forth above.

In one aspect of this embodiment, the kit further comprises at least one additional therapeutic agent, preferably an inhibitor of the PI3K/Akt pathway, as disclosed herein.

In another aspect of this embodiment, administration of the first and second anti-cancer agents provides a synergistic effect compared to administration of either anti-cancer agent alone.

Another embodiment of the present invention is a pharmaceutical composition for treating or ameliorating the effects of a cancer in a subject in need thereof. The pharmaceutical composition comprises a pharmaceutically acceptable diluent or carrier and an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, wherein administration of the first and second anti-cancer agents provides a synergistic effect compared to administration of either anti-cancer agent alone.

Suitable and preferred subjects and type 2 MEK inhibitors are as disclosed herein. The pharmaceutical compositions of the invention may be used to treat the cancers disclosed above, including those cancers with the mutational backgrounds identified herein. Methods of identifying such mutations are also as set forth above.

In a further aspect of this embodiment, the pharmaceutical composition further comprises at least one additional therapeutic agent, preferably an inhibitor of the PI3K/Akt pathway, as disclosed herein.

The pharmaceutical compositions according to the present invention may be in a unit dosage form comprising both anti-cancer agents. In another aspect of this embodiment, the first anti-cancer agent is in a first unit dosage form and the second anti-cancer agent is in a second unit dosage form, separate from the first.

The first and second anti-cancer agents may be co-administered to the subject, either simultaneously or at different times, as deemed most appropriate by a physician. If the first and second anti-cancer agents are administered at different times, for example, by serial administration, the first anti-cancer agent may be administered to the subject before the second anti-cancer agent. Alternatively, the second anti-cancer agent may be administered to the subject before the first anti-cancer agent.

A further embodiment of the present invention is a method of treating or ameliorating the effects of a cancer in a subject in need thereof. The method comprises administering to the subject an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof, to treat or ameliorate the effects of the cancer.

Suitable and preferred subjects are as disclosed herein. In this embodiment, the methods may be used to treat the cancers disclosed above, including those cancers with the mutational backgrounds identified above. Methods of identifying such mutations are also as set forth above.

In an additional aspect of this embodiment, the method further comprises administering to the subject at least one additional therapeutic agent, preferably an inhibitor of the PI3K/Akt pathway, as disclosed herein.

In another aspect of this embodiment, administration of the first and second anti-cancer agents provides a synergistic effect compared to administration of either anti-cancer agent alone.

An additional embodiment of the present invention is a method of effecting cancer cell death. The method comprises contacting the cancer cell with an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof.

In this embodiment, the methods may be used to effect cell death in any of the cancers disclosed above, including those cancers with the mutational backgrounds identified herein. Methods of identifying such mutations are also as set forth above.

In one aspect of this embodiment, the cancer cell is a mammalian cancer cell. Preferably, the mammalian cancer cell is obtained from a mammal selected from the group consisting of humans, primates, farm animals, and domestic animals. More preferably, the mammalian cancer cell is a human cancer cell.

In another aspect of this embodiment, the method further comprises contacting the cancer cell with at least one additional therapeutic agent, preferably an inhibitor of the PI3K/Akt pathway, as disclosed herein.

In a further aspect of this embodiment, contacting the cancer with the first and second anti-cancer agents provides a synergistic effect compared to contacting the cancer cell with either anti-cancer agent alone. In this embodiment, "contacting" means bringing BVD-523 and the MEK inhibitors, and optionally one or more additional therapeutic agents into close proximity to the cancer cells. This may be accomplished using conventional techniques of drug delivery to mammals or in the *in vitro* situation by, e.g., providing BVD-523 and the MEK inhibitors, and optionally other therapeutic agents to a culture media in which the cancer cells are located.

The methods of this embodiment, which may be carried out *in vitro* or *in vivo,* may be used to effect cancer cell death, by e.g., killing cancer cells, in cells of the types of cancer disclosed herein.

Another embodiment of the present invention is a kit for treating or ameliorating the effects of a cancer in a subject in need thereof. The kit comprises an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof, packaged together with instructions for their use.

Suitable and preferred subjects are as disclosed herein. In this embodiment, the kit may be used to treat the cancers disclosed above, including those cancers with the mutational backgrounds identified herein. Methods of identifying such mutations are also as set forth above.

In another aspect of this embodiment, the kit further comprises at least one additional therapeutic agent, preferably an inhibitor of the PI3K/Akt pathway, as disclosed herein.

In a further aspect of this embodiment, administration of the first and second anti-cancer agents provides a synergistic effect compared to administration of either anti-cancer agent alone.

A further embodiment of the present invention is a pharmaceutical composition for treating or ameliorating the effects of a cancer in a subject in need thereof. The pharmaceutical composition comprises a pharmaceutically acceptable diluent or carrier and an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof, wherein administration of the first and second anti-cancer agents provides a synergistic effect compared to administration of either anti-cancer agent alone.

Suitable and preferred subjects are as disclosed herein. In this embodiment, the pharmaceutical composition may be used to treat the cancers disclosed above, including those cancers with the mutational backgrounds identified herein. Methods of identifying such mutations are also as set forth above.

In another aspect of this embodiment, the pharmaceutical composition further comprises at least one additional therapeutic agent, preferably an inhibitor of the PI3K/Akt pathway, as disclosed herein.

The pharmaceutical compositions according to this embodiment may be in a unit dosage form comprising both anti-cancer agents. In another aspect of this embodiment, the first anti-cancer agent is in a first unit dosage form and the second anti-cancer agent is in a second unit dosage form, separate from the first.

The first and second anti-cancer agents may be co-administered to the subject, either simultaneously or at different times, as deemed most appropriate by a physician. If the first and second anti-cancer agents are administered at different times, for example, by serial administration, the first anti-cancer agent may be administered to the subject before the second anti-cancer agent. Alternatively, the second anti-cancer agent may be administered to the subject before the first anti-cancer agent.

In the present invention, an "effective amount" or a "therapeutically effective amount" of a compound or composition disclosed herein is an amount of such compound or composition that is sufficient to effect beneficial or desired results as described herein when administered to a subject. Effective dosage forms, modes of administration, and dosage amounts may be determined empirically, and making such determinations is within the skill of the art. It is understood by those skilled in the art that the dosage amount will vary with the route of administration, the rate of excretion, the duration of the treatment, the identity of any other drugs being administered, the age, size, and species of mammal, e.g., human patient, and like factors well known in the arts of medicine and veterinary medicine. In general, a suitable dose of a composition according to the invention will be that amount of the composition, which is the lowest dose effective to produce the desired effect. The effective dose of a compound or composition of the present invention may be administered as two, three, four, five, six or more sub-doses, administered separately at appropriate intervals throughout the day.

A suitable, non-limiting example of a dosage of an anti-cancer agent disclosed herein is from about 1 mg/kg to about 2400 mg/kg per day, such as from about 1 mg/kg to about 1200 mg/kg per day, 75 mg/kg per day to about 300 mg/kg per day, including from about 1 mg/kg to about 100 mg/kg per day. Other representative dosages of such agents include about 1 mg/kg, 5 mg/kg, 10 mg/kg, 15 mg/kg, 20 mg/kg, 25 mg/kg, 30 mg/kg, 35 mg/kg, 40 mg/kg, 45 mg/kg, 50 mg/kg, 60 mg/kg, 70 mg/kg, 75 mg/kg, 80 mg/kg, 90 mg/kg, 100 mg/kg, 125 mg/kg, 150 mg/kg, 175 mg/kg, 200 mg/kg, 250 mg/kg, 300 mg/kg, 400 mg/kg, 500 mg/kg, 600 mg/kg, 700 mg/kg, 800 mg/kg, 900 mg/kg, 1000 mg/kg, 1100 mg/kg, 1200 mg/kg, 1300 mg/kg, 1400 mg/kg, 1500 mg/kg, 1600 mg/kg, 1700 mg/kg, 1800 mg/kg, 1900 mg/kg, 2000 mg/kg, 2100 mg/kg, 2200 mg/kg, and 2300 mg/kg per day. The effective dose of an anti-cancer agent disclosed herein, e.g., BVD-523 and a MEK inhibitor, may be administered as two, three, four, five, six or more sub-doses, administered separately at appropriate intervals throughout the day.

The anti-cancer agents or the pharmaceutical compositions of the present invention may be administered in any desired and effective manner: for oral ingestion, or as an ointment or drop for local administration to the eyes, or for parenteral or other administration in any appropriate manner such as intraperitoneal, subcutaneous, topical, intradermal, inhalation, intrapulmonary, rectal, vaginal, sublingual, intramuscular, intravenous, intraarterial, intrathecal, or intralymphatic. Further, the anti-cancer agents or the pharmaceutical compositions of the present invention may be administered in conjunction with other treatments. The anti-cancer agents or the pharmaceutical compositions of the present invention may be encapsulated or otherwise protected against gastric or other secretions, if desired.

The pharmaceutical compositions of the invention comprise one or more active ingredients, e.g. anti-cancer agents, in admixture with one or more pharmaceutically-acceptable diluents or carriers and, optionally, one or more other compounds, drugs, ingredients and/or materials. Regardless of the route of administration selected, the agents/compounds of the present invention are formulated into pharmaceutically-acceptable dosage forms by conventional methods known to those of skill in the art. See, e.g., Remington, The Science and Practice of Pharmacy (21st Edition, Lippincott Williams and Wilkins, Philadelphia, PA.).

Pharmaceutically acceptable diluents or carriers are well known in the art (see, e.g., Remington, The Science and Practice of Pharmacy (21st Edition, Lippincott Williams and Wilkins, Philadelphia, PA.) and The National Formulary (American Pharmaceutical Association, Washington, D.C.)) and include sugars *(e.g.,* lactose, sucrose, mannitol, and sorbitol), starches, cellulose preparations, calcium phosphates (e.g., dicalcium phosphate, tricalcium phosphate and calcium hydrogen phosphate), sodium citrate, water, aqueous solutions (e.g., saline, sodium chloride injection, Ringer's injection, dextrose injection, dextrose and sodium chloride injection, lactated Ringer's injection), alcohols (e.g., ethyl alcohol, propyl alcohol, and benzyl alcohol), polyols (e.g., glycerol, propylene glycol, and polyethylene glycol), organic esters (e.g., ethyl oleate and tryglycerides), biodegradable polymers (e.g., polylactide-polyglycolide, poly(orthoesters), and poly(anhydrides)), elastomeric matrices, liposomes, microspheres, oils (e.g., corn, germ, olive, castor, sesame, cottonseed, and groundnut), cocoa butter, waxes (e.g., suppository waxes), paraffins, silicones, talc, silicylate, etc. Each pharmaceutically acceptable diluent or carrier used in a pharmaceutical composition of the invention must be "acceptable" in the sense of being compatible with the other ingredients of the formulation and not injurious to the subject. Diluents or carriers suitable for a selected dosage form and intended route of administration are well known in the art, and acceptable diluents or carriers for a chosen dosage form and method of administration can be determined using ordinary skill in the art.

The pharmaceutical compositions of the invention may, optionally, contain additional ingredients and/or materials commonly used in pharmaceutical compositions. These ingredients and materials are well known in the art and include (1) fillers or extenders, such as starches, lactose, sucrose, glucose, mannitol, and silicic acid; (2) binders, such as carboxymethylcellulose, alginates, gelatin, polyvinyl pyrrolidone, hydroxypropylmethyl cellulose, sucrose and acacia; (3) humectants, such as glycerol; (4) disintegrating agents, such as agar-agar, calcium carbonate, potato or tapioca starch, alginic acid, certain silicates, sodium starch glycolate, cross-linked sodium carboxymethyl cellulose and sodium carbonate; (5) solution retarding agents, such as paraffin; (6) absorption accelerators, such as quaternary ammonium compounds; (7) wetting agents, such as cetyl alcohol and glycerol monostearate; (8) absorbents, such as kaolin and bentonite clay; (9) lubricants, such as talc, calcium stearate, magnesium stearate, solid polyethylene glycols, and sodium lauryl sulfate; (10) suspending agents, such as ethoxylated isostearyl alcohols, polyoxyethylene sorbitol and sorbitan esters, microcrystalline cellulose, aluminum metahydroxide, bentonite, agar-agar and tragacanth; (11) buffering agents; (12) excipients, such as lactose, milk sugars, polyethylene glycols, animal and vegetable fats, oils, waxes, paraffins, cocoa butter, starches, tragacanth, cellulose derivatives, polyethylene glycol, silicones, bentonites, silicic acid, talc, salicylate, zinc oxide, aluminum hydroxide, calcium silicates, and polyamide powder; (13) inert diluents, such as water or other solvents; (14) preservatives; (15) surface-active agents; (16) dispersing agents; (17) control-release or absorption-delaying agents, such as hydroxypropylmethyl cellulose, other polymer matrices, biodegradable polymers, liposomes, microspheres, aluminum monostearate, gelatin, and waxes; (18) opacifying agents; (19) adjuvants; (20) wetting agents; (21) emulsifying and suspending agents; (22), solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan; (23) propellants, such as chlorofluorohydrocarbons and volatile unsubstituted hydrocarbons, such as butane and propane; (24) antioxidants; (25) agents which render the formulation isotonic with the blood of the intended recipient, such as sugars and sodium chloride; (26) thickening agents; (27) coating materials, such as lecithin; and (28) sweetening, flavoring, coloring, perfuming and preservative agents. Each such ingredient or material must be "acceptable" in the sense of being compatible with the other ingredients of the formulation and not injurious to the subject. Ingredients and materials suitable for a selected dosage form and intended route of administration are well known in the art, and acceptable ingredients and materials for a chosen dosage form and method of administration may be determined using ordinary skill in the art.

The pharmaceutical compositions of the present invention suitable for oral administration may be in the form of capsules, cachets, pills, tablets, powders, granules, a solution or a suspension in an aqueous or non-aqueous liquid, an oil-in-water or water-in-oil liquid emulsion, an elixir or syrup, a pastille, a bolus, an electuary or a paste. These formulations may be prepared by methods known in the art, e.g., by means of conventional pan-coating, mixing, granulation or lyophilization processes.

Solid dosage forms for oral administration (capsules, tablets, pills, dragees, powders, granules and the like) may be prepared, e.g., by mixing the active ingredient(s) with one or more pharmaceutically-acceptable diluents or carriers and, optionally, one or more fillers, extenders, binders, humectants, disintegrating agents, solution retarding agents, absorption accelerators, wetting agents, absorbents, lubricants, and/or coloring agents. Solid compositions of a similar type may be employed as fillers in soft and hard-filled gelatin capsules using a suitable excipient. A tablet may be made by compression or molding, optionally with one or more accessory ingredients. Compressed tablets may be prepared using a suitable binder, lubricant, inert diluent, preservative, disintegrant, surface-active or dispersing agent. Molded tablets may be made by molding in a suitable machine. The tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein. They may be sterilized by, for example, filtration through a bacteria-retaining filter. These compositions may also optionally contain opacifying agents and may be of a composition such that they release the active ingredient only, or preferentially, in a certain portion of the gastrointestinal tract, optionally, in a delayed manner. The active ingredient can also be in microencapsulated form.

Liquid dosage forms for oral administration include pharmaceutically-acceptable emulsions, microemulsions, solutions, suspensions, syrups and elixirs. The liquid dosage forms may contain suitable inert diluents commonly used in the art. Besides inert diluents, the oral compositions may also include adjuvants, such as wetting agents, emulsifying and suspending agents, sweetening, flavoring, coloring, perfuming and preservative agents. Suspensions may contain suspending agents.

The pharmaceutical compositions of the present invention for rectal or vaginal administration may be presented as a suppository, which may be prepared by mixing one or more active ingredient(s) with one or more suitable nonirritating diluents or carriers which are solid at room temperature, but liquid at body temperature and, therefore, will melt in the rectum or vaginal cavity and release the active compound. The pharmaceutical compositions of the present invention which are suitable for vaginal administration also include pessaries, tampons, creams, gels, pastes, foams or spray formulations containing such pharmaceutically-acceptable diluents or carriers as are known in the art to be appropriate.

Dosage forms for the topical or transdermal administration include powders, sprays, ointments, pastes, creams, lotions, gels, solutions, patches, drops and inhalants. The active agent(s)/compound(s) may be mixed under sterile conditions with a suitable pharmaceutically-acceptable diluent or carrier. The ointments, pastes, creams and gels may contain excipients. Powders and sprays may contain excipients and propellants.

The pharmaceutical compositions of the present invention suitable for parenteral administrations may comprise one or more agent(s)/compound(s) in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain suitable antioxidants, buffers, solutes which render the formulation isotonic with the blood of the intended recipient, or suspending or thickening agents. Proper fluidity can be maintained, for example, by the use of coating materials, by the maintenance of the required particle size in the case of dispersions, and by the use of surfactants. These pharmaceutical compositions may also contain suitable adjuvants, such as wetting agents, emulsifying agents and dispersing agents. It may also be desirable to include isotonic agents. In addition, prolonged absorption of the injectable pharmaceutical form may be brought about by the inclusion of agents which delay absorption.

In some cases, in order to prolong the effect of a drug (e.g., pharmaceutical formulation), it is desirable to slow its absorption from subcutaneous or intramuscular injection. This may be accomplished by the use of a liquid suspension of crystalline or amorphous material having poor water solubility.

The rate of absorption of the active agent/drug then depends upon its rate of dissolution which, in turn, may depend upon crystal size and crystalline form. Alternatively, delayed absorption of a parenterally-administered agent/drug may be accomplished by dissolving or suspending the active agent/drug in an oil vehicle. Injectable depot forms may be made by forming microencapsule matrices of the active ingredient in biodegradable polymers. Depending on the ratio of the active ingredient to polymer, and the nature of the particular polymer employed, the rate of active ingredient release can be controlled. Depot injectable formulations are also prepared by entrapping the drug in liposomes or microemulsions which are compatible with body tissue. The injectable materials can be sterilized for example, by filtration through a bacterial-retaining filter.

The formulations may be presented in unit-dose or multi-dose sealed containers, for example, ampules and vials, and may be stored in a lyophilized condition requiring only the addition of the sterile liquid diluent or carrier, for example water for injection, immediately prior to use. Extemporaneous injection solutions and suspensions may be prepared from sterile powders, granules and tablets of the type described above.

### Nucleic Acid

"Nucleic acid" or "oligonucleotide" or "polynucleotide" used herein mean at least two nucleotides covalently linked together. Many variants of a nucleic acid may be used for the same purpose as a given nucleic acid. Thus, a nucleic acid also encompasses substantially identical nucleic acids and complements thereof.

Nucleic acids may be single stranded or double stranded, or may contain portions of both double stranded and single stranded sequences. The nucleic acid may be DNA, both genomic and cDNA, RNA, or a hybrid, where the nucleic acid may contain combinations of deoxyribo- and ribonucleotides, and combinations of bases including uracil, adenine, thymine, cytosine, guanine, inosine, xanthine hypoxanthine, isocytosine and isoguanine. Nucleic acids may be synthesized as a single stranded molecule or expressed in a cell *(in vitro* or *in vivo)* using a synthetic gene. Nucleic acids may be obtained by chemical synthesis methods or by recombinant methods.

A nucleic acid will generally contain phosphodiester bonds, although nucleic acid analogs may be included that may have at least one different linkage, *e.g.,* phosphoramidate, phosphorothioate, phosphorodithioate, or O-methylphosphoroamidite linkages and peptide nucleic acid backbones and linkages. Other analog nucleic acids include those with positive backbones; non-ionic backbones, and non-ribose backbones, including those disclosed in U.S. Pat. Nos. 5,235,033 and 5,034,506. Nucleic acids containing one or more non-naturally occurring or modified nucleotides are also included within the definition of nucleic acid.

A nucleic acid molecule is "complementary" to another nucleic acid molecule if it hybridizes with the second nucleic acid molecule, although some level of mismatch is permitted. Hybridization may be under conditions of low stringency, moderate stringency or high stringency. Suitable stringency conditions are, in general, determined by the length of the nucleic acid molecules, the degree of complementation, and other factors readily understood by those of skill in the art. In some embodiments, for example, for preliminary screening, low stringency conditions, such as a temperature of about 48 to about 55°C, in a buffer including about 5x SSC, about 0.1 to about 0.5 % SDS, and about 0 to about 30% formamide. Moderate stringency hybridization conditions may be at a temperature of about 60°C in a buffer including about 5x to about 6x SSC, about 0.1 to about 0.5 % SDS, and about 40% formamide. High stringency hybridization conditions may be at a temperature of about 65°C in a buffer including about 5x to about 6x SSC, about 0.1 to about 0.5 % SDS, and about 50% formamide. In some embodiments, high stringency conditions are as described herein or are, for example, conditions that allow hybridization comparable with the hybridization that occurs using a DNA probe of at least 500 nucleotides in length, in a buffer containing 0.5 M NaHP0₄, pH 7.2, 7% SDS, 1 mM EDTA, and 1 % BSA (fraction V), at a temperature of 65°C, or a buffer containing 48% formamide, 4.8x SSC, 0.2 M Tris-Cl, pH 7.6, 1x Denhardt's solution, 10% dextran sulfate, and 0.1 % SDS, at a temperature of 42°C.

Hybridizations may be carried out over a period of about 20 to 30 minutes, or about 2 to 6 hours, or about 10 to 15 hours, or over 24 hours or more. High stringency hybridization is also relied upon for the success of numerous techniques routinely performed by molecular biologists, such as high stringency PCR, DNA sequencing, single strand conformational polymorphism analysis, and *in situ* hybridization. In contrast to northern and Southern hybridizations, these techniques are usually performed with relatively short probes (e.g., usually about 15 nucleotides or longer for PCR or sequencing and about 40 nucleotides or longer for *in situ* hybridization).

A probe or primer is a single-stranded DNA or RNA molecule (e.g., an oligonucleotide) of defined sequence that can base pair to a second DNA or RNA molecule that contains a complementary sequence (the target). The stability of the resulting hybrid molecule depends upon the extent of the base pairing that occurs, and is affected by parameters such as the degree of complementarity between the probe and target molecule, and the degree of stringency of the hybridization conditions. The degree of hybridization stringency is affected by parameters such as the temperature, salt concentration, and concentration of organic molecules, such as formamide, and is determined by methods that are known to those skilled in the art. Probes or primers specific for the nucleic acid sequences described herein, or portions thereof, may vary in length by any integer from at least 8 nucleotides to over 500 nucleotides, including any value in between, depending on the purpose for which, and conditions under which, the probe or primer is used. For example, a probe or primer may be 8, 10, 15, 20, or 25 nucleotides in length, or may be at least 30, 40, 50, or 60 nucleotides in length, or may be over 100, 200, 500, or 1000 nucleotides in length. Probes or primers specific for the nucleic acid molecules described herein may have greater than 55-75% sequence identity, or at least 75-85% sequence identity, or at least 85-99% sequence identity, or 100% sequence identity to the nucleic acid sequences described herein.

Probes or primers may be derived from a gene, chromosomal segment, or chromosome that is used as a reference, for example, in variance detection to determine whether a test sample of the same gene, chromosomal segment, or chromosome derived from a particular individual contains the identical sequence or a different sequence at one or more nucleotide positions. Probes may be derived from genomic DNA or cDNA, for example, by amplification, or from cloned DNA segments, and may contain either genomic DNA or cDNA sequences representing all or a portion of a single gene from a single individual. Probes or primers may be chemically synthesized.

Probes or primers can be detectably-labeled, either radioactively or nonradioactive, by methods that are known to those skilled in the art.

The present invention provides combinations shown to enhance the effects of ERK inhibitors. Herein, applicants have also shown that the combination of different ERK inhibitors is likewise synergistic. Therefore, it is contemplated that the effects of the combinations described herein can be further improved by the use of one or more additional ERK inhibitors. Accordingly, some embodiments of the present invention include one or more additional ERK inhibitors.

The following examples are provided to further illustrate the methods of the present invention. These examples are illustrative only and are not intended to limit the scope of the invention in any way.

### EXAMPLES

### Example 1

### MATERIALS AND METHODS

Cancer cell lines were maintained in cell culture under standard media and serum conditions. For dose escalation studies, A375 cells were split, grown to about 40-60% confluence, and then treated with the initial dose of the specified drug. Table 5 shows a summary of drug treatments that were escalated.

**Table 5 - Summary of Treatments Being Escalated**

| **Treatment** | **Inhibitor** |
|---|---|
| **1** | Trametinib (MEKi) |
| **2** | Dabrafenib (BRAFi) |
| **3** | BVD-523 (ERKi) |
| **4** | Dabrafenib (BRAFi) + Trametinib (MEKi) |
| **5** | Dabrafenib (BRAFi) + BVD-523 (ERKi) |
| **6** | Trametinib (MEKi) + BVD-523 (ERKi) |

Single agent dose escalations were performed based on Little *et* al., 2011 and is outlined in FIG. 19. Cells were then allowed to grow until 70-90% confluence and split. Split ratios were kept as "normal" as possible and reasonably consistent between treatments (e.g. a minimum of 50% of the normal split ratio of the parentals). Medium was refreshed every 3-4 days. When cells again reached about 40-60% confluence, the dose was escalated. In the event that the 40-60% window was missed, the cells were split again and dosed once they reached 40-60% confluence. Again, medium was refreshed every 3-4 days. The process was repeated as required (FIG. 19).

For single agent treatments, starting concentrations and dose increases were conducted by starting with the approximate IC₅₀, escalating in small increments or, gently, for the initial 4-5 doses, doubling the dose, increasing by the same increment for the next 4 doses, then moving to 1.5-fold increases in concentration for subsequent doses.

For combination treatments, starting concentrations and dose increases were conducted by starting with half of the approximate IC₅₀ of each compound (combination assay suggests this will result in about 40-70% inhibition range), escalating as per single agents (*i*.*e*. doing an initial doubling and then increasing by the same increment for the next 4 doses, then moving to 1.5-fold increases in concentration). Table 6 shows the projected dose increases using these schemes.

**Table 6 - Projected Dose Increases - Month 1**

| | | | | **Dab/Tram** | | **Dab/523** | | **Tram/523** | |
|---|---|---|---|---|---|---|---|---|---|
| **Dose** | **Tram (nM)** | **Dab (nM)** | **BVD-523 (µM)** | **Dab (nM)** | **Tram (nM)** | **Dab (nM)** | **523 (µM)** | **Tram (nM)** | **523 (µM)** |
| **1** | 1 | 5 | 0.16 | 2.5 | 0.5 | 2.5 | 0.08 | 0.5 | 0.08 |
| **2** | 2 | 10 | 0.32 | 5 | 1 | 5 | 0.16 | 1 | 0.16 |
| **3** | 3 | 15 | 0.48 | 7.5 | 1.5 | 7.5 | 0.24 | 1.5 | 0.24 |
| **4** | 4 | 20 | 0.64 | 10 | 2 | 10 | 0.32 | 2 | 0.32 |
| **5** | 5 | 25 | 0.80 | 12.5 | 2.5 | 12.5 | 0.40 | 2.5 | 0.40 |
| **6** | 8 | 38 | 1.2 | 19 | 4 | 19 | 0.6 | 4 | 0.6 |
| **7** | 11 | 56 | 1.8 | 28 | 6 | 28 | 0.9 | 6 | 0.9 |
| **8** | 17 | 84 | 2.7 | 42 | 8 | 42 | 1.4 | 8 | 1.4 |
| **9** | 25 | 127 | 4.1 | 63 | 13 | 63 | 2.0 | 13 | 2.0 |
| **10** | 38 | 190 | 6.1 | 95 | 19 | 95 | 3.0 | 19 | 3.0 |
| **11** | 57 | 285 | 9.1 | 142 | 28 | 142 | 4.6 | 28 | 4.6 |
| **12** | 85 | 427 | 13.7 | 214 | 43 | 214 | 6.8 | 43 | 6.8 |
| **13** | 128 | 641 | 20.5 | 320 | 64 | 320 | 10.3 | 64 | 10.3 |
| **14** | 192 | 961 | 30.8 | 481 | 96 | 481 | 15.4 | 96 | 15.4 |
| **15** | 288 | 1442 | 46.1 | 721 | 144 | 721 | 23.1 | 144 | 23.1 |
| **16** | 432 | 2162 | 69.2 | 1081 | 216 | 1081 | 34.6 | 216 | 34.6 |
| **17** | 649 | 3244 | 103.8 | 1622 | 324 | 1622 | 51.9 | 324 | 51.9 |
| **18** | 973 | 4865 | 155.7 | 2433 | 487 | 2433 | 77.8 | 487 | 77.8 |
| **19** | 1460 | 7298 | 233.5 | 3649 | 730 | 3649 | 116.8 | 730 | 116.8 |
| **20** | 2189 | 10947 | 350.3 | 5474 | 1095 | 5474 | 175.2 | 1095 | 175.2 |

Clonal resistant cell populations were derived from resistant cell pools by limiting dilution.

Proliferation assays were used to track changes in sensitivity to the escalated agent(s) at appropriate time intervals (e.g. each month, although the timing is dependent on adequate cell numbers being available). For proliferation assays, cells were seeded in 96-well plates at 3000 cells per well in drug-free DMEM medium containing 10% FBS and allowed to adhere overnight prior to addition of compound or vehicle control. Compounds were prepared from DMSO stocks to give a final concentration range as shown in FIGS. 2A-H. The final DMSO concentration was constant at 0.1%. Test compounds were incubated with the cells for 96 hours at 37°C and 5% CO₂ in a humidified atmosphere. Alamar Blue 10% (v/v) was then added and incubated for 4 hours and fluorescent product was detected using a BMG FLUOstar plate reader. The average media only background value was deducted and the data analyzed using a 4-parameter logistic equation in GraphPad Prism. Paclitaxel was used as a positive control.

Proliferation assays for month 1 were initiated at day 28 using cells growing in the concentrations of each agent indicated in Table 7.

**Table 7 - Initial Concentrations of Drugs Used in Proliferation Assays - Month 1**

| **Line** | **Dab** | **Tram** | **BVD-523** |
|---|---|---|---|
| **Parental** | - | - | - |
| **Tram** | - | 2 nM | - |
| **Dab** | 15 nM | - | - |
| **BVD-523** | - | - | 0.48 µM |
| **Tram + Dab** | 5 nM | 1 nM | - |
| **Dab + BVD-523** | 7.5 nM | - | 0.24 µM |
| **Tram + BVD-523** | - | 1 nM | 0.16 µM |

Proliferation assays for month 2 were initiated at day 56 using cells growing in the concentrations of each agent indicated in Table 8.

**Table 8 - Initial Concentrations of Drugs Used in Proliferation Assays - Month 2**

| **Line** | **Dab** | **Tram** | **BVD-523** |
|---|---|---|---|
| **Parental** | - | - | - |
| **Tram** | - | 8 nM | - |
| **Dab** | 127 nM | - | - |
| **BVD-523** | - | - | 0.8 µM |
| **Tram + Dab** | 10 nM | 2 nM | - |
| **Dab** + **BVD-523** | 12.5 nM | - | 0.4 µM |
| **Tram + BVD-523** | - | 2 nM | 0.32 µM |

At the end of the 3 month escalation period, cultures were maintained at the top concentration for 2 weeks prior to the final round of proliferation assays and potential single cell cloning. As the proliferation assays/single cell cloning required actively proliferating cells, for treatments where cells were proliferating very slowly at the top concentration or that were only recently escalated, a backup culture was also maintained at a lower concentration (Table 9). For the BVD-523 treatment, where cells appeared to have almost completely stopped growing and looked particularly fragile at the top concentration (1.8µM), cultures were maintained at a lower concentration for the 2 week period.

**Table 9 - Details of Treatments Being Cultured at a Fixed Concentration for 2 Weeks**

| **Treatment** | **Inhibitor** | **Culture 1** | **Backup Culture** |
|---|---|---|---|
| **1** | Tram | 160 nM | 80 nM |
| **2** | Dab | 3.2 µM | - |
| **3** | BVD-523 | 1.2 µM | 0.8 µM |
| **4** | Dab + Tram | D: 160 nM | D: 80 nM |
| | | T: 30 nM | T: 16 nM |
| **5** | Dab + BVD-523 | D: 42 nM | D: 28 nM |
| | | 523: 1.4 µM | 523: 0.9 µM |
| **6** | Tram + BVD-523 | T: 4 nM | T: 2.5 nM |
| | | 523: 0.6 µM | 523: 0.4 µM |

Proliferation assays for month 3 used cells growing in the concentrations of each agent indicated in Table 10.

**Table 10 - Initial Concentrations of Drugs Used in Proliferation Assays - Month 3**

| **Line** | **Dab** | **Tram** | **BVD-523** |
|---|---|---|---|
| **Parental** | - | - | - |
| **Tram** | - | 160 nM | - |
| **Dab** | 3.2 µM | - | - |
| **BVD-523** | - | - | 1.2 µM |
| **Tram + Dab** | 80 nM | 16 nM | - |
| **Dab + BVD-523** | 28 nM | - | 0.9 µM |
| **Tram + BVD-523** | - | 2.5 nM | 0.4 µM |

For combination studies, A375 cells (ATCC) were seeded into triplicate 96-well plates at a cell density of 3000 cells/well in DMEM plus 10% FBS and allowed to adhere overnight prior to addition of test compound or vehicle control. Combinations were tested using a 10x8 dose matrix with a final DMSO concentration of 0.2%. A 96 hour assay incubation period followed, with subsequent addition of Alamar Blue 10% (v/v) and 4 hours incubation prior to reading on a fluorescent plate reader. After reading Alamar Blue, the medium/Alamar Blue mix was flicked off and 100µl of CellTiter-Glo/PBS (1:1) added and the plates processed as per the manufacturer's instructions (Promega). Media only background values were subtracted before the data was analysed. The Bliss additivity model was then applied.

In brief, predicted fractional inhibition values for combined inhibition were calculated using the equation C_{bliss} =A + B - (A x B) where A and B are the fractional inhibitions obtained by drug A alone or drug B alone at specific concentrations. C_{bliss} is the fractional inhibition that would be expected if the combination of the two drugs were exactly additive. C_{bliss} values are subtracted from the experimentally observed fractional inhibition values to give an 'excess over Bliss' value. Excess over Bliss values greater than 0 indicate synergy, whereas values less than 0 indicate antagonism. Excess over Bliss values are plotted as heat maps ± SD.

The single and combination data are also presented as dose-response curves generated in GraphPad Prism (plotted using % viability relative to DMSO only treated controls).

For focused combination studies, the Alamar Blue viability assays were performed as described above for combination studies. Additionally, Caspase-Glo 3/7 assays were performed. In brief, HCT116 cells were seeded in triplicate in white 96-well plates at a cell density of 5000 cells/well in McCoy's 5A plus 10% FBS. A375 cells were seeded at a density of 5000 cells/well in DMEM plus 10% FBS. Cells were allowed to adhere overnight prior to addition of test compound or vehicle control. The final concentration of DMSO was 0.2%, and 800nM staurosporine was included as a positive control. 24 and 48 hour assay incubation periods were used. Then, Caspase-Glo^{®} 3/7 50% (v/v) was added, plates were mixed for 5 minutes on an orbital shaker and incubated for 1 hour at room temperature prior to reading on a luminescent plate reader. Media only background values were subtracted before the data was analysed.

### Example 2

### Dose Escalation and Proliferation Assays - Month 1

### Dose Escalation Progress - Month 1

A375 cells were dose escalated using BVD-523, dabrafenib, and trametinib either as single agents or in combination. Doses were increased in small increments during the first month. Other than a marked reduction in growth rate, cells generally tolerated the escalations well and the doses were planned to be more aggressively escalated using larger increments in month 2. FIGS. 1A-C show month 1 progress for the dose escalation studies.

### Proliferation Assay Results - Month 1

Proliferation assays were performed to assess the response of the escalated cells lines vs. parental cell line, to BVD-523, dabrafenib, and trametinib treatments.

FIGS. 2A-H show normalized and raw proliferation assay results from month 1 of the studies. Note that differences in max signals in DMSO controls between different treatments (FIGS. 2D-F, 2H) suggest differential growth rates between treatments. These differences may influence the responses of lines to inhibitors in the proliferation assays.

Table 11 shows IC₅₀ data for month 1 of the studies.

**Table 11 - IC₅₀ Data - Month 1**

| **Compoun d** | **Cell Line, Relative IC₅₀ (nM)** | | | | | | |
|---|---|---|---|---|---|---|---|
| | **Par *** | **Tra m** | **Dab** | **BVD-52 3** | **Dab/Tra m** | **Dab/52 3** | **Tram/52 3** |
| **Dabrafeni b** | 6 | 29 | abou t 161 | 8 | 58 | 68 | 11 |
| **Trametinib** | 0.5 | 2.2 | 2.5 | 0.7 | 3.9 | 3.1 | 2.5 |
| BVD-523 | 189 | 335 | 350 | 268 | 300 | 412 | 263 |
| Paclitaxel | 2.2 | 3.0 | 3.3 | 3.4 | 3.5 | 3.4 | 3.4 |

| | | | | | | | |
|---|---|---|---|---|---|---|---|
| *Par = Parental cell line | | | | | | | |

There were early hints that cells grown in the presence of escalating doses of dabrafenib or trametinib, either as single agents or in combinations, were exhibiting decreased responses to these two agents in proliferation assays.

In the early stages of month 2, the growth rate of cells in the dabrafenib only treatment notably increased relative to the early stages of month 1. This enabled an increased rate of progression and suggested that resistance was becoming apparent.

### Example 3

### Dose Escalation and Proliferation Assays - Month 2

### Dose Escalation Progress - Month 2

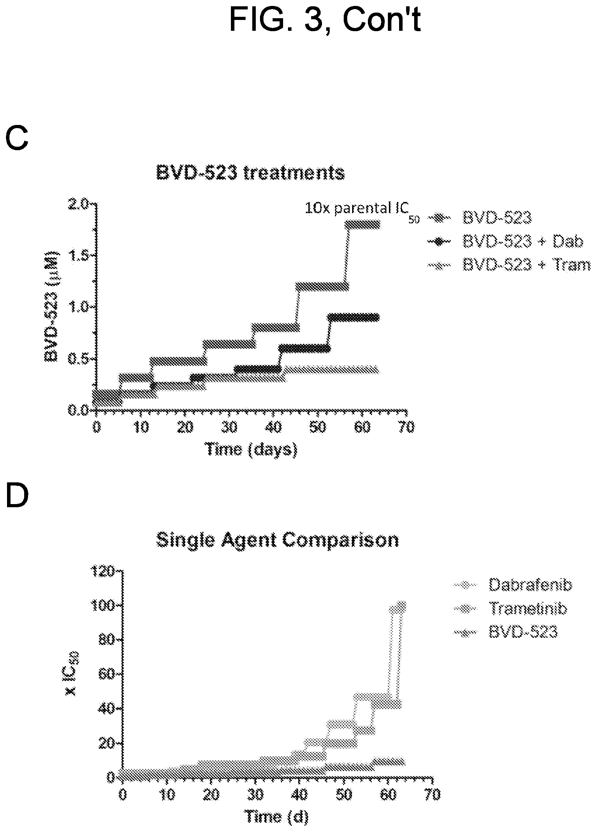

The second month of studies saw most treatments move into a phase where doses were increased in greater increments (1.5-fold) compared to the initial gentle escalation phase. The single agent escalation of dabrafenib and trametinib was quickest, with cells growing in concentrations equivalent to 100x parental cell IC₅₀ (FIGS. 3A,B). The single agent escalation of BVD-523 progressed more slowly compared to dabrafenib and trametinib (FIG. 3C). See FIG. 3D for a comparison of the single agent escalations. BVD-523 escalated cells had a more "fragile" appearance and there was a greater number of floating cells compared to the dabrafenib and trametinib escalated populations.

The combined agent escalations progressed more slowly than the single agent treatments. The BVD-523/trametinib combination was particularly effective in preventing cells from progressing.

### Proliferation Assay Results - Month 2

Proliferation assays on single agent escalated dabrafenib and trametinib cell populations revealed modest shifts in the dose response curves, suggesting that an additional period of escalation would be beneficial to further enrich for resistant cells. Interestingly, in the proliferations assay, there was evidence to suggest that cells exposed to BVD-523 grew less well upon inhibitor withdrawal, perhaps indicating a level of addiction.

FIGS. 4A-H show normalized and raw proliferation assay results from month 2 of the studies. Note that differences in max signals in DMSO controls between different treatments (FIGS. 4D-F, 4H) suggest differential growth rates between treatments. These differences may influence the responses of lines to inhibitors in the proliferation assays.

FIGS. 5A-H show normalized and raw proliferation assay results from month 2 of the studies with a focus on parental and BVD-523 line data only.

Table 12 shows IC₅₀ data for month 2 of the studies. Relative IC₅₀s were determined from 4-parameter curve fits in Prism.

**Table 12 - IC₅₀ Data - Month 2**

| **Compound** | **Cell Line, Relative IC₅₀ (nM)** | | | | | | |
|---|---|---|---|---|---|---|---|
| | **Par*** | **Tra** | **Dab** | **BVD-523** | **Dab/Tram** | **Dab/523** | **Tram/523** |
| **Dabrafenib** | 4.1 | 6.2 | 11.5 | 697 | 256 | 218 | 68 |
| **Trametinib** | 0.4 | 0.7 | 1.1 | 24.3 | 12.6 | 6.2 | 4.6 |
| **BVD-523** | 187 | 252 | 284 | 1706 | 561 | 678 | 435 |
| **Paclitaxel** | 3.7 | 8.9 | 1.9 | 6.5 | 4.7 | 4.2 | 8.9 |

| | | | | | | | |
|---|---|---|---|---|---|---|---|
| *Par = Parental cell line | | | | | | | |

### Example 4

### Dose Escalation and Proliferation Assays - Month 3

### Dose Escalation Progress - Month 3

FIGS. 6A-C show single and combination agent escalation for month 3 of the studies. FIG. 6D shows a comparison of single agent escalations.

### Proliferation Assay Results - Month 3

FIG. 7 shows an assessment of growth during the proliferation assay in DMSO control wells. FIGS. 8A-D show results from month 3 of the studies. FIGS. 9A-D show results from month 3 of the studies with a focus on single treatment cell lines.

Table 13 shows IC₅₀ data for month 3 of the studies. Relative IC₅₀s were determined from 4-parameter curve fits in Prism. IC₅₀ values were not determined for the cell line escalated with trametinib due to a lack of growth during the assay (ND: not done).

**Table 13 - IC₅₀ Data - Month 3**

| **Compound** | **Cell Line, Relative IC₅₀ (nM)** | | | | | | |
|---|---|---|---|---|---|---|---|
| | **Par *** | **Tram** | **Dab** | **BVD-523** | **Dab/Tram** | **Dab/523** | **Tram/523** |
| **Dabrafenib** | 2.1 | ND | 2.5 | 18.4 | 17.9 | 337 | 73 |
| **Trametinib** | 0.2 | ND | 0.4 | 1.7 | 2.7 | 90 | 11.2 |
| **BVD-523** | 129 | ND | 198 | 433 | 323 | 1151 | 296 |
| **Paclitaxel** | 1.9 | ND | 1.9 | 6.5 | 4.7 | 4.2 | 8.9 |

| | | | | | | | |
|---|---|---|---|---|---|---|---|
| *Par = Parental cell line | | | | | | | |

### Example 5

### Combination Study Results

As expected, A375 cells, which carry a BRAF (V600E) mutation, were sensitive to dabrafenib. Single agent IC₅₀ values calculated using Alamar Blue (FIGS. 10, 12, 14) were generally slightly lower for Dabrafenib and BVD-523 compared to those derived using CellTiter-Glo (FIGS. 11, 13, 15). Published IC₅₀ values for Dabrafenib and Trametinib in a 72 hour CellTiter-Glo assay were 28 ± 16nM and 5 ± 3nM respectively (Greger *et al.,* 2012; King *et al.,* 2013) - the single agent results reported here are consistent with these values. There was some evidence for a window of synergy in all treatments. Variation between triplicates was low, however, there was some evidence of edge effects that likely explains the apparent enhanced growth observed in some treatments versus the no drug control (e.g. particularly apparent in the Trametinib/BVD-523 combination). This makes the interpretation of the Bliss analysis more challenging as in some treatments it may have resulted in the artefactual enhancement in the level of synergy.

The combination assays were repeated for A375 cells. Single agent BVD-523, Trametinib and Dabrafenib potencies were consistent with those reported in the previous studies.

HCT116 cells are human colorectal cancer cells with mutations in KRAS. Dabrafenib and Trametinib were antagonist at relevant on-target concentrations. In contrast, Trametinib exhibited synergy with AZ628 over a broad range of combinations, and with higher concentrations of Sorafenib. BVD-523 exhibited windows of synergy with both AZ628 and Sorafenib.

In A375 cells, trametinib exhibited pockets of synergy at lower concentrations of Dabrafenib and AZ628. BVD-523 exhibited a window of synergy with the lower concentrations of Sorafenib.

### Example 6

### BVD-523 altered markers of MAPK kinase activity and effector function

For Western blot studies, HCT116 cells (5 x 10⁶) were seeded into 10 cm dishes in McCoy's 5A plus 10% FBS. A375 cells (2.5 x 10⁶) were seeded into 10 cm dishes in DMEM plus 10% FBS. Cells were allowed to adhere overnight prior to addition of the indicated amount of test compound (BVD-523) or vehicle control. Cells were treated for either 4 or 24 hours before isolation of whole-cell protein lysates, as specified below. Cells were harvested by trypsinisation, pelleted and snap frozen. Lysates were prepared with RIPA (Radio-Immunoprecipitation Assay) buffer, clarified by centrifugation and quantitated by bicinchoninic acid assay (BCA) assay. 20-50 µg of protein was resolved by SDS-PAGE electrophoresis, blotted onto PVDF membrane and probed using the antibodies detailed in Table 14 (for the 4-hour treatment) and Table 15 (for the 24-hour treatment) below.

**Table 14 - Antibody Details**

| **Antigen** | **Size (kDa)** | **Supplier** | **Cat No** | **Dilution** | **Incubation / Block Conditions** | **Secondary** |
|---|---|---|---|---|---|---|
| pRSK1/2 pS380 | 90 | Cell Signaling | 9335 | 1:1000 | o/n 4°C 5% BSA | anti-rabbit |
| pRSK1/2 pS380 | 90 | Cell Signaling | 11989 | 1:2000 | o/n 4°C 5% BSA | anti-rabbit |
| pRSK-T359/S363 | 90 | Millipore | 04-419 | 1:40000 | o/n 4°C 5% BSA | anti-rabbit |
| Total RSK | 90 | Cell Signaling | 9333 | 1:1000 | o/n 4°C 5% BSA | anti-rabbit |
| pErk 1/2 | 42/44 | Cell Signaling | 9106S | 1:500 | o/n 4°C 5% milk | anti-mouse |
| Total ERK | 42/44 | Cell Signaling | 9102 | 1:2000 | o/n 4°C 5% milk | anti-rabbit |
| pMEK1/2 | 45 | Cell Signaling | 9154 | 1:1000 | o/n 4°C 5% BSA | anti-rabbit |
| Total MEK | 45 | Cell Signaling | 9126 | 1:1000 | o/n 4°C 5% BSA | anti-rabbit |
| pS6-pS235 | 32 | Cell Signaling | 2211S | 1:3000 | o/n 4°C 5% milk | anti-rabbit |
| Total S6 | 32 | Cell Signaling | 2217 | 1:2000 | o/n 4°C 5% milk | anti-rabbit |
| DUSP6 | 48 | Cell Signaling | 3058S | 1:1000 | o/n 4°C 5% BSA | anti-rabbit |
| Total CRAF | 73 | BD Bio-sciences | 610152 | 1:2000 | o/n 4°C 5% milk | anti-mouse |
| pCRAF-Ser338 | 73 | Cell Signaling | 9427 | 1:1000 | o/n 4°C 5% BSA | anti-rabbit |
| pRB (Ser780) | 105 | Cell Signaling | 9307 | 1:2000 | o/n 4°C 5% BSA | anti-rabbit |
| β-Actin | 42 | Sigma | A5441 | 1:500,000 | o/n 4°C 5% milk | anti-mouse |

**Table 15 - Antibody details**

| **Antigen** | **(kDa)** | **Supplier** | **Cat No** | **Dilution** | **Incubation / Block Conditions** | **Secondary** |
|---|---|---|---|---|---|---|
| pRB (Ser780) | 105 | Cell SignalinG | 9307 | 1:2000 | o/n 4°C 5% BSA | anti-rabbit |
| CCND1 | 34 | Abcam | ab6152 | 1:500 | o/n 4°C 5% milk | anti-mouse |
| Bim-EL | 23 | Millipore | AB17003 | 1:1000 | o/n 4°C 5% BSA | anti-rabbit |
| Bim-EL | 23 | Cell Signaling | 2933 | 1:1000 | o/n 4°C 5% BSA | anti-rabbit |
| BCL-xL | 30 | Cell Signaling | 2762 | 1:2000 | o/n 4°C 5% BSA | anti-rabbit |
| PARP | 116/ 89 | Cell Signaling | 9542 | 1:1000 | o/n 4°C 5% milk | anti-rabbit |
| Cleaved Caspase 3 | 17,19 | Cell Signaling | 9664X | 1:1000 | o/n 4°C 5% milk | anti-rabbit |
| DUSP6 | 48 | Cell Signaling | 3058S | 1:1000 | o/n 4°C 5% BSA | anti-rabbit |
| pRSK1/2 pS380 | 90 | Cell Signaling | 9335 | 1:1000 | o/n 4°C 5% BSA | anti-rabbit |
| pRSK1/2 pS380 | 90 | Cell Signaling | 11989 | 1:2000 | o/n 4°C 5% BSA | anti-rabbit |
| pRSK-T359/S363 | 90 | Millipore | 04-419 | 1:40000 | o/n 4°C 5% BSA | anti-rabbit |
| Total RSK | 90 | Cell Signaling | 9333 | 1:1000 | o/n 4°C 5% BSA | anti-rabbit |
| pErk 1/2 | 42/44 | Cell Signaling | 9106S | 1:500 | o/n 4°C 5% milk | anti-mouse |
| Total ERK | 42/44 | Cell Signaling | 9102 | 1:2000 | o/n 4°C 5% milk | anti-rabbit |
| B-Actin | 42 | Sigma | A5441 | 1:500,000 | o/n 4°C 5% milk | anti-mouse |

FIGS. 16-18 show Western blot analyses of cells treated with BVD-523 at various concentrations for the following: 1) MAPK signaling components in A375 cells after 4 hours; 2) cell cycle and apoptosis signaling in A375 24 hours treatment with various amounts of BVD-523; and 3) MAPK signaling in HCT-116 cells treated for 4 hours. The results show that acute and prolonged treatment with BVD-523 in RAF and RAS mutant cancer cells in-vitro affects both substrate phosphorylation and effector targets of ERK kinases. The concentrations of BVD-523 required to induce these changes is typically in the low micromolar range.

Changes in several specific activity markers are noteworthy. First, the abundance of slowly migrating isoforms of ERK kinase increase following BVD-523 treatment; modest changes can be observed acutely, and increase following prolonged treatment. While this could indicate an increase in enzymatically active, phosphorylated forms of ERK, it remains noteworthy that multiple proteins subject to both direct and indirect regulation by ERK remain "off" following BVD-523 treatment. First, RSK1/2 proteins exhibit reduced phosphorylation at residues that are strictly dependent on ERK for protein modification (T359/S363). Second, BVD-523 treatment induces complex changes in the MAPK feedback phosphatase, DUSP6: slowly migrating protein isoforms are reduced following acute treatment, while total protein levels are greatly reduced following prolonged BVD-523 treatment. Both of these findings are consistent with reduced activity of ERK kinases, which control DUSP6 function through both post-translational and transcriptional mechanisms. Overall, despite increases in cellular forms of ERK that are typically thought to be active, it appears likely that cellular ERK enzyme activity is fully inhibited following either acute or prolonged treatment with BVD-523.

Consistent with these observations, effector genes that require MAPK pathway signaling are altered following treatment with BVD-523. The G1/S cell-cycle apparatus is regulated at both post-translational and transcriptional levels by MAPK signaling, and cyclin-D1 protein levels are greatly reduced following prolonged BVD-523 treatment. Similarly, gene expression and protein abundance of apoptosis effectors often require intact MAPK signaling, and total levels of Bim-EL increase following prolonged BVD-523 treatment. As noted above, however, PARP protein cleavage and increased apoptosis were not noted in the A375 cell background; this suggests that additional factors may influence whether changes in BVD-523/ERK-dependent effector signaling are translated into definitive events such as cell death and cell cycle arrest.

Consistent with the cellular activity of BVD-523, marker analysis suggests that ERK inhibition alters a variety of molecular signaling events in cancer cells, making them susceptible to both decreased cell proliferation and survival.

In sum, FIGS. 16-18 show that BVD-523 inhibits the MAPK signaling pathway and may be more favorable compared to RAF or MEK inhibition in this setting.

Finally, properties of BVD-523 may make this a preferred agent for use as an ERK inhibitor, compared to other agents with a similar activity. It is known that kinase inhibitor drugs display unique and specific interactions with their enzyme targets, and that drug efficacy is strongly influenced by both the mode of direct inhibition, as well as susceptibility to adaptive changes that occur following treatment. For example, inhibitors of ABL, KIT, EGFR and ALK kinases are effective only when their cognate target is found in active or inactive configurations. Likewise, certain of these inhibitors are uniquely sensitive to either secondary genetic mutation, or post-translational adaptive changes, of the protein target. Finally, RAF inhibitors show differential potency to RAF kinases present in certain protein complexes and/or subcellular localizations. In summary, as ERK kinases are similarly known to exist in diverse, variable, and complex biochemical states, it appears likely that BVD-523 may interact with and inhibit these targets in a fashion that is distinct and highly preferable to other agents.

### Example 7

### Cell culture studies of MEK and ERK inhibitors

### Single Agent Proliferation Assay

Cells were seeded in 96-well plates at the densities and media conditions indicated in Table 16 and allowed to adhere overnight prior to addition of compound or vehicle control. Compounds were prepared from DMSO stocks to give the desired final concentrations The final DMSO concentration was constant at 0.1%. Test compounds were incubated with the cells for 72h at 37°C, 5% CO2 in a humidified atmosphere. CellTiter-Glo^{®} reagent (Promega, Madison, WI) was added according to manufacturer's instructions and luminescence detected using the BMG FLUOstar plate reader (BMG Labtech, Ortenberg, Germany). The average media only background value was deducted and the data analysed using a 4-parameter logistic equation in GraphPad Prism (GraphPad Software, La Jolla, CA).

### Combination Proliferation Assay

Cells were seeded in triplicate 96-well plates at the densities and media conditions indicated in Table 16 and allowed to adhere overnight prior to addition of compound or vehicle control. Compounds were prepared from DMSO stocks to give the desired final concentrations The final DMSO concentration was constant at 0.2%. Combinations were tested using a 10 x 8 dose matrix or a 10 x 6 dose matrix. Test compounds were incubated with the cells for 72h at 37°C, 5% CO2 in a humidified atmosphere. CellTiter-Glo^{®} reagent (Promega, Madison, WI) was added according to manufacturer's instructions and luminescence detected using the BMG FLUOstar plate reader (BMG Labtech, Ortenberg, Germany). The average media only background value was deducted and the data analysed.

Combination interactions across the dose matrix were determined by the Loewe Additivity and Bliss independence models using Chalice ^{™} Combination Analysis Software (Horizon Discovery Group, Cambridge, MA) as outlined in the user manual (available at chalice. horizondiscovery.com/chalice-portal/documentation/analyzer/home.jsp). Synergy is determined by comparing the experimentally observed level of inhibition at each combination point with the value expected for additivity, which is derived from the single-agent responses along the edges of the matrix. Potential synergistic interactions were identified by displaying the calculated excess inhibition over that predicted as being additive across the dose matrix as a heat map, and by reporting a quantitative 'Synergy Score' based on the Loewe model. The single agent data derived from the combination assay plates were presented as dose-response curves generated in GraphPad Prism (GraphPad Software, La Jolla, CA) (plotted using percentage viability relative to DMSO only treated controls).

**Table 16 - Cell Line Seeding Density and Growth Media**

| Cell Line | Seeding Density (cells/well) | Media |
|---|---|---|
| HCT116 Parental | 1000 | McCoy's 5A + 10% FBS |
| HCT116 KRAS KO (+/-) | 2000 | McCoy's 5A + 10% FBS |
| RKO Parental | 2000 | McCoy's 5A + 10% FBS |
| RKO BRAF KO (+/-/-) | 2000 | McCoy's 5A + 10% FBS |
| A375 Parental | 2000 | DMEM + 10% FBS |
| A375 NRAS (Q61 K/+/+) | 2000 | DMEM + 10% FBS |
| G-361 | 5000 | McCoy's 5A + 10% FBS |
| A549 | 750 | RPMI 1640 + 10% FBS |
| H2212 | 4000 | RPMI 1640 + 10% FBS |
| H1437 | 1500 | RPMI 1640 + 10% FBS |
| H226 | 750 | RPMI 1640 + 10% FBS |

### Results

The aim of this study was to assess the effects on cell viability of combining ERK inhibitors with MEK inhibitors in a panel of isogenic and non-isogenic cancer cell lines (Table 17).

**Table 17 - Description of Cell Lines Studied**

| Cell Line | Cancer Type | Description |
|---|---|---|
| HCT116 Parental | CRC | Heterozygous parental cells containing one mutant KRAS allele (G13D) and one wild type allele |
| HCT116 KRAS KO (+/-) | CRC | Knock out of mutant KRAS allele in heterozygous parental cells |
| RKO Parental | CRC | Triploid parental cells containing two mutant BRAF alleles (V600E) and one wild type allele |
| RKO BRAF KO (+/-/-) | CRC | Knock out of both mutant BRAF alleles (V600E) in triploid parental cells |
| A375 Parental | Melanoma | Hypotriploid parental line carrying BRAF (V600E) mutation |
| A375 NRAS (Q61 K/+/+) | Melanoma | Heterozygous knock-in of NRAS activating mutation (Q61K) |
| G-361 | Melanoma | BRAF (V600E) mutant |
| A549 | NSCLC | BRAF mutant |
| H2212 | NSCLC | BRAF mutant |
| H1437 | NSCLC | KRAS wild type |
| H226 | NSCLC | KRAS wild type |

An initial round of single agent assays was performed in the A375 (FIG. 20), HCT116 (FIG. 21) and RKO-isogenic (FIG. 22) cell line pairs. IC₅₀ values are shown in Table 18. These revealed no differentials in response to ERK or MEK inhibition between the two cell lines within the A375 and HCT116 isogenic pair. This suggests that under the assay conditions tested 1) the knocked-in mutant NRAS allele does not drive resistance to MEK or ERK inhibition in A375 cells and 2) sensitivity of HCT116 to MEK/ERK inhibition is not coupled to the mutant KRAS allele.

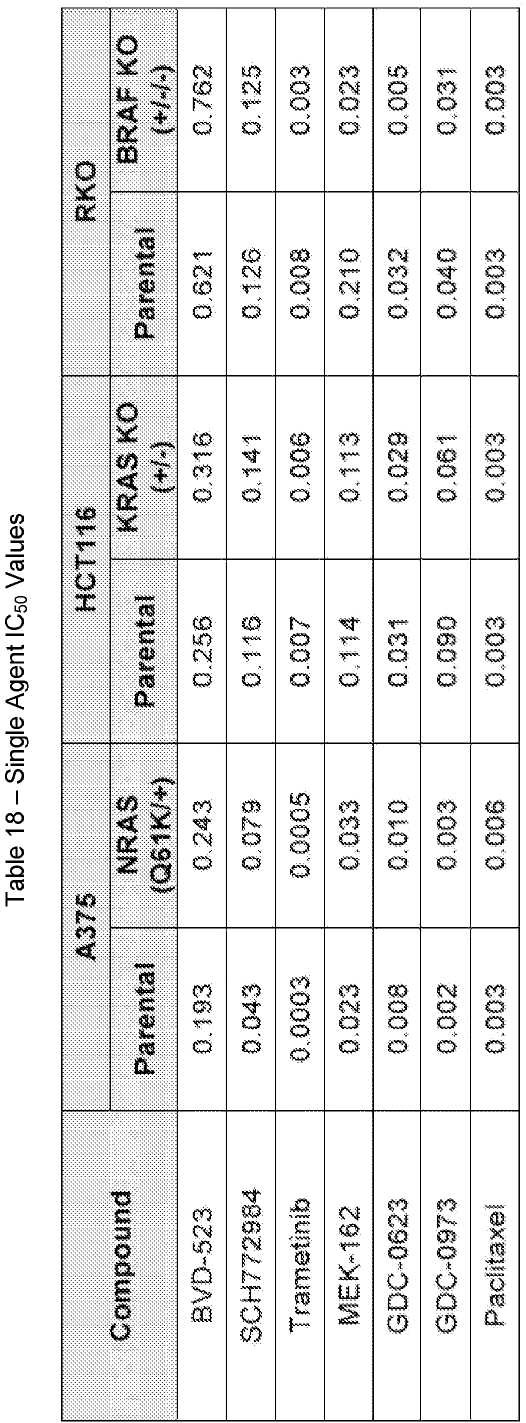

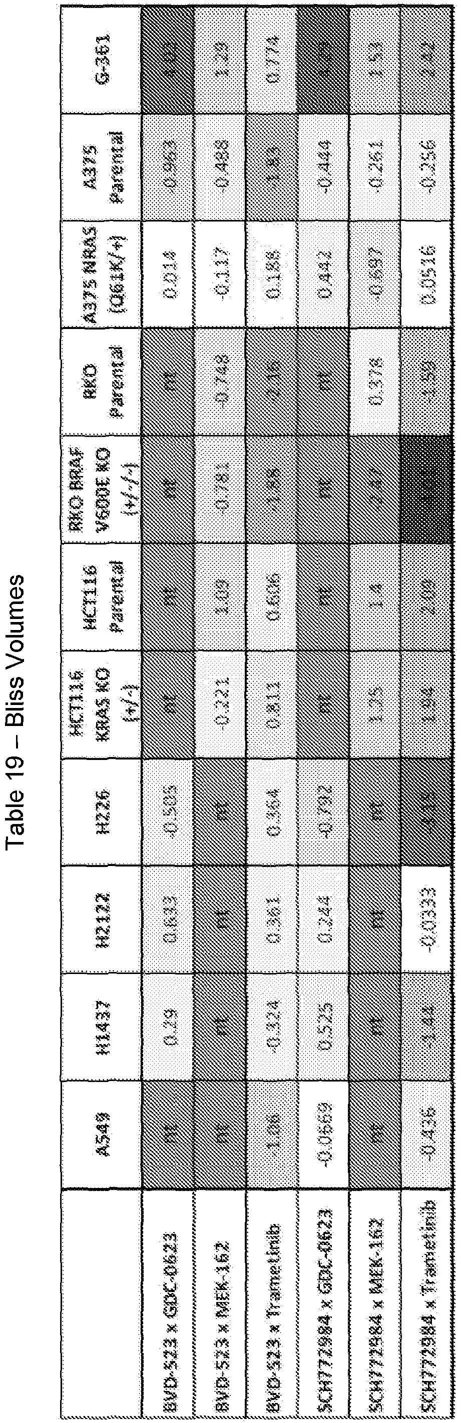

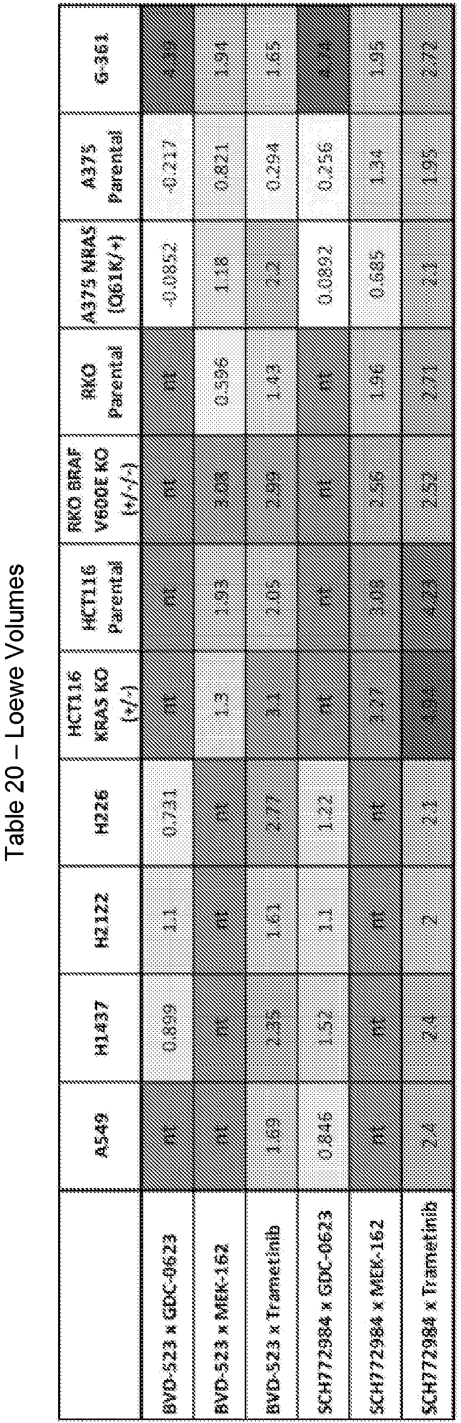

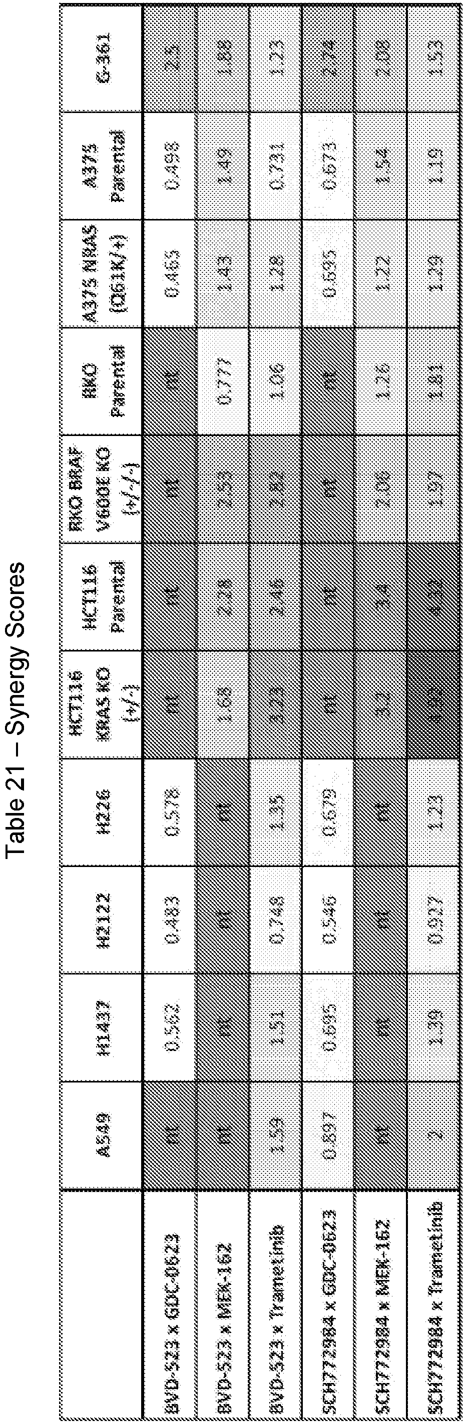

Surprisingly, deletion of the mutant BRAF (V600E) alleles in RKO cells increased the sensitivity to several of the MEK inhibitors, but did not markedly alter the response to ERK inhibition (FIG. 22). This is consistent with the general observation that upstream modulations of the MAPK pathway that alter sensitivity to MEK inhibitors do not markedly affect sensitivity to ERK inhibition.

Combination interactions between two compounds were assessed across a matrix of concentrations using the Loewe Additivity and Bliss Independence Models with Chalice^{™} Bioinformatics Software (Horizon Discovery Group, Cambridge, MA). Chalice^{™} enables potential synergistic interactions to be identified by displaying the calculated excess inhibition over that predicted as being additive across the dose matrix as a heat map, and by reporting a quantitative 'Synergy Score' based on the Loewe model.

Visualization of the Bliss 'excess inhibition' heat maps for the A375 parental and NRAS mutant (Q61K) cell lines revealed a small window of synergy between BVD-523 and all three MEK inhibitors tested (FIG. 23, FIG. 25, FIG. 27). These observations were confirmed in a second BRAF mutant cell line G-361 (FIG. 37, FIG. 39, FIG. 41) and using a second benchmark ERK inhibitor SCH772984 (FIG. 24, FIG. 26, FIG. 28 and FIG. 38, FIG. 40, FIG. 42, respectively). Although not as strong, these windows of synergy were also mostly detected when the data was analyzed using the Loewe model.

In summary, these results suggest that interactions between BVD-523 and MEK inhibitors may potentially be synergistic in melanoma cell lines mutated for BRAF.

In contrast, when assessed using the Bliss model, interactions between BVD-523 or SCH772984 and MEK inhibitors in HCT116 (FIG. 29 - FIG. 32 ) and the lung lines (FIG. 44 - FIG. 57) appeared to be mostly additive. In the RKO cells (FIG. 33 - FIG. 36) there were pockets of mild antagonism at higher concentrations. Excess scores were generally more positive, but still mainly additive, when the BVD-523 combinations were analyzed using the Loewe model. Similar results were also obtained for the SCH772984 combinations in these cell lines using the Bliss model, however, the Loewe model suggested the possible presence of regions of synergy in HCT116 and some of the lung lines that were not apparent from the Bliss model.

Synergistic interactions were scored in two ways. Excess activity over that predicted if a combination was additive can be calculated using a simple volume score, which calculates the volume between the measured and the predicted response surface. This volume score shows whether the overall response to a combination is synergistic (positive values), antagonistic (negative values) or additive (values ~ 0). Table 19 shows Bliss volumes and Table 20 shows Loewe volumes; nt = not tested. Additionally, a 'Synergy Score', a positive-gated inhibition-weighted volume over Loewe additivity, is calculated and results are shown in Table 21; nt = not tested. This provides an additional prioritization favoring combinations whose synergy occurs at high effect levels, ignoring antagonistic portions of the response surface.

### Example 8

### Combination Interactions Between ERK inhibitors

RAF mutant melanoma cell line A375 cells were cultured in DMEM with 10% FBS and seeded into triplicate 96-well plates at an initial density of 2000 cells per well. Combination interactions between ERK inhibitors BVD-523 and SCH772984 were analized after 72 hours as described above in Example 7. Viability was determined using CellTiter-Glo^{®} reagent (Promega, Madison, WI) according to manufacturer's instructions and luminescence was detected using the BMG FLUOstar plate reader (BMG Labtech, Ortenberg, Germany).

Visualization of the Loewe and Bliss 'excess inhibition' heat maps suggested that the combination of BVD-523 and SCH772984 was mainly additive with windows of potential synergy in mid-range doses (FIG. 58).

In summary, these results suggest that interactions between BVD-523 and SCH772984 are at least additive, and in some cases synergistic.

### Documents

ABSALAN, Farnaz; Mostafa Ronaghi (2008). Molecular Inversion Probe Assay. Methods in Molecular Biology 396. Humana Press. pp. 315-330.
GREGER, James G., et al. "Combinations of BRAF, MEK, and PI3K/mTOR inhibitors overcome acquired resistance to the BRAF inhibitor GSK2118436 dabrafenib, mediated by NRAS or MEK mutations." Molecular cancer therapeutics 11.4 (2012): 909-920.
HARDENBOL, P. et al. Multiplexed genotyping with sequence-tagged molecular inversion probes. Nat. Biotechnol. 2003, no. 21 , pp. 673-678.
HATZIVASSILIOU, G. et al., "RAF inhibitors prime wild-type RAF to activate the MAPK pathway and enhance growth." Nature 464.7287 (2010): 431-435.
KING, Alastair J., et al. "Dabrafenib; preclinical characterization, increased efficacy when combined with trametinib, while BRAF/MEK tool combination reduced skin lesions." PloS one 8.7 (2013): e67583.
LITTLE, A.S. et al., Amplification of the Driving Oncogene, KRAS or BRAF, Underpins Acquired Resistance to MEK1/2 Inhibitors in Colorectal Cancer Cells. Sci. Signal. 4, ra17 (2011).
MANANDHAR SP, Hildebrandt ER, Schmidt WK. Small-molecule inhibitors of the Rce1p CaaX protease. J Biomol Screen. 2007;12(7):983-993.
MAURER, T, Garrenton, LS, Oh, A, Pitts, K, Anderson, DJ, Skelton, NJ, Fauber, BP, Pan, B, Malek, S, Stokoe, D, Ludlam, MJC, Bowman, KK, Wu, J, Giannetti, AM, Starovasnik, MA, Mellman, I, Jackson, PK, Rudolph, J, Wang, W, Fang, G. Small-molecule ligands bind to a distinct pocket in Ras and inhibit SOS-mediated nucleotide exchange activity. PNAS. 2012;109(14):5299-304.
METZKER, Emerging technologies in DNA sequencing Genome Res. 2005. 15: 1767-1776
MITTAL, Rohit et al. "The acetyltransferase activity of the bacterial toxin YopJ of Yersinia is activated by eukaryotic host cell inositol hexakisphosphate." Journal of Biological Chemistry 285.26 (2010): 19927-19934.
NILSSON, M. et al., Padlock probes: circularizing oligonucleotides for localized DNA detection. Science. 1994, no.265, p.2085-2088.
OTA et al., Single nucleotide polymorphism detection by polymerase chain reaction-restriction fragment length polymorphism. Nat Protoc. 2007;2(11):2857-64.
PATGIRI, A, Yadav, KK, Arora, PS, Bar-Sagi, D. An orthosteric inhibitor of the Ras-Sos interaction. Nat Chem Biol. 2011;7:585-587.
PORTER SB, Hildebrandt ER, Breevoort SR, Mokry DZ, Dore TM, Schmidt WK. Inhibition of the CaaX proteases Rce1p and Ste24p by peptidyl (acyloxy)methyl ketones. Biochim Biophys Acta.2007;1773(6):853-862.
SHIMA, F, Yoshikawa, Y, Ye, M, Araki, M, Matsumoto, S, Liao, J, Hu, L, Sugimoto, T, Ijiri, Y, Takeda, A, Nishiyama, Y, Sato, C, Muraoka, S, Tamura, A, Osoda, T, Tsuda, K-I, Miyakawa, T, Fukunishi, H, Shimada, J, Kumasaka, Yamamoto, M, Kataoka, T. In silico discovery of small-molecule Ras inhibitors that display antitumor activity by blocking the Ras-effector interaction. PNAS. 2013;110(20):8182-7.

All documents cited in this application are hereby incorporated by reference as if recited in full herein.

Although illustrative embodiments of the present invention have been described herein, it should be understood that the invention is not limited to those described, and that various other changes or modifications may be made by one skilled in the art without departing from the scope or spirit of the invention.

Furthermore, the present invention relates to the following items:
1. A method of treating or ameliorating the effects of a cancer in a subject in need thereof comprising administering to the subject an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, to treat or ameliorate the effects of the cancer.
2. The method according to item 1, wherein the subject is a mammal.
3. The method according to item 2, wherein the mammal is selected from the group consisting of humans, primates, farm animals, and domestic animals.
4. The method according to item 2, wherein the mammal is a human.
5. The method according to item 1, wherein the type 2 MEK inhibitor is selected from the group consisting of anthrax toxin, lethal factor portion of anthrax toxin, ARRY-142886 (6-(4-bromo-2-chloro-phenylamino)-7-fluoro-3-methyl-3H-benzoimidazole-5-carboxylic acid (2-hydroxy-ethoxy)-amide), ARRY-438162 (Array BioPharma), AS-1940477 (Astellas), MEK162, PD 098059 (2-(2'-amino-3'-methoxyphenyl)-oxanaphthalen-4-one), PD 184352 (CI-1040), PD-0325901 (Pfizer), pimasertib (Santhera Pharmaceuticals), refametinib (AstraZeneca), selumetinib (AZD6244) (AstraZeneca), TAK-733 (Takeda), trametinib (Japan Tobacco), U0126 (1,4-diamino-2,3-dicyano-1,4-bis(2-aminophenylthio)butadiene), RDEA119 (Ardea Biosciences/Bayer), pharmaceutically acceptable salts thereof, and combinations thereof.
6. The method according to item 1, wherein the type 2 MEK inhibitor is trametinib or a pharmaceutically acceptable salt thereof.
7. The method according to item 1, wherein the subject with cancer has a somatic RAS or BRAF mutation.
8. The method according to item 1, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
9. The method according to item 1, wherein the cancer is melanoma.
10. The method according to item 1 further comprising administering to the subject at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
11. The method according to item 10, wherein the additional therapeutic agent is an inhibitor of the PI3K/Akt pathway.
12. The method according to item 11, wherein the inhibitor of the PI3K/Akt pathway is selected from the group consisting of A-674563 (CAS # 552325-73-2), AGL 2263, AMG-319 (Amgen, Thousand Oaks, CA), AS-041164 (5-benzo[1,3]dioxol-5-ylmethylene-thiazolidine-2,4-dione), AS-604850 (5-(2,2-Difluoro-benzo[1,3]dioxol-5-ylmethylene)-thiazolidine-2,4-dione), AS-605240 (5-quinoxilin-6-methylene-1,3-thiazolidine-2,4-dione), AT7867 (CAS # 857531-00-1), benzimidazole series, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 (CAS # 1007207-67-1), CHR-4432 (Chroma Therapeutics, Ltd., Abingdon, UK), FPA 124 (CAS # 902779-59-3), GS-1101 (CAL-101) (Gilead Sciences), GSK 690693 (CAS # 937174-76-0), H-89 (CAS # 127243-85-0), Honokiol, IC87114 (Gilead Science), IPI-145 (Intellikine Inc.), KAR-4139 (Karus Therapeutics, Chilworth, UK), KAR-4141 (Karus Therapeutics), KIN-1 (Karus Therapeutics), KT 5720 (CAS # 108068-98-0), Miltefosine, MK-2206 dihydrochloride (CAS # 1032350-13-2), ML-9 (CAS # 105637-50-1), Naltrindole Hydrochloride, OXY-111A (NormOxys Inc., Brighton, MA), perifosine, PHT-427 (CAS # 1191951-57-1), PI3 kinase delta inhibitor, Merck KGaA (Merck & Co., Whitehouse Station, NJ), PI3 kinase delta inhibitors, Genentech (Roche Holdings Inc.), PI3 kinase delta inhibitors, Incozen (Incozen Therapeutics, Pvt. Ltd., Hyderabad, India), PI3 kinase delta inhibitors-2, Incozen (Incozen Therapeutics), PI3 kinase inhibitor, Roche-4 (Roche Holdings Inc.), PI3 kinase inhibitors, Roche (Roche Holdings Inc.), PI3 kinase inhibitors, Roche-5 (Roche Holdings Inc.), PI3-alpha/delta inhibitors, Pathway Therapeutics (Pathway Therapeutics Ltd., South San Francisco, CA), PI3-delta inhibitors, Cellzome (Cellzome AG, Heidelberg, Germany), PI3-delta inhibitors, Intellikine (Intellikine Inc., La Jolla, CA), PI3-delta inhibitors, Pathway Therapeutics-1 (Pathway Therapeutics Ltd.), PI3-delta inhibitors, Pathway Therapeutics-2 (Pathway Therapeutics Ltd.), PI3-delta/gamma inhibitors, Cellzome (Cellzome AG), PI3-delta/gamma inhibitors, Cellzome (Cellzome AG), PI3-delta/gamma inhibitors, Intellikine (Intellikine Inc.), PI3-delta/gamma inhibitors, Intellikine (Intellikine Inc.), PI3-delta/gamma inhibitors, Pathway Therapeutics (Pathway Therapeutics Ltd.), PI3-delta/gamma inhibitors, Pathway Therapeutics (Pathway Therapeutics Ltd.), PI3-gamma inhibitor Evotec (Evotec), PI3-gamma inhibitor, Cellzome (Cellzome AG), PI3-gamma inhibitors, Pathway Therapeutics (Pathway Therapeutics Ltd.), PI3K delta/gamma inhibitors, Intellikine-1 (Intellikine Inc.), PI3K delta/gamma inhibitors, Intellikine-1 (Intellikine Inc.), pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, Tetrahydro Curcumin, TG100-115 (Targegen Inc., San Diego, CA), Triciribine, X-339 (Xcovery, West Palm Beach, FL), XL-499 (Evotech, Hamburg, Germany), pharmaceutically acceptable salts thereof, and combinations thereof.
13. The method according to item 1, wherein administration of the first and second anti-cancer agents provides a synergistic effect compared to administration of either anti-cancer agent alone.
14. A method of treating or ameliorating the effects of a cancer in a subject in need thereof comprising administering to the subject an effective amount of (i) a first anti-cancer agent, which is BVD-523 or a pharmaceutically acceptable salt thereof and (ii) a second anti-cancer agent, which is trametinib or a pharmaceutically acceptable salt thereof, to treat or ameliorate the effects of the cancer.
15. The method according to item 14, wherein the subject is a mammal.
16. The method according to item 15, wherein the mammal is selected from the group consisting of humans, primates, farm animals, and domestic animals.
17. The method according to item 15, wherein the mammal is a human.
18. The method according to item 14, wherein the BVD-523 or a pharmaceutically acceptable salt thereof is administered in the form of a pharmaceutical composition further comprising a pharmaceutically acceptable carrier or diluent.
19. The method according to item 14, wherein the trametinib or a pharmaceutically acceptable salt thereof is administered in the form of a pharmaceutical composition further comprising a pharmaceutically acceptable carrier or diluent.
20. The method according to item 14, wherein the subject with cancer has a somatic RAS mutation or BRAF mutation.
21. The method according to item 14, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
22. The method according to item 14, wherein the cancer is melanoma.
23. The method according to item 14 further comprising administering to the subject at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
24. The method according to item 23, wherein the additional therapeutic agent is an inhibitor of the PI3K/Akt pathway.
25. The method according to item 24, wherein the inhibitor of the PI3K/Akt pathway is selected from the group consisting of A-674563 (CAS # 552325-73-2), AGL 2263, AMG-319 (Amgen, Thousand Oaks, CA), AS-041164 (5-benzo[1,3]dioxol-5-ylmethylene-thiazolidine-2,4-dione), AS-604850 (5-(2,2-Difluoro-benzo[1,3]dioxol-5-ylmethylene)-thiazolidine-2,4-dione), AS-605240 (5-quinoxilin-6-methylene-1,3-thiazolidine-2,4-dione), AT7867 (CAS # 857531-00-1), benzimidazole series, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 (CAS # 1007207-67-1), CHR-4432 (Chroma Therapeutics, Ltd., Abingdon, UK), FPA 124 (CAS # 902779-59-3), GS-1101 (CAL-101) (Gilead Sciences), GSK 690693 (CAS # 937174-76-0), H-89 (CAS # 127243-85-0), Honokiol, IC87114 (Gilead Science), IPI-145 (Intellikine Inc.), KAR-4139 (Karus Therapeutics, Chilworth, UK), KAR-4141 (Karus Therapeutics), KIN-1 (Karus Therapeutics), KT 5720 (CAS # 108068-98-0), Miltefosine, MK-2206 dihydrochloride (CAS # 1032350-13-2), ML-9 (CAS # 105637-50-1), Naltrindole Hydrochloride, OXY-111A (NormOxys Inc., Brighton, MA), perifosine, PHT-427 (CAS # 1191951-57-1), PI3 kinase delta inhibitor, Merck KGaA (Merck & Co., Whitehouse Station, NJ), PI3 kinase delta inhibitors, Genentech (Roche Holdings Inc.), PI3 kinase delta inhibitors, Incozen (Incozen Therapeutics, Pvt. Ltd., Hyderabad, India), PI3 kinase delta inhibitors-2, Incozen (Incozen Therapeutics), PI3 kinase inhibitor, Roche-4 (Roche Holdings Inc.), PI3 kinase inhibitors, Roche (Roche Holdings Inc.), PI3 kinase inhibitors, Roche-5 (Roche Holdings Inc.), PI3-alpha/delta inhibitors, Pathway Therapeutics (Pathway Therapeutics Ltd., South San Francisco, CA), PI3-delta inhibitors, Cellzome (Cellzome AG, Heidelberg, Germany), Pl3-delta inhibitors, Intellikine (Intellikine Inc., La Jolla, CA), PI3-delta inhibitors, Pathway Therapeutics-1 (Pathway Therapeutics Ltd.), PI3-delta inhibitors, Pathway Therapeutics-2 (Pathway Therapeutics Ltd.), PI3-delta/gamma inhibitors, Cellzome (Cellzome AG), PI3-delta/gamma inhibitors, Cellzome (Cellzome AG), PI3-delta/gamma inhibitors, Intellikine (Intellikine Inc.), PI3-delta/gamma inhibitors, Intellikine (Intellikine Inc.), PI3-delta/gamma inhibitors, Pathway Therapeutics (Pathway Therapeutics Ltd.), PI3-delta/gamma inhibitors, Pathway Therapeutics (Pathway Therapeutics Ltd.), PI3-gamma inhibitor Evotec (Evotec), PI3-gamma inhibitor, Cellzome (Cellzome AG), PI3-gamma inhibitors, Pathway Therapeutics (Pathway Therapeutics Ltd.), PI3K delta/gamma inhibitors, Intellikine-1 (Intellikine Inc.), PI3K delta/gamma inhibitors, Intellikine-1 (Intellikine Inc.), pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, Tetrahydro Curcumin, TG100-115 (Targegen Inc., San Diego, CA), Triciribine, X-339 (Xcovery, West Palm Beach, FL), XL-499 (Evotech, Hamburg, Germany), pharmaceutically acceptable salts thereof, and combinations thereof.
26. The method according to item 14, wherein administration of the first and second anti-cancer agents provides a synergistic effect compared to administration of either anti-cancer agent alone.
27. A method of effecting cancer cell death comprising contacting the cancer cell with an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof.
28. The method according to item 27, wherein the subject is a mammal.
29. The method according to item 28, wherein the mammal is selected from the group consisting of humans, primates, farm animals, and domestic animals.
30. The method according to item 28, wherein the mammal is a human.
31. The method according to item 27, wherein the type 2 MEK inhibitor is selected from the group consisting of anthrax toxin, lethal factor portion of anthrax toxin, ARRY-142886 (6-(4-bromo-2-chloro-phenylamino)-7-fluoro-3-methyl-3H-benzoimidazole-5-carboxylic acid (2-hydroxy-ethoxy)-amide), ARRY-438162 (Array BioPharma), AS-1940477 (Astellas), MEK162, PD 098059 (2-(2'-amino-3'-methoxyphenyl)-oxanaphthalen-4-one), PD 184352 (CI-1040), PD-0325901 (Pfizer), pimasertib (Santhera Pharmaceuticals), refametinib (AstraZeneca), selumetinib (AZD6244) (AstraZeneca), TAK-733 (Takeda), trametinib (Japan Tobacco), U0126 (1,4-diamino-2,3-dicyano-1,4-bis(2-aminophenylthio)butadiene), RDEA119 (Ardea Biosciences/Bayer), pharmaceutically acceptable salts thereof, and combinations thereof.
32. The method according to item 27, wherein the type 2 MEK inhibitor is selected from the group consisting of trametinib or a pharmaceutically acceptable salt thereof.
33. The method according to item 27, wherein the subject with cancer has a somatic RAS mutation or BRAF mutation.
34. The method according to item 27, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
35. The method according to item 27, wherein the cancer is melanoma.
36. The method according to item 27 further comprising administering to the subject at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
37. The method according to item 36, wherein the additional therapeutic agent is an inhibitor of the PI3K/Akt pathway.
38. The method according to item 37, wherein the inhibitor of the PI3K/Akt pathway is selected from the group consisting of A-674563 (CAS # 552325-73-2), AGL 2263, AMG-319 (Amgen, Thousand Oaks, CA), AS-041164 (5-benzo[1,3]dioxol-5-ylmethylene-thiazolidine-2,4-dione), AS-604850 (5-(2,2-Difluoro-benzo[1,3]dioxol-5-ylmethylene)-thiazolidine-2,4-dione), AS-605240 (5-quinoxilin-6-methylene-1,3-thiazolidine-2,4-dione), AT7867 (CAS # 857531-00-1), benzimidazole series, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 (CAS # 1007207-67-1), CHR-4432 (Chroma Therapeutics, Ltd., Abingdon, UK), FPA 124 (CAS # 902779-59-3), GS-1101 (CAL-101) (Gilead Sciences), GSK 690693 (CAS # 937174-76-0), H-89 (CAS # 127243-85-0), Honokiol, IC87114 (Gilead Science), IPI-145 (Intellikine Inc.), KAR-4139 (Karus Therapeutics, Chilworth, UK), KAR-4141 (Karus Therapeutics), KIN-1 (Karus Therapeutics), KT 5720 (CAS # 108068-98-0), Miltefosine, MK-2206 dihydrochloride (CAS # 1032350-13-2), ML-9 (CAS # 105637-50-1), Naltrindole Hydrochloride, OXY-111A (NormOxys Inc., Brighton, MA), perifosine, PHT-427 (CAS # 1191951-57-1), PI3 kinase delta inhibitor, Merck KGaA (Merck & Co., Whitehouse Station, NJ), PI3 kinase delta inhibitors, Genentech (Roche Holdings Inc.), PI3 kinase delta inhibitors, Incozen (Incozen Therapeutics, Pvt. Ltd., Hyderabad, India), PI3 kinase delta inhibitors-2, Incozen (Incozen Therapeutics), PI3 kinase inhibitor, Roche-4 (Roche Holdings Inc.), PI3 kinase inhibitors, Roche (Roche Holdings Inc.), PI3 kinase inhibitors, Roche-5 (Roche Holdings Inc.), PI3-alpha/delta inhibitors, Pathway Therapeutics (Pathway Therapeutics Ltd., South San Francisco, CA), PI3-delta inhibitors, Cellzome (Cellzome AG, Heidelberg, Germany), PI3-delta inhibitors, Intellikine (Intellikine Inc., La Jolla, CA), PI3-delta inhibitors, Pathway Therapeutics-1 (Pathway Therapeutics Ltd.), PI3-delta inhibitors, Pathway Therapeutics-2 (Pathway Therapeutics Ltd.), PI3-delta/gamma inhibitors, Cellzome (Cellzome AG), PI3-delta/gamma inhibitors, Cellzome (Cellzome AG), PI3-delta/gamma inhibitors, Intellikine (Intellikine Inc.), PI3-delta/gamma inhibitors, Intellikine (Intellikine Inc.), PI3-delta/gamma inhibitors, Pathway Therapeutics (Pathway Therapeutics Ltd.), PI3-delta/gamma inhibitors, Pathway Therapeutics (Pathway Therapeutics Ltd.), PI3-gamma inhibitor Evotec (Evotec), PI3-gamma inhibitor, Cellzome (Cellzome AG), PI3-gamma inhibitors, Pathway Therapeutics (Pathway Therapeutics Ltd.), PI3K delta/gamma inhibitors, Intellikine-1 (Intellikine Inc.), PI3K delta/gamma inhibitors, Intellikine-1 (Intellikine Inc.), pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, Tetrahydro Curcumin, TG100-115 (Targegen Inc., San Diego, CA), Triciribine, X-339 (Xcovery, West Palm Beach, FL), XL-499 (Evotech, Hamburg, Germany), pharmaceutically acceptable salts thereof, and combinations thereof.
39. The method according to item 27, wherein contacting the cancer cell with the first and second anti-cancer agents provides a synergistic effect compared to contacting the cancer cell with either anti-cancer agent alone.
40. A kit for treating or ameliorating the effects of a cancer in a subject in need thereof comprising an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, packaged together with instructions for their use.
41. The kit according to item 40, wherein the subject is a mammal.
42. The kit according to item 41, wherein the mammal is selected from the group consisting of humans, primates, farm animals, and domestic animals.
43. The kit according to item 41, wherein the mammal is a human.
44. The kit according to item 40, wherein the type 2 MEK inhibitor is selected from the group consisting of anthrax toxin, lethal factor portion of anthrax toxin, ARRY-142886 (6-(4-bromo-2-chloro-phenylamino)-7-fluoro-3-methyl-3H-benzoimidazole-5-carboxylic acid (2-hydroxy-ethoxy)-amide), ARRY-438162 (Array BioPharma), AS-1940477 (Astellas), MEK162, PD 098059 (2-(2'-amino-3'-methoxyphenyl)-oxanaphthalen-4-one), PD 184352 (CI-1040), PD-0325901 (Pfizer), pimasertib (Santhera Pharmaceuticals), refametinib (AstraZeneca), selumetinib (AZD6244) (AstraZeneca), TAK-733 (Takeda), trametinib (Japan Tobacco), U0126 (1,4-diamino-2,3-dicyano-1,4-bis(2-aminophenylthio)butadiene), RDEA119 (Ardea Biosciences/Bayer), pharmaceutically acceptable salts thereof, and combinations thereof.
45. The kit according to item 40, wherein the type 2 MEK inhibitor is trametinib or a pharmaceutically acceptable salt thereof.
46. The kit according to item 40, wherein the subject with cancer has a somatic RAS mutation or BRAF mutation.
47. The kit according to item 40, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
48. The kit according to item 40, wherein the cancer is melanoma.
49. The kit according to item 40 further comprising at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
50. The kit according to item 49, wherein the additional therapeutic agent is an inhibitor of the PI3K/Akt pathway.
51. The kit according to item 50, wherein the inhibitor of the PI3K/Akt pathway is selected from the group consisting of A-674563 (CAS # 552325-73-2), AGL 2263, AMG-319 (Amgen, Thousand Oaks, CA), AS-041164 (5-benzo[1,3]dioxol-5-ylmethylene-thiazolidine-2,4-dione), AS-604850 (5-(2,2-Difluoro-benzo[1,3]dioxol-5-ylmethylene)-thiazolidine-2,4-dione), AS-605240 (5-quinoxilin-6-methylene-1,3-thiazolidine-2,4-dione), AT7867 (CAS # 857531-00-1), benzimidazole series, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 (CAS # 1007207-67-1), CHR-4432 (Chroma Therapeutics, Ltd., Abingdon, UK), FPA 124 (CAS # 902779-59-3), GS-1101 (CAL-101) (Gilead Sciences), GSK 690693 (CAS # 937174-76-0), H-89 (CAS # 127243-85-0), Honokiol, IC87114 (Gilead Science), IPI-145 (Intellikine Inc.), KAR-4139 (Karus Therapeutics, Chilworth, UK), KAR-4141 (Karus Therapeutics), KIN-1 (Karus Therapeutics), KT 5720 (CAS # 108068-98-0), Miltefosine, MK-2206 dihydrochloride (CAS # 1032350-13-2), ML-9 (CAS # 105637-50-1), Naltrindole Hydrochloride, OXY-111A (NormOxys Inc., Brighton, MA), perifosine, PHT-427 (CAS # 1191951-57-1), PI3 kinase delta inhibitor, Merck KGaA (Merck & Co., Whitehouse Station, NJ), PI3 kinase delta inhibitors, Genentech (Roche Holdings Inc.), PI3 kinase delta inhibitors, Incozen (Incozen Therapeutics, Pvt. Ltd., Hyderabad, India), PI3 kinase delta inhibitors-2, Incozen (Incozen Therapeutics), PI3 kinase inhibitor, Roche-4 (Roche Holdings Inc.), PI3 kinase inhibitors, Roche (Roche Holdings Inc.), PI3 kinase inhibitors, Roche-5 (Roche Holdings Inc.), PI3-alpha/delta inhibitors, Pathway Therapeutics (Pathway Therapeutics Ltd., South San Francisco, CA), PI3-delta inhibitors, Cellzome (Cellzome AG, Heidelberg, Germany), PI3-delta inhibitors, Intellikine (Intellikine Inc., La Jolla, CA), PI3-delta inhibitors, Pathway Therapeutics-1 (Pathway Therapeutics Ltd.), PI3-delta inhibitors, Pathway Therapeutics-2 (Pathway Therapeutics Ltd.), PI3-delta/gamma inhibitors, Cellzome (Cellzome AG), PI3-delta/gamma inhibitors, Cellzome (Cellzome AG), PI3-delta/gamma inhibitors, Intellikine (Intellikine Inc.), PI3-delta/gamma inhibitors, Intellikine (Intellikine Inc.), PI3-delta/gamma inhibitors, Pathway Therapeutics (Pathway Therapeutics Ltd.), PI3-delta/gamma inhibitors, Pathway Therapeutics (Pathway Therapeutics Ltd.), PI3-gamma inhibitor Evotec (Evotec), PI3-gamma inhibitor, Cellzome (Cellzome AG), PI3-gamma inhibitors, Pathway Therapeutics (Pathway Therapeutics Ltd.), PI3K delta/gamma inhibitors, Intellikine-1 (Intellikine Inc.), PI3K delta/gamma inhibitors, Intellikine-1 (Intellikine Inc.), pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, Tetrahydro Curcumin, TG100-115 (Targegen Inc., San Diego, CA), Triciribine, X-339 (Xcovery, West Palm Beach, FL), XL-499 (Evotech, Hamburg, Germany), pharmaceutically acceptable salts thereof, and combinations thereof.
52. The kit according to item 40, wherein administration of the first and second anti-cancer agents provides a synergistic effect compared to administration of either anti-cancer agent alone.
53. A pharmaceutical composition for treating or ameliorating the effects of a cancer in a subject in need thereof, the pharmaceutical composition comprising a pharmaceutically acceptable diluent or carrier and an effective amount of (i) a first anti-cancer agent, which is BVD-523 or a pharmaceutically acceptable salt thereof and (ii) a second anti-cancer agent, which is a type 2 MEK inhibitor or a pharmaceutically acceptable salt thereof, wherein administration of the first and second anti-cancer agents provides a synergistic effect compared to administration of either anti-cancer agent alone.
54. The pharmaceutical composition according to item 53, wherein the subject is a mammal.
55. The pharmaceutical composition according to item 54, wherein the mammal is selected from the group consisting of humans, primates, farm animals, and domestic animals.
56. The pharmaceutical composition according to item 54, wherein the mammal is a human.
57. The pharmaceutical composition according to item 53, wherein the type 2 MEK inhibitor is selected from the group consisting of anthrax toxin, lethal factor portion of anthrax toxin, ARRY-142886 (6-(4-bromo-2-chlorophenylamino)-7-fluoro-3-methyl-3H-benzoimidazole-5-carboxylic acid (2-hydroxy-ethoxy)-amide), ARRY-438162 (Array BioPharma), AS-1940477 (Astellas), MEK162, PD 098059 (2-(2'-amino-3'-methoxyphenyl)-oxanaphthalen-4-one), PD 184352 (CI-1040), PD-0325901 (Pfizer), pimasertib (Santhera Pharmaceuticals), refametinib (AstraZeneca), selumetinib (AZD6244) (AstraZeneca), TAK-733 (Takeda), trametinib (Japan Tobacco), U0126 (1,4-diamino-2,3-dicyano-1,4-bis(2-aminophenylthio)butadiene), RDEA119 (Ardea Biosciences/Bayer), pharmaceutically acceptable salts thereof, and combinations thereof.
58. The pharmaceutical composition according to item 53, wherein the type 2 MEK inhibitor is trametinib or a pharmaceutically acceptable salt thereof.
59. The pharmaceutical composition according to item 53, wherein the subject with cancer has a somatic RAS mutation or BRAF mutation
60. The pharmaceutical composition according to item 53, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
61. The pharmaceutical composition according to item 53, wherein the cancer is melanoma.
62. The pharmaceutical composition according to item 53 further comprising at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
63. The pharmaceutical composition according to item 62, wherein the additional therapeutic agent is an inhibitor of the PI3K/Akt pathway.
64. The pharmaceutical composition according to item 63, wherein the inhibitor of the PI3K/Akt pathway is selected from the group consisting of A-674563 (CAS # 552325-73-2), AGL 2263, AMG-319 (Amgen, Thousand Oaks, CA), AS-041164 (5-benzo[1,3]dioxol-5-ylmethylene-thiazolidine-2,4-dione), AS-604850 (5-(2,2-Difluoro-benzo[1,3]dioxol-5-ylmethylene)-thiazolidine-2,4-dione), AS-605240 (5-quinoxilin-6-methylene-1,3-thiazolidine-2,4-dione), AT7867 (CAS # 857531-00-1), benzimidazole series, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 (CAS # 1007207-67-1), CHR-4432 (Chroma Therapeutics, Ltd., Abingdon, UK), FPA 124 (CAS # 902779-59-3), GS-1101 (CAL-101) (Gilead Sciences), GSK 690693 (CAS # 937174-76-0), H-89 (CAS # 127243-85-0), Honokiol, IC87114 (Gilead Science), IPI-145 (Intellikine Inc.), KAR-4139 (Karus Therapeutics, Chilworth, UK), KAR-4141 (Karus Therapeutics), KIN-1 (Karus Therapeutics), KT 5720 (CAS # 108068-98-0), Miltefosine, MK-2206 dihydrochloride (CAS # 1032350-13-2), ML-9 (CAS # 105637-50-1), Naltrindole Hydrochloride, OXY-111A (NormOxys Inc., Brighton, MA), perifosine, PHT-427 (CAS # 1191951-57-1), PI3 kinase delta inhibitor, Merck KGaA (Merck & Co., Whitehouse Station, NJ), PI3 kinase delta inhibitors, Genentech (Roche Holdings Inc.), PI3 kinase delta inhibitors, Incozen (Incozen Therapeutics, Pvt. Ltd., Hyderabad, India), PI3 kinase delta inhibitors-2, Incozen (Incozen Therapeutics), PI3 kinase inhibitor, Roche-4 (Roche Holdings Inc.), PI3 kinase inhibitors, Roche (Roche Holdings Inc.), PI3 kinase inhibitors, Roche-5 (Roche Holdings Inc.), PI3-alpha/delta inhibitors, Pathway Therapeutics (Pathway Therapeutics Ltd., South San Francisco, CA), PI3-delta inhibitors, Cellzome (Cellzome AG, Heidelberg, Germany), PI3-delta inhibitors, Intellikine (Intellikine Inc., La Jolla, CA), PI3-delta inhibitors, Pathway Therapeutics-1 (Pathway Therapeutics Ltd.), PI3-delta inhibitors, Pathway Therapeutics-2 (Pathway Therapeutics Ltd.), PI3-delta/gamma inhibitors, Cellzome (Cellzome AG), PI3-delta/gamma inhibitors, Cellzome (Cellzome AG), PI3-delta/gamma inhibitors, Intellikine (Intellikine Inc.), PI3-delta/gamma inhibitors, Intellikine (Intellikine Inc.), PI3-delta/gamma inhibitors, Pathway Therapeutics (Pathway Therapeutics Ltd.), PI3-delta/gamma inhibitors, Pathway Therapeutics (Pathway Therapeutics Ltd.), PI3-gamma inhibitor Evotec (Evotec), PI3-gamma inhibitor, Cellzome (Cellzome AG), PI3-gamma inhibitors, Pathway Therapeutics (Pathway Therapeutics Ltd.), PI3K delta/gamma inhibitors, Intellikine-1 (Intellikine Inc.), PI3K delta/gamma inhibitors, Intellikine-1 (Intellikine Inc.), pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, Tetrahydro Curcumin, TG100-115 (Targegen Inc., San Diego, CA), Triciribine, X-339 (Xcovery, West Palm Beach, FL), XL-499 (Evotech, Hamburg, Germany), pharmaceutically acceptable salts thereof, and combinations thereof.
65. The pharmaceutical composition according to item 53, which is in a unit dosage form comprising both anti-cancer agents.
66. The pharmaceutical composition according to item 53 in which the first anti-cancer agent is in a first unit dosage form and the second anti-cancer agent is in a second unit dosage form, separate from the first.
67. The pharmaceutical composition according to item 53, wherein the first and second anti-cancer agents are co-administered to the subject.
68. The pharmaceutical composition according to item 53, wherein the first and second anti-cancer agents are administered to the subject serially.
69. The pharmaceutical composition according to item 68, wherein the first anti-cancer agent is administered to the subject before the second anti-cancer agent.
70. The pharmaceutical composition according to item 68, wherein the second anti-cancer agent is administered to the subject before the first anti-cancer agent.
71. A method of treating or ameliorating the effects of a cancer in a subject in need thereof comprising administering to the subject an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof, to treat or ameliorate the effects of the cancer.
72. The method according to item 71, wherein the subject is a mammal.
73. The method according to item 72, wherein the mammal is selected from the group consisting of humans, primates, farm animals, and domestic animals.
74. The method according to item 72, wherein the mammal is a human.
75. The method according to item 71, wherein the subject with cancer has a somatic RAS or BRAF mutation.
76. The method according to item 71, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
77. The method according to item 71, wherein the cancer is melanoma.
78. The method according to item 71 further comprising administering to the subject at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
79. The method according to item 71, wherein administration of the first and second anti-cancer agents provides a synergistic effect compared to administration of either anti-cancer agent alone.
80. A method of effecting cancer cell death comprising contacting the cancer cell with an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof.
81. The method according to item 80, wherein the subject is a mammal.
82. The method according to item 81, wherein the mammal is selected from the group consisting of humans, primates, farm animals, and domestic animals.
83. The method according to item 81, wherein the mammal is a human.
84. The method according to item 80, wherein the subject with cancer has a somatic RAS mutation or BRAF mutation.
85. The method according to item 80, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
86. The method according to item 80, wherein the cancer is melanoma.
87. The method according to item 80 further comprising contacting the cancer cell with at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
88. The method according to item 80, wherein contacting the cancer cell with the first and second anti-cancer agents provides a synergistic effect compared to contacting the cancer cell with either anti-cancer agent alone.
89. A kit for treating or ameliorating the effects of a cancer in a subject in need thereof comprising an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof, packaged together with instructions for their use.
90. The kit according to item 89, wherein the subject is a mammal.
91. The kit according to item 90, wherein the mammal is selected from the group consisting of humans, primates, farm animals, and domestic animals.
92. The kit according to item 90, wherein the mammal is a human.
93. The kit according to item 89, wherein the subject with cancer has a somatic RAS or BRAF mutation.
94. The kit according to item 89, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
95. The kit according to item 89, wherein the cancer is melanoma.
96. The kit according to item 89 further comprising at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
97. The kit according to item 89, wherein administration of the first and second anti-cancer agents provides a synergistic effect compared to administration of either anti-cancer agent alone.
98. A pharmaceutical composition for treating or ameliorating the effects of a cancer in a subject in need thereof, the pharmaceutical composition comprising a pharmaceutically acceptable diluent or carrier and an effective amount of (i) a first anti-cancer agent, which is BVD-523 or a pharmaceutically acceptable salt thereof and (ii) a second anti-cancer agent, which is a MEK inhibitor selected from the group consisting of antroquinonol (Golden Biotechnology), AS-1940477 (Astellas), AS-703988 (Merck KGaA), BI-847325 (Boehringer Ingelheim), E-6201 (Eisai), GDC-0623 (Hoffmann-La Roche), GDC-0973, RG422, RO4987655, RO5126766, SL327, WX-554 (Wilex), YopJ polypeptide, pharmaceutically acceptable salts thereof, and combinations thereof, wherein administration of the first and second anti-cancer agents provides a synergistic effect compared to administration of either anti-cancer agent alone.
99. The pharmaceutical composition according to item 98, wherein the subject is a mammal.
100. The pharmaceutical composition according to item 99, wherein the mammal is selected from the group consisting of humans, primates, farm animals, and domestic animals.
101. The pharmaceutical composition according to item 99, wherein the mammal is a human.
102. The pharmaceutical composition according to item 98, wherein the subject with cancer has a somatic RAS mutation or BRAF mutation
103. The pharmaceutical composition according to item 98, wherein the cancer is selected from the group consisting of a cancer of the large intestine, breast cancer, pancreatic cancer, skin cancer, endometrial cancer, neuroblastoma, leukemia, lymphoma, liver cancer, lung cancer, testicular cancer, and thyroid cancer.
104. The pharmaceutical composition according to item 98, wherein the cancer is melanoma.
105. The pharmaceutical composition according to item 98 further comprising at least one additional therapeutic agent selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof.
106. The pharmaceutical composition according to item 98, which is in a unit dosage form comprising both anti-cancer agents.
107. The pharmaceutical composition according to item 98, in which the first anti-cancer agent is in a first unit dosage form and the second anti-cancer agent is in a second unit dosage form, separate from the first.
108. The pharmaceutical composition according to item 98, wherein the first and second anti-cancer agents are co-administered to the subject.
109. The pharmaceutical composition according to item 98, wherein the first and second anti-cancer agents are administered to the subject serially.
110. The pharmaceutical composition according to item 109, wherein the first anti-cancer agent is administered to the subject before the second anti-cancer agent.
111. The pharmaceutical composition according to item 109, wherein the second anti-cancer agent is administered to the subject before the first anti-cancer agent.

## Claims

1. A first anti-cancer agent (i), which is BVD-523 or a pharmaceutically acceptable salt thereof, a second anti-cancer agent (ii), and one or more additional ERK inhibitors, for use in treating or ameliorating the effects of a cancer in a subject, wherein the second anti-cancer agent is
(ii-1) a type 2 MEK inhibitor selected from the group consisting of trametinib, MEK162, pharmaceutically acceptable salts thereof and combinations thereof; or
(ii-2) GDC-0623 (Hoffmann-La Roche) or a pharmaceutically acceptable salt thereof.

2. An *in vitro* method of effecting cancer cell death comprising contacting the cancer cell with an effective amount of the first and second anti-cancer agents and the one or more additional ERK inhibitors as defined in claim 1.

3. A kit for use in treating or ameliorating the effects of a cancer in a subject comprising an effective amount of the first and second anti-cancer agents and the one or more additional ERK inhibitors as defined in claim 1, packaged together with instructions for their use.

4. A pharmaceutical composition for use in treating or ameliorating the effects of a cancer in a subject, the pharmaceutical composition comprising a pharmaceutically acceptable diluent or carrier and an effective amount of the first and second anti-cancer agent and the one or more additional ERK inhibitors as defined in claim 1, wherein administration of the first and second anti-cancer agents provides a synergistic effect compared to administration of either anti-cancer agent alone.

5. The first and second anti-cancer agents and the one or more additional ERK inhibitors for the use according to claim 1, the *in vitro* method according to claim 2, the kit for the use according to claim 3, or the pharmaceutical composition for the use according to claim 4, wherein
(i) the subject is a mammal, or wherein the cancer cell has been obtained from a subject, which is a mammal; and wherein the mammal is, preferably, selected from the group consisting of humans, primates, farm animals, and domestic animals; and/or
(ii) the subject with cancer, or the subject from which the cancer cell has been obtained, has a somatic RAS or BRAF mutation.

6. The first and second anti-cancer agents and the one or more additional ERK inhibitors for the use according to claims 1 or 5, the *in vitro* method according to claims 2 or 5, the kit for the use according to claims 3 or 5, or the pharmaceutical composition for the use according to claims 4 or 5, wherein the cancer is selected from the group consisting of skin cancer such as melanoma, a cancer of the large intestine, breast cancer, pancreatic cancer, endometrial cancer, neuroblastoma, leukemia, lymphoma, liver cancer, lung cancer, testicular cancer, and thyroid cancer.

7. The first and second anti-cancer agents and the one or more additional ERK inhibitors for the use according to any one of claims 1 and 5-6, the *in vitro* method according to any one of claims 2 and 5-6, the kit for the use according to any one of claims 3 and 5-6, or the pharmaceutical composition for the use according to any one of claims 4-6, wherein at least one additional therapeutic agent is to be administered to the subject or the cancer cell, or wherein the kit or the pharmaceutical composition comprises at least one additional therapeutic agent; wherein the additional therapeutic agent is selected from the group consisting of an antibody or fragment thereof, a cytotoxic agent, a drug, a toxin, a radionuclide, an immunomodulator, a photoactive therapeutic agent, a radiosensitizing agent, a hormone, an anti-angiogenesis agent, and combinations thereof; preferably, wherein the additional therapeutic agent is an inhibitor of the PI3K/Akt pathway, more preferably an inhibitor of the PI3K/Akt pathway selected from the group consisting of A-674563 (CAS # 552325-73-2), AGL 2263, AMG-319 (Amgen, Thousand Oaks, CA), AS-041164 (5-benzo[1,3]dioxol-5-ylmethylene-thiazolidine-2,4-dione), AS-604850 (5-(2,2-Difluoro-benzo[1,3]dioxol-5-ylmethylene)-thiazolidine-2,4-dione), AS-605240 (5-quinoxilin-6-methylene-1,3-thiazolidine-2,4-dione), AT7867 (CAS # 857531-00-1), benzimidazole series, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 (CAS # 1007207-67-1), CHR-4432 (Chroma Therapeutics, Ltd., Abingdon, UK), FPA 124 (CAS # 902779-59-3), GS-1101 (CAL-101) (Gilead Sciences), GSK 690693 (CAS # 937174-76-0), H-89 (CAS # 127243-85-0), Honokiol, IC87114 (Gilead Science), IPI-145 (Intellikine Inc.), KAR-4139 (Karus Therapeutics, Chilworth, UK), KAR-4141 (Karus Therapeutics), KIN-1 (Karus Therapeutics), KT 5720 (CAS # 108068-98-0), Miltefosine, MK-2206 dihydrochloride (CAS # 1032350-13-2), ML-9 (CAS # 105637-50-1), Naltrindole Hydrochloride, OXY-111A (NormOxys Inc., Brighton, MA), perifosine, PHT-427 (CAS # 1191951-57-1), PI3 kinase delta inhibitor, Merck KGaA (Merck & Co., Whitehouse Station, NJ), PI3 kinase delta inhibitors, Genentech (Roche Holdings Inc.), PI3 kinase delta inhibitors, Incozen (Incozen Therapeutics, Pvt. Ltd., Hyderabad, India), PI3 kinase delta inhibitors-2, Incozen (Incozen Therapeutics), PI3 kinase inhibitor, Roche-4 (Roche Holdings Inc.), PI3 kinase inhibitors, Roche (Roche Holdings Inc.), PI3 kinase inhibitors, Roche-5 (Roche Holdings Inc.), PI3-alpha/delta inhibitors, Pathway Therapeutics (Pathway Therapeutics Ltd., South San Francisco, CA), PI3-delta inhibitors, Cellzome (Cellzome AG, Heidelberg, Germany), PI3-delta inhibitors, Intellikine (Intellikine Inc., La Jolla, CA), PI3-delta inhibitors, Pathway Therapeutics-1 (Pathway Therapeutics Ltd.), PI3-delta inhibitors, Pathway Therapeutics-2 (Pathway Therapeutics Ltd.), PI3-delta/gamma inhibitors, Cellzome (Cellzome AG), PI3-delta/gamma inhibitors, Intellikine (Intellikine Inc.), PI3-delta/gamma inhibitors, Pathway Therapeutics (Pathway Therapeutics Ltd.), PI3-gamma inhibitor Evotec (Evotec), PI3-gamma inhibitor, Cellzome (Cellzome AG), PI3-gamma inhibitors, Pathway Therapeutics (Pathway Therapeutics Ltd.), PI3K delta/gamma inhibitors, Intellikine-1 (Intellikine Inc.), pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, Tetrahydro Curcumin, TG100-115 (Targegen Inc., San Diego, CA), Triciribine, X-339 (Xcovery, West Palm Beach, FL), XL-499 (Evotech, Hamburg, Germany), pharmaceutically acceptable salts thereof, and combinations thereof.

8. The first and second anti-cancer agents and the one or more additional ERK inhibitors for the use according to any one of claims 1 and 5-7, the *in vitro* method according to any one of claims 2 and 5-7, or the kit for the use according to any one of claims 3 and 5-7, wherein administration of the first and second anti-cancer agents provides a synergistic effect compared to administration of either anti-cancer agent alone,
or wherein contacting the cancer cell with the first and second anti-cancer agents provides a synergistic effect compared to contacting the cancer cell with either anti-cancer agent alone.

9. The first and second anti-cancer agents and the one or more additional ERK inhibitors for the use according to any one of claims 1 and 5-8, the *in vitro* method according to any one of claims 2 and 5-8, or the kit for the use according to any one of claims 3 and 5-8, wherein the second anti-cancer agent is trametinib or a pharmaceutically acceptable salt thereof,
and wherein BVD-523 or a pharmaceutically acceptable salt thereof, trametinib or a pharmaceutically acceptable salt thereof and/or the one or more additional ERK inhibitors is to be administered in the form of a pharmaceutical composition further comprising a pharmaceutically acceptable carrier or diluent.

10. The pharmaceutical composition for the use according to any one of claims 4-7, wherein
(i) the pharmaceutical composition is in a unit dosage form comprising both anti-cancer agents; or
(ii) the first anti-cancer agent is in a first unit dosage form and the second anti-cancer agent is in a second unit dosage form, separate from the first.

11. The pharmaceutical composition for the use according to any one of claims 4-7, wherein
(i) the first and second anti-cancer agents are to be co-administered to the subject; or
(ii) wherein the first and second anti-cancer agents are to be administered to the subject serially; wherein the first anti-cancer agent may be administered to the subject before the second anti-cancer agent; or wherein the second anti-cancer agent may be administered to the subject before the first anti-cancer agent.

12. The first and second anti-cancer agents and the one or more additional ERK inhibitors for the use according to any one of claims 1 and 5-9, the *in vitro* method according to any one of claims 2 and 5-9, the kit for the use according to any one of claims 3 and 5-9, or the pharmaceutical composition for the use according to any one of claims 4-7, 10 and 11, wherein the one or more additional ERK inhibitors comprise(s) or consist(s) of SCH772984.

## Patentansprüche

1. Erstes Antikrebsmittel (i), das BVD-523 oder ein pharmazeutisch verträgliches Salz davon ist, zweites Antikrebsmittel (ii) und ein oder mehrere zusätzliche ERK-Inhibitoren, zur Verwendung bei der Behandlung oder Verbesserung der Auswirkungen einer Krebserkrankung bei einem Individuum, wobei das zweite Antikrebsmittel
(ii-1) ein Typ-2-MEK-Inhibitor ausgewählt aus der Gruppe bestehend aus Trametinib, MEK162, pharmazeutisch verträgliche Salze davon und Kombinationen davon; oder
(ii-2) GDC-0623 (Hoffmann-La Roche) oder ein pharmazeutisch verträgliches Salz davon
ist.

2. *In vitro*-Verfahren, das Krebszelltod bewirkt, umfassend das Inkontaktbringen der Krebszelle mit einer wirksamen Menge des ersten und des zweiten Antikrebsmittels und des einen oder der mehreren zusätzlichen ERK-Inhibitoren wie in Anspruch 1 definiert.

3. Kit zur Verwendung bei der Behandlung oder Verbesserung der Auswirkungen einer Krebserkrankung bei einem Individuum, umfassend eine wirksame Menge des ersten und des zweiten Antikrebsmittels und des einen oder der mehreren zusätzlichen ERK-Inhibitoren wie in Anspruch 1 definiert, verpackt zusammen mit einer Anleitung für deren Verwendung.

4. Arzneimittel zur Verwendung bei der Behandlung oder Verbesserung der Auswirkungen einer Krebserkrankung bei einem Individuum, wobei das Arzneimittel ein pharmazeutisch verträgliches Verdünnungsmittel oder einen pharmazeutisch verträglichen Träger und eine wirksame Menge des ersten und des zweiten Antikrebsmittels und des einen oder der mehreren zusätzlichen ERK-Inhibitoren wie in Anspruch 1 definiert umfasst, wobei die Verabreichung des ersten und des zweiten Antikrebsmittels im Vergleich zur Verabreichung eines der beiden Antikrebsmittel allein einen Synergieeffekt bereitstellt.

5. Erstes und zweites Antikrebsmittel und ein oder mehrere zusätzliche ERK-Inhibitoren zur Verwendung nach Anspruch 1, *in vitro*-Verfahren nach Anspruch 2, Kit zur Verwendung nach Anspruch 3 oder Arzneimittel zur Verwendung nach Anspruch 4, wobei
(i) das Individuum ein Säuger ist, oder wobei die Krebszelle von einem Individuum erhalten wurde, das ein Säuger ist; und wobei der Säuger bevorzugt ausgewählt ist aus der Gruppe bestehend aus Menschen, Primaten, Nutztieren und Haustieren; und/oder
(ii) das Individuum mit der Krebserkrankung oder das Individuum, von dem die Krebszelle erhalten wurde, eine somatische RAS- oder BRAF-Mutation aufweist.

6. Erstes und zweites Antikrebsmittel und ein oder mehrere zusätzliche ERK-Inhibitoren zur Verwendung nach Anspruch 1 oder 5, *in vitro*-Verfahren nach Anspruch 2 oder 5, Kit zur Verwendung nach Anspruch 3 oder 5 oder Arzneimittel zur Verwendung nach Anspruch 4 oder 5, wobei die Krebserkrankung ausgewählt ist aus der Gruppe bestehend aus Hautkrebs wie Melanom, einem Dickdarmkrebs, Brustkrebs, Bauchspeicheldrüsenkrebs, Endometriumkarzinom, Neuroblastom, Leukämie, Lymphom, Leberkrebs, Lungenkrebs, Hodenkrebs und Schilddrüsenkrebs.

7. Erstes und zweites Antikrebsmittel und ein oder mehrere zusätzliche ERK-Inhibitoren zur Verwendung nach einem der Ansprüche 1 und 5 bis 6, *in vitro*-Verfahren nach einem der Ansprüche 2 und 5 bis 6, Kit zur Verwendung nach einem der Ansprüche 3 und 5 bis 6 oder Arzneimittel zur Verwendung nach einem der Ansprüche 4 bis 6, wobei dem Individuum oder der Krebszelle mindestens ein zusätzliches therapeutisches Agens zu verabreichen ist, oder wobei der Kit oder das Arzneimittel mindestens ein zusätzliches therapeutisches Agens umfasst; wobei das zusätzliche therapeutische Agens ausgewählt ist aus der Gruppe bestehend aus einem Antikörper oder einem Fragment davon, einem cytotoxischen Agens, einem Arzneistoff, einem Toxin, einem Radionuklid, einem Immunmodulator, einem photoaktiven therapeutischen Agens, einem radiosensitivierenden Agens, einem Hormon, einem Anti-Angiogenese-Agens und Kombinationen davon; bevorzugt wobei das zusätzliche therapeutische Agens ein Inhibitor des PI3K/Akt-Signalwegs ist, stärker bevorzugt ein Inhibitor des PI3K/Akt-Signalwegs, der ausgewählt ist aus der Gruppe bestehend aus A-674563 (CAS # 552325-73-2), AGL 2263, AMG-319 (Amgen, Thousand Oaks, CA), AS-041164 (5-Benzo[1,3]dioxol-5-ylmethylen-thiazolidin-2,4-dion), AS-604850 (5-(2,2-Difluor-benzo[1,3]dioxol-5-ylmethylen)-thiazolidin-2,4-dion), AS-605240 (5-Quinoxilin-6-methylen-1,3-thiazolidin-2,4-dion), AT7867 (CAS # 857531-00-1), Benzimidazol-Reihe, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 (CAS # 1007207-67-1), CHR-4432 (Chroma Therapeutics, Ltd., Abingdon, UK), FPA 124 (CAS # 902779-59-3), GS-1101 (CAL-101) (Gilead Sciences), GSK 690693 (CAS # 937174-76-0), H-89 (CAS # 127243-85-0), Honokiol, IC87114 (Gilead Science), IPI-145 (Intellikine Inc.), KAR-4139 (Karus Therapeutics, Chilworth, UK), KAR-4141 (Karus Therapeutics), KIN-1 (Karus Therapeutics), KT 5720 (CAS # 108068-98-0), Miltefosin, MK-2206-Dihydrochlorid (CAS # 1032350-13-2), ML-9 (CAS # 105637-50-1), Naltrindolhydrochlorid, OXY-111A (NormOxys Inc., Brighton, MA), Perifosin, PHT-427 (CAS # 1191951-57-1), PI3-Kinase-delta-Inhibitor, Merck KGaA (Merck & Co., Whitehouse Station, NJ), PI3-Kinase-delta-Inhibitoren, Genentech (Roche Holdings Inc.), PI3-Kinase-delta-Inhibitoren, Incozen (Incozen Therapeutics, Pvt. Ltd., Hyderabad, Indien), PI3-Kinase-delta-Inhibitoren-2, Incozen (Incozen Therapeutics), PI3-Kinase-Inhibitor, Roche-4 (Roche Holdings Inc.), PI3-Kinase-Inhibitoren, Roche (Roche Holdings Inc.), PI3-Kinase-Inhibitoren, Roche-5 (Roche Holdings Inc.), PI3-alpha/delta-Inhibitoren, Pathway Therapeutics (Pathway Therapeutics Ltd., South San Francisco, CA), PI3-delta-Inhibitoren, Cellzome (Cellzome AG, Heidelberg, Germany), PI3-delta-Inhibitoren, Intellikine (Intellikine Inc., La Jolla, CA), PI3-delta-Inhibitoren, Pathway Therapeutics-1 (Pathway Therapeutics Ltd.), PI3-delta-Inhibitoren, Pathway Therapeutics-2 (Pathway Therapeutics Ltd.), PI3-delta/gamma-Inhibitoren, Cellzome (Cellzome AG), PI3-delta/gamma-Inhibitoren, Intellikine (Intellikine Inc.), PI3-delta/gamma-Inhibitoren, Pathway Therapeutics (Pathway Therapeutics Ltd.), PI3-gamma-Inhibitor Evotec (Evotec), PI3-gamma-Inhibitor, Cellzome (Cellzome AG), PI3-gamma-Inhibitoren, Pathway Therapeutics (Pathway Therapeutics Ltd.), PI3K-delta/gamma-Inhibitoren, lntellikine-1 (Intellikine Inc.), Pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, Tetrahydrocurcumin, TG100-115 (Targegen Inc., San Diego, CA), Triciribin, X-339 (Xcovery, West Palm Beach, FL), XL-499 (Evotech, Hamburg, Germany), pharmazeutisch verträglichen Salzen davon und Kombinationen davon.

8. Erstes und zweites Antikrebsmittel und ein oder mehrere zusätzliche ERK-Inhibitoren zur Verwendung nach einem der Ansprüche 1 und 5 bis 7, *in vitro*-Verfahren nach einem der Ansprüche 2 und 5 bis 7 oder Kit zur Verwendung nach einem der Ansprüche 3 und 5 bis 7, wobei die Verabreichung des ersten und des zweiten Antikrebsmittels im Vergleich zur Verabreichung eines der Antikrebsmittel allein einen Synergieeffekt bereitstellt,
oder wobei das Inkontaktbringen der Krebszelle mit dem ersten und zweiten Antikrebsmittel im Vergleich zum Inkontaktbringen der Krebszelle mit einem der Antikrebsmittel alleine einen Synergieeffekt bereitstellt.

9. Erstes und zweites Antikrebsmittel und ein oder mehrere zusätzliche ERK-Inhibitoren zur Verwendung nach einem der Ansprüche 1 und 5 bis 8, *in vitro*-Verfahren nach einem der Ansprüche 2 und 5 bis 8 oder Kit zur Verwendung nach einem der Ansprüche 3 und 5 bis 8, wobei das zweite Antikrebsmittel Trametinib oder ein pharmazeutisch verträgliches Salz davon ist,
und wobei BVD-523 oder ein pharmazeutisch verträgliches Salz davon, Trametinib oder ein pharmazeutisch verträgliches Salz davon und/oder der eine oder die mehreren zusätzlichen ERK-Inhibitoren in Form eines Arzneimittels, das des Weiteren einen pharmazeutisch verträglichen Träger oder ein pharmazeutisch verträgliches Verdünnungsmittel umfasst, zu verabreichen ist.

10. Arzneimittel zur Verwendung nach einem der Ansprüche 4 bis 7, wobei
(i) das Arzneimittel in einer Darreichungsform vorliegt, die beide Antikrebsmittel umfasst; oder
(ii) das erste Antikrebsmittel in einer ersten Darreichungsform vorliegt und das zweite Antikrebsmittel in einer zweiten Darreichungsform vorliegt, getrennt von der Ersten.

11. Arzneimittel zur Verwendung nach einem der Ansprüche 4 bis 7, wobei
(i) das erste und das zweite Antikrebsmittel dem Individuum zusammen zu verabreichen sind; oder
(ii) wobei das erste und das zweite Antikrebsmittel dem Individuum nacheinander zu verabreichen sind; wobei das erste Antikrebsmittel dem Individuum vor dem zweiten Antikrebsmittel verabreicht werden kann; oder wobei das zweite Antikrebsmittel dem Individuum vor dem ersten Antikrebsmittel verabreicht werden kann.

12. Erstes und zweites Antikrebsmittel und ein oder mehrere zusätzliche ERK-Inhibitoren zur Verwendung nach einem der Ansprüche 1 und 5 bis 9, *in vitro*-Verfahren nach einem der Ansprüche 2 und 5 bis 9, Kit zur Verwendung nach einem der Ansprüche 3 und 5 bis 9 oder Arzneimittel zur Verwendung nach einem der Ansprüche 4 bis 7, 10 und 11, wobei der eine oder die mehreren zusätzlichen ERK-Inhibitoren SCH772984 umfasst/umfassen oder daraus besteht/bestehen.

## Revendications

1. Un premier agent anticancéreux (i), qui est le BVD-523 ou un sel pharmaceutiquement acceptable de celui-ci, un deuxième agent anticancéreux (ii), et un ou plusieurs inhibiteurs d'ERK additionnels, pour une utilisation dans le traitement ou l'amélioration des effets d'un cancer chez un sujet, dans lesquels le deuxième agent anticancéreux est
(ii-1) un inhibiteur de MEK de type 2 choisi dans le groupe constitué par le tramétinib, MEK162, leurs sels pharmaceutiquement acceptables, et leurs combinaisons ; ou
(ii-2) GDC-0623 (Hoffmann-La Roche) ou un sel pharmaceutiquement acceptable de celui-ci.

2. Méthode *in vitro* provoquant la mort de cellule cancéreuse, comprenant la mise en contact de la cellule cancéreuse avec une quantité efficace des premier et deuxième agents anticancéreux et du ou des inhibiteurs d'ERK additionnels tels que définis dans la revendication 1.

3. Kit pour une utilisation dans le traitement ou l'amélioration des effets d'un cancer chez un sujet, comprenant une quantité efficace des premier et deuxième agents anticancéreux et du ou des inhibiteurs d'ERK additionnels tels que définis dans la revendication 1, conditionnés conjointement avec des instructions pour leur utilisation.

4. Composition pharmaceutique pour une utilisation dans le traitement ou l'amélioration des effets d'un cancer chez un sujet, la composition pharmaceutique comprenant un diluant ou véhicule pharmaceutiquement acceptable et une quantité efficace des premier et deuxième agents anticancéreux et du ou des inhibiteurs d'ERK additionnels tels que définis dans la revendication 1, dans laquelle l'administration des premier et deuxième agents anticancéreux apporte un effet synergique en comparaison avec l'administration de l'un ou l'autre agent anticancéreux seul.

5. Les premier et deuxième agents anticancéreux et le ou les inhibiteurs d'ERK additionnels pour une utilisation selon la revendication 1, la méthode *in vitro* selon la revendication 2, le kit pour une utilisation selon la revendication 3, ou la composition pharmaceutique pour une utilisation selon la revendication 4, dans lesquels
(i) le sujet est un mammifère, ou la cellule cancéreuse a été obtenue auprès d'un sujet qui est un mammifère ; et le mammifère est de préférence choisi dans le groupe constitué par les humains, les primates, les animaux de ferme, et les animaux domestiques ; et/ou
(ii) le sujet cancéreux, ou le sujet auprès duquel la cellule cancéreuse a été obtenue, a une mutation BRAF ou RAS somatique.

6. Les premier et deuxième agents anticancéreux et le ou les inhibiteurs d'ERK additionnels pour une utilisation selon la revendication 1 ou 5, la méthode *in vitro* selon la revendication 2 ou 5, le kit pour une utilisation selon la revendication 3 ou 5, ou la composition pharmaceutique pour une utilisation selon la revendication 4 ou 5, dans lesquels le cancer est choisi dans le groupe constitué par un cancer de la peau tel qu'un mélanome, un cancer du gros intestin, un cancer du sein, un cancer du pancréas, un cancer de l'endomètre, un neuroblastome, une leucémie, un lymphome, un cancer du foie, un cancer du poumon, un cancer des testicules, et un cancer de la thyroïde.

7. Les premier et deuxième agents anticancéreux et le ou les inhibiteurs d'ERK additionnels pour une utilisation selon l'une quelconque des revendications 1, 5 et 6, la méthode *in vitro* selon l'une quelconque des revendications 2, 5 et 6, le kit pour une utilisation selon l'une quelconque des revendications 3, 5 et 6, ou la composition pharmaceutique pour une utilisation selon l'une quelconque des revendications 4 à 6, dans lesquels au moins un agent thérapeutique additionnel est destiné à être administré au sujet ou à la cellule cancéreuse, ou dans lesquels le kit ou la composition pharmaceutique comprend au moins un agent thérapeutique additionnel, dans lesquels l'agent thérapeutique additionnel est choisi dans le groupe constitué par un anticorps ou un fragment de celui-ci, un agent cytotoxique, un médicament, une toxine, un radionucléide, un immunomodulateur, un agent thérapeutique photoactif, un agent radiosensibilisateur, une hormone, un agent anti-angiogenèse, et leurs combinaisons ; de préférence dans lesquels l'agent thérapeutique additionnel est un inhibiteur de la voie PI3K/Akt, mieux encore un inhibiteur de la voie PI3K/Akt choisi dans le groupe constitué par A-674563 (CAS # 552325-73-2), AGL 2263, AMG-319 (Amgen, Thousand Oaks, CA), AS-041164 (5-benzo[1,3]dioxol-5-ylméthylène-thiazolidine-2,4-dione), AS-604850 (5-(2,2-difluorobenzo[1,3]dioxol-5-ylméthylène)thiazolidine-2,4-dione), AS-605240 (5-quinoxiline-6-méthylène-1,3-thiazolidine-2,4-dione), AT7867 (CAS # 857531-00-1), la série des benzimidazoles, Genentech (Roche Holdings Inc., South San Francisco, CA), BML-257 (CAS # 32387-96-5), CAL-120 (Gilead Sciences, Foster City, CA), CAL-129 (Gilead Sciences), CAL-130 (Gilead Sciences), CAL-253 (Gilead Sciences), CAL-263 (Gilead Sciences), CAS # 612847-09-3, CAS # 681281-88-9, CAS # 75747-14-7, CAS # 925681-41-0, CAS # 98510-80-6, CCT128930 (CAS # 885499-61-6), CH5132799 CAS #1007207-67-1), CHR-4432 (Chroma Therapeutics, Ltd., Abingdon, Royaume-Uni), FPA 124 (CAS # 902779-59-3), GS-1101 (CAL-101) (Gilead Sciences), GSK 690693 (CAS # 937174-76-0), H-89 (CAS # 127243-85-0), le honokiol, IC87114 (Gilead Science), IPI-145 (Intellikine Inc.), KAR-4139 (Karus Therapeutics, Chilworth, Royaume-Uni), KAR-4141 (Karus Therapeutics), KIN-1 (Karus Therapeutics), KT 5720 (CAS # 108068-98-0), la miltéfosine, le dichlorhydrate de MK-2206 (CAS # 1032350-13-2), ML-9 (CAS # 105637-50-1), le chlorhydrate de naltrindole, OXY-111A (NormOxys Inc., Brighton, MA), la périfosine, PHT-427 (CAS # 1191951-57-1), l'inhibiteur de kinase PI3 delta, Merck KGaA (Merck & Co., Whitehouse Station, NJ), les inhibiteurs de kinase PI3 delta, Genentech (Roche Holdings, Inc.), les inhibiteurs de kinase PI3 delta, Incozen (Incozen Therapeutics, Pvt. Ltd., Hyderabad, Inde), les inhibiteurs de kinase PI3 delta 2, Incozen (Incozen Therapeutics), l'inhibiteur de kinase PI3, Roche-4 (Roche Holdings Inc.), les inhibiteurs de kinase PI3, Roche (Roche Holdings Inc.), les inhibiteurs de kinase PI3, Roche-5 (Roche Holdings, Inc.), les inhibiteurs de PI3 alpha/delta, Pathway Therapeutics (Pathway Therapeutics Ltd., South San Francisco, CA), les inhibiteurs de PI3 delta, Cellzome (Cellzome AG, Heidelberg, Allemagne), les inhibiteurs de PI3 delta, Intellikine (Intellikine Inc., La Jolla, CA), les inhibiteurs de PI3 delta, Pathway Therapeutics-1 (Pathway Therapeutics Ltd.), les inhibiteurs de PI3 delta, Pathway Therapeutics-2 (Pathway Therapeutics Ltd.), les inhibiteurs de PI3 delta/gamma, Cellzome (Cellzome AG), les inhibiteurs de PI3 delta/gamma, Intellikine (Intellikine Inc.), les inhibiteurs de PI3-delta/gamma, Pathway Therapeutics (Pathway Therapeutics Ltd.), l'inhibiteur de PI3 gamma Evotec (Evotec), l'inhibiteur de PI3 gamma, Cellzome (Cellzome AG), les inhibiteurs de PI3 gamma, Pathway Therapeutics (Pathway Therapeutics Ltd.), les inhibiteurs de PI3K delta/gamma, Intellikine-1 (Intellikine Inc.), le pictilisib (Roche Holdings Inc.), PIK-90 (CAS # 677338-12-4), SC-103980 (Pfizer, New York, NY), SF-1126 (Semafore Pharmaceuticals, Indianapolis, IN), SH-5, SH-6, la tétrahydrocurcumine, TG100-115 (Targegen Inc., San Diego, CA), la triciribine, X-339 (Xcovery, West Palm Beach, FL), XL-499 (Evotech, Hambourg, Allemagne), leurs sels pharmaceutiquement acceptables, et leurs combinaisons.

8. Les premier et deuxième agents anticancéreux et le ou les inhibiteurs d'ERK additionnels pour une utilisation selon l'une quelconque des revendications 1 et 5 à 7, la méthode *in vitro* selon l'une quelconque des revendications 2 et 5 à 7, ou le kit pour une utilisation selon l'une quelconque des revendications 3 et 5 à 7, dans lesquels l'administration des premier et deuxième agents anticancéreux apporte un effet synergique en comparaison avec l'administration de l'un ou l'autre agent anticancéreux seul,
ou dans lesquels la mise en contact de la cellule cancéreuse avec les premier et deuxième agents anticancéreux apporte un effet synergique en comparaison avec la mise en contact de la cellule cancéreuse avec l'un ou l'autre agent anticancéreux seul.

9. Les premier et deuxième agents anticancéreux et le ou les inhibiteurs d'ERK additionnels pour une utilisation selon l'une quelconque des revendications 1 et 5 à 8, la méthode *in vitro* selon l'une quelconque des revendications 2 et 5 à 8, ou le kit pour une utilisation selon l'une quelconque des revendications 3 et 5 à 8, dans lesquels le deuxième agent anticancéreux est le tramétinib ou un sel pharmaceutiquement acceptable de celui-ci,
et dans lesquels le BVD-523 ou un sel pharmaceutiquement acceptable de celui-ci, le tramétinib ou un sel pharmaceutiquement acceptable de celui-ci, et/ou le ou les inhibiteurs d'ERK additionnels, sont destinés à être administrés sous la forme d'une composition pharmaceutique comprenant en outre un véhicule ou diluant pharmaceutiquement acceptable.

10. Composition pharmaceutique pour une utilisation selon l'une quelconque des revendications 4 à 7, dans laquelle
(i) la composition pharmaceutique est dans une unité posologique comprenant les deux agents anticancéreux ; ou
(ii) le premier agent anticancéreux est dans une première unité posologique et le deuxième agent anticancéreux est dans une deuxième unité posologique, séparée de la première.

11. Composition pharmaceutique pour une utilisation selon l'une quelconque des revendications 4 à 7, dans laquelle
(i) les premier et deuxième agents anticancéreux sont destinés à être coadministrés au sujet ; ou
(ii) les premier et deuxième agents anticancéreux sont destinés à être administrés au sujet en série ; dans laquelle le premier agent anticancéreux peut être administré au sujet avant le deuxième agent anticancéreux ; ou dans laquelle le deuxième agent anticancéreux peut être administré au sujet avant le premier agent anticancéreux.

12. Les premier et deuxième agents anticancéreux et le ou les inhibiteurs d'ERK additionnels pour une utilisation selon l'une quelconque des revendications 1 et 5 à 9, la méthode *in vitro* selon l'une quelconque des revendications 2 et 5 à 9, le kit pour une utilisation selon l'une quelconque des revendications 3 et 5 à 9, ou la composition pharmaceutique pour une utilisation selon l'une quelconque des revendications 4 à 7, 10 et 11, dans lesquels le ou les inhibiteurs d'ERK additionnels comprennent ou consistent en SCH772984.
